# EUROPÄISCHE PATENTSCHRIFT

(11) **EP 1 745 010 B1**
(45) Veröffentlichungstag und Bekanntmachung des Hinweises auf die Patenterteilung: **05.08.2009**
(21) Anmeldenummer: 05739598.0
(22) Anmeldetag: 06.05.2005
(51) Int. Cl.: C07C 233/41, C07D 295/12, C07C 233/47, C07D 295/08, C07D 295/18, C07C 255/58, C07D 209/14, C07D 277/62, C07D 333/58, A61K 31/132, A61K 31/165, A61K 31/404, A61K 31/425, A61P 25/04

(54) **SUBSTITUIERTE CYCLOHEXYL-1,4-DIAMIN-DERIVATE**
SUBSTITUTED CYCLOHEXYL-1,4-DIAMINE DERIVATIVES
DERIVES DE CYCLOHEXYL-1,4-DIAMINE SUBSTITUES

(30) Priorität: 10.05.2004 DE 102004023522
(43) Veröffentlichungstag der Anmeldung: 24.01.2007
(73) Patentinhaber: Grünenthal GmbH, 52078 Aachen (DE)
(72) Erfinder: SUNDERMANN, Corinna, 52074 Aachen (DE); SUNDERMANN, Bernd, 52074 Aachen (DE)
(74) Vertreter: Bülle, Jan
(86) Internationale Anmeldenummer: PCT/EP2005/004913
(87) Internationale Veröffentlichungsnummer: WO 2005/110974

(56) Entgegenhaltungen:
- WO-A-02/090317

## Beschreibung

Die vorliegende Erfindung betrifft substituierte Cyclohexyl-1,4-diamin-Derivate, Verfahren zu deren Herstellung, Arzneimittel enthaltend diese Verbindungen und die Verwendung von substituierten Cyclohexyl-1,4-diamin-Derivate-Derivaten zur Herstellung von Arzneimitteln.

Die Behandlung chronischer und nichtchronischer Schmerzzustände hat in der Medizin eine große Bedeutung. Es besteht ein weltweiter Bedarf an gut wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind.

Klassische µ-Opioide wie Morphin sind bei der Therapie starker bis stärkster Schmerzen gut wirksam und von größter Bedeutung für die Schmerztherapie. Es kann jedoch von Vorteil sein, wenn neben dem µ-Opioid-Rezeptor auch andere Opioid-Rezeptoren, insbesondere der ORL-1-Rezeptor, beeinflusst werden, da die reinen µ-Opioide auch unerwünschte Nebenwirkungen wie Obstipation und Atemdepression aufweisen, aber auch zu Abhängigkeit führen können. Auch die Opioid-Rezeptoren δ, κ und ORL-1 sind am Schmerzgeschehen beteiligt (Opioids: Introduction, S. 127-150, Further Opioid Receptors, 455-476 in: Analgesics - From Chemistry and Pharmacology to Clinical Application, Wiley VCH, 2002).

Darüber hinaus ist bekannt, dass eine sich eine Beeinflussung der Serotonin- und/oder Noradrenalin-Wiederaufnahme günstig auf das Wirk- und Nebenwirkungsspektrum von Opioiden auswirken kann (Beispiel: Tramadol, s. Opioids with Clinical Relevance: Tramadol, 228-230 in: Analgesics - From Chemistry and Pharmacology to Clinical Application, Wiley VCH 2002).

Der ORL1-Rezeptor ist außerdem noch an der Regulation weiterer physiologischer und pathophysiologischer Prozesse beteiligt. Hierzu gehören unter anderem Lernen und Gedächtnisbildung (Manabe et al., Nature, 394, 1997, S. 577-581), Hörvermögen (Nishi et al., EMBO J., 16, 1997, S. 1858-1864) sowie zahlreiche weitere Prozesse. In einem Übersichtsartikel von Calo et al. (Br.J. Pharmacol., 129, 2000, 1261 - 1283) wird ein Überblick über die Indikationen oder biologischen Vorgänge gegeben, in denen der ORL1-Rezeptor eine Rolle spielt oder mit hoher Wahrscheinlichkeit spielen könnte. Genannt werden u.a.: Analgesie, Stimulation und Regulation der Nahrungsaufnahme, Einfluß auf µ-Agonisten wie Morphin, Behandlung von Entzugserscheinungen, Reduzierung des Suchtpotentials von Opioiden, Anxiolyse, Modulation der Bewegungsaktivität, Gedächtnis-Störungen, Epilepsie; Modulation der Neurotransmitter-Ausschüttung, insbesondere von Glutamat, Serotonin und Dopamin, und damit neurodegenerative Erkrankungen; Beeinflußung des cardiovaskulären Systems, Auslösung einer Erektion, Diurese, Antinatriurese, Elektrolyt-Haushalt, arterieller Blutdruck, Wasserspeicher-Krankheiten, intestinale Motilität (Diarrhoe), relaxierende Effekte auf die Atemwege, Mikturations Reflex (Harninkontinenz). Weiter wird die Verwendung von Agonisten und Antagonisten als Anoretika, Analgetika (auch in Coadministration mit Opioiden) oder Nootroplka diskutiert.

Aus dem Stand der Technik (WO 02090317) sind strukturell verwandte Verbindungen bekannt, die eine Affinität zum ORL-1-Rezeptor besitzen. Für diese Strukturklasse wurde bislang kein Einfluss auf die Noradrenalin- und Serotonin-Wiederaufnahme beschrieben.

Aufgabe der vorliegenden Erfindung war es, Arzneimittel zur Verfügung zu stellen, die auf das Opioid-Rezeptor-System wirken und damit für Arzneimittel insbesondere zur Behandlung der verschiedenen mit diesem System nach dem Stand der Technik in Verbindung stehenden Krankeiten bzw. zum Einsatz in den dort genannten Indikationen geeignet sind. Darüber hinaus sollten die Verbindungen die Noradrenaiin- und Serotonin-Wiederaufnahme beeinflussen.

Gegenstand der Erfindung sind daher substituierte Cyclohexyl-1,4-diamin-Derivate-Derivate der allgemeinen Formel I, 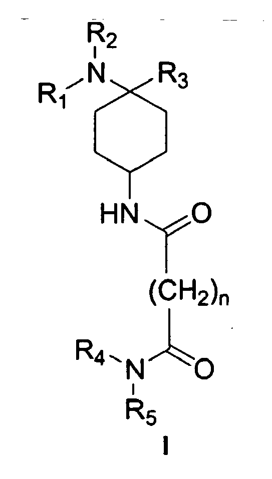 worin
n für 1, 2, 3, 4 oder 5 steht
R¹ und R², unabhängig voneinander für H; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils mehrfach oder mehrfach substituiert oder unsubstituiert; C(O)Phenyl, C(O)Heteroaryl, C(O)C₁₋₅-Alkyl, jeweils substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
R⁴ für -(CR⁶R⁷)ₚR⁸ steht,
wobei p 0, 1, 2, 3 oder 4 bedeutet
R⁶ H oder C₁₋₅-Alkyl, jeweils gesättigt oder ungesättigt, verzweigt oder unverzweigt, unsubstituiert oder einfach oder mehrfach substituiert, bedeutet
R⁷ H, C₁₋₅-Alkyl, jeweils gesättigt oder ungesättigt, verzweigt oder unverzweigt, unsubstituiert oder einfach oder mehrfach substituiert, oder COOR⁹ bedeutet,
oder R⁶ und R⁷ einen Ring (CH₂)ₖCHR⁸(CH₂)ₘ bilden, mit k = 1, 2, 3 und
m = 1, 2
R⁸ C₁₋₆-Alkyl, jeweils gesättigt oder ungesättigt, verzweigt oder unverzweigt, unsubstituiert oder einfach oder mehrfach substituiert; C₃₋₈-Cycloalkyl, Aryl oder Heteroaryl, jeweils unsubstituiert oder einfach oder mehfach substituiert, bedeutet,
R⁹ H oder C₁₋₅-Alkyl bedeutet
R⁵ für H oder für-(CH₂)ₗR⁸ steht,
wobei I für 1, 2 oder 3 steht,
oder zusammen mit R⁴ für CH₂CHR¹⁴OCHR¹⁴CH₂, CH₂CH₂SCH₂CH₂, CH₂CH₂NR¹¹CH₂CH₂, (CR¹²R¹³)₃₋₆ oder 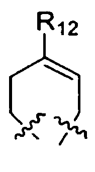 stehen,
wobei R¹¹ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R¹² H; C₁₋₅-Alkyl, jeweils gesättigt oder ungesättigt, verzweigt oder unverzweigt, einfach oder mehrfach substituiert oder unsubstituiert; OH; C(O)Phenyl, unsubstituiert oder einfach oder mehrfach substituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
und R¹³ H oder OH bedeutet oder mit R¹² zusammen über dasselbe C-Atom (Spiro-Verbindung) oder ein benachbartes C-Atom einen fünfgliedrigen oder sechsgliedrigen Ring bildet, der Heteroatome enthalten, gesättigt oder ungesättigt, substituiert oder unsubstituiert und/oder Teil eines polycyclischen Systems sein kann;
R¹⁴ H oder C₁₋₃-Alkyl, jeweils gesättigt oder ungesättigt, verzweigt oder unverzweigt, einfach oder mehrfach substituiert oder unsubstituiert, bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Verbindungen zeigen gute Bindung an den µ-Rezeptor und den ORL-1-Rezeptor, aber auch an andere Opioidrezeptoren. Überraschenderweise zeigte es sich, dass die Verbindungen auch gute Inhibitoren der Noradrenalin- und der Serotonin-Wiederaufnahme sind. Damit eignen sie sich auch zur Behandlung von Depressionen, und/oder Bulimie und/oder Anorexie und/oder Katalepsie und/oder zur Anxiolyse und/oder zur Vigilanz- und/oder Libidosteigerung.

Die Ausdrücke "C₁₋₆-Alkyl," "C₁₋₅-Alkyl" und "C₁₋₃-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1, 2, 3, 4, 5 oder 6 C-Atomen bzw. 1, 2, 3, 4 oder 5 C-Atomen bzw. 1, 2 oder 3 C-Atomen, d.h. C₁₋₆-Alkanyle, C₂₋₆-Alkenyle und C₂₋₆-Alkinyle bzw. C₁₋₅-Alkanyle, C₂₋₅-Alkenyle und C₂₋₅-Alkinyle bzw. C₁₋₃-Alkanyle, C₂₋₃-Alkenyle und C₂₋₃-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl, 2,3-Dimethylbutyl; Ethylenyl (Vinyl), Ethinyl, Propenyl (-CH₂CH=CH₂, -CH=CH-CH₃, -C(=CH₂)-CH₃), Propinyl (-CH-C=CH, -C=C-CH₃), 1,1-Dimethylethyl, 1,1-Dimethylpropyl, Butenyl, Butinyl, Pentenyl, Pentinyl, Hexenyl und Hexinyl umfaßt.

Der Ausdruck "Cycloalkyl" oder "C₃₋₈-Cycloalkyl" bedeutet für die Zwecke dieser Erfindung cyclische Kohlenwasserstoffe mit 3, 4, 5, 6, 7 oder 8 Kohlenstoffatomen, wobei die Kohlenwasserstoffe gesättigt oder ungesättigt (aber nicht aromatisch), unsubstituiert oder ein- oder mehrfach substituiert sein können. In Bezug auf Cycloalkyl umfasst der Begriff auch gesättigte oder ungesättigte (aber nicht aromatische) Cycloalkyle, in denen ein oder zwei Kohlenstoffatome durch ein Heteroatom S, N oder O ersetzt sind. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl, aber auch Tetrahydropyranyl, Dioxanyl, Dioxolanyl, Morpholinyl, Piperidinyl, Piperazinyl, Pyrazolinonyl und Pyrrolidinyl enthält.

Unter dem Begriff (CH₂)₃₋₆ ist -CH₂-CH₂-CH₂-, -CH₂-CH₂-CH₂-CH₂-, -CH₂-CH₂-CH₂-CH₂-CH₂- und CH₂-CH₂-CH₂-CH₂-CH₂-CH₂- zu verstehen.

Der Ausdruck "Aryl" bedeutet im Sinne dieser Erfindung carbocyclische Ringsysteme mit mindestens einem aromatischen Ring, aber ohne Heteroatome in nur einem der Ringe, u.a. Phenyle, Naphthyle und Phenanthrenyle, Fluoranthenyle, Fluorenyle, Indanyle und Tetralinyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Besonders vorteilhaft sind Phenyl- oder Naphthyl-Reste.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein oder mehrfach substituiert sein kann; im Falle der Substitution am Heterocyclus können die Substituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Benzothiadiazolyl, Benzothiazolyl, Benzotriazolyl, Benzodioxolanyl, Benzodioxanyl, Phtalazinyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indazolyl, Purinyl, Indolizinyl, Chinolinyl, Isochinolinyl, Chinazolinyl, Carbazolyl, Phenazinyl, Phenothiazinyl oder Oxadiazolyl enthält, wobei die Bindung an die Verbindungen der allgemeinen Struktur I über jedes beliebige und mögliche Ringglied des Heteroaryl-Restes erfolgen kann.

Im Zusammenhang mit "Alkyl" versteht man unter dem Begriff "substituiert" im Sinne dieser Erfindung die Substitution eines oder mehrerer Wasserstoffreste durch F, Cl, Br, I, -CN, =O, =S, NH₂, NH-Alkyl, NH-Aryl, NH-Heteroaryl, NH-Cycloalkyl, NH-Alkyl-Aryl, NH-Alkyl-Heteroaryl, NH-Alkyl-OH, N(Alkyl)₂, N(Alkyl-Aryl)₂, N(Alky)-Heteroaryl)₂, N(Cycloalkyl)₂, N(Alkyl-OH)₂, NH(C=O)Alkyl, NH(C=O)Aryl), NO₂, SH, S-Alkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, SO-Alkyl, SO₂-Alkyl, SO₂NH₂, SO₃H, PO(O-C₁₋₆-Alkyl)₂, Cycloalkyl, Aryl oder Heteroaryl, wobei unter mehrfach substituierten Resten solche Reste zu verstehen sind, die entweder an verschiedenen oder an gleichen Atomen mehrfach, z. B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -O-CH₂-CH₂-O-CH₂-CH₂-OH.

In Bezug auf "Aryl", "Heteroaryl" sowie "Cycloalkyl" versteht man im Sinne dieser Erfindung unter "ein- oder mehrfach substituiert" die ein- oder mehrfache, z.B. zwei-, drei- vier- oder fünffache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Cycloalkyl, NH-Alkyl-OH, N(Alkyl)₂, N(Alkyl-Aryl)₂, N(Alkyl-Heteroaryl)₂, N(Cycloalkyl)₂, N(Alkyl-OH)₂, NO₂, SH, S-Alkyl, S-Cycloalkyl, S-Aryl, S-Heteroaryl, S-Alkyl-Aryl, S-Alkyl-Heteroaryl, S-Cycloalkyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Cycloalkyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, C(=S)C₁₋₆-Alkyl-Aryl, C(=O)-Heteroaryl, C(=S)-Heteroaryl, C(=O)-Cycloalkyl, C(=S)-Cycloalkyl, CO₂H, CO₂-Alkyl, CO₂-Alkyl-Aryl, C(=O)NH₂, C(=O)NH-Alkyl, C(=O)NHAryl, C(=O)NH-Cycloalkyl, C(=O)N(Alkyl)₂, C(=O)N(Alkyl-Aryl)₂, C(=O)N(Alkyl-Heteroaryl)₂, C(=O)N(Cycloalkyl)₂, S(O)-Alkyl, S(O)-Aryl, SO₂-Alkyl, SO₂-Aryl, SO₂NH₂, SO₃H, CF₃, =O, =S; 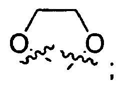 Alkyl, Cycloalkyl, Aryl und/oder Heteroaryl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten.

Unter dem Begriff Salz ist jegliche Form des erfindungsgemäßen Wirkstoffes zu verstehen, in dem dieser eine ionische Form annimmt bzw. geladen ist und mit einem Gegenion (einem Kation oder Anion) gekoppelt ist bzw. sich in Lösung befindet. Darunter sind auch Komplexe des Wirkstoffes mit anderen Molekülen und Ionen zu verstehen, insbesondere Komplexe, die über ionische Wechselwirkungen komplexiert sind. Insbesondere versteht man darunter (und dies ist auch eine bevorzugte Ausführungsform dieser Erfindung) physiologisch verträgliche Salze, insbesondere physiologisch verträgliche Salze mit Kationen oder Basen und physiologisch verträgliche Salze mit Anionen oder Säuren oder auch ein mit einer physiologisch verträglichen Säure oder einem physiologisch verträglichen Kation gebildetes Salz.

Unter dem Begriff des physiologisch verträglichen Salzes mit Anionen oder Säuren versteht man im Sinne dieser Erfindung Salze mindestens einer der erfindungsgemäßen Verbindungen - meist, beispielsweise am Stickstoff, protoniert - als Kation mit mindestens einem Anion, die physiologisch - insbesondere bei Anwendung im Menschen und/oder Säugetier - verträglich sind. Insbesondere versteht man darunter im Sinne dieser Erfindung das mit einer physiologisch verträglichen Säure gebildete Salz, nämlich Salze des jeweiligen Wirkstoffes mit anorganischen bzw. organischen Säuren, die physiologisch - insbesondere bei Anwendung im Menschen und/oder Säugetier - verträglich sind. Beispiele für physiologisch verträgliche Salze bestimmter Säuren sind Salze der: Salzsäure, Bromwasserstoffsäure, Schwefelsäure, Methansulfonsäure, Ameisensäure, Essigsäure, Oxalsäure, Bernsteinsäure, Apfelsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Phosphorsäure, Maleinsäure, Malonsäure, Hippursäure und/oder Asparaginsäure. Besonders bevorzugt ist das Hydrochlorid-Salz, das Citrat und das Hemicitrat.

Unter dem Begriff des mit einer physiologisch verträglichen Säure gebildeten Salzes versteht man im Sinne dieser Erfindung Salze des jeweiligen Wirkstoffes mit anorganischen bzw. organischen Säuren, die physiologisch - insbesondere bei Anwendung im Menschen und/oder Säugetier - verträglich sind. Besonders bevorzugt ist das Hydrochlorid und das Citrat. Beispiele für physiologisch verträgliche Säuren sind: Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Saccharinsäure, Monomethylsebacinsäure, 5-Oxo-prolin, Hexan-1-sulfonsäure, Nicotinsäure, 2-, 3- oder 4-Aminobenzoesäure, 2,4,6-Trimethyl-benzoesäure, α-Liponsäure, Acetylglycin, Hippursäure und/oder Asparaginsäure.

Unter dem Begriff des physiologisch verträglichen Salzes mit Kationen oder Basen versteht man im Sinne dieser Erfindung Salze mindestens einer der erfindungsgemäßen Verbindungen - meist einer (deprotonierten) Säure - als Anion mit mindestens einem, vorzugsweise anorganischen, Kation, die physiologisch - insbesondere bei Anwendung im Menschen und/oder Säugetier - verträglich sind. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Unter dem Begriff des mit einem physiologisch verträglichen Kation gebildeten Salzes versteht man im Sinne dieser Erfindung Salze mindestens einer der jeweiligen Verbindungen als Anion mit mindestens einem anorganischen Kation, das physiologisch - insbesondere bei Anwendung im Menschen und/oder Säugetier - veträglich ist. Besonders bevorzugt sind die Salze der Alkali- und Erdalkalimetalle aber auch Ammoniumsalze, insbesondere aber (Mono-) oder (Di-) Natrium-, (Mono-) oder (Di-) Kalium-, Magnesium- oder Calzium-Salze.

Für eine bevorzugte Ausführungsform der erfindungsgemäßen substituierten Cyclohexyl-1,4-diamin-Derivate gilt, dass
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten,
wobei R¹⁰ H; C₁₋₅-Alkyl, gesättigt oder ungesättigt, verzweigt oder unverzweigt, einfach oder mehrfach substituiert oder unsubstituiert, bedeutet.

Besonders bevorzugt sind substituierte Cyclohexyl-1,4-diamin -Derivate, worin R¹ und R² unabhängig voneinander für CH₃ oder H stehen, wobei R¹ und R² nicht gleichzeitig H bedeuten, oder R¹ und R² für CH₂CH₂OCH₂CH₂ oder (CH₂)₆ stehen.

Weiterhin bevorzugt sind substituierte Cyclohexyl-1,4-diamin -Derivate, worin
R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.
Besonders bevorzugt sind substituierte Cyclohexyl-1,4-diamin -Derivate, worin R³ für Phenyl, Thiophenyl, Pyridyl oder Benzyl, jeweils substituiert oder unsubstituiert, steht, besonders bevorzugt für 4-Methylbenzyl, 3-Chlorbenzyl, 4-Chlorbenzyl, 3-Methylbenzyl, Benzyl, Phenyl, Thiophenyl und 3-Fluorphenyl.

Bevorzugt sind substituierte Cyclohexyl-1,4-diamin-Derivate, worin n für 2 oder 3 steht.

Bevorzugt sind auch substituierte Cyclohexyl-1,4-diamin-Derivate, worin R⁴ für - (CR⁶R⁷)ₚR⁸ und R⁵ für H steht.

Außerdem bevorzugt sind substituierte Cyclohexyl-1,4-diamin-Derivate, worin R⁴ (CH₂)ₚR⁸ und R⁵ für-(CH₂)ₗR⁸ steht.

Bevorzugt sind darüber hinaus substituierte Cyclohexyl-1,4-diamin-Derivate, worin R⁴ und R⁵ zusammen für CH₂CHR¹⁴OCHR¹⁴CH₂, CH₂CH₂SCH₂CH₂, CH₂CH₂NR¹¹CH₂CH₂, (CR¹²R¹³)₃₋₆ oder 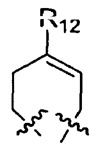 stehen.

Bevorzugt sind auch substituierte Cyclohexyl-1,4-diamin-Derivate, bei denen R⁶ H und R⁷ H, CH₃, Benzyl, unsubstituiert oder einfach oder mehrfach substituiert, oder COOR⁹ bedeutet oder R⁶ und R⁷ einen Ring (CH₂)ₖCHR⁸(CH₂)ₘ bilden.

Weiterhin bevorzugt sind substituierte Cyclohexyl-1,4-diamin-Derivate, bei denen R⁸ C₁₋₆-Alkyl, jeweils gesättigt oder ungesättigt, verzweigt oder unverzweigt, unsubstituiert oder einfach oder mehrfach substituiert; Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyrrolidinyl, Tetrahydrofuryl, Piperazinyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet.

Besonders bevorzugt sind substituierte Cyclohexyl-1,4-diamin-Derivate, bei denen R⁸ C₁₋₆-Alkyl, jeweils gesättigt oder ungesättigt, verzweigt oder unverzweigt, unsubstituiert oder einfach oder mehrfach substituiert; Pyrrolidinyl, Morpholinyl, Tetrahydrofuryl, Tetrahydronaphthyl, Dihydroindolyl, Pyridyl, Thienyl, Piperazinyl, Naphthyl, Indanyl, Chinolinyl, Cyclopropyl, Cyclopentyl, Cyclohexyl, Cycloheptyl, Cyclooctyl, Adamantyl, Furyl, Benzofuryl, Phenyl oder Indolyl, jeweils unsubstituiert oder einfach oder mehrfach substituiert, bedeutet.

Ganz besonders bevorzugt sind substituierte Cyclohexyl-1,4-diamin-Derivate aus der Gruppe
N-[1-(2,6-Dichlor-benzyl)-pyrrolidin-3-yl]-N'-[4-dimethylamino-4-(4-methyl-benzyl)-cyclohexyl]-succinamid
N-[4-(3-Chlor-benzyl)-4-dimethylamino-cyclohexyl]-N'-(2-oxo-tetrahydro-furan-3-yl)-succinamid
N-[4-(4-Chlor-benzyl)-4-dimethylamino-cyclohexyl]-N'-(3,3-diphenyl-propyl)-succinamid
N-[4-Dimethylamino-4-(4-methyl-benzyl)-cyclohexyl]-4-[4-(7-methoxy-benzo[1,3]dioxol-5-ylmethyl)-piperazin-1-yl]-4-oxo-butyramid
N-[4-(4-Chlor-benzyl)-4-dimethylamino-cyclohexyl]-4-(3,6-dihydro-2H-pyridin-1-yl)-4-oxo-butyramid
N-[4-Dimethylamino-4-(4-methyl-benzyl)-cyclohexyl]-4-oxo-4-(4-m-tolyl-piperazin-1-yl)-butyramid
2-{3-[4-(4-Chlor-benzyl)-4-dimethylamino-cyclohexylcarbamoyl]-propionylamino}-propionsäureethylester
N-[4-(3-Chlor-benzyl)-4-dimethylamino-cyclohexyl]-4-(8-fluor-1,3,4,5-tetrahydro-pyrido[4,3-b]indol-2-yl)-4-oxo-butyramid
3-{3-[4-Dimethylamino-4-(4-methyl-benzyl)-cyclohexylcarbamoyl]-propionylamino}-propionsäureethylester
N-[4-Dimethylamino-4-(4-methyl-benzyl)-cyclohexyl]-4-[4-(5-methyl-pyrazin-2-carbonyl)-piperazin-1-yl]-4-oxo-butyramid
N-[4-Dimethylamino-4-(4-methyl-benzyl)-cyclohexyl]-N'-methyl-N'-[2-(2,2,2-trifluor-acetylamino)-ethyl]-succinamid
N-[4-(3-Chlor-benzyl)-4-dimethylamino-cyclohexyl]-N'-(2-pyridin-2-yl-ethyl)-succinamid
N-[4-(4-Chlor-benzyl)-4-dimethylamino-cyclohexyl]-4-oxo-4-thiomorpholin-4-yl-butyramid
N-[4-(4-Chlor-benzyl)-4-dimethylamino-cyclohexyl]-4-[4-(5-methyl-pyrazin-2-carbonyl)-piperazin-1-yl]-4-oxo-butyramid
N-[4-Dimethylamino-4-(3-methyl-benzyl)-cyclohexyl]-N'-(2-thiophen-2-yl-ethyl)-succinamid
N-[4-(4-Chlor-benzyl)-4-dimethylamino-cyclohexyl]-4-[4-(4-ethoxy-phenyl)-piperazin-1-yl]-4-oxo-butyramid
N-Allyl-N'-[4-(4-chlor-benzyl)-4-dimethylamino-cyclohexyl]-N-methyl-succinamid
5-[4-(2,5-Dimethoxy-benzyl)-piperazin-1-yl]-5-oxo-valeriansäure (4-benzyl-4-dimethylamino-cyclohexyl)-amid
4-(3,6-Dihydro-2H-pyridin-1-yl)-N-[4-dimethylamino-4-(4-methyl-benzyl)-cyclohexyl]-4-oxo-butyramid
N-[4-Dimethylamino-4-(3-methyl-benzyl)-cyclohexyl]-4-(4-methyl-piperidin-1-yl)-4-oxo-butyramid
N-[4-Dimethylamino-4-(4-methyl-benzyl)-cyclohexyl]-4-oxo-4-(4-pyridin-2-yl-piperazin-1-yl)-butyramid
({3-[4-Dimethylamino-4-(3-methyl-benzyl)-cyclohexylcarbamoyl]-propionyl}-methyl-amino)-essigsäure benzyl ester
4-[4-(2,6-Dichlor-benzyl)-piperazin-1-yl]-N-[4-dimethylamino-4-(3-methyl-benzyl)-cyclohexyl]-4-oxo-butyramid
{[4-(4-Benzyl-4-dimethylamino-cyclohexylcarbamoyl)-butyryl]-methyl-amino}-essigsäure tert-butyl ester
5-[4-(2-Fluor-5-methoxy-benzyl)-piperazin-1-yl]-5-oxo-valeriansäure (4-benzyl-4-dimethylamino-cyclohexyl)-amid
N-[4-(4-Chlor-benzyl)-4-dimethylamino-cyclohexyl]-N'-(3-methoxy-propyl)-succinamid
4-[4-(4-Acetyl-phenyl)-piperazin-1-yl]-N-[4-dimethylamino-4-(4-methyl-benzyl)-cyclohexyl]-4-oxo-butyramid
4-{3-[4-Dimethylamino-4-(4-methyl-benzyl)-cyclohexylcarbamoyl]-propionyl}-piperazin-1-carbonsäure-*tert*-butylester
N-[4-(3-Chlor-benzyl)-4-dimethylamino-cyclohexyl]-N'-(tetrahydro-furan-2-ylmethyl)-succinamid
2-{3-[4-(3-Chlor-benzyl)-4-dimethylamino-cyclohexylcarbamoyl]-propionylamino}-propionsäureethylester
N-(3-Brom-benzyl)-N'-[4-dimethylamino-4-(3-methyl-benzyl)-cyclohexyl]-succinamid
3-(4-Chlor-phenyl)-2-{3-[4-dimethylamino-4-(4-methyl-benzyl)-cyclohexylcarbamoyl]-propionylamino}-propionsäuremethylester
N-[4-(3-Chlor-benzyl)-4-dimethylamino-cyclohexyl]-4-[4-(5-methyl-pyrazin-2-carbonyl)-piperazin-1-yl]-4-oxo-butyramid
N-[4-(4-Chlor-benzyl)-4-dimethylamino-cyclohexyl]-N'-naphthalen-1-ylmethyl-succinamid
N-[4-Dimethylamino-4-(3-methyl-benzyl)-cyclohexyl]-4-[4-(3-fluor-4-methoxy-benzyl)-piperazin-1-yl]-4-oxo-butyramid
N-[4-Dimethylamino-4-(4-methyl-benzyl)-cyclohexyl]-N'-[2-(1H-indol-3-yl)-ethyl]-N'-methyl-succinamid
(4-{3-[4-(3-Chlor-benzyl)-4-dimethylamino-cyclohexylcarbamoyl]-propionylamino}-benzyl)-phosphonsäurediethylester
N-[4-Dimethylamino-4-(4-methyl-benzyl)-cyclohexyl]-N'-methyl-N'-(2-pyridin-2-yl-ethyl)-succinamid
N-[4-(3-Chlor-benzyl)-4-dimethylamino-cyclohexyl]-4-(3,6-dihydro-2H-pyridin-1-yl)-4-oxo-butyramid
N-[4-Dimethylamino-4-(3-methyl-benzyl)-cyclohexyl]-N'-(1-methyl-3-phenyl-propyl)-succinamid
5-[4-(5-Methyl-pyrazin-2-carbonyl)-piperazin-1-yl]-5-oxo-valeriansäure (4-benzyl-4-dimethylamino-cyclohexyl)-amid
N-[4-(3-Chlor-benzyl)-4-dimethylamino-cyclohexyl]-N'-(2-chlor-6-methyl-benzyl)-succinamid
N-Allyl-N'-[4-(3-chlor-benzyl)-4-dimethylamino-cyclohexyl]-N-methyl-Succinamia
N-[4-(4-Chlor-benzyl)-4-dimethylamino-cyclohexyl]-N'-cyclopentyl-succinamid
N-[4-Dimethylamino-4-(4-methyl-benzyl)-cyclohexyl]-N'-(tetrahydro-furan-2-ylmethyl)-succinamid
(4-{3-[4-Dimethylamino-4-(3-methyl-benzyl)-cyclohexylcarbamoyl]-propionylamino}-benzyl)-phosphonsäurediethylester
N-[4-(4-Chlor-benzyl)-4-dimethylamino-cyclohexyl]-4-[4-(2-methoxy-phenyl)-piperazin-1-yl]-4-oxo-butyramid
N-[4-Dimethylamino-4-(3-methyl-benzyl)-cyclohexyl]-4-[4-(3-methoxy-phenyl)-piperazin-1-yl]-4-oxo-butyramid
N-[4-(4-Chlor-benzyl)-4-dimethylamino-cyclohexyl]-N'-(3,5-difluor-benzyl)-succinamid
N-[4-(4-Chlor-benzyl)-4-dimethylamino-cyclohexyl]-N'-(2-methyl-benzyl)-succinamid
N-[4-Dimethylamino-4-(4-methyl-benzyl)-cyclohexyl]-4-oxo-4-{4-[(2,2,2-trifluor-acetylamino)-methyl]-piperidin-1-yl}-butyramid
N-[4-(4-Chlor-benzyl)-4-dimethylamino-cyclohexyl]-N'-[2-(1H-indol-3-yl)-ethyl]-succinamid
N-[4-(3-Chlor-benzyl)-4-dimethylamino-cyclohexyl]-N'-[2-(1H-indol-3-yl)-ethyl]-N'-methyl-succinamid
5-[4-(7-Methoxy-benzo[1,3]dioxol-5-ylmethyl)-piperazin-1-yl]-5-oxo-valeriansäure (4-benzyl-4-dimethylamino-cyclohexyl)-amid
N-Benzyl-N'-[4-dimethylamino-4-(3-methyl-benzyl)-cyclohexyl]-N-(2-hydroxy-ethyl)-succinamid
5-[4-(2,3-Dimethyl-phenyl)-piperazin-1-yl]-5-oxo-valeriansäure (4-benzyl-4-dimethylamino-cyclohexyl)-amid
N-[4-(4-Chlor-benzyl)-4-dimethylamino-cyclohexyl]-N'-[2-(1H-indol-3-yl)-ethyl]-N'-methyl-succinamid
N-[4-Dimethylamino-4-(4-methyl-benzyl)-cyclohexyl]-4-[4-(2,3-dimethyl-phenyl)-piperazin-1-yl]-4-oxo-butyramid
Glutarsäure-(4-benzyl-4-dimethylamino-cyclohexyl)-amid (1,2,3,4-tetrahydro-naphthalen-1-yl)-amid
N-[4-(4-Chlor-berizyl)-4-dimethylamino-cyclohexyl]-4-(4-methyl-piperidin-1-yl)-4-oxo-butyramid
2-{3-[4-Dimethylamino-4-(4-methyl-benzyl)-cyclohexylcarbamoyl]-propionylamino}-propionsäure-tert-butylester
5-(4-Cycloheptyl-piperazin-1-yl)-5-oxo-valeriansäure (4-benzyl-4-dimethylamino-cyclohexyl)-amid
Glutarsäure-(4-benzyl-4-dimethylamino-cyclohexyl)-amid [2-(4-fluor-phenyl)-ethyl]-amid
Glutarsäure-(4-benzyl-4-dimethylamino-cyclohexyl)-amid [1-(2,6-dichlor-benzyl)-pyrrolidin-3-yl]-amid
N-[4-(4-Chlor-benzyl)-4-dimethylamino-cyclohexyl]-4-(1,4-dioxa-8-aza-spiro[4.5]dec-8-yl)-4-oxo-butyramid
N-[4-(3-Chlor-benzyl)-4-dimethylamino-cyclohexyl]-4-oxo-4-thiomorpholin-4-yl-butyramid
4-(4-Benzoyl-piperidin-1-yl)-N-[4-dimethylamino-4-(3-methyl-benzyl)-cyclohexyl]-4-oxo-butyramid
N-[4-(4-Chlor-benzyl)-4-dimethylamino-cyclohexyl]-N'-(2,4-dimethoxy-benzyl)-succinamid
N-[4-Dimethylamino-4-(3-methyl-benzyl)-cyclohexyl]-N'-(1,2,3,4-tetrahydro-naphthalen-1-yl)-succinamid
N-[4-(4-Chlor-benzyl)-4-dimethylamino-cyclohexyl]-N'-ethyl-N'-pyridin-4-ylmethyl-succinamid
N-[2-(3,4-Dimethoxy-phenyl)-ethyl]-N'-[4-dimethylamino-4-(3-methyl-benzyl)-cyclohexyl]-N-methyl-succinamid
N-[4-(4-Chlor-benzyl)-4-dimethylamino-cyclohexyl]-N'-methyl-N'-pyridin-3-ylmethyl-succinamid
N-[4-(3-Chlor-benzyl)-4-dimethylamino-cydohexyl]-N'-pyridin-2-ylmethyl-succinamid
N-[4-(3-Chlor-benzyl)-4-dimethylamino-cyclohexyl]-N'-(2-methyl-benzyl)-succinamid
N-[4-Dimethylamino-4-(3-methyl-benzyl)-cyclohexyl]-N'-(4-methoxy-phenyl)-succinamid
N-[4-Dimethylamino-4-(3-methyl-benzyl)-cyclohexyl]-N'-indan-2-yl-succinamid
N-[4-(3-Chlor-benzyl)-4-dimethylamino-cyclohexyl]-N'-(1-methyl-3-phenyl-propyl)-succinamid
N-(2-Chlor-6-methyl-benzyl)-N'-[4-dimethylamino-4-(3-methyl-benzyl)-cyclohexyl]-succinamid
N-[4-Dimethylamino-4-(4-methyl-benzyl)-cyclohexyl]-4-[4-(4-methoxy-phenyl)-piperazin-1-yl]-4-oxo-butyramid
N-[4-Dimethylamino-4-(4-methyl-benzyl)-cyclohexyl]-N'-[2-(4-fluor-phenyl)-ethyl]-succinamid
Glutarsäure-(4-benzyl-4-dimethylamino-cyclohexyl)-amid 4-dimethylamino-benzylamid
N-[4-(3-Chlor-benzyl)-4-dimethylamino-cyclohexyl]-N'-(3,5-dichlor-benzyl)-succinamid
N-[4-(4-Chlor-benzyl)-4-dimethylamino-cyclohexyl]-N'-(1-methyl-3-phenyl-propyl)-succinamid
4-{3-[4-(3-Chlor-benzyl)-4-dimethylamino-cyclohexylcarbamoyl]-propionyl}-piperazin-1-carbonsäureethylester
N-[4-Dimethylamino-4-(3-methyl-benzyl)-cyclohexyl]-N'-(4-methoxy-benzyl)-succinamid
N-[4-(4-Chlor-benzyl)-4-dimethylamino-cyclohexyl]-4-(2,6-dimethyl-morpholin-4-yl)-4-oxo-butyramid
N-[4-Dimethylamino-4-(4-methyl-benzyl)-cyclohexyl]-4-oxo-4-(4-phenyl-3,6-dihydro-2H-pyridin-1-yl)-butyramid
N-[4-(4-Chlor-benzyl)-4-dimethylamino-cyclohexyl]-N'-[2-(3-fluor-phenyl)-ethyl]-succinamid
4-[4-(4-Allyloxy-benzyl)-piperazin-1-yl]-N-[4-dimethylamino-4-(3-methyl-benzyl)-cyclohexyl]-4-oxo-butyramid
Glutarsäure-(4-benzyl-4-dimethylamino-cyclohexyl)-amid [2-(5-brom-2-methoxy-phenyl)-ethyl]-amid
5-[4-(3-Methoxy-phenyl)-piperazin-1-yl]-5-oxo-valeriansäure (4-benzyl-4-dimethylamino-cyclohexyl)-amid
N-[4-(3-Chlor-benzyl)-4-dimethylamino-cyclohexyl]-4-(4-methyl-pi perid in-1-yl)-4-oxo-butyramid
(4-{3-[4-(4-Chlor-benzyl)-4-dimethylamino-cyclohexylcarbamoyl]-propionylamino}-benzyl)-phosphonsäurediethylester
N-[4-Dimethylamino-4-(4-methyl-benzyl)-cyclohexyl]-4-oxo-4-(4-o-tolyl-piperazin-1-yl)-butyramid
N-[4-Dimethylamino-4-(3-methyl-benzyl)-cyclohexyl]-N'-[2-(3-fluor-phenyl)-ethyl]-succinamid
4-[4-(4-Chlor-phenyl)-piperazin-1-yl]-N-[4-dimethylamino-4-(4-methyl-benzyl)-cyclohexyl]-4-oxo-butyramid
N-[4-(3-Chlor-benzyl)-4-dimethylamino-cyclohexyl]-N'-[1-(4-fluor-phenyl)-ethyl]-succinamid
N-[4-(4-Chlor-benzyl)-4-dimethylamino-cyclohexyl]-N'-[1-(4-trifluormethyl-benzyl)-pyrrolidin-3-yl]-succinamid
N-[4-(3-Chlor-benzyl)-4-dimethylamino-cyclohexyl]-N'-[2-(1H-indol-3-yl)-ethyl]-succinamid
N-[4-(4-Chlor-benzyl)-4-dimethylamino-cyclohexyl]-N'-indan-5-yl-succinamid
N-(4-Benzyloxy-phenyl)-N'-[4-dimethylamino-4-(4-methyl-benzyl)-cyclohexyl]-succinamid
2-{3-[4-Dimethylamino-4-(3-methyl-benzyl)-cyclohexylcarbamoyl]-propionylamino}-3-methyl-valeriansäure tert-butyl ester
4-(4-Benzyl-piperidin-1-yl)-N-[4-dimethylamino-4-(3-methyl-benzyl)-cyclohexyl]-4-oxo-butyramid
N-[4-(4-Chlor-benzyl)-4-dimethylamino-cyclohexyl]-4-[4-(4-fluor-phenyl)-3,6-dihydro-2H-pyridin-1-yl]-4-oxo-butyramid
N-[4-Dimethylamino-4-(4-methyl-benzyl)-cyclohexyl]-N'-(1,2-dimethyl-propyl)-succinamid
N-[4-Dimethylamino-4-(3-methyl-benzyl)-cyclohexyl]-4-(8-fluor-1,3,4,5-tetrahydro-pyrido[4,3-b]indol-2-yl)-4-oxo-butyramid
4-[4-(3-Chlor-benzoyl)-piperazin-1-yl]-N-[4-dimethylamino-4-(3-methyl-benzyl)-cyclohexyl]-4-oxo-butyramid
N-[4-(4-Chlor-benzyl)-4-dimethylamino-cyclohexyl]-N'-(4-methoxy-phenyl)-succinamid
5-[4-(2,6-Dichlor-benzyl)-piperazin-1-yl]-5-oxo-valeriansäure (4-benzyl-4-dimethylamino-cyclohexyl)-amid
N-[4-Dimethylamino-4-(3-methyl-benzyl)-cyclohexyl]-N'-[2-(1H-indol-3-yl)-ethyl]-N'-methyl-succinamid
N-[4-Dimethylamino-4-(3-methyl-benzyl)-cyclohexyl]-N'-[1-(4-fluor-phenyl)-ethyl]-succinamid
4-[4-(4-Chlor-phenyl)-piperazin-1-yl]-N-[4-dimethylamino-4-(3-methyl-benzyl)-cyclohexyl]-4-oxo-butyramid
N-[4-(3-Chlor-benzyl)-4-dimethylamino-cyclohexyl]-4-oxo-4-(4-oxo-1-phenyl-1,3,8-triaza-spiro[4.5]dec-8-yl)-butyramid
N-Benzyl-N'-[4-dimethylamino-4-(3-methyl-benzyl)-cyclohexyl]-N-methyl-succinamid
N-[4-(3-Chlor-benzyl)-4-dimethylamino-cyclohexyl]-N'-(3,5-difluor-benzyl)-succinamid
N-[4-Dimethylamino-4-(3-methyl-benzyl)-cyclohexyl]-N'-[2-(4-fluor-phenyl)-ethyl]-succinamid
N-[4-Dimethylamino-4-(3-methyl-benzyl)-cyclohexyl]-N'-[2-(1H-indol-3-yl)-ethyl]-succinamid
N-(2-Benzylsulfanyl-ethyl)-N'-[4-dimethylamino-4-(3-methyl-benzyl)-cyclohexyl]-succinamid
N-[4-(3-Chlor-benzyl)-4-dimethylamino-cyclohexyl]-N'-(2,4-difluor-benzyl)-succinamid
N-[4-(3-Chlor-benzyl)-4-dimethylamino-cyclohexyl]-N'-phenethyl-succinamid
N-[4-Dimethylamino-4-(3-methyl-benzyl)-cyclohexyl]-N'-(1-naphthalen-2-yl-ethyl)-succinamid
N-[4-(3-Chlor-benzyl)-4-dimethylamino-cyclohexyl]-N'-(1-methoxymethyl-2-phenyl-ethyl)-succinamid
N-[4-(3-Chlor-benzyl)-4-dimethylamino-cyclohexyl]-N'-(3,4-dichlor-benzyl)-succinamid
N-[4-(3-Chlor-benzyl)-4-dimethylamino-cyclohexyl]-N'-furan-2-ylmethyl-succinamid
N-[4-Dimethylamino-4-(4-methyl-benzyl)-cyclohexyl]-N'-(1,2,3,4-tetrahydro-naphthalen-1-yl)-succinamid
N-[4-(3-Chlr-benzyl)-4-dimethylamino-cyclohexyl]-N'-(4-fluor-benzyl)-succinamid
N-[4-Dimethylamino-4-(3-methyl-benzyl)-cyclohexyl]-N'-indan-1-yl-succinamid
N-[4-(3-Chlor-benzyl)-4-dimethylamino-cyclohexyl]-4-(3,5-dimethyl-piperidin-1-yl)-4-oxo-butyramid
N-[4-Dimethylamino-4-(3-methyl-benzyl)-cyclohexyl]-N'-phenethyl-succinamid
N-[4-Dimethylamino-4-(4-methyl-benzyl)-cyclohexyl]-N'-phenethyl-succinamid
N-(2-Benzylsulfanyl-ethyl)-N'-[4-(3-chlor-benzyl)-4-dimethylamino-cyclohexyl]-succinamid
N-[4-(3-Chlor-benzyl)-4-dimethylamino-cyclohexyl]-N'-(2,5-dichlor-benzyl)-succinamid
4-[4-(3-Chlor-benzoyl)-piperazin-1-yl]-N-[4-(4-chlor-benzyl)-4-dimethylamino-cyclohexyl]-4-oxo-butyramid
N-[4-(4-Chlor-benzyl)-4-dimethylamino-cyclohexyl]-N'-(1-phenyl-propyl)-succinamid
N-(4-Brom-2-fluor-benzyl)-N'-[4-dimethylamino-4-(3-methyl-benzyl)-cyclohexyl]-succinamid
N-[4-(3-Chlor-benzyl)-4-dimethylamino-cyclohexyl]-N'-methyl-N'-pyridin-3-ylmethyl-succinamid
N-[4-Dimethylamino-4-(4-methyl-benzyl)-cyclohexyl]-N'-(2-ethoxy-benzyl)-succinamid
N-[4-Dimethylamino-4-(3-methyl-benzyl)-cyclohexyl]-N'-[2-(5-methoxy-1 H-indol-3-yl)-ethyl]-succinamid
N-(4-sec-Butyl-phenyl)-N'-[4-(4-chlor-benzyl)-4-dimethylamino-cyclohexyl]-succinamid
5-Oxo-5-(4-phenyl-3,6-dihydro-2H-pyridin-1-yl)-valeriansäure (4-benzyl-4-dimethylamino-cyclohexyl)-amid
5-Oxo-5-(4-phenyl-3,6-dihydro-2H-pyridin-1-yl)-valeriansäure (4-benzyl-4-dimethylamino-cyclohexyl)-amid
N-[4-(3-Chlor-benzyl)-4-dimethylamino-cyclohexyl]-N'-[2-(3-fluor-phenyl)-ethyl]-succinamid
N-(3-Brom-benzyl)-N'-[4-(3-chlor-benzyl)-4-dimethylamino-cyclohexyl]-succinamid
N-(4-Brom-2-fluor-benzyl)-N'-[4-(3-chlor-benzyl)-4-dimethylamino-cyclohexyl]-succinamid
N-[4-(3-Chlor-benzyl)-4-dimethylamino-cyclohexyl]-N'-(1-phenyl-propyl)-succinamid
N-[4-Dimethylamino-4-(3-methyl-benzyl)-cyclohexyl]-N'-(4-fluor-2-trifluormethyl-benzyl)-succinamid
N-[4-(3-Chlor-benzyl)-4-dimethylamino-cyclohexyl]-N'-indan-2-yl-succinamid
N-[4-Dimethylamino-4-(3-methyl-benzyl)-cyclohexyl]-4-oxo-4-(4-phenyl-3,6-dihydro-2H-pyridin-1-yl)-butyramid
N-[4-(4-Chlor-benzyl)-4-dimethylamino-cyclohexyl]-N'-[2-(2-fluor-phenyl)-ethyl]-succinamid
N-[4-Dimethylamino-4-(4-methyl-benzyl)-cyclohexyl]-N'-[2-(2-fluor-phenyl)-ethyl]-succinamid
Glutarsäure-(4-benzyl-4-dimethylamino-cyclohexyl)-amid [2-(2-fluor-phenyl)-ethyl]-amid
N-[2-(2-Chlor-phenyl)-ethyl]-N'-[4-dimethylamino-4-(4-methyl-benzyl)-cyclohexyl]-succinamid
N-[4-(3-Chlor-benzyl)-4-dimethylamino-cyclohexyl]-N'-(3,4-difluor-benzyl)-succinamid
N-Benzyl-N-(2-cyano-ethyl)-N'-[4-dimethylamino-4-(4-methyl-benzyl)-cyclohexyl]-succinamid
N-[4-(3-Chlor-benzyl)-4-dimethylamino-cyclohexyl]-N'-(3-methyl-benzyl)-succinamid
N-[4-(3-Chlor-benzyl)-4-dimethylamino-cyclohexyl]-N'-(4-fluor-3-trifluormethyl-benzyl)-succinamid
N-[4-(3-Chlor-benzyl)-4-dimethylamino-cyclohexyl]-N'-[2-(2-chlor-phenyl)-ethyl]-succinamid
N-[4-(3-Chlor-benzyl)-4-dimethylamino-cyclohexyl]-N'-[2-(4-chlor-phenyl)-ethyl]-succinamid
N-[4-(3-Chlor-benzyl)-4-dimethytamino-cyclohexyl]-N'-[2-(2,4-dichlor-phenyl)-ethyl]-succinamid
N-[4-(3-Chlor-benzyl)-4-dimethylamino-cyclohexyl]-N'-indan-1-yl-succinamid
N-[4-(4-Chlor-benzyl)-4-dimethylamino-cyclohexyl]-N'-indan-1-yl-succinamid
N-[2-(4-Chlor-phenyl)-propyl]-N'-[4-dimethylamino-4-(3-methyl-benzyl)-cyclohexyl]-succinamid
N-(3-Brom-benzyl)-N'-[4-(4-chlor-benzyl)-4-dimethylamino-cyclohexyl]-succinamid
N-[4-(3-Chlor-benzyl)-4-dimethylamino-cyclohexyl]-N'-(3-phenyl-propyl)-succinamid
N-[4-Dimethylamino-4-(4-methyl-benzyl)-cyclohexyl]-N'-(2-p-tolyl-ethyl)-succinamid
N-[4-(3-Chlor-benzyl)-4-dimethylamino-cyclohexyl]-N'-(2-thiophen-2-yl-ethyl)-succinamid
Glutarsäure-(4-benzyl-4-dimethylamino-cyclohexyl)-amid [2-(4-chlor-phenyl)-propyl]-amid
N-Benzyl-N'-[4-(3-chlor-benzyl)-4-dimethylamino-cyclohexyl]-N-methyl-succinamid
N-[4-(3-Chlor-benzyl)-4-dimethylamino-cyclohexyl]-N'-[2-(3-chlor-phenyl)-ethyl]-succinamid
Glutarsäure-(4-benzyl-4-dimethylamino-cyclohexyl)-amid [2-(2-ethoxy-phenyl)-ethyl]-amid
Glutarsäure-(4-benzyl-4-dimethylamino-cyclohexyl)-amid phenethyl-amid
Giutarsäure-(4-benzyi-4-dimethylamino-cyclohexyl)-amid [2-(3-chlor-phenyl)-ethyl]-amid
N-[4-(3-Chlor-benzyl)-4-dimethylamino-cyclohexyl]-N'-[1-(3-methoxy-phenyl)-ethyl]-succinamid
Glutarsäure-(4-benzyl-4-dimethylamino-cyclohexyl)-amid (1,3-dimethyl-butyl)-amid
N-Benzyl-N'-[4-dimethylamino-4-(3-methyl-benzyl)-cyclohexyl]-N-ethyl-succinamid
N-(4-Chlor-benzyl)-N'-[4-(3-chlor-benzyl)-4-dimethylamino-cyclohexyl]-succinamid
N-[4-(3-Chlor-benzyl)-4-dimethylamino-cyclohexyl]-N'-[2-(2-fluor-phenyl)-ethyl]-succinamid
N-[4-Dimethylamino-4-(3-methyl-benzyl)-cyclohexyl]-N'-(2-fluor-benzyl)-succinamid
N-[2-(4-Chlor-phenyl)-ethyl]-N'-[4-dimethylamino-4-(3-methyl-benzyl)-cyclohexyl]-succinamid
N-[2-(2-Chlor-phenyl)-ethyl]-N'-[4-dimethylamino-4-(3-methyl-benzyl)-cyclohexyl]-succinamid
N-[2-(3-Chlor-phenyl)-ethyl]-N'-[4-dimethylamino-4-(3-methyl-benzyl)-cyclohexyl]-succinamid
N-Benzyl-N'-[4-(3-chlor-benzyl)-4-dimethylamino-cyclohexyl]-N-(2-cyano-ethyl)-succinamid
N-[4-(3-Chlor-benzyl)-4-dimethylamino-cyclohexyl]-N'-(3-chlor-4-fluor-benzyl)-succinamid
N-[4-Dimethylamino-4-(3-methyl-benzyl)-cyclohexyl]-N'-(2-trifluormethyl-benzyl)-succinamid
N-[2-(2,4-Dichlor-phenyl)-ethyl]-N'-[4-dimethylamino-4-(3-methyl-benzyl)-cyclohexyl]-succinamid
N-Benzyl-N'-[4-(3-chlor-benzyl)-4-dimethylamino-cyclohexyl]-N-ethyl-succinamid
N-[2-(4-Chlor-phenyl)-ethyl]-N'-[4-dimethylamino-4-(4-methyl-benzyl)-cyclohexyl]-succinamid
5-Oxo-5-(4-o-tolyl-piperazin-1-yl)-valeriansäure (4-benzyl-4-dimethylamino-cyclohexyl)-amid
N-[4-(3-Chlor-benzyl)-4-dimethylamino-cyclohexyl]-N'-[3-(methyl-phenyl-amino)-propyl]-succinamid
N-[4-(3-Chlor-benzyl)-4-dimethylamino-cyclohexyl]-4-[4-(5-chlor-2-methyl-phenyl)-piperazin-1-yl]-4-oxo-butyramid
N-[4-Dimethylamino-4-(3-methyl-benzyl)-cyclohexyl]-N'-[2-(2-ethoxy-phenyl)-ethyl]-succinamid
4-[4-(4-Chlor-benzoyl)-piperidin-1-yl]-N-[4-(3-chlor-benzyl)-4-dimethylamino-cyclohexyl]-4-oxo-butyramid
N-[4-(3-Chlor-benzyl)-4-dimethylamino-cyclohexyl]-N'-[2-(2-ethoxy-phenyl)-ethyl]-succinamid
N-[2-(2,6-Dichlor-phenyl)-ethyl]-N'-[4-dimethylamino-4-(3-methyl-benzyl)-cyclohexyl]-succinamid
N-Benzhydryl-N'-[4-(3-chlor-benzyl)-4-dimethylamino-cyclohexyl]-succinamid
N-[4-(3-Chlor-benzyl)-4-dimethylamino-cyclohexyl]-N'-cyclooctyl-succinamid
N-[4-(3-Chlor-benzyl)-4-dimethylamino-cyclohexyl]-N'-[2-(4-phenoxy-phenyl)-ethyl]-succinamid
N-[4-Dimethylamino-4-(3-methyl-benzyl)-cyclohexyl]-N'-(2,3-dimethyl-benzyl)-succinamid
4-[4-(5-Chlor-2-methyl-phenyl)-piperazin-1-yl]-N-[4-dimethylamino-4-(3-methyl-benzyl)-cyclohexyl]-4-oxo-butyramid
4-[4-(4-Chlor-benzoyl)-piperidin-1-yl]-N-[4-dimethylamino-4-(3-methyl-benzyl)-cyclohexyl]-4-oxo-butyramid
N-[4-(3-Chlor-benzyl)-4-dimethylamino-cyclohexyl]-N'-(1,2,3,4-tetrahydro-naphthalen-1-yl)-succinamid
N-(2-Chlor-benzyl)-N'-[4-dimethylamino-4-(3-methyl-benzyl)-cyclohexyl]-succinamid
N-(2-Chlor-benzyl)-N'-[4-(3-chlor-benzyl)-4-dimethylamino-cyclohexyl]-succinamid
N-[4-(3-Chlor-benzyl)-4-dimethylamino-cyclohexyl]-N'-(3-fluor-5-trifluormethyl-benzyl)-succinamid
N-[4-(3-Chlor-benzyl)-4-dimethylamino-cyclohexyl]-N'-(1-p-tolyl-ethyl)-succinamid
N-[2-(2,6-Dichlor-benzylsulfanyl)-ethyl]-N'-[4-dimethylamino-4-(4-methyl-benzyl)-cyclohexyl]-succinamid
N-[4-(3-Chlor-benzyl)-4-dimethylamino-cyclohexyl]-N'-[2-(2,6-dichlor-benzylsulfanyl)-ethyl]-succinamid
N-[4-(3-Chlor-benzyl)-4-dimethylamino-cyclohexyl]-N'-[2-(3-trifluormethyl-phenyl)-ethyl]-succinamid
N-[2-(3,4-Dichlor-phenyl)-ethyl]-N'-[4-dimethylamino-4-(3-methyl-benzyl)-cyclohexyl]-succinamid
N-[4-(3-Chlor-benzyl)-4-dimethylamino-cyclohexyl]-4-[4-(2-fluor-phenyl)-piperazin-1-yl]-4-oxo-butyramid
5-[4-(2,4-Dimethoxy-phenyl)-piperazin-1-yl]-5-oxo-valeriansäure (4-benzyl-4-dimethylamino-cyclohexyl)-amid
N-[4-(3-Chlor-benzyl)-4-dimethylamino-cyclohexyl]-N'-(1-naphthalen-1-yl-ethyl)-succinamid
N-[4-Dimethylamino-4-(3-methyl-benzyl)-cyclohexyl]-N'-(1-naphthalen-1-yl-ethyl)-succinamid
N-[4-(3-Chlor-benzyl)-4-dimethylamino-cyclohexyl]-N'-(2-fluor-benzyl)-N'-furan-2-ylmethyl-succinamid
N-[4-Dimethylamino-4-(3-methyl-benzyl)-cyclohexyl]-N'-(2-fluor-benzyl)-N'-furan-2-ylmethyl-succinamid
N-[4-(3-Chlor-benzyl)-4-dimethylamino-cyclohexyl]-N'-(2,5-dimethoxy-benzyl)-N'-furan-2-ylmethyl-succinamid
N-(4-Azepan-1-yl-4-benzyl-cyclohexyl)-4-(2,3-dihydro-indol-1-yl)-4-oxo-butyramid
4-(2,3-Dihydro-indol-1-yl)-N-[4-dimethylamino-4-(3-fluor-phenyl)-cyclohexyl]-4-oxo-butyramid
N-(4-Benzyl-4-morpholin-4-yl-cyclohexyl)-N'-(2-chlor-6-methyl-benzyl)-succinamid
N-(4-Benzyl-4-morpholin-4-yl-cyclohexyl)-N'-[2-(3-methoxy-phenyl)-ethyl]-succinamid
N-(4-Benzyl-4-morpholin-4-yl-cyclohexyl)-N'-(3-methyl-benzyl)-succinamid
N-(4-Benzyl-4-morpholin-4-yl-cyclohexyl)-N'-(4-brom-2-fluor-benzyl)-succinamid
N-(4-Benzyl-4-morpholin-4-yl-cyclohexyl)-N'-pyridin-4-ylmethyl-succinamid
N-(4-Benzyl-4-morpholin-4-yl-cyclohexyl)-N'-indan-1-yl-succinamid
N-(4-Benzyl-4-morpholin-4-yl-cyclohexyl)-N'-(2,4-dimethoxy-benzyl)-succinamid
N-(4-Benzyl-4-morpholin-4-yl-cyclohexyl)-N'-thiophen-2-ylmethyl-succinamid
N-Adamantan-1-ylmethyl-N'-(4-benzyl-4-morpholin-4-yl-cyclohexyl)-succinamid
N-(4-Benzyl-4-morpholin-4-yl-cyclohexyl)-N'-(2,5-difluor-benzyl)-succinamid
N-(4-Benzyl-4-morpholin-4-yl-cyclohexyl)-N'-(4-tert-butyl-phenyl)-succinamid
N-(4-Benzyl-4-morpholin-4-yl-cyclohexyl)-N'-(3,5-bis-trifluormethyl-benzyl)-succinamid
N-(4-Benzyl-4-morpholin-4-yl-cyclohexyl)-N'-[2-(2-ethoxy-phenyl)-ethyl]-succinamid
N-(4-Benzyl-4-morpholin-4-yl-cyclohexyl)-N'-(3-methoxy-propyl)-succinamid
N-(4-Benzyl-4-morpholin-4-yl-cyclohexyl)-N'-(2-morpholin-4-yl-ethyl)-succinamid
N-(4-Benzyl-4-morpholin-4-yl-cyclohexyl)-N'-(2,5-dichlor-benzyl)-succinamid
N-(4-Benzyl-4-morpholin-4-yl-cyclohexyl)-N'-[1-(2-benzyloxy-benzyl)-pyrrolidin-3-yl]-N'-methyl-succinamid
N-(4-Benzyl-4-morpholin-4-yl-cyclohexyl)-4-oxo-4-(2-pyrrolidin-1-ylmethyl-pyrrolidin-1-yl)-butyramid
N-(4-Benzyl-4-morpholin-4-yl-cyclohexyl)-4-morpholin-4-yl-4-oxo-butyramid
N-(4-Benzyl-4-morpholin-4-yl-cyclohexyl)-N'-(3-dimethylamino-propyl)-N'-methyl-succinamid
N-(4-Benzyl-4-morpholin-4-yl-cyclohexyl)-N'-(2-phenyl-cyclopropyl)-succinamid
N-(4-Benzyl-4-morpholin-4-yl-cyclohexyl)-N'-naphthalen-1-ylmethyl-succinamid
N-(4-Benzyl-4-morpholin-4-yl-cyclohexyl)-4-(4-methyl-[1,4]diazepan-1-yl)-4-oxo-butyramid
N-(4-Benzyl-4-morpholin-4-yl-cyclohexyl)-4-(4-hydroxy-piperidin-1-yl)-4-oxo-butyramid
N-(4-Benzyl-4-morpholin-4-yl-cyclohexyl)-N'-[2-(7-methyl-1H-indol-3-yl)-ethyl]-succinamid
N-(4-Benzyl-4-morpholin-4-yl-cyclohexyl)-N'-[2-(5-methoxy-1H-indol-3-yl)-ethyl]-succinamid
N-(4-Benzyl-4-morpholin-4-yl-cyclohexyl)-4-oxo-4-(4-chinolin-2-ylmethyl-piperazin-1-yl)-butyramid
N-(4-Dimethylamino-4-thiophen-2-yl-cyclohexyl)-4-oxo-4-[4-(5-trifluormethyl-pyridin-2-yl)-piperazin-1-yl]-butyramid
Glutarsäure-(1-adamantan-1-yl-ethyl)-amid (4-dimethylamino-4-thiophen-2-yl-cyclohexyl)-amid
N-[2-(2,6-Dichlor-phenyl)-ethyl]-N'-(4-dimethylamino-4-thiophen-2-yl-cyclohexyl)-succinamid
N-(4-Dimethylamino-4-thiophen-2-yl-cyclohexyl)-N'-(1-methyl-3-phenyl-propyl)-succinamid
N-Benzyl-N'-(4-dimethylamino-4-thiophen-2-yl-cyclohexyl)-N-ethyl-succinamid
N-[2-(4-Brom-phenyl)-ethyl]-N'-(4-dimethylamino-4-thiophen-2-yl-cyclohexyl)-succinamid
4-[4-(4-Chlor-benzoyl)-piperidin-1-yl]-N-(4-dimethylamino-4-thiophen-2-yl-cyclohexyl)-4-oxo-butyramid
Glutarsäure-[1-(2,6-dichlor-benzyl)-pyrrolidin-3-yl]-amid (4-dimethylamino-4-thiophen-2-yl-cyclohexyl)-amid
Glutarsäure-(4-dimethylamino-4-thiophen-2-yl-cyclohexyl)-amid (3-phenyl-propyl)-amid
N-(4-Dimethylamino-4-phenyl-cyclohexyl)-N'-pentyl-succinamid
3-(4-Chlor-phenyl)-2-[4-(4-dimethylamino-4-thiophen-2-yl-cyclohexylcarbamoyl)-butyrylamino]-propionsäureethylester
Glutarsäure-(4-dimethylamino-4-thiophen-2-yl-cyclohexyl)-amid indan-1-ylamid
N-Adamantan-1-ylmethyl-N'-(4-dimethylamino-4-thiophen-2-yl-cyclohexyl)-succinamid
5-(4-Benzofuran-2-ylmethyl-piperazin-1-yl)-5-oxo-valeriansäure (4-dimethylamino-4-thiophen-2-yl-cyclohexyl)-amid
4-[4-(4-tert-Butyl-benzyl)-piperazin-1-yl]-N-(4-dimethylamino-4-phenyl-cyclohexyl)-4-oxo-butyramid
Glutarsäure-(4-dimethylamino-4-thiophen-2-yl-cyclohexyl)-amid [2-(1H-indol-3-yl)-ethyl]-amid
N-(4-Dimethylamino-4-phenyl-cyclohexyl)-4-[4-hydroxy-4-(3-trifluormethyl-phenyl)-piperidin-1-yl]-4-oxo-butyramid
5-(3,4-Dihydro-1H-isochinolin-2-yl)-5-oxo-valeriansäure (4-dimethylamino-4-thiophen-2-yl-cyclohexyl)-amid
4-(4-Benzyl-piperidin-1-yl)-N-(4-dimethylamino-4-phenyl-cyclohexyl)-4-oxo-butyramid
{[4-(4-Dimethylamino-4-thiophen-2-yl-cyclohexylcarbamoyl)-butyryl]-methyl-amino}-essigsäurebenzylester
N-Benzyl-N'-(4-dimethylamino-4-phenyl-cyclohexyl)-N-phenethyl-succinamid
Glutarsäure-(4-dimethylamino-4-thiophen-2-yl-cyclohexyl)-amid 3-trifluormethoxy-benzylamid
Glutarsäure-(4-dimethylamino-4-thiophen-2-yl-cyclohexyl)-amid ethyl-(2-methyl-allyl)-amid
N,N-Bis-(2-cyano-ethyl)-N'-(4-dimethylamino-4-thiophen-2-yl-cyclohexyl)-succinamid
Glutarsäure-benzyl-methyl-amid (4-dimethylamino-4-thiophen-2-yl-cyclohexyl)-amid
Glutarsäure-(2-benzyloxy-cyclopentyl)-amid (4-dimethylamino-4-thiophen-2-yl-cyclohexyl)-amid
Glutarsäure-(4-dimethylamino-4-thiophen-2-yl-cyclohexyl)-amid 4-trifluormethyl-benzylamid
N-(4-Dimethylamino-4-thiophen-2-yl-cyclohexyl)-N'-[2-(4-phenoxy-phenyl)-ethyl]-succinamid
Glutarsäure-(4-dimethylamino-4-thiophen-2-yl-cyclohexyl)-amid (3,3-diphenyl-propyl)-amid
4-(4-Benzyl-piperidin-1-yl)-N-(4-dimethylamino-4-thiophen-2-yl-cyclohexyl)-4-oxo-butyramid
N-(4-Dimethylamino-4-thiophen-2-yl-cyclohexyl)-4-[4-hydroxy-4-(3-trifluormethyl-phenyl)-piperidin-1-yl]-4-oxo-butyramid
Glutarsäure-(4-dimethylamino-4-thiophen-2-yl-cyclohexyl)-amid 4-fluor-2-trifluormethyl-benzylamid
N-(4-Dimethylamino-4-thiophen-2-yl-cyclohexyl)-N'-pentyl-succinamid
5-[4-(2-Methoxy-naphthalen-1-ylmethyl)-piperazin-1-yl]-5-oxo-valeriansäure (4-dimethylamino-4-thiophen-2-yl-cyclohexyl)-amid
N-(4-Dimethylamino-4-thiophen-2-yl-cyclohexyl)-N'-(1-ethoxy-benzyl)-N-furan-2-ylmethyl-succinamid
Glutarsäure-(4-dimethylamino-4-thiophen-2-yl-cyclohexyl)-amid (2-phenyl-cyclopropyl)-amid
N-[2-(3,4-Dichlor-phenyl)-ethyl]-N'-(4-dimethylamino-4-phenyl-cyclohexyl)-succinamid
N-Adamantan-1-ylmethyl-N'-(4-dimethylamino-4-phenyl-cyclohexyl)-succinamid
5-[4-(4-Fluor-phenyl)-3,6-dihydro-2H-pyridin-1-yl]-5-oxo-valeriansäure (4-dimethylamino-4-thiophen-2-yl-cyclohexyl)-amid
2-[4-(4-Dimethylamino-4-thiophen-2-yl-cyclohexylcarbamoyl)-butyrylamino]-4-methyl-valeriansäure benzyl ester
{4-[4-(4-Dimethylamino-4-thiophen-2-yl-cyclohexylcarbamoyl)-butyrylamino]-phenyl}-carbaminsäure-tert-butylester
Glutarsäure-(4-dimethylamino-4-thiophen-2-yl-cyclohexyl)-amid [2-(3-methoxy-phenyl)-ethyl]-amid
Glutarsäure-benzyl-ethyl-amid (4-dimethylamino-4-thiophen-2-yl-cyclohexyl)-amid
Glutarsäure-3-chlor-benzylamid (4-dimethylamino-4-thiophen-2-yl-cyclohexyl)-amid
Glutarsäure-(4-dimethylamino-4-thiophen-2-yl-cyclohexyl)-amid (4-methyl-cyclohexyl)-amid
N-(4-Dimethylamino-4-phenyl-cyclohexyl)-N'-[2-(1H-indol-3-yl)-ethyl]-N'-methyl-succinamid
Glutarsäure-2,5-dichlor-benzylamid (4-dimethylamino-4-thiophen-2-yl-cyclohexyl)-amid
Glutarsäure-(4-dimethylamino-4-thiophen-2-yl-cyclohexyl)-amid [2-(3-fluor-phenyl)-ethyl]-amid
N-(4-Dimethylamino-4-phenyl-cyclohexyl)-N'-[2-(4-methoxy-phenoxy)-ethyl]-succinamid
N-(4-Dimethylamino-4-thiophen-2-yl-cyclohexyl)-N'-[2-(ethyl-m-tolyl-amino)-ethyl]-succinamid
Glutarsäure-(4-benzyloxy-phenyl)-amid (4-dimethylamino-4-thiophen-2-yl-cyclohexyl)-amid
Glutarsäure-(4-dimethylamino-4-thiophen-2-yl-cyclohexyl)-amid [2-(4-fluor-phenyl)-ethyl]-amid
Glutarsäure-[2-(4-brom-phenyl)-ethyl]-amid (4-dimethylamino-4-thiophen-2-yl-cyclohexyl)-amid
N-(4-Dimethylamino-4-thiophen-2-yl-cyclohexyl)-N'-(4-phenoxy-phenyl)-succinamid
N-(4-Dimethylamino-4-phenyl-cyclohexyl)-N'-[2-(3-trifluormethyl-phenyl)-ethyl]-succinamid
Glutarsäure-[2-(4-chlor-phenyl)-propyl]-amid (4-dimethylamino-4-thiophen-2-yl-cyclohexyl)-amid
N-(4-Dimethylamino-4-thiophen-2-yl-cyclohexyl)-N'-[2-(1H-indol-3-yl)-ethyl]-N'-methyl-succinamid
N-(4-Dimethylamino-4-thiophen-2-yl-cyclohexyl)-N'-[2-(4-methoxy-phenoxy)-ethyl]-succinamid
Glutarsäure-(4-dimethylamino-4-thiophen-2-yl-cyclohexyl)-amid (3-phenoxy-phenyl)-amid
N-(4-Dimethylamino-4-phenyl-cyclohexyl)-N'-[2-(ethyl-m-tolyl-amino)-ethyl]-succinamid
Glutarsäure-[2-(5-brom-2-methoxy-phenyl)-ethyl]-amid (4-dimethylamino-4-thiophen-2-yl-cyclohexyl)-amid
N-(4-Dimethylamino-4-thiophen-2-yl-cyclohexyl)-N'-[2-(3-trifluormethyl-phenyl)-ethyl]-succinamid
Glutarsäure-(4-dimethylamino-4-thiophen-2-yl-cyclohexyl)-amid (1-methyl-3-phenyl-propyl)-amid
N-[2-(2,6-Dichlor-benzylsulfanyl)-ethyl]-N'-(4-dimethylamino-4-thiophen-2-yl-cyclohexyl)-succinamid
Glutarsäure-(4-dimethylamino-4-thiophen-2-yl-cyclohexyl)-amid (2,2-diphenyl-propyl)-amid
N-(4-Dimethylamino-4-thiophen-2-yl-cyclohexyl)-N'-[3-(methyl-phenyl-amino)-propyl]-succinamid
N-(1-Biphenyl-4-ylmethyl-pyrrolidin-3-yl)-N'-(4-dimethylamino-4-thiophen-2-yl-cyclohexyl)-N-methyl-succinamid
Glutarsäure-[4-(cyano-phenyl-methyl)-phenyl]-amid (4-dimethylamino-4-thiophen-2-yl-cyclohexyl)-amid
Glutarsäure-(4-tert-butyl-cyclohexyl)-amid (4-dimethylamino-4-thiophen-2-yl-cyclohexyl)-amid
Glutarsäure-(4-sec-butyl-phenyl)-amid (4-dimethylamino-4-thiophen-2-yl-cyclohexyl)-amid
Glutarsäure-(4-butyl-phenyl)-amid (4-dimethylamino-4-thiophen-2-yl-cyclohexyl)-amid
N-(2-Benzylsulfanyl-ethyl)-N'-(4-dimethylamino-4-thiophen-2-yl-cyclohexyl)-succinamid
N-(4-Dimethylamino-4-thiophen-2-yl-cyclohexyl)-N'-(2-phenoxy-ethyl)-succinamid
Glutarsäure-(4-dimethylamino-4-thiophen-2-yl-cyclohexyl)-amid (naphthalen-1-ylmethyl)-amid
Glutarsäure-3-brom-benzylamid (4-dimethylamino-4-thiophen-2-yl-cyclohexyl)-amid
N-(4-Dimethylamino-4-thiophen-2-yl-cyclohexyl)-N'-(3-phenyl-propyl)-succinamid
N-[2-(3-Chlor-phenyl)-ethyl]-N'-(4-dimethylamino-4-phenyl-cyclohexyl)-succinamid
2-[3-(4-Dimethylamino-4-thiophen-2-yl-cyclohexylcarbamoyl)-propionylamino]-propionsäurebenzylester
N-[2-(2-Chlor-phenyl)-ethyl]-N'-(4-dimethylamino-4-phenyl-cyclohexyl)-succinamid
N-(4-Dimethylamino-4-phenyl-cyclohexyl)-N'-[2-(3-fluor-phenyl)-ethyl]-succinamid
Glutarsäure-(4-dimethylamino-4-thiophen-2-yl-cyclohexyl)-amid (2-thiophen-2-yl-ethyl)-amid
Glutarsäure-(4-dimethylamino-4-thiophen-2-yl-cyclohexyl)-amid [2-(4-phenoxy-phenyl)-ethyl]-amid
N-[2-(4-Chlor-phenyl)-ethyl]-N'-(4-dimethylamino-4-phenyl-cyclohexyl)-succinamid
N-[2-(3-Chlor-phenyl)-ethyl]-N'-(4-dimethylamino-4-thiophen-2-yl-cyclohexyl)-succinamid
N-Benzyl-N'-(4-dimethylamino-4-thiophen-2-yl-cyclohexyl)-N-ethyl-succinamid
Glutarsäure-[1-(2,6-dichlor-benzyl)-pyrrolidin-3-yl]-amid (4-dimethylamino-4-thiophen-2-yl-cyclohexyl)-amid
Glutarsäure-(4-dimethylamino-4-thiophen-2-yl-cyclohexyl)-amid (3-phenyl-propyl)-amid
Glutarsäure-(4-dimethylamino-4-thiophen-2-yl-cyclohexyl)-amid [2-(1H-indol-3-yl)-ethyl]-amid
5-(3,4-Dihydro-1H-isochinolin-2-yl)-5-oxo-valeriansäure (4-dimethylamino-4-thiophen-2-yl-cyclohexyl)-amid
{[4-(4-Dimethylamino-4-thiophen-2-yl-cyclohexylcarbamoyl)-butyryl]-methyl-amino}-essigsäurebenzylester
N-(4-Dimethylamino-4-thiophen-2-yl-cyclohexyl)-N'-[2-(4-phenoxy-phenyl)-ethyl]-succinamid
Glutarsäure-(4-dimethylamino-4-thiophen-2-yl-cyclohexyl)-amid (2-phenyl-cyclopropyl)-amid
N-[4-(3-Chlorbenzyl)-4-dimethylaminocyclohexyl]-N'-furan-2-ylmethylbernsteinsäureamid Hydrochlorid, unpolareres Diastereoisomer
N-[4-(3-Chlorbenzyl)-4-dimethylaminocyclohexyl]-N'-furan-2-ylmethylbernsteinsäureamid Hydrochlorid, polareres Diastereoisomer
Glutarsäure (4-benzyl-4-dimethylaminocyclohexyl)amid [2-(2-fluorphenyl)ethyl]amid Hydrochlorid, unpolareres Diastereoisomer
Glutarsäure (4-benzyl-4-dimethylaminocyclohexyl)amid [2-(2-fluorphenyl)ethyl]amid Hydrochlorid, polareres Diastereoisomer
N-[4-(3-Chlorbenzyl)-4-dimethylaminocyclohexyl]-N'-[2-(4-chlorphenyl)ethyl]-bernsteinsäureamid Hydrochlorid, unpolareres Diastereoisomer
N-[4-(3-Chlorbenzyl)-4-dimethylaminocyclohexyl]-N'-[2-(4-chlorphenyl)ethyl]-bernsteinsäureamid Hydrochlorid, polareres Diastereoisomer
Glutarsäure (4-benzyl-4-dimethylaminocyclohexyl)amid [2-(4-chlorphenyl)ethyl]amid Hydrochlorid, unpolareres Diastereoisomer
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

Die erfindungsgemäßen Substanzen wirken beispielsweise auf den im Zusammenhang mit verschiedenen Erkrankungen relevanten ORL-1-Rezeptor, sodass sie sich als pharmazeutischer Wirkstoff in einem Arzneimittel eignen. Ein weiterer Gegenstand der Erfindung sind daher Arzneimittel enthaltend wenigstens ein erfindungsgemäßes substituiertes Cyclohexycarbonsäure-Derivat, sowie gegebenenfalls geeignete Zusatz- und/oder Hilfsstoffe und/oder gegebenenfalls weitere Wirkstoffe.

Die erfindungsgemäßen Arzneimittel enthalten neben mindestens einem erfindungsgemäßen substituierten Cyclohexyl-1,4-diamin-Derivat gegebenenfalls geeignete Zusatz- und/oder Hilfsstoffe, so auch Trägermaterialien, Füllstoffe, Lösungsmittel, Verdünnungsmittel, Farbstoffe und/oder Bindemittel und können als flüssige Arzneiformen in Form von Injektionslösungen, Tropfen oder Säfte, als halbfeste Arzneiformen in Form von Granulaten, Tabletten, Pellets, Patches, Kapseln, Pflaster/Sprühpflaster oder Aerosolen verabreicht werden. Die Auswahl der Hilfsstoffe etc. sowie die einzusetzenden Mengen derselben hängen davon ab, ob das Arzneimittel oral, peroral, parenteral, intravenös, intraperitoneal, intradermal, intramuskulär, intranasal, buccal, rektal oder örtlich, zum Beispiel auf die Haut, die Schleimhäute oder in die Augen, appliziert werden soll. Für die orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße substituierte Cyclohexyl-1,4-diamin -Derivate in einem Depot, in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die erfindungsgemäßen substituierten Cyclohexyl-1,4-diamin -Derivate verzögert freisetzen. Die erfindungsgemäßen substituierten Cyclohexyl-1,4-diamin -Derivate können auch in parenteralen Langzeitdepotformen wie z. B. Implantaten oder implantierten Pumpen angewendet werden. Prinzipiell können den erfindungsgemäßen Arzneimitteln andere dem Fachmann bekannte weitere Wirkstoffe zugesetzt werden.

Die an den Patienten zu verabreichende Wirkstoffmenge variiert in Abhängigkeit vom Gewicht des Patienten, von der Applikationsart, der Indikation und dem Schweregrad der Erkrankung. Üblicherweise werden 0,00005 bis 50 mg/kg, bevorzugt 0,01 bis 5 mg/kg wenigstens eines erfindungsgemäßen substituierten Cyclohexyl-1,4-diamin-Derivats appliziert.

Für alle vorstehenden Formen der erfindungsgemäßen Arzneimittel ist es besonders bevorzugt, wenn das Arzneimittel neben wenigstens einem substituierten Cyclohexyl-1,4-diamin-Derivat noch einen weiteren Wirkstoff, insbesondere ein Opioid, vorzugsweise ein starkes Opioid, insbesondere Morphin, oder ein Anesthetikum, vorzugsweise Hexobarbital oder Halothan, enthält.

In einer bevorzugten Form des Arzneimittel liegt ein enthaltenes erfindungsgemäßes substituiertes Cyclohexyl-1,4-diamin-Derivat als reines Diastereomer und/oder Enantiomer, als Razemat oder als nicht-äquimolare oder äquimolare Mischung der Diastereomere und/oder Enantiomere vor.

Der ORL-1-Rezeptor, aber auch die anderen Opioid-Rezeptoren, wurden insbesondere im Schmerzgeschehen identifiziert. Entsprechend können erfindungsgemäße substituierte Cyclohexyl-1,4-diamin-Derivate zur Herstellung eines Arzneimittels zur Behandlung von Schmerz, insbesondere von akutem, neuropathischem oder chronischem Schmerz, verwendet werden.

Ein weiterer Gegenstand der Erfindung ist daher die Verwendung eines erfindungsgemäßen substituierten Cyclohexyl-1,4-diamin-Derivats zur Herstellung eines Arzneimittels zur Behandlung von Schmerz, insbesondere von akutem, viszeralem, neuropathischem oder chronischem Schmerz.

Ein weiterer Gegenstand der Erfindung ist die Verwendung eines erfindungsgemäßen substituierten Cyclohexyl-1,4-diamin-Derivats zur Herstellung eines Arzneimittels zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, lokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

Dabei kann es in einer der vorstehenden Verwendungen bevorzugt sein, wenn ein verwendetes substituiertes Cyclohexyl-1,4-diamin-Derivat als reines Diastereomer und/oder Enantiomer, als Razemat oder als nicht-äquimolare oder äquimolare Mischung der Diastereomere und/oder Enantiomere vorliegt.

Weiterhin bezieht sich die Erfindung auf ein Verfahren zur Behandlung, insbesondere in einer der vorgenannten Indikationen, eines nichthumanen Säugetieres oder Menschen, das oder der eine Behandlung von Schmerzen, insbesondere chronischer Schmerzen, benötigt, durch Verabreichung einer therapeutisch wiksamen Dosis eines erfindungsgemäßen substituierten Cyclohexyl-1,4-diamin-Derivats, oder eines erfindungsgemäßen Arzneimittels.

Ein weiterer Gegenstand der Erfindung ist ein Verfahren zur Herstellung der erfindungsgemäßen substituierten Cyclohexyl-1,4-diamin-Derivate wie in der folgenden Beschreibung und Beispielen ausgeführt.

Die Reste R⁰¹ und R⁰² haben die für erfindungsgemäße Verbindungen gemäß Formel I für R¹ und R² angegebene Bedeutung und können zusätzlich unabhängig voneinander für eine Schutzgruppe stehen. Die übrigen Reste haben die in Formel I angegebene Bedeutung:

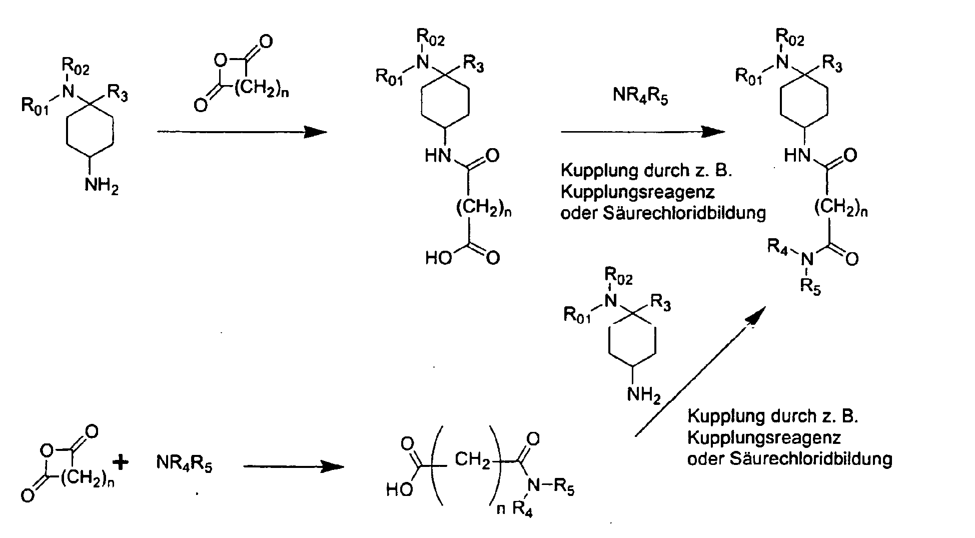

Zur Darstellung der erfindungsgemäßen Diamide sind grundsätzlich die vielfältigen, dem Fachmann bekannten Methoden zur Herstellung von Amiden geeignet. Das erfindungsgemäße Verfahren beruht darauf, substituierte Cyclohexan-1,4-diamine (WO 02090317) über Anhydride, offenkettige Dicarbonsäuren oder vorzugsweise deren aktivierte Analoga, insbesondere deren Säurehalogenide, mit weiteren primären oder sekundären Aminen zu verknüpfen und so in erfindungsgemäße Verbindungen zu überführen. Vorzugsweise werden Anhydride zunächst mit einem Cyclohexan-1,4-diamin oder einem primären oder sekundären Amin zu den entsprechenden Halbamiden geöffnet, bevor im zweiten Schritt in Gegenwart wasserentziehender Mittel mit einem komplementären primären oder sekundären Aminen bzw. einem Cyclohexan-1,4-diamin zum Diamid umgesetzt wird. Insbesondere beim zweiten Schritt kann es vorteilhaft sein, die Carbonsäurefunktion des intermediären Halbamids vor der Darstellung des Diamids durch Überführung in ein Carbonsäureäquivalent (z.B. Säurechlorid oder Aktivester) zu aktivieren.
Die Umsetzungen mit Anhydriden finden vorzugsweise in polaren oder unpolaren aprotischen Lösungsmitteln wie DMF, DMSO, Diethylether, Diisopropylether, THF, Toluol, Dichlormethan oder Acetonitril bei Temperaturen zwischen -20 und +110 °C, bevorzugt -10 und +40 °C statt.
Bei Umsetzungen mit Säurechloriden werden ebenfalls polare oder unpolare aprotische Lösungsmittel eingesetzt, denen eine organische oder anorganische Hilfsbase, vorzugsweise tertiäre Amine wie Triethylamin, Diisopropylethylamin oder DMAP, zugesetzt wurde. Neben solchen Aminen ist auch beispielsweise Pyridin als Base wie auch als Lösungsmittel geeignet. Vorzugsweise werden Säurechloride mit Aminen bei -10 und +40 °C in Dichlormethan oder Chloroform in Gegenwart von Triethylamin oder Pyridin und ggf. katalytischer Mengen DMAP umgesetzt.
Für die Umsetzung der Carbonsäurefunktion eines intermediären Halbamids mit einem weiteren Amin steht die gesamte Bandbreite der dem Fachmann bekannten Methoden zur Herstellung von Amiden zur Verfügung. Vorteilhaft ist dabei der Einsatz organischer oder anorganischer wasserentziehender Mittel wie z.B. Molsiob, Magnesiumsulfat, Schwefelsäure oder Carbodiimiden wie DCC oder DIC, letztere ggf. in Gegenwart von HOBt (1-Hydroxybenzotriazol). Auch diese Umsetzungen werden vorzugsweise in polaren oder unpolaren aprotischen Lösungsmitteln bei Temperaturen zwischen -20 und +110 °C, bevorzugt -10 und +40 °C durchgeführt. Gegebenenfalls werden anschließend die Schutzgruppen abgespalten.

### Beispiele

Die folgenden Beispiele dienen zur näheren Erläuterung der Erfindung, schränken aber den allgemeinen Erfindungsgedanken nicht ein.

Die Ausbeuten der hergestellten Verbindungen sind nicht optimiert.

Alle Temperaturen sind unkorrigiert.

Die Angabe "Ether" bedeutet Diethylether, "EE" Ethylacetat und "DCM" Dichlormethan. Die Angabe "Äquivalente" bedeutet Stoffmengenäquivalente, "Smp." Schmelzpunkt bzw. Schmelzbereich, "Zers." Zersetzung, "RT" Raumtemperatur , "abs." absolut (wasserfrei), ,"rac." racemisch , "konz." konzentriert, "min" Minuten, "h" Stunden, "d" Tage, "Vol.%" Volumenprozent, "m%" Massenprozent und "M" ist eine Konzentrationsangabe in mol/l.

Als stationäre Phase für die Säulenchromatographie wurde Kieselgel 60 (0.040 - 0.063 mm) der Firma E. Merck, Darmstadt, eingesetzt.

Die dünnschicht-chromatographischen Untersuchungen wurden mit HPTLC-Fertigplatten, Kieselgel 60 F 254, der Firma E. Merck, Darmstadt, durchgeführt.

Die Mischungsverhältnisse von Laufmitteln für chromatographische Untersuchungen sind stets in VolumenNolumen angegeben.

Die im folgenden eingesetzten Verbindungen waren entweder kommerziell erhältlich, oder ihre Herstellung ist aus dem Stand der Technik bekannt oder in für den Fachmann offensichtlicher Weise aus dem Stand der Technik abgeleitet worden.

### Allgemeine Vorschrift:

Zu 0,1 mmol des Anhydrids wurden 0,1 mmol des Cyclohexan-1,4-diamins sowie 0,1 mmol Diisopropylethylamin in THF zugetropft. Es wurde 12 h gerührt und anschließend die Reaktionslösung mit 2 M HCl versetzt. Durch Extraktion mit 3 x 2 ml Dichlormethan und Entfernen den Lösungsmittels wurde das Carbonsäurederivat erhalten.

Zu dem erhaltenen Produkt wurde 0,1 mmol des entsprechenden Amins (s. Tabelle 1) sowie 1 Equivalent Diisopropylcarbodiimid und 1 Equivalent 1-Hydroxybenzotriazol zugegeben. Es wurde 12 h gerührt und anschließend mit einer 1 M Natriumcarbonatlösung versetzt. Durch Extraktion mit je 3 x 2 ml Ethylacetat/THF und Entfernen des Lösungsmittels wurde das Produkt erhalten.

In Tabelle 1 sind die für den letzten Schritt eingesetzten Amine für die Beispiele genannt.

**Tabelle 1: Auflistung der Beispiele und Abbildung des im letzten Syntheseschritt eingesetzten Amins.**

| **Beispiel** | **eingesetztes Amin** | **Name der Beispielverbindung** |
|---|---|---|
| **1** | 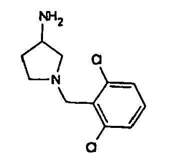 | N-[1-(2,6-Dichlor-benzyl)-pyrrolidin-3-yl]-N'-[4-dimethylamino-4-(4-methyl-benzyl)-cyclohexyl]-succinamid |
| **2** | 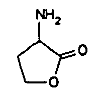 | N-[4-(3-Chlor-benzyl)-4-dimethylamino-cyclohexyl]-N'-(2-oxo-tetrahydro-furan-3-yl)-succinamid |
| **3** | 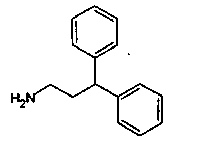 | N-[4-(4-Chlor-benzyl)-4-dimethylamino-cyclohexyl]-N'-(3,3-diphenyl-propyl)-succinamid |
| **4** | 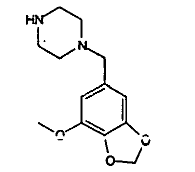 | N-[4-Dimethylamino-4-(4-methyl-benzyl)-cyclohexyl]-4-[4-(7-methoxy-benzo[1,3]dioxol-5-ylmethyl)-piperazin-1-yl]-4-oxo-butyramid |
| **5** | 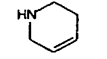 | N-[4-(4-Chlor-benzyl)-4-dimethylamino-cyclohexyl]-4-(3,6-dihydro-2H-pyridin-1-yl)-4-oxo-butyramid |
| **6** | 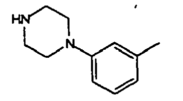 | N-[4-Dimethylamino-4-(4-methyl-benzyl)-cyclohexyl]-4-oxo-4-(4-m-tolyl-piperazin-1-yl)-butyramid |
| **7** | 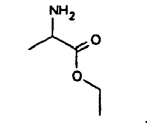 | 2-{3-[4-(4-Chlor-benzyl)-4-dimethylamino-cyclohexylcarbamoyl]-propionylamino}-propionsäure ethyl ester |
| **8** | 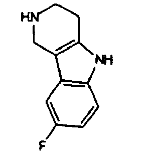 | N-[4-(3-Chlor-benzyl)-4-dimethylamino-cyclohexyl]-4-(8-fluor-1,3,4,5-tetrahydro-pyrido[4,3-b]indol-2-yl)-4-oxo-butyramid |
| **9** | 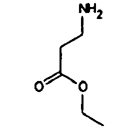 | 3-{3-[4-Dimethylamino-4-(4-methyl-benzyl)-cyclohexylcarbamoyl]-propionylamino}-propionsäure ethyl ester |
| **10** | 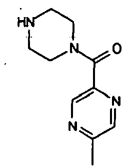 | N-[4-Dimethylamino-4-(4-methyl-benzyl)-cyclohexyl]-4-[4-(5-methyl-pyrazin-2-carbonyl)-piperazin-1-yl]-4-oxo-butyramid |
| **11** | 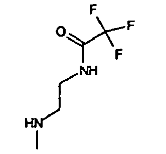 | N-[4-Dimethylamino-4-(4-methyl-benzyl)-cyclohexyl]-N'-methyl-N'-[2-(2,2,2-trifluor-acetylamino)-ethyl]-succinamid |
| **12** | 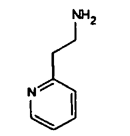 | N-[4-(3-Chlor-benzyl)-4-dimethylamino-cyclohexyl]-N'-(2-pyridin-2-yl-ethyl)-succinamid |
| **13** | 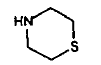 | N-[4-(4-Chlor-benzyl)-4-dimethylamino-cyclohexyl]-4-oxo-4-thiomorpholin-4-yl-butyramid |
| **14** | 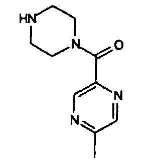 | N-[4-(4-Chlor-benzyl)-4-dimethylamino-cyclohexyl]-4-[4-(5-methyl-pyrazin-2-carbonyl)-piperazin-1-yl]-4-oxo-butyramid |
| **15** | 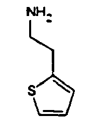 | N-[4-Dimethylamino-4-(3-methyl-benzyl)-cyclohexyl]-N'-(2-thiophen-2-yl-ethyl)-succinamid |
| **16** | 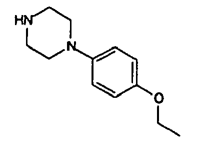 | N-[4-(4-Chlor-benzyl)-4-dimethylamino-cyclohexyl]-4-[4-(4- ethoxy-phenyl)-piperazin-1-yl]-4-oxo-butyramid |
| **17** | 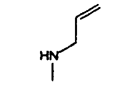 | N-Allyl-N'-[4-(4-chlor-benzyl)-4-dimethylamino-cyclohexyl]-N-methyl-succinamid |
| **18** | 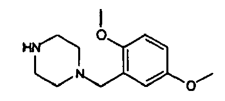 | 5-[4-(2,5-Dimethoxy-benzyl)-piperazin-1-yl]-5-oxo-valeriansäure (4-benzyl-4-dimethylamino-cyclohexyl)-amid |
| **19** | 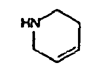 | 4-(3,6-Dihydro-2H-pyridin-1-yl)-N-[4-dimethylamino-4-(4-methyl-benzyl)-cyclohexyl]-4-oxo-butyramid |
| **20** | 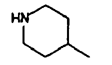 | N-[4-Dimethylamino-4-(3-methyl-benzyl)-cyclohexyl]-4-(4-methyl-piperidin-1-yl)-4-oxo-butyramid |
| **21** | 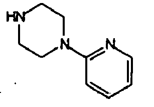 | N-[4-Dimethylamino-4-(4-methyl-benzyl)-cyclohexyl]-4-oxo-4-(4-pyridin-2-yl-piperazin-1-yl)-butyramid |
| **22** | 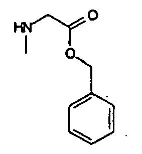 | ({3-[4-Dimethylamino-4-(3-methyl-benzyl)-cyclohexylcarbamoyl]-propionyl}-methyl-amino)-essigsäure benzyl ester |
| **23** | 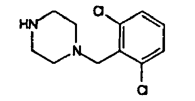 | 4-[4-(2,6-Dichlor-benzyl)-piperazin-1-yl]-N-[4-dimethylamino-4-(3-methyl-benzyl)-cyclohexyl]-4-oxo-butyramid |
| **24** | 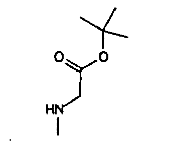 | {[4-(4-Benzyl-4-dimethylamino-cyclohexylcarbamoyl)-butyryl]-methyl-amino}-essigsäure tert-butyl ester |
| **25** | 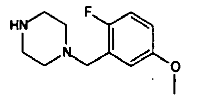 | 5-[4-(2-Fluor-5-methoxy-benryl)-piperazin-1-yl]-5-oxo-valeriansäure (4-benzyl-4-dimethylamino-cyclohexyl)-amid |
| **26** | 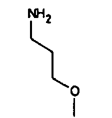 | N-[4-(4-Chlor-benzyl)-4-dimethylamino-cyclohexyl]-N'-(3-methoxy-propyl)-succinamid |
| **27** | 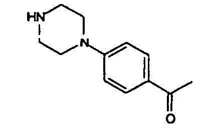 | 4-[4-(4-Acetyl-phenyl)-piperazin-1-yl]-N-[4-dimethylamino-4-(4-methyl-benzyl)-cyclohexyl]-4-oxo-butyramid |
| **28** | 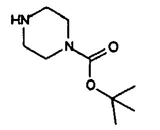 | 4-{3-[4-Dimethylamino-4-(4-methyl-benzyl)-cyclohexylcarbamoyl]-propionyl}-piperazin-1-carbonsäure tert-butyl ester |
| **29** | 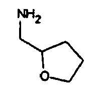 | N-[4-(3-Chlor-benzyl)-4-dimethylamino-cyclohexyl]-N'-(tetrahydro-furan-2-ylmethyl)-succinamid |
| **30** | 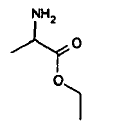 | 2-{3-[4-(3-Chlor-benzyl)-4-dimethylamino-cyclohexylcarbamoyl]-propionylamino}-propionsäure ethyl ester |
| 31 | 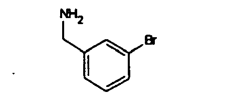 | N-(3-Brom-benzyl)-N'-[4-dimethylamino-4-(3-methyl-benzyl)-cyclohexyl]-succinamid |
| **32** | 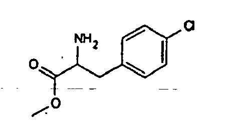 | 3-(4-Chlor-phenyl)-2-{3-[4-dimethylamino-4-(4-methyl-benzyl)-cyclohexylcarbamoyl]-propionylamino}-propionsäure methyl ester |
| **33** | 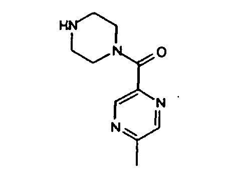 | N-[4-(3-Chlor-benzyl)-4-dimethylamino-cyclohexyl]-4-[4-(5-methyl-pyrazin-2-carbonyl)-piperazin-1-yl]-4-oxo-butyramid |
| **34** | 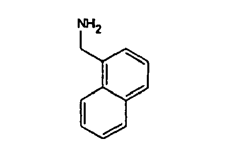 | N-[4-(4-Chlor-benzyl)-4-dimethylamino-cyclohexyl]-N'-naphthalen-1-ylmethyl-succinamid |
| **35** | 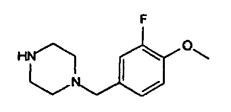 | N-[4-Dimethylamino-4-(3-methyl-benzyl)-cyclohexyl]-4-[4-(3-fluor-4-methoxy-benzyl)-piperazin-1-yl]-4-oxo-butyramid |
| **36** | 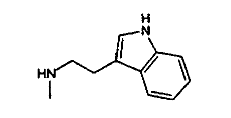 | N-[4-Dimethylamino-4-(4-methyl-benzyl)-cyclohexyl]-N'-[2-(1 H-indol-3-yl)-ethyl]-N'-methyl-succinamid |
| **37** | 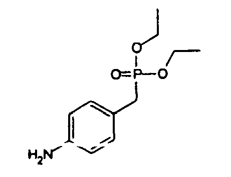 | (4-{3-[4-(3-Chlor-benzyl)-4-dimethylamino-cyclohexylcarbamoyl]-propionylamino}-benzyl)-phosphonsäurediethylester |
| **38** | 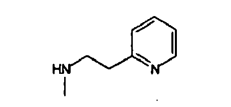 | N-[4-Dimethylamino-4-(4-methyl-benzyl)-cyclohexyl]-N'-methyl-N'-(2-pyridin-2-yl-ethyl)-succinamid |
| **39** | 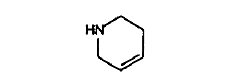 | N-[4-(3-Chlor-benzyl)-4-dimethylamino-cyclohexyl]-4-(3,6-dihydro-2H-pyridin-1-yl)-4-oxo-butyramid |
| **40** | 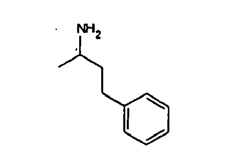 | N-[4-Dimethylamino-4-(3-methyl-benzyl)-cyclohexyl]-N'-(1-methyl-3-phenyl-propyl)-succinamid |
| **41** | 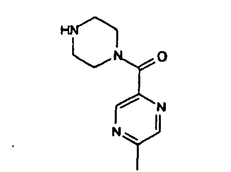 | 5-[4-(5-Methyl-pyrazin-2-carbonyl)-piperazin-1-yl]-5-oxo-valeriansäure (4-benzyl-4-dimethylamino-cyclohexyl)-amid |
| **42** | 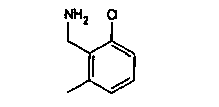 | N-[4-(3-Chlor-benzyl)-4-dimethylamino-cyclohexyl]-N'-(2-chlor-6-methyl-benzyl)-succinamid |
| **43** | 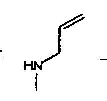 | N-Allyl-N'-[4-(3-chlor-benzyl)-4-dimethylamino-cyclohexyl]-N-methyl-succinamid |
| **44** | 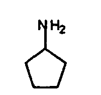 | N-[4-(4-Chlor-benzyl)-4-dimethylamino-cyclohexyl]-N'-cyclopentyl-succinamid |
| **45** | 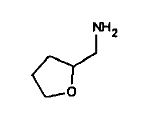 | N-[4-Dimethylamino-4-(4-methyl-benzyl)-cyclohexyl]-N'-(tetrahydro-furan-2-ylmethyl)-succinamid |
| **46** | 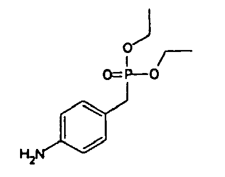 | (4-{3-[4-Dimethylamino-4-(3-methyl-benzyl)-cyclohexylcarbamoyl]-propionylamino}-benzyl)-phosphonsäurediethylester |
| **47** | 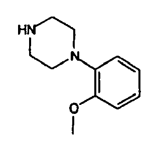 | N-[4-(4-Chlor-benzyl)-4-dimethylamino-cyclohexyl]-4-[4-(2- methoxy-phenyl)-piperazin-1-yl]-4-oxo-butyramid |
| **48** | 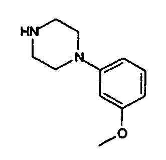 | N-[4-Dimethylamino-4-(3-methyl-benzyl)-cyclohexyl]-4-[4-(3-methoxy-phenyl)-piperazin-1-yl]-4-oxo-butyramid |
| **49** | 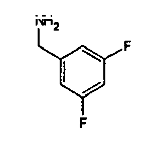 | N-[4-(4-Chlor-benzyl)-4-dimethylamino-cyclohexyl]-N'-(3,5-difluor-benzyl)-succinamid |
| **50** | 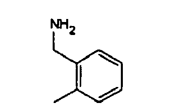 | N-[4-(4-Chlor-benzyl)-4-dimethylamino-cyclohexyl]-N'-(2- methyl-benzyl)-succinamid |
| **51** | 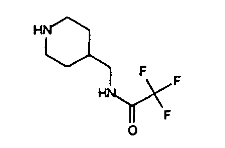 | N-[4-Dimethylamino-4-(4-methyl-benzyl)-cyclohexyl]-4-oxo-4-{4-[(2,2,2-trifluor-acetylamino)-methyl]-piperidin-1-yl}-butyramid |
| **52** | 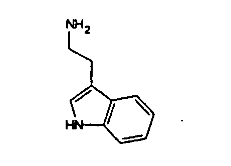 | N-[4-(4-Chlor-benzyl)-4-dimethylamino-cyclohexyl]-N'-[2-(1H-indol-3-yl)-ethyl]-succinamid |
| **53** | 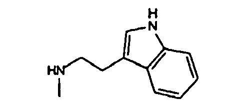 | N-[4-(3-Chlor-benzyl)-4-dimethylamino-cyclohexyl]-N'-[2-(1H-indol-3-yl)-ethyl]-N'-methyl-succinamid |
| **54** | 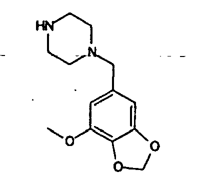 | 5-[4-(7-Methoxy-benzo[1,3]dioxol-5-ylmethyl)-piperazin-1-yl]-5-oxo-valeriansäure (4-benzyl-4-dimethylamino-cyclohexyl)-amid |
| **55** | 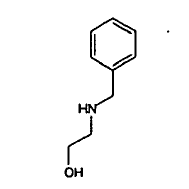 | N-Benzyl-N'-[4-dimethylamino-4-(3-methyl-benzyl)-cyclohexyl]-N-(2-hydroxy-ethyl)-succinamid |
| **56** | 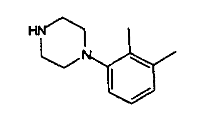 | 5-[4-(2,3-Dimethyl-phenyl)-piperazin-1-yl]-5-oxo-valeriansäure (4-benzyl-4-dimethylamino-cyclohexyl)-amid |
| **57** | 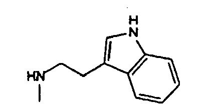 | N-[4-(4-Chlor-benzyl)-4-dimethylamino-cyclohexyl]-N'-[2-(1H-indol-3-yl)-ethyl]-N'-methyl-succinamid |
| **58** | 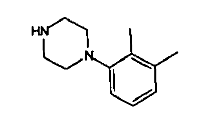 | N-[4-Dimethylamino-4-(4-methyl-benzyl)-cyclohexyl]-4-[4-(2,3-dimethyl-phenyl)-piperazin-1-yl]-4-oxo-butyramid |
| **59** | 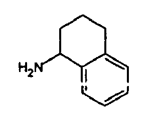 | Glutarsäure-(4-benzyl-4-dimethylamino-cyclohexyl)-amid (1,2,3,4-tetrahydro-naphthalen-1-yl)-amid |
| **60** | 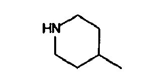 | N-[4-(4-Chlor-benzyl)-4-dimethylamino-cyclohexyl]-4-(4-methyl-piperidin-1-yl)-4-oxo-butyramid |
| **61** | 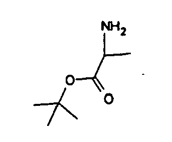 | 2-{3-[4-Dimethylamino-4-(4-methyl-benzyl)-cyclohexylcarbamoyl]-propionylamino}-propionsäure tert-butyl ester |
| **62** | 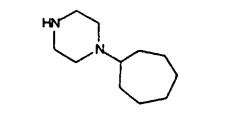 | 5-(4-Cycloheptyl-piperazin-1-yl)-5-oxo-valeriansäure (4-benzyl-4-dimethylamino-cyclohexyl)-amid |
| **63** | 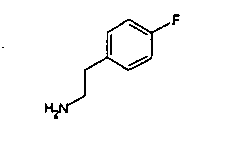 | Glutarsäure-(4-benzyl-4-dimethylamino-cyclohexyl)-amid [2-(4-fluor-phenyl)-ethyl]-amid |
| **64** | 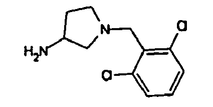 | Glutarsäure-(4-benzyl-4-dimethylamino-cyclohexyl)-amid [1-(2,6-dichlor-benzyl)-pyrrolidin-3-yl]-amid |
| **65** | 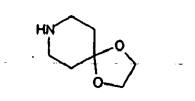 | N-[4-(4-Chlor-benzyl)-4-dimethylamino-cyclohexyl]-4-(1,4-dioxa-8-aza-spiro[4.5]dec-8-yl)-4-oxo-butyramid |
| **66** | 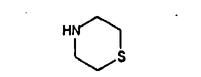 | N-[4-(3-Chlor-benzyl)-4-dimethylamino-cyclohexyi]-4-oxo-4-thiomorpholin-4-yl-butyramid |
| **67** | 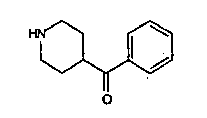 | 4-(4-Benzoyl-piperidin-1-yl)-N-[4-dimethylamino-4-(3-methyl-benzyl)-cyclohexyl]-4-oxo-butyramid |
| **68** | 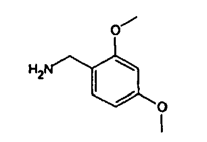 | N-[4-(4-Chlor-benzyl)-4-dimethylamino-cyclohexyl]-N'-(2,4-dimethoxy-benzyl)-succinamid |
| **69** | 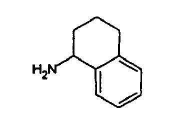 | N-[4-Dimethylamino-4-(3-methyl-benzyl)-cyclohexyl]-N'-(1,2,3,4-tetrahydro-naphthalen-1-yl)-succinamid |
| **70** | 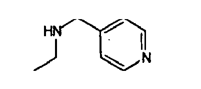 | N-[4-(4-Chlor-benzyl)-4-dimethylamino-cyclohexyl]-N'-ethyl-N'-pyridin-4-ylmethyl-succinamid |
| **71** | 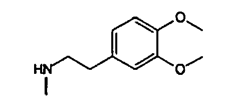 | N-[2-(3,4-Dimethoxy-phenyl)-ethyl]-N'-[4-dimethylamino-4-(3-methyl-benzyl)-cyclohexyl]-N-methyl-succinamid |
| **72** | 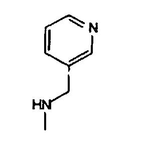 | N-[4-(4-Chlor-benzyl)-4-dimethylamino-cyclohexyl]-N'-methyl-N'-pyridin-3-ylmethyl-succinamid |
| **73** | 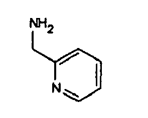 | N-[4-(3-Chlor-benzyl)-4-dimethylamino-cyclohexyl]-N'-pyridin-2-ylmethyl-succinamid |
| **74** | 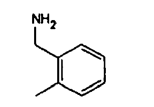 | N-[4-(3-Chlor-benzyl)-4-dimethylamino-cyclohexyl]-N'-(2-methyl-benzyl)-succinamid |
| **75** | 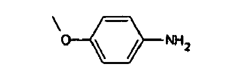 | N-[4-Dimethylamino-4-(3-methyl-benzyl)-cyclohexyl]-N'-(4-methoxy-phenyl)-succinamid |
| **76** | 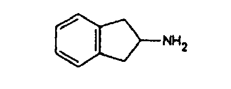 | N-[4-Dimethylamino-4-(3-methyl-benzyl)-cyclohexyl]-N'-indan-2-yl-succinamid |
| **77** | 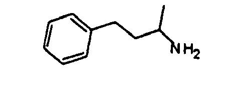 | N-[4-(3-Chlor-benzyl)-4-dimethylamino-cyclohexyl]-N'-(1-methyl-3-phenyl-propyl)-succinamid |
| **78** | 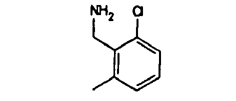 | N-(2-Chlor-6-methyl-benzyl)-N'-[4-dimethylamino-4-(3-methyl-benzyl)-cyclohexyl]-succinamid |
| **79** | 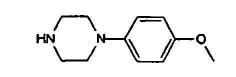 | N-[4-Dimethylamino-4-(4-methyl-benzyl)-cyclohexyl]-4-[4-(4-methoxy-phenyl)-piperazin-1-yl]-4-oxo-butyramid |
| **80** | 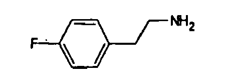 | N-[4-Dimethylamino-4-(4-methyl-benzyl)-cyclohexyl]-N'-[2-(4-fluor-phenyl)-ethyl]-succinamid |
| **81** | 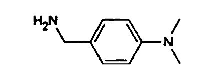 | Glutarsäure-(4-benzyl-4-dimethylamino-cyclohexyl)-amid 4-dimethylamino-benzylamid |
| **82** | 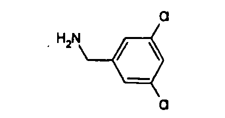 | N-[4-(3-Chlor-benzyl)-4-dimethylamino-cyclohexyl]-N'-(3,5-dichlor-benzyl)-succinamid |
| **83** | 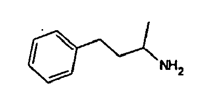 | N-[4-(4-Chlor-benzyl)-4-dimethylamino-cyclohexyl]-N'-(1-methyl-3-phenyl-propyl)-succinamid |
| **84** | 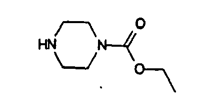 | 4-{3-[4-(3-Chlor-benzyl)-4-dimethylamino-cyclohexylcarbamoyl]-propionyl}-piperazin-1-carbonsäureethylester |
| **85** | 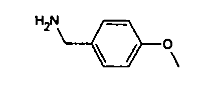 | N-[4-Dimethylamino-4-(3-methyl-benzyl)-cyclohexyl]-N'-(4-methoxy-benzyl)-succinamid |
| **86** | 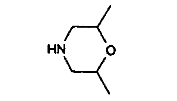 | N-[4-(4-Chlor-benzyl)-4-dimethylamino-cyclohexyl]-4-(2,6-dimethyl-morpholin-4-yl)-4-oxo-butyramid |
| **87** | 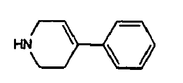 | N-[4-Dimethylamino-4-(4-methyl-benzyl)-cyclohexyl]-4-oxo-4-(4-phenyl-3,6-dihydro-2H-pyridin-1-yl)-butyramid |
| **88** | 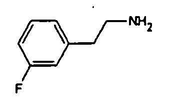 | N-[4-(4-Chlor-benzyl)-4-dimethylamino-cyclohexyl]-N'-[2-(3-fluor-phenyl)-ethyl]-succinamid |
| **89** | 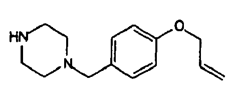 | 4-[4-(4-Allyloxy-benzyl)-piperazin-1-yl]-N-[4-dimethylamino-4-(3-methyl-benzyl)-cyclohexyl]-4-oxo-butyramid |
| **90** | 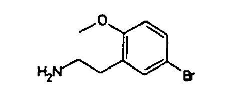 | Glutarsäure-(4-benzyl-4-dimethylamino-cyclohexyl)-amid [2-(5-brom-2-methoxy-phenyl)-ethyl]-amid |
| **91** | 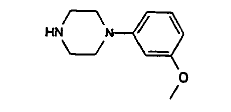 | 5-[4-(3-Methoxy-phenyl)-piperazin-1-yl]-5-oxo-valeriansäure (4-benzyl-4-dimethylamino-cyclohexyl)-amid |
| **92** | 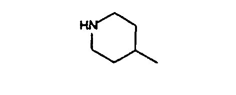 | N-[4-(3-Chlor-benzyl)-4-dimethylamino-cyclohexyl]-4-(4-methyl-piperidin-1-yl)-4-oxo-butyramid |
| **93** | 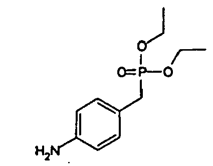 | (4-{3-[4-(4-Chlor-benzyl)-4-dimethylamino-cyclohexylcarbamoyl]-propionylamino}-benzyl)-phosphonsäurediethylester |
| **94** | 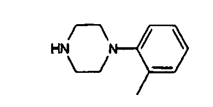 | N-[4-Dimethylamino-4-(4-methyl-benzyl)-cyclohexyl]-4-oxo-4-(4-o-tolyl-piperazin-1-yl)-butyramid |
| **95** | 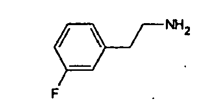 | N-[4-Dimethylamino-4-(3-methyl-benzyl)-cyclohexyl]-N'-[2-(3-fluor-phenyl)-ethyl]-succinamid |
| **96** | 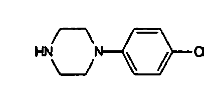 | 4-[4-(4-Chlor-phenyl)-piperazin-1-yl]-N-[4-dimethylamino-4-(4-methyl-benzyl)-cyclohexyl]-4-oxo-butyramid |
| **97** | 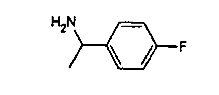 | N-[4-(3-Chlor-benzyl)-4-dimethylamino-cyclohexyl]-N'-[1-(4-fluor-phenyl)-ethyl]-succinamid |
| **98** | 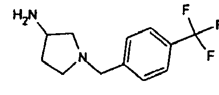 | N-[4-(4-Chlor-benzyl)-4-dimethylamino-cyclohexyl]-N'-[1-(4-trifluormethyl-benzyl)-pyrrolidin-3-yl]-succinamid |
| **99** | 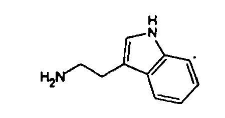 | N-[4-(3-Chlor-benzyl)-4-dimethylamino-cyclohexyl]-N'-[2-(1H-indol-3-yl)-ethyl]-succinamid |
| **100** | 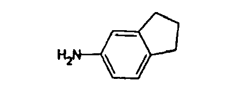 | N-[4-(4-Chlor-benzyl)-4-dimethylamino-cyclohexyl]-N'-indan-5-yl-succinamid |
| **101** | 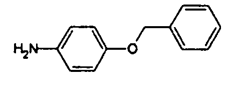 | N-(4-Benzyloxy-phenyl)-N'-[4-dimethylamino-4-(4-methyl-benzyl)-cyclohexyl]-succinamid |
| **102** | 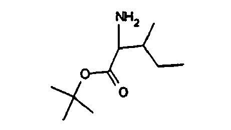 | 2-{3-[4-Dimethylamino-4-(3-methyl-benzyl)-cyclohexylcarbamoyl]-propionylamino}-3-methyl-valeriansäure tert-butyl ester |
| **103** | 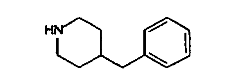 | 4-(4-Benzyl-piperidin-1-yl)-N-[4-dimethylamino-4-(3-methyl-benzyl)-cyclohexyl]-4-oxo-butyramid |
| **104** | 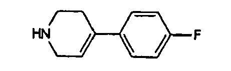 | N-[4-(4-Chlor-benzyl)-4-dimethylamino-cyclohexyl]-4-[4-(4-fluor-phenyl)-3,6-dihydro-2H-pyridin-1-yl]-4-oxo-butyramid |
| **105** | 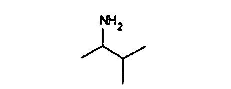 | N-[4-Dimethylamino-4-(4-methyl-benzyl)-cyclohexyl]-N'-(1,2-dimethyl-propyl)-succinamid |
| **106** | 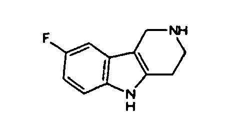 | N-[4-Dimethylamino-4-(3-methyl-benzyl)-cyclohexyl]-4-(8-fluor-1,3,4,5-tetrahydro-pyrido[4,3-b]indol-2-yl)-4-oxo-butyramid |
| **107** | 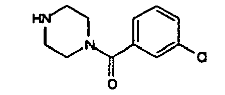 | 4-[4-(3-Chlor-benzoyl)-piperazin-1-yl]-N-[4-dimethylamino-4-(3-methyl-benzyl)-cyclohexyl]-4-oxo-butyramid |
| **108** | 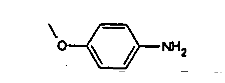 | N-[4-(4-Chlor-benzyl)-4-dimethylamino-cyclohexyl]-N'-(4-methoxy-phenyl)-succinamid |
| **110** | 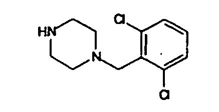 | 5-[4-(2,6-Dichlor-benzyl)-piperazin-1-yl]-5-oxo-valeriansäure (4-benzyl-4-dimethylamino-cyclohexyl)-amid |
| **111** | 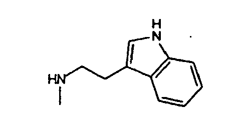 | N-[4-Dimethylamino-4-(3-methyl-benzyl)-cyclohexyl]-N'-[2-(1H-indol-3-yl)-ethyl]-N'-methyl-succinamid |
| **112** | 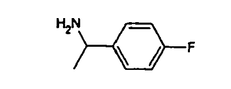 | N-[4-Dimethylamino-4-(3-methyl-benzyl)-cyclohexyl]-N'-[1-(4-fluor-phenyl)-ethyl]-succinamid |
| **113** | 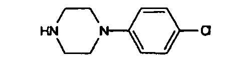 | 4-[4-(4-Chlor-phenyl)-piperazin-1-yl]-N-[4-dimethylamino-4-(3-methyl-benzyl)-cyclohexyl]-4-oxo-butyramid |
| **114** | 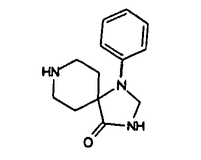 | N-[4-(3-Chlor-benzyl)-4-dimethylamino-cyclohexyl]-4-oxo-4-(4-oxo-1-phenyl-1,3,8-triaza-spiro[4.5]dec-8-yl)-butyramid |
| **115** | 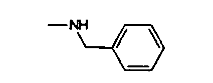 | N-Benzyl-N'-[4-dimethylamino-4-(3-methyl-benzyl)-cyclohexyl]-N-methyl-succinamid |
| **116** | 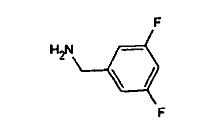 | N-[4-(3-Chlor-benzyl)-4-dimethylamino-cyclohexyl]-N'-(3,5-difluor-benzyl)-succinamid |
| **117** | 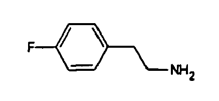 | N-[4-Dimethylamino-4-(3-methyl-benzyl)-cyclohexyl]-N'-[2-(4-fluor-phenyl)-ethyl]-succinamid |
| **118** | 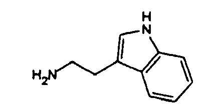 | N-[4-Dimethylamino-4-(3-methyl-benzyl)-cyclohexyl]-N'-[2-(1H-indol-3-yl)-ethyl]-succinamid |
| **119** | 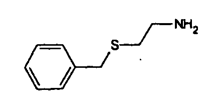 | N-(2-Benzylsulfanyl-ethyl)-N'-[4-dimethylamino-4-(3-methyl-benzyl)-cyclohexyl]-succinamid |
| **120** | 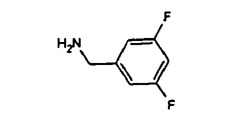 | N-[4-(3-Chlor-benzyl)-4-dimethylamino-cyclohexyl]-N'-(2,4-difluor-benzyl)-succinamid |
| **121** | 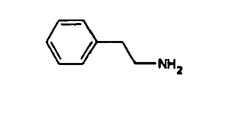 | N-[4-(3-Chlor-benzyl)-4-dimethylamino-cyclohexyl]-N'-phenethyl-succinamid |
| **122** | 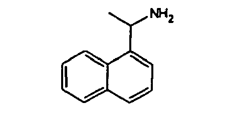 | N-[4-Dimethylamino-4-(3-methyl-benzyl)-cyclohexyl]-N'-(1-naphthalen-2-yl-ethyl)-succinamid |
| **123** | 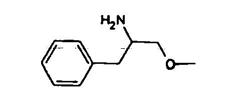 | N-[4-(3-Chlor-benzyl)-4-dimethylamino-cyclohexyl]-N'-(1-methoxymethyl-2-phenyl-ethyl)-succinamid |
| **124** | 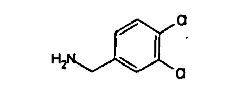 | N-[4-(3-Chlor-benzyl)-4-dimethylamino-cyclohexyl]-N'-(3,4-dichlor-benzyl)-succinamid |
| **125** | 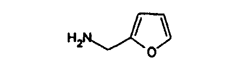 | N-[4-(3-Chlor-benzyl)-4-dimethylamino-cyclohexyl]-N'-furan-2-ylmethyl-succinamid |
| **126** | 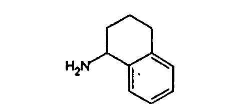 | N-[4-Dimethylamino-4-(4-methyl-benzyl)-cyclohexyl]-N'-(1,2,3,4-tetrahydro-naphthalen-1-yl)-succinamid |
| **127** | 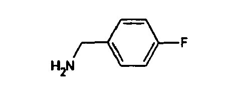 | N-[4-(3-Chlor-benzyl)-4-dimethylamino-cyclohexyl]-N'-(4-fluor-benzyl)-succinamid |
| **128** | 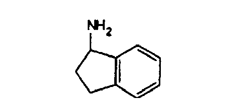 | N-[4-Dimethylamino-4-(3-methyl-benzyl)-cyclohexyl]-N'-indan-1-yl-succinamid |
| **129** | 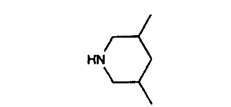 | N-[4-(3-Chlor-benzyl)-4-dimethylamino-cyclohexyl]-4-(3,5-dimethyl-piperidin-1-yl)-4-oxo-butyramid |
| **130** | 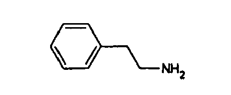 | N-[4-Dimethylamino-4-(3-methyl-benzyl)-cyclohexyl]-N'-phenethyl-succinamid |
| **131** | 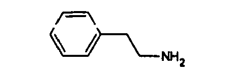 | N-[4-Dimethylamino-4-(4-methyl-benzyl)-cyclohexyl]-N'-phenethyl-succinamid |
| **132** | 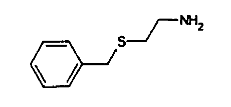 | N-(2-Benzylsulfanyl-ethyl)-N'-[4-(3-chlor-benzyl)-4-dimethylamino-cyclohexyl]-succinamid |
| **133** | 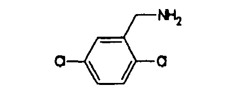 | N-[4-(3-Chlor-benzyl)-4-dimethylamino-cyclohexyl]-N'-(2,5-dichlor-benzyl)-succinamid |
| **134** | 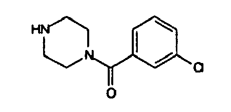 | 4-[4-(3-Chlor-benzoyl)-piperazin-1-yl]-N-[4-(4-chlor-benzyl)-4-dimethylamino-cyclohexyl]-4-oxo-butyramid |
| **135** | 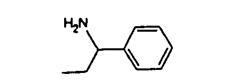 | N-[4-(4-Chlor-benzyl)-4-dimethylamino-cyclohexyl]-N'-(1-phenyl-propyl)-succinamid |
| **136** | 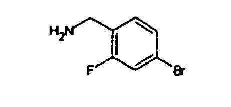 | N-(4-Brom-2-fluor-benzyl)-N'-[4-dimethylamino-4-(3-methyl-benzyl)-cyclohexyl]-succinamid |
| **137** | 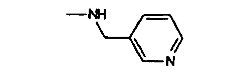 | N-[4-(3-Chlor-benzyl)-4-dimethylamino-cyclohexyl]-N'-methyl-N'-pyridin-3-ylmethyl-succinamid |
| **138** | 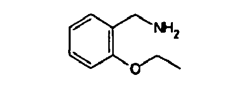 | N-[4-Dimethylamino-4-(4-methyl-benzyl)-cyclohexyl]-N'-(2-ethoxy-benzyl)-succinamid |
| **139** | 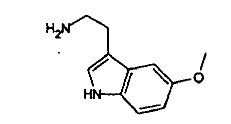 | N-[4-Dimethylamino-4-(3-methyl-benzyl)-cyclohexyl]-N'-[2-(5-methoxy-1H-indol-3-yl)-ethyl]-succinamid |
| **140** | 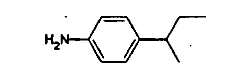 | N-(4-sec-Butyl-phenyl)-N'-[4-(4-chlor-benzyl)-4-dimethylamino-cyclohexyl]-succinamid |
| **141** | 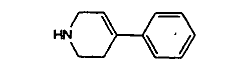 | 5-Oxo-5-(4-phenyl-3,6-dihydro-2H-pyridin-1-yl)-valeriansäure (4-benzyl-4-dimethylamino-cyclohexyl)-amid |
| **142** | 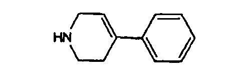 | 5-Oxo-5-(4-phenyl-3,6-dihydro-2H-pyridin-1-yl)-valeriansäure (4-benzyl-4-dimethylamino-cyclohexyl)-amid |
| **143** | 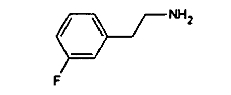 | N-[4-(3-Chlor-benzyl)-4-dimethylamino-cyclohexyl]-N'-[2-(3-fluor-phenyl)-ethyl]-succinamid |
| **144** | 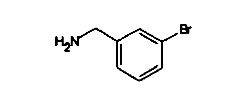 | N-(3-Brom-benzyl)-N'-[4-(3-chlor-benzyl)-4-dimethylamino-cyclohexyl]-succinamid |
| **145** | 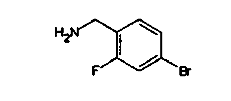 | N-(4-Brom-2-fluor-benzyl)-N'-[4-(3-chlor-benzyl)-4-dimethylamino-cyclohexyl]-succinamid |
| **146** | 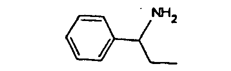 | N-[4-(3-Chlor-benzyl)-4-dimethylamino-cyclohexyl]-N'-(1-phenyl-propyl)-succinamid |
| **147** | 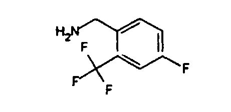 | N-[4-Dimethylamino-4-(3-methyl-benzyl)-cyclohexyl]-N'-(4-fluor-2-trifluormethyl-benzyl)-succinamid |
| **148** | 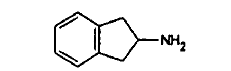 | N-[4-(3-Chlor-benzyl)-4-dimethylamino-cyclohexyl]-N'-indan-2-yl-succinamid |
| **149** | 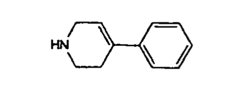 | N-[4-Dimethylamino-4-(3-methyl-benzyl)-cyclohexyl]-4-oxo-4-(4-phenyl-3,6-dihydro-2H-pyridin-1-yl)-butyramid |
| **150** | 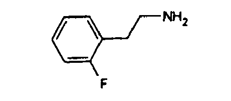 | N-[4-(4-Chlor-benzyl)-4-dimethylamino-cyclohexyl]-N'-[2-(2-fluor-phenyl)-ethyl]-succinamid |
| **151** | 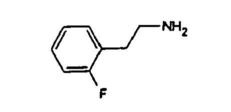 | N-[4-Dimethylamino-4-(4-methyl-benzyl)-cyclohexyl]-N'-[2-(2-fluor-phenyl)-ethyl]-succinamid |
| **152** | 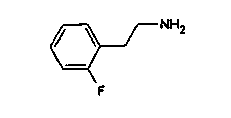 | Glutarsäure-(4-benzyl-4-dimethylamino-cyclohexyl)-amid [2-(2-fluor-phenyl)-ethyl]-amid |
| **153** | 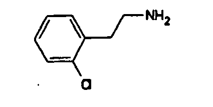 | N-[2-(2-Chlor-phenyl)-ethyl]-N'-[4-dimethylamino-4-(4-methyl-benzyl)-cyclohexyl]-succinamid |
| **154** | 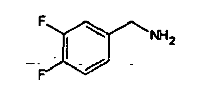 | N-[4-(3-Chlor-benzyl)-4-dimethylamino-cyclohexyl]-N'-(3,4-difluor-benzyl)-succinamid |
| **155** | 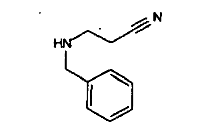 | N-Benzyl-N-(2-cyano-ethyl)-N'-[4-dimethylamino-4-(4-methyl-benzyl)-cyclohexyl]-succinamid |
| **156** | 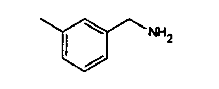 | N-[4-(3-Chlor-benzyl)-4-dimethylamino-cyclohexyl]-N'-(3-methyl-benzyl)-succinamid |
| **157** | 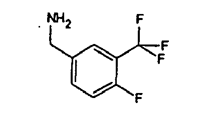 | N-[4-(3-Chlor-benzyl)-4-dimethylamino-cyclohexyl]-N'-(4-fluor-3-trifluormethyl-benzyl)-succinamid |
| **159** | 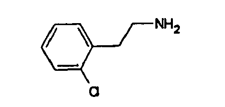 | N-[4-(3-Chlor-benzyl)-4-dimethylamino-cyclohexyl]-N'-[2-(2-chlor-phenyl)-ethyl]-succinamid |
| **160** | 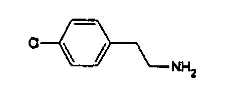 | N-[4-(3-Chlor-benzyl)-4-dimethylamino-cyclohexyl]-N'-[2-(4-chlor-phenyl)-ethyl]-succinamid |
| **161** | 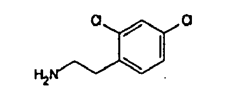 | N-[4-(3-Chlor-benzyl)-4-dimethylamino-cyclohexyl]-N'-[2-(2,4-dichlor-phenyl)-ethyl]-succinamid |
| **162** | 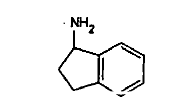 | N-[4-(3-Chlor-benzyl)-4-dimethylamino-cyclohexyl]-N'-indan-1-yl-succinamid |
| **163** | 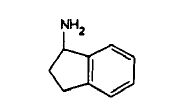 | N-[4-(4-Chlor-benzyl)-4-dimethylamino-cyclohexyl]-N'-indan-1-yl-succinamid |
| **164** | 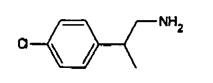 | N-[2-(4-Chlor-phenyl)-propyl]-N'-[4-dimethylamino-4-(3-methyl-benzyl)-cyclohexyl]-succinamid |
| **165** | 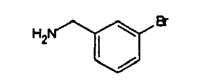 | N-(3-Brom-benzyl)-N'-[4-(4-chlor-benzyl)-4-dimethylamino-cyclohexyl]-succinamid |
| **167** | 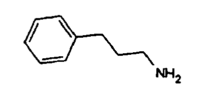 | N-[4-(3-Chlor-benzyl)-4-dimethylamino-cyclohexyl]-N'-(3-phenyl-propyl)-succinamid |
| **168** | 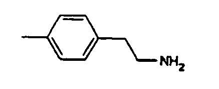 | N-[4-Dimethylamino-4-(4-methyl-benzyl)-cyclohexyl]-N'-(2-p-tolyl-ethyl)-succinamid |
| **169** | 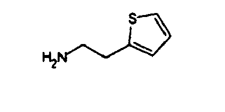 | N-[4-(3-Chlor-benzyl)-4-dimethylamino-cyclohexyl]-N'-(2-thiophen-2-yl-ethyl)-succinamid |
| **170** | 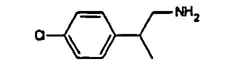 | Glutarsäure-(4-benzyl-4-dimethylamino-cyclohexyl)-amid [2-(4-chlor-phenyl)-propyl]-amid |
| **171** | 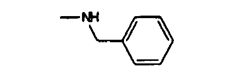 | N-Benzyl-N'-[4-(3-chlor-benzyl)-4-dimethylamino-cyclohexyl]-N-methyl-succinamid |
| **172** | 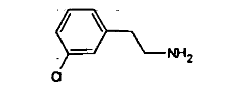 | N-[4-(3-Chlor-benzyl)-4-dimethylamino-cyclohexyl]-N'-[2-(3-chlor-phenyl)-ethyl]-succinamid |
| **173** | 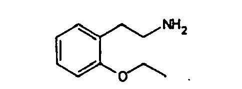 | Glutarsäure-(4-benzyl-4-dimethylamino-cyclohexyl)-amid [2-(2-ethoxy-phenyl)-ethyl]-amid |
| **174** | 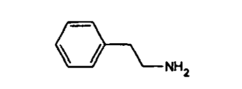 | Glutarsäure-(4-benzyl-4-dimethylamino-cyclohexyl)-amid phenethyl-amid |
| **175** | 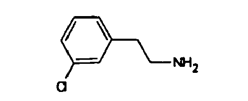 | Glutarsäure-(4-benzyl-4-dimethylamino-cyclohexyl)-amid [2-(3-chlor-phenyl)-ethyl]-amid |
| **176** | 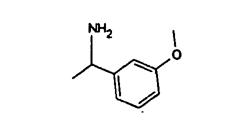 | N-[4-(3-Chlor-benzyl)-4-dimethylamino-cyclohexyl]-N'-[1-(3-methoxy-phenyl)-ethyl]-succinamid |
| **177** | 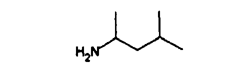 | Glutarsäure-(4-benzyl-4-dimethylamino-cyclohexyl)-amid (1,3-dimethyl-butyl)-amid |
| **178** | 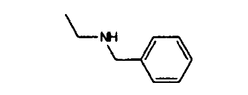 | N-Benzyl-N'-[4-dimethylamino-4-(3-methyl-benzyl)-cyclohexyl]-N-ethyl-succinamid |
| **179** | 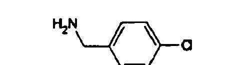 | N-(4-Chlor-benzyl)-N'-[4-(3-chlor-benzyl)-4-dimethylamino-cyclohexyl]-succinamid |
| **180** | 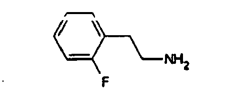 | N-[4-(3-Chlor-benzyl)-4-dimethylamino-cyclohexyl]-N'-[2-(2-fluor-phenyl)-ethyl]-succinamid |
| **181** | 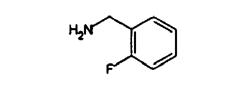 | N-[4-Dimethylamino-4-(3-methyl-benzyl)-cyclohexyl]-N'-(2-fluor-benzyl)-succinamid |
| **182** | 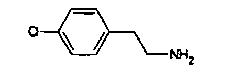 | N-[2-(4-Chlor-phenyl)-ethyl]-N'-[4-dimethylamino-4-(3-methyl-benzyl)-cyclohexyl]-succinamid |
| **183** | 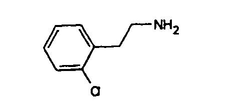 | N-[2-(2-Chlor-phenyl)-ethyl]-N'-[4-dimethylamino-4-(3-methyl-benzyl)-cyclohexyl]-succinamid |
| **184** | 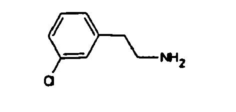 | N-[2-(3-Chlor-phenyl)-ethyl]-N'-[4-dimethylamino-4-(3-methyl-benzyl)-cyclohexyl]-succinamid |
| **185** | 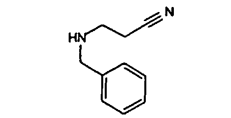 | N-Benzyl-N'-[4-(3-chlor-benzyl)-4-dimethylamino-cyclohexyl]-N-(2-cyano-ethyl)-succinamid |
| **186** | 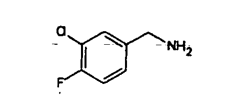 | N-[4-(3-Chlor-benzyl)-4-dimethylamino-cyclohexyl]-N'-(3-chlor-4-fluor-benzyl)-succinamid |
| **187** | 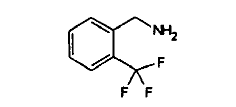 | N-[4-Dimethylamino-4-(3-methyl-benzyl)-cyclohexyl]-N'-(2-trifluormethyl-benzyl)-succinamid |
| **188** | 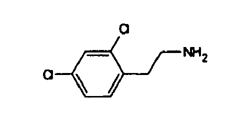 | N-[2-(2,4-Dichlor-phenyl)-ethyl]-N'-[4-dimethylamino-4-(3-methyl-benzyl)-cyclohexyl]-succinamid |
| **189** | 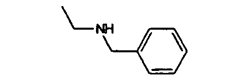 | N-Benzyl-N'-[4-(3-chlor-benzyl)-4-dimethylamino-cyclohexyl]-N-ethyl-succinamid |
| **190** | 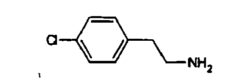 | N-[2-(4-Chlor-phenyl)-ethyl]-N'-[4-dimethylamino-4-(4-methyl-benzyl)-cyclohexyl]-succinamid |
| **191** | 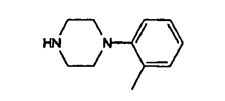 | 5-Oxo-5-(4-o-tolyl-piperazin-1-yl)-valeriansäure (4-benzyl-4-dimethylamino-cyclohexyl)-amid |
| **192** | 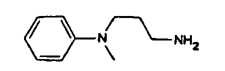 | N-[4-(3-Chlor-benzyl)-4-dimethylamino-cyclohexyl]-N'-[3-(methyl-phenyl-amino)-propyl]-succinamid |
| **193** | 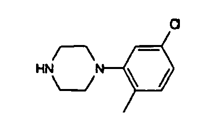 | N-[4-(3-Chlor-benzyl)-4-dimethylamino-cyclohexyl]-4-[4-(5-chlor-2-methyl-phenyl)-piperazin-1-yl]-4-oxo-butyramid |
| **194** | 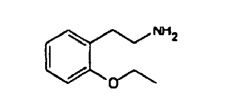 | N-[4-Dimethylamino-4-(3-methyl-benzyl)-cyclohexyl]-N'-[2-(2-ethoxy-phenyl)-ethyl]-succinamid |
| **195** | 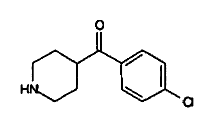 | 4-[4-(4-Chlor-benzoyl)-piperidin-1-yl]-N-[4-(3-chlor-benzyl)-4-dimethylamino-cyclohexyl]-4-oxo-butyramid |
| **196** | 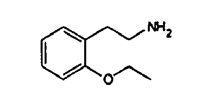 | N-[4-(3-Chlor-benzyl)-4-dimethylamino-cyclohexyl]-N'-[2-(2-ethoxy-phenyl)-ethyl]-succinamid |
| **197** | 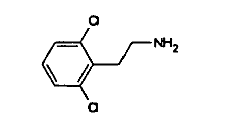 | N-[2-(2,6-Dichlor-phenyl)-ethyl]-N'-[4-dimethylamino-4-(3-methyl-benzyl)-cyclohexyl]-succinamid |
| **198** | 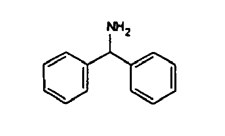 | N-Benzhydryl-N'-[4-(3-chlor-benzyl)-4-dimethylamino-cyclohexyl]-succinamid |
| **199** | 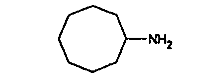 | N-[4-(3-Chlor-benzyl)-4-dimethylamino-cyclohexyl]-N'-cyclooctyl-succinamid |
| **200** | 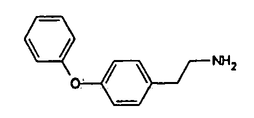 | N-[4-(3-Chlor-benzyl)-4-dimethylamino-cyclohexyl]-N'-[2-(4-phenoxy-phenyl)-ethyl]-succinamid |
| **201** | 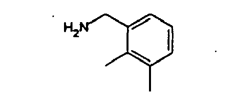 | N-[4-Dimethylamino-4-(3-methyl-benzyl)-cyclohexyl]-N'-(2,3-dimethyl-benzyl)-succinamid |
| **202** | 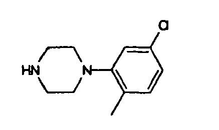 | 4-[4-(5-Chlor-2-methyl-phenyl)-piperazin-1-yl]-N-[4-dimethylamino-4-(3-methyl-benzyl)-cyclohexyl]-4-oxo-butyramid |
| **203** | 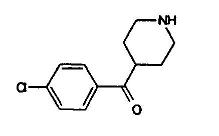 | 4-[4-(4-Chlor-benzoyl)-piperidin-1-yl]-N-[4-dimethylamino-4-(3-methyl-benzyl)-cyclohexyl]-4-oxo-butyramid |
| **204** | 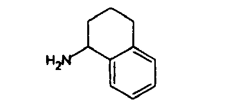 | N-[4-(3-Chlor-benzyl)-4-dimethylamino-cyclohexyl]-N'-(1,2,3,4-tetrahydro-naphthalen-1-yl)-succinamid |
| **205** | 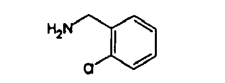 | N-(2-Chlor-benzyl)-N'-[4-dimethylamino-4-(3-methyl-benzyl)-cyclohexyl]-succinamid |
| **206** | 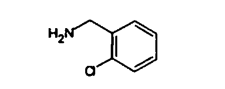 | N-(2-Chlor-benzyl)-N'-[4-(3-chlor-benzyl)-4-dimethylamino-cyclohexyl]-succinamid |
| **207** | 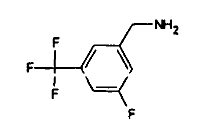 | N-[4-(3-Chlor-benzyl)-4-dimethylamino-cyclohexyl]-N'-(3-fluor-5-trifluormethyl-benzyl)-succinamid |
| **208** | 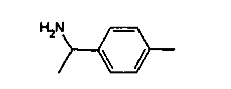 | N-[4-(3-Chlor-benzyl)-4-dimethylamino-cyclohexyl]-N'-(1-p-tolyl-ethyl)-succinamid |
| **209** | 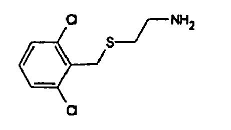 | N-[2-(2,6-Dichlor-benzylsulfanyl)-ethyl]-N'-[4-dimethylamino-4-(4-methyl-benzyl)-cyclohexyl]-succinamid |
| **210** | 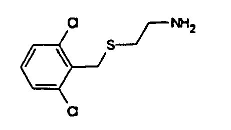 | N-[4-(3-Chlor-benzyl)-4-dimethylamino-cyclohexyl]-N'-[2-(2,6-dichlor-benzylsulfanyl)-ethyl]-succinamid |
| **211** | 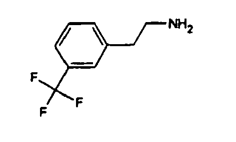 | N-[4-(3-Chlor-benzyl)-4-dimethylamino-cyclohexyl]-N'-[2-(3-trifluormethyl-phenyl)-ethyl]-succinamid |
| **212** | 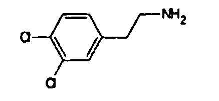 | N-[2-(3,4-Dichlor-phenyl)-ethyl]-N'-[4-dimethylamino-4-(3- methyl-benzyl)-cyclohexyl]-succinamid |
| **213** | 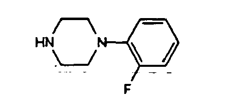 | N-[4-(3-Chlor-benzyl)-4-dimethylamino-cyclohexyl]-4-[4-(2- fluor-phenyl)-piperazin-1-yl]-4-oxo-butyramid 5-[4-(2,4-Dimethoxy-phenyl)-piperazin-1-yl]-5-oxo- |
| **214** | 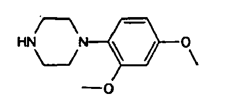 | valeriansäure (4-benzyl-4-dimethylamino-cyclohexyl)-amid |
| **215** | 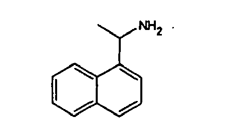 | N-[4-(3-Chlor-benzyl)-4-dimethylamino-cyclohexyl]-N'-(1- naphthalen-1-yl-ethyl)-succinamid |
| **216** | 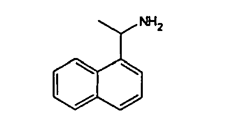 | N-[4-Dimethylamino-4-(3-methyl-benzyl)-cyclohexyl]-N'-(1- naphthalen-1-yl-ethyl)-succinamid |
| **217** | 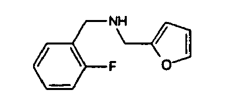 | N-[4-(3-Chlor-benzyl)-4-dimethylamino-cyclohexyl]-N'-(2-fluor-benzyl)-N'-furan-2-ylmethyl-succinamid |
| **218** | 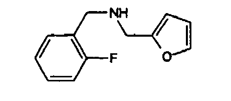 | N-[4-Dimethylamino-4-(3-methyl-benzyl)-cyclohexyl]-N'-(2- fluor-benzyl)-N'-furan-2-ylmethyl-succinamid |
| **219** | 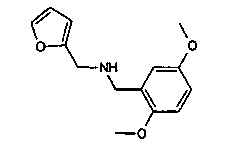 | N-[4-(3-Chlor-benzyl)-4-dimethylamino-cyclohexyl]-N'-(2,5- dimethoxy-benzyl)-N'-furan-2-ylmethyl-succinamid |
| **220** | 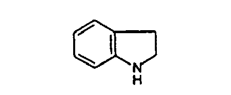 | N-(4-Azepan-1-yl-4-benzyl-cyclohexyl)-4-(2,3-dihydro-indol-1-yl)-4-oxo-butyramid |
| **221** | 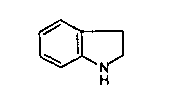 | 4-(2,3-Dihydro-indol-1-yl)-N-[4-dimethylamino-4-(3-fluor- phenyl)-cyclohexyl]-4-oxo-butyramid |
| **222** | 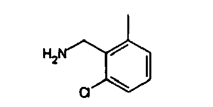 | N-(4-Benzyl-4-morpholin-4-yl-cyclohexyl)-N'-(2-chlor-6-methyl-benzyl)-succinamid |
| **223** | 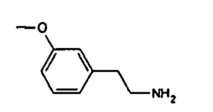 | N-(4-Benzyl-4-morpholin-4-yl-cyclohexyl)-N'-[2-(3-methoxy-phenyl)-ethyl]-succinamid |
| **224** | 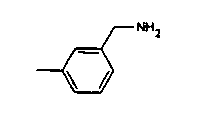 | N-(4-Benzyl-4-morpholin-4-yl-cyclohexyl)-N'-(3-methyl-benzyl)-succinamid |
| **225** | 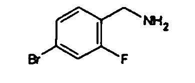 | N-(4-Benzyl-4-morpholin-4-yl-cyclohexyl)-N'-(4-brom-2-fluor-benzyl)-succinamid |
| **226** | 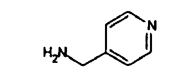 | N-(4-Benzyl-4-morpholin-4-yl-cyclohexyl)-N'-pyridin-4-ylmethyl-succinamid |
| **227** | 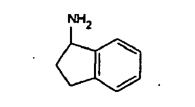 | N-(4-Benzyl-4-morpholin-4-yl-cyclohexyl)-N'-indan-1-yl- succinamid |
| **228** | 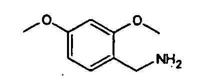 | N-(4-Benzyl-4-morpholin-4-yl-cyclohexyl)-N'-(2,4-dimethoxy-benzyl)-succinamid |
| **229** | 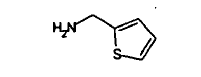 | N-(4-Benzyl-4-morpholin-4-yl-cyclohexyl)-N'-thiophen-2- ylmethyl-succinamid |
| **230** | 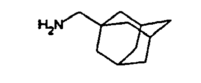 | N-Adamahtan-1-ylmethyl-N'-(4-benzyl-4-morpholin-4-yl- cyclohexyl)-succinamid |
| **231** | 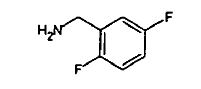 | N-(4-Benzyl-4-morpholin-4-yl-cyclohexyl)-N'-(2,5-difluor- benzyl)-succinamid |
| **232** | 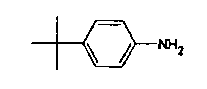 | N-(4-Benzyl-4-morpholin-4-yl-cyclohexyl)-N'-(4-tert-butyl- phenyl)-succinamid |
| **233** | 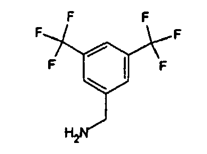 | N-(4-Benzyl-4-morpholin-4-yl-cyclohexyl)-N'-(3,5-bis- trifluormethyl-benzyl)-succinamid |
| **234** | 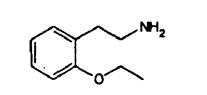 | N-(4-Benzyl-4-morpholin-4-yl-cyclohexyl)-N'-[2-(2-ethoxy- phenyl)-ethyl]-succinamid |
| **235** | 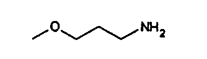 | N-(4-Benzyl-4-morpholin-4-yl-cyclohexyl)-N'-(3-methoxy- propyl)-succinamid |
| **236** | 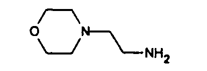 | N-(4-Benzyl-4-morpholin-4-yl-cyclohexyl)-N'-(2-morpholin-4-yl-ethyl)-succinamid |
| **237** | 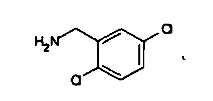 | N-(4-Benzyl-4-morpholin-4-yl-cyclohexyl)-N'-(2,5-dichlor- benzyl)-succinamid |
| **238** | 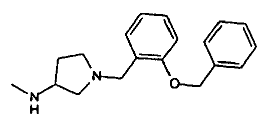 | N-(4-Benzyl-4-morpholin-4-yl-cyclohexyl)-N'-[1-(2-benzyloxy-benzyl)-pyrrolidin-3-yl]-N'-methyl-succinamid |
| **239** | 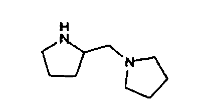 | N-(4-Benzyl-4-morpholin-4-yl-cyclohexyl)-4-oxo-4-(2-pyrrolidin-1-ylmethyl-pyrrolidin-1-yl)-butyramid |
| **240** | 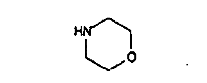 | N-(4-Benzyl-4-morpholin-4-yl-cyclohexyl)-4-morpholin-4-yl-4-oxo-butyramid |
| **241** | 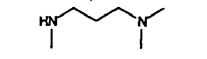 | N-(4-Benzyl-4-morpholin-4-yl-cyclohexyl)-N'-(3-dimethylamino-propyl)-N'-methyl-succinamid |
| **242** | 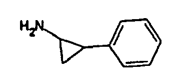 | N-(4-Benzyl-4-morpholin-4-yl-cyclohexyl)-N'-(2-phenyl-cyclopropyl)-succinamid |
| **243** | 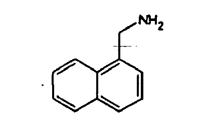 | N-(4-Benzyl-4-morpholin-4-yl-cyclohexyl)-N'-naphthalen-1-ylmethyl-succinamid |
| **244** | 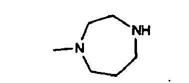 | N-(4-Benzyl-4-morpholin-4-yl-cyclohexyl)-4-(4-methyl-[1,4]diazepan-1-yl)-4-oxo-butyramid |
| **245** | 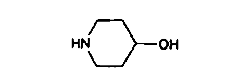 | N-(4-Benzyl-4-morpholin-4-yl-cyclohexyl)-4-(4-hydroxy-piperidin-1-yl)-4-oxo-butyramid |
| **246** | 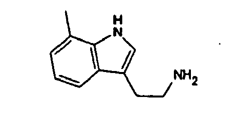 | N-(4-Benzyl-4-morpholin-4-yl-cyclohexyl)-N'-[2-(7-methyl-1H-indol-3-yl)-ethyl]-succinamid |
| **247** | 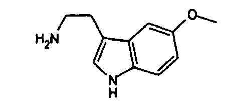 | N-(4-Benzyl-4-morpholin-4-yl-cyclohexyl)-N'-[2-(5-methoxy-1H-indol-3-yl)-ethyl]-succinamid |
| **248** | 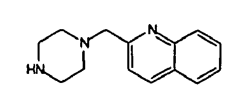 | N-(4-Benzyl-4-morpholin-4-yl-cyclohexyl)-4-oxo-4-(4-chinolin-2-ylmethyl-piperazin-1-yl)-butyramid |
| **249** | 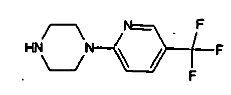 | N-(4-Dimethylamino-4-thiophen-2-yl-cyclohexyl)-4-oxo-4-[4- (5-trifluormethyl-pyridin-2-yl)-piperazin-1-yl]-butyramid |
| **250** | 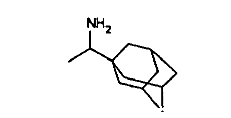 | Glutarsäure-(1-adamantan-1-yl-ethyl)-amid (4-dimethylamino-4-thiophen-2-yl-cyclohexyl)-amid |
| **251** | 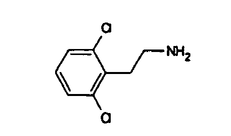 | N-[2-(2,6-Dichlor-phenyl)-ethyl]-N'-(4-dimethylamino-4-thiophen-2-yl-cyclohexyl)-succinamid |
| **252** | 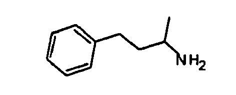 | N-(4-Dimethylamino-4-thiophen-2-yl-cyclohexyl)-N'-(1-methyl-3-phenyl-propyl)-succinamid |
| **253** | 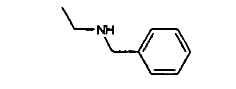 | N-Benzyl-N'-(4-dimethylamino-4-thiophen-2-yl-cyclohexyl)-N-ethyl-succinamid |
| **254** | 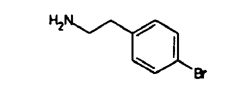 | N-[2-(4-Brom-phenyl)-ethyl]-N'-(4-dimethylamino-4-thiophen-2-yl-cyclohexyl)-succinamid |
| **255** | 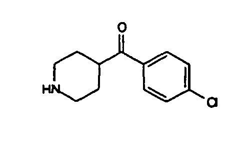 | 4-[4-(4-Chlor-benzoyl)-piperidin-1-yl]-N-(4-dimethylamino-4-thiophen-2-yl-cyclohexyl)-4-oxo-butyramid |
| **256** | 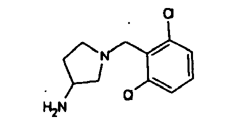 | Glutarsäure-[1-(2,6-dichlor-benzyl)-pyrrolidin-3-yl]-amid (4-dimethylamino-4-thiophen-2-yl-cyclohexyl)-amid |
| **257** | 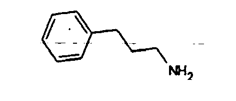 | Glutarsäure-(4-dimethylamino-4-thiophen-2-yl-cyclohexyl)-amid (3-phenyl-propyl)-amid |
| **258** | 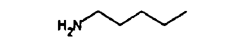 | N-(4-Dimethylamino-4-phenyl-cyclohexyl)-N'-pentyl- succinamid |
| **259** | 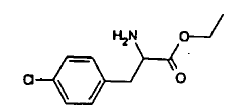 | 3-(4-Chlor-phenyl)-2-[4-(4-dimethylamino-4-thiophen-2-yl- cyclohexylcarbamoyl)-butyrylamino]-propionsäureethylester |
| **260** | 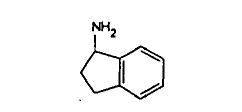 | Glutarsäure-(4-dimethylamino-4-thiophen-2-yl-cyclohexyl)- amid indan-1-ylamid |
| **261** | 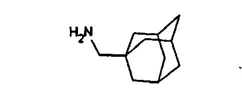 | N-Adamantan-1-ylmethyl-N'-(4-dimethylamino-4-thiophen-2-yl-cyclohexyl)-succinamid |
| **262** | 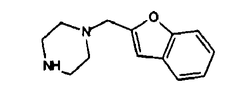 | 5-(4-Benzofuran-2-ylmethyl-piperazin-1-yl)-5-oxo- valeriansäure (4-dimethylamino-4-thiophen-2-yl-cyclohexyl)-amid |
| **263** | 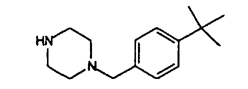 | 4-[4-(4-tert-Butyl-benzyl)-piperazin-1-yl]-N-(4-dimethylamino-4-phenyl-cyclohexyl)-4-oxo-butyramid |
| **264** | 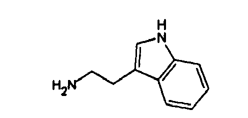 | Glutarsäure-(4-dimethylamino-4-thiophen-2-yl-cyclohexyl)- amid [2-(1 H-indol-3-yl)-ethyl]-amid |
| **265** | 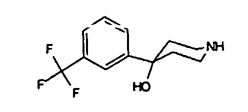 | N-(4-Dimethylamino-4-phenyl-cyclohexyl)-4-[4-hydroxy-4-(3-trifluormethyl-phenyl)-piperidin-1-yl]-4-oxo-butyramid |
| **266** | 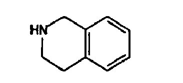 | 5-(3,4-Dihydro-1 H-isochinolin-2-yl)-5-oxo-valeriansäure (4-dimethylamino-4-thiophen-2-yl-cyclohexyl)-amid |
| **267** | 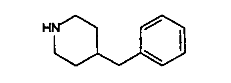 | 4-(4-Benzyl-piperidin-1-yl)-N-(4-dimethylamino-4-phenyl-cyclohexyl)-4-oxo-butyramid |
| **268** | 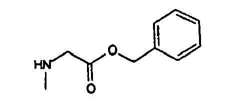 | {[4-(4-Dimethylamino-4-thiophen-2-yl-cyclohexylcarbamoyl)-butyryl]-methyl-amino}-essigsäurebenzylester |
| **269** | 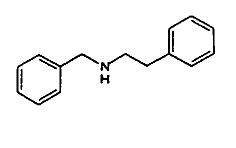 | N-Benzyl-N'-(4-dimethylamino-4-phenyl-cyclohexyl)-N- phenethyl-succinamid |
| **270** | 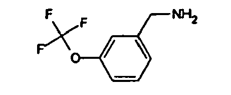 | Glutarsäure-(4-dimethylamino-4-thiophen-2-yl-cyclohexyl)- amid 3-trifluormethoxy-benzylamid |
| **271** | 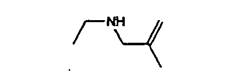 | Glutarsäure-(4-dimethylamino-4-thiophen-2-yl-cyclohexyl)- amid ethyl-(2-methyl-allyl)-amid |
| **272** | 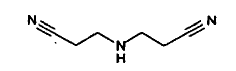 | N,N-Bis-(2-cyano-ethyl)-N'-(4-dimethylamino-4-thiophen-2-yl-cyclohexyl)-succinamid |
| **273** | 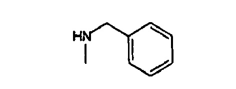 | Glutarsäure-benzyl-methyl-amid (4-dimethylamino-4-thiophen-2-yl-cyclohexyl)-amid |
| **274** | 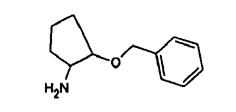 | Glutarsäure-(2-benzyloxy-cyclopentyl)-amid (4- dimethylamino-4-thiophen-2-yl-cyclohexyl)-amid |
| **275** | 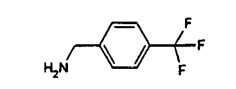 | Glutarsäure-(4-dimethylamino-4-thiophen-2-yl-cyclohexyl)- amid 4-trifluormethyl-benzylamid |
| **276** | 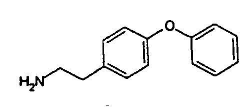 | N-(4-Dimethylamino-4-thiophen-2-yl-cyclohexyl)-N'-[2-(4- phenoxy-phenyl)-ethyl]-succinamid |
| **277** | 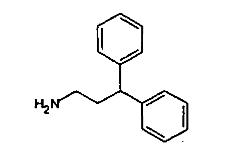 | Glutarsäure-(4-dimethylamino-4-thiophen-2-yl-cyclohexyl)- amid (3,3-diphenyl-propyl)-amid |
| **278** | 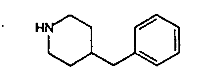 | 4-(4-Benzyl-piperidin-1-yl)-N-(4-dimethylamino-4-thiophen-2-yl-cyclohexyl)-4-oxo-butyramid |
| **279** | 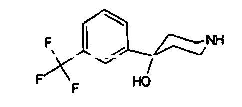 | N-(4-Dimethylamino-4-thiophen-2-yl-cyclohexyl)-4-[4-hydroxy-4-(3-trifluormethyl-phenyl)-piperidin-1-yl]-4-oxo-butyramid |
| **280** | 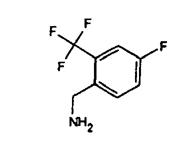 | Glutarsäure-(4-dimethylamino-4-thiophen-2-yl-cyclohexyl)- amid 4-fluor-2-trifluormethyl-benzylamid |
| **281** | 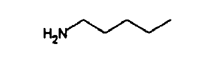 | N-(4-Dimethylamino-4-thiophen-2-yl-cyclohexyl)-N'-pentyl- succinamid |
| **282** | 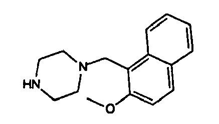 | 5-[4-(2-Methoxy-naphthalen-1-ylmethyl)-piperazin-1-yl]-5-oxo-valeriansäure (4-dimethylamino-4-thiophen-2-yl-cyclohexyl)-amid |
| **283** | 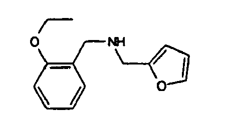 | N-(4-Dimethylamino-4-thiophen-2-yl-cyclohexyl)-N'-(2-ethoxy-benzyl)-N'-furan-2-ylmethyl-succinamid |
| **284** | 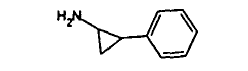 | Glutarsäure-(4-dimethylamino-4-thiophen-2-yl-cyclohexyl)-amid (2-phenyl-cyclopropyl)-amid |
| **285** | 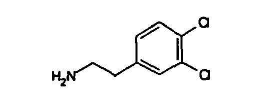 | N-[2-(3,4-Dichlor-phenyl)-ethyl]-N'-(4-dimethylamino-4-phenyl-cyclohexyl)-succinamid |
| **286** | 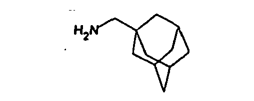 | N-Adamantan-1-ylmethyl-N'-(4-dimethylamino-4-phenyl-cyclohexyl)-succinamid |
| **287** | 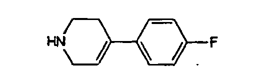 | 5-[4-(4-Fluor-phenyl)-3,6-dihydro-2H-pyridin-1-yl]-5-oxo- valeriansäure (4-dimethylamino-4-thiophen-2-yl-cyclohexyl)-amid |
| **288** | 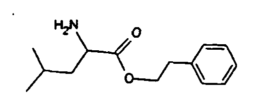 | 2-[4-(4-Dimethylamino-4-thiophen-2-yl-cyclohexylcarbamoyl)-butyrylamino]-4-methyl-valeriansäure benzyl ester |
| **289** | 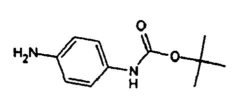 | {4-[4-(4-Dimethylamino-4-thiophen-2-yl-cyclohexylcarbamoyl)-butyrylamino]-phenyl}-carbaminsäure-tert-butylester |
| **290** | 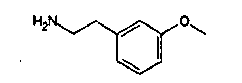 | Glutarsäure-(4-dimethylamino-4-thiophen-2-yl-cyclohexyl)-amid [2-(3-methoxy-phenyl)-ethyl]-amid |
| **291** | 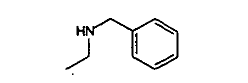 | Glutarsäure-benzyl-ethyl-amid (4-dimethylamino-4-thiophen-2-yl-cyclohexyl)-amid |
| **292** | 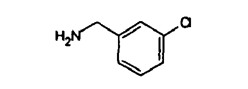 | Glutarsäure-3-chlor-benzylamid (4-dimethylamino-4-thiophen-2-yl-cyclohexyl)-amid |
| **293** | 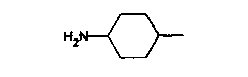 | Glutarsäure-(4-dimethylamino-4-thiophen-2-yl-cyclohexyl)-amid (4-methyl-cyclohexyl)-amid |
| **294** | 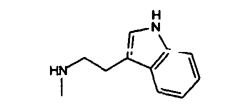 | N-(4-Dimethylamino-4-phenyl-cyclohexyl)-N'-[2-(1H-indol-3-yl)-ethyl]-N'-methyl-succinamid |
| **295** | 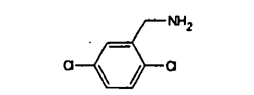 | Glutarsäure-2,5-dichlor-benzylamid (4-dimethylamino-4-thiophen-2-yl-cyclohexyl)-amid |
| **296** | 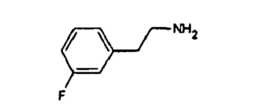 | Glutarsäure-(4-dimethylamino-4-thiophen-2-yl-cyclohexyl)-amid [2-(3-fluor-phenyl)-ethyl]-amid |
| **297** | 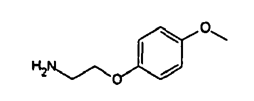 | N-(4-Dimethylamino-4-phenyl-cyclohexyl)-N'-[2-(4-methoxy-phenoxy)-ethyl]-succinamid |
| **298** | 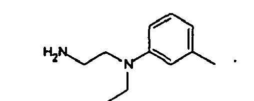 | N-(4-Dimethylamino-4-thiophen-2-yl-cyclohexyl)-N'-[2-(ethyl-m-tolyl-amino)-ethyl]-succinamid |
| **299** | 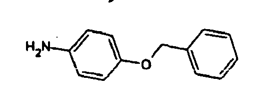 | Glutarsäure-(4-benzyloxy-phenyl)-amid (4-dimethylamino-4-thiophen-2-yl-cyclohexyl)-amid |
| **300** | 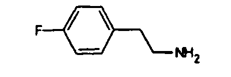 | Glutarsäure-(4-dimethylamino-4-thiophen-2-yl-cyclohexyl)-amid [2-(4-fluor-phenyl)-ethyl]-amid |
| **301** | 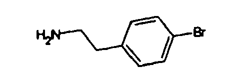 | Glutarsäure-[2-(4-brom-phenyl)-ethyl]-amid (4-dimethylamino-4-thiophen-2-yl-cyclohexyl)-amid |
| **302** | 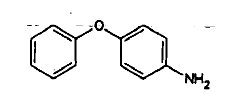 | N-(4-Dimethylamino-4-thiophen-2-yl-cyclohexyl)-N'-(4-phenoxy-phenyl)-succinamid |
| **303** | 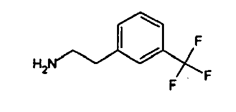 | N-(4-Dimethylamino-4-phenyl-cyclohexyl)-N'-[2-(3-trifluormethyl-phenyl)-ethyl]-succinamid |
| **304** | 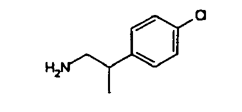 | Glutarsäure-[2-(4-chlor-phenyl)-propyl]-amid (4-dimethylamino-4-thiophen-2-yl-cyclohexyl)-amid |
| **305** | 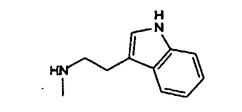 | N-(4-Dimethylamino-4-thiophen-2-yl-cyclohexyl)-N'-[2-(1H-indol-3-yl)-ethyl]-N'-methyl-succinamid |
| **306** | 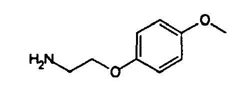 | N-(4-Dimethylamino-4-thiophen-2-yl-cyclohexyl)-N'-[2-(4-methoxy-phenoxy)-ethyl]-succinamid |
| **307** | 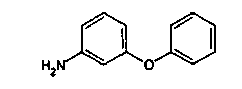 | Glutarsäure-(4-dimethylamino-4-thiophen-2-yl-cyclohexyl)- amid (3-phenoxy-phenyl)-amid |
| **308** | 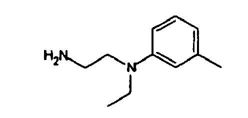 | N-(4-Dimethylamino-4-phenyl-cyclohexyl)-N'-[2-(ethyl-m-tolyl-amino)-ethyl]-succinamid |
| **309** | 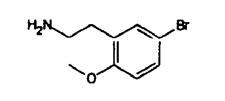 | Glutarsäure-[2-(5-brom-2-methoxy-phenyl)-ethyl]-amid (4- dimethylamino-4-thiophen 2-yl-cyclohexyl)-amid |
| **310** | 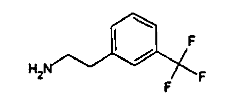 | N-(4-Dimethylamino-4-thiophen-2-yl-cyclohexyl)-N'-[2-(3- trifluormethyl-phenyl)-ethyl]-succinamid |
| **311** | 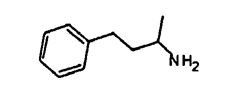 | Glutarsäure-(4-dimethylamino-4-thiophen-2-yl-cyclohexyl)- amid (1-methyl-3-phenyl-propyl)-amid |
| **312** | 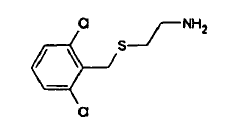 | N-[2-(2,6-Dichlor-benzylsulfanyl)-ethyl]-N'-(4-dimethylamino-4-thiophen-2-yl-cyclohexyl)-succinamid |
| **313** | 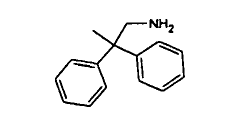 | Glutarsäure-(4-dimethylamino-4-thiophen-2-yl-cyclohexyl)- amid (2,2-diphenyl-propyl)-amid |
| **314** | 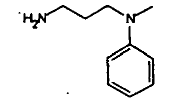 | N-(4-Dimethylamino-4-thiophen-2-yl-cyclohexyl)-N'-[3- (methyl-phenyl-amino)-propyl]-succinamid |
| **315** | 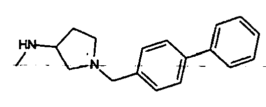 | N-(1-Biphenyl-4-ylmethyl-pyrrolidin-3-yl)-N'-(4-dimethylamino-4-thiophen-2-yl-cyclohexyl)-N-methyl-succinamid |
| **316** | 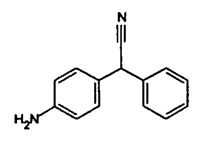 | Glutarsäure-[4-(cyano-phenyl-methyl)-phenyl]-amid (4- dimethylamino-4-thiophen-2-yl-cyclohexyl)-amid |
| **317** | 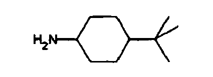 | Glutarsäure-(4-*tert*-butyl-cyclohexyl)-amid (4-dimethylamino-4-thiophen-2-yl-cyclohexyl)-amid |
| **318** | 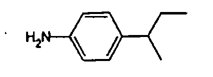 | Glutarsäure-(4-sec-butyl-phenyl)-amid (4-dimethylamino-4- thiophen-2-yl-cyclohexyl)-amid |
| **319** | 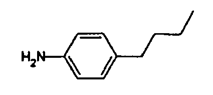 | Glutarsäure-(4-butyl-phenyl)-amid (4-dimethylamino-4- thiophen-2-yl-cyclohexyl)-amid |
| **320** | 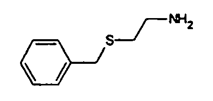 | N-(2-Benzylsulfanyl-ethyl)-N'-(4-dimethylamino-4-thiophen-2-yl-cyclohexyl)-succinamid |
| **321** | 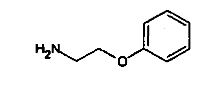 | N-(4-Dimethylamino-4-thiophen-2-yl-cyclohexyl)-N'-(2- phenoxy-ethyl)-succinamid |
| **322** | 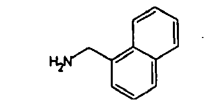 | Glutarsäure-(4-dimethylamino-4-thiophen-2-yl-cyclohexyl)- amid (naphthalen-1-ylmethyl)-amid |
| **323** | 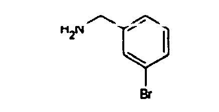 | Glutarsäure-3-brom-benzylamid (4-dimethylamino-4- thiophen-2-yl-cyclohexyl)-amid |
| **324** | 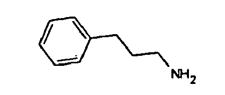 | N-(4-Dimethylamino-4-thiophen-2-yl-cyclohexyl)-N'-(3-phenyl-propyl)-succinamid |
| **325** | 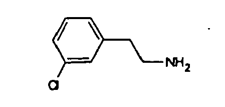 | N-[2-(3-Chlor-phenyl)-ethyl]-N'-(4-dimethylamino-4-phenyl- cyclohexyl)-succinamid |
| **326** | 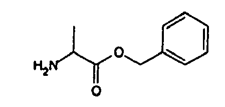 | 2-[3-(4-Dimethylamino-4-thiophen-2-yl-cyclohexylcarbamoyl)-propionylamino]-propionsäurebenzylester |
| **327** | 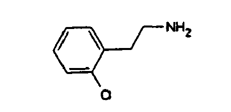 | N-[2-(2-Chlor-phenyl)-ethyl]-N'-(4-dimethylamino-4-phenyl- cyclohexyl)-succinamid |
| **328** | 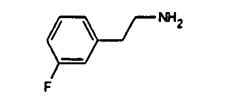 | N-(4-Dimethylamino-4-phenyl-cyclohexyl)-N'-[2-(3-fluor- phenyl)-ethyl]-succinamid |
| **329** | 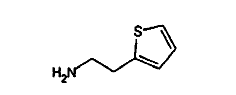 | Glutarsäure-(4-dimethylamino-4-thiophen-2-yl-cyclohexyl)- amid (2-thiophen-2-yl-ethyl)-amid |
| **330** | 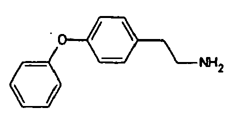 | Glutarsäure-(4-dimethylamino-4-thiophen-2-yl-cyclohexyl)- amid [2-(4-phenoxy-phenyl)-ethyl]-amid |
| **331** | 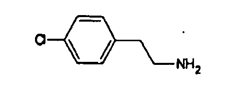 | N-[2-(4-Chlor-phenyl)-ethyl]-N'-(4-dimethylamino-4-phenyl- cyclohexyl)-succinamid |
| **332** | 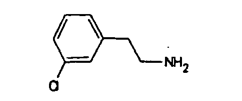 | N-[2-(3-Chlor-phenyl)-ethyl]-N'-(4-dimethylamino-4-thiophen-2-yl-cyclohexyl)-succinamid |
| **333** | 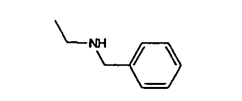 | N-Benryl-N'-(4-dimethylamino-4-thiophen-2-yl-cyclohexyl)-N-ethyl-succinamid |
| **334** | 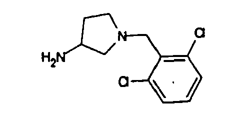 | Glutarsäure-[1-(2,6-dichlor-benzyl)-pyrrolidin-3-yl]-amid (4-dimethylamino-4-thiophen-2-yl-cyclohexyl)-amid |
| **335** | 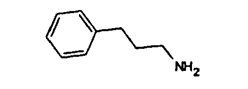 | Glutarsäure-(4-dimethylamino-4-thiophen-2-yl-cyclohexyl)- amid (3-phenyl-propyl)-amid |
| **336** | 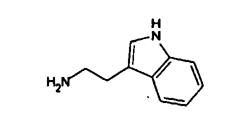 | Glutarsäure-(4-dimethylamino-4-thiophen-2-yl-cyclohexyl)- amid [2-(1 H-indol-3-yl)-ethyl]-amid |
| **337** | 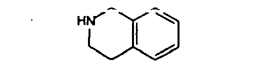 | 5-(3,4-Dihydro-1H-isochinolin-2-yl)-5-oxo-valeriansäure (4-dimethylamino-4-thiophen-2-yl-cyclohexyl)-amid |
| **338** | 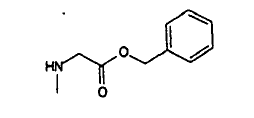 | {[4-(4-Dimethylamino-4-thiophen-2-yl-cyclohexylcarbamoyl)-butyryl]-methyl-amino}-essigsäurebenzylester |
| **339** | 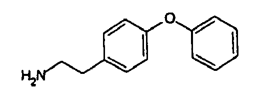 | N-(4-Dimethylamino-4-thiophen-2-yl-cyclohexyl)-N'-[2-(4- phenoxy-phenyl)-ethyl]-succinamid |
| **340** | 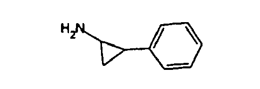 | Glutarsäure-(4-dimethylamino-4-thiophen-2-yl-cyclohexyl)- amid (2-phenyl-cyclopropyl)-amid |

Ausgewählte Beispiele wurden auch in größerem Maßstab synthetisiert:

### Beispiel 341: N-[4-(3-Chlorbenzyl)-4-dimethylaminocyclohexyl]-N'-furan-2-yl-methylbernsteinsäureamid Hydrochlorid, unpolareres Diastereoisomer

Zu 3,92 g Bernsteinsäureanhydrid in 40 ml THF p.a. wurde eine Lösung von 5,0 ml C-Furan-2-yl-methylamin und 6,7 ml Diisopropylethylamin in 25 ml THF p.a. zugetropft und die Lösung über Nacht gerührt. Nach Zugabe von 25 ml zweimolarer Salzsäure wurde dreimal mit je 40 ml Dichlormethan extrahiert, die Extrakte über Magnesiumsulfat getrocknet, filtriert und eingeengt. Es wurden 7,0 g N-Furan-2-yl-methylbernsteinsäure erhalten.

Zu 0,74 g N-Furan-2-ylmethylbernsteinsäure wurden bei 0 °C 1,0 g 1-(3-Chlorbenzyl)-N,N-dimethylcyclohexan-1,4-diamin, als cis/trans-Gemisch aus 4-(3-Chlorbenzyl)-4-dimethylaminocyclohexanon erhalten durch Umsetzung mit Hydroxylamin und Reduktion des erhaltenen Oxims mit Lithiumaluminiumhydrid (vgl. WO 02090317), gelöst in 5 ml DMF gegeben und unter Rühren 0,59 ml N,N-Diisopropylcarbodiimid gefolgt von 0,5 g 1-Hydroxybenzotriazol (HOBt) zugegeben. Nach drei Stunden im Eisbad wurde über Nacht bei Raumtemperatur gerührt. Zur Aufarbeitung wurde einmolare Natriumcarbonatlösung zugegeben (pH > 10) und das Rohprodukt (1,75 g) durch Extraktion mit Ethylacetat/THF (V:V = 1:1), anschließende Trocknung über Natriumsulfat und Einengen isoliert. Die nach Säulenchromatographie an Kieselgel (3,0 x 20 cm) mit je 250 ml Diethylether, Diethylether /Methanol (V:V = 4:1) und Diethylether /Methanol (V:V = 1.1) erhaltene Hauptfraktion von 718 mg wurde in 50 ml 2-Butanon, 50 ml Ethylacetat und 2 ml Methanol gelöst durch Zugabe von 29 µl Wasser und 200 µl Chlortrimethylsilan in das korrespondierende Hydrochlorid von N-[4-(3-Chlorbenzyl)-4-dimethylaminocyclohexyl]-N'-furan-2-ylmethylbernsteinsäureamid überführt wurden (648 mg weißer Feststoff, Smp. 233 - 234,5 °C).

### Beispiel 342: N-[4-(3-Chlorbenzyl)-4-dimethylaminocyclohexyl]-N'-furan-2-yl-methylbernsteinsäureamid Hydrochlorid, polareres Diastereoisomer

Wie für Beispiel 341 beschrieben wurden auch 357 mg des polareren Diastereoisomers von N-[4-(3-Chlorbenzyl)-4-dimethylaminocyclohexyl]-N'-furan-2-yl-methylbernsteinsäureamid erhalten, die in 25 ml 2-Butanon gelöst durch Zugabe von 14,4 µl Wasser und 100 µl Chlortrimethylsilan in das korrespondierende Hydrochlorid überführt wurden (230 mg weißer Feststoff, Smp. 186 - 188 °C)..

### Beispiel 343: Glutarsäure (4-benzyl-4-dimethylaminocyclohexyl)amid [2-(2-fluorphenyl)ethyl]amid Hydrochlorid, unpolareres Diastereoisomer

Wie für Beispiel 341 beschrieben wurden 0,95 mg 4-[2-(2-Fluorphenyl)ethyl-carbamoyl]buttersäure und 0,87 g 1-Benzyl-N,N-dimethylcyclohexan-1,4-diamin in 5 ml DMF in Gegenwart von 0,59 ml N,N-Diisopropylcarbodiimid und 0,50 g1-Hydroxybenzotriazol umgesetzt und das Rohprodukt (1,65 g gelber Feststoff) analog isoliert. Durch Chromatographie an Kieselgel (3,0 x 20 cm) mit 450 ml Diethylether gefolgt von 450 ml Diethylether/Methanol (V:V = 4:1) und 450 ml Diethylether/Methanol (V:V = 1:1) wurden 720 mg des unpolareren Diastereoisomers von Glutarsäure (4-benzyl-4-dimethylaminocyclohexyl)amid erhalten, die in 25 ml 2-Butanon und 25 ml Ethylacetat gelöst durch Zugabe von 28µl Wasser und 198 µl Chlortrimethylsilan in das korrespondierende Hydrochlorid überführt wurden (657 mg glasartiger Feststoff).

### Beispiel 344: Glutarsäure (4-benzyl-4-dimethylaminocyclohexyl)amid [2-(2-fluorphenyl)ethyl]amid Hydrochlorid, polareres Diastereoisomer

Wie für Beispiel 343 beschrieben wurden auch 260 mg des polareren Diastereoisomers von Glutarsäure (4-benzyl-4-dimethylaminocyclohexyl)amid erhalten, die in 5 ml 2-Butanon und 45 ml Ethylacetat gelöst durch Zugabe von 10µl Wasser und 71 µl Chlortrimethylsilan in das korrespondierende Hydrochlorid überführt wurden (110 mg glasartiger Feststoff).

### Beispiel 345: N-[4-(3-Chlorbenzyl)-4-dimethylaminocyclohexyl]-N'-[2-(4-chlor-phenyl)ethyl]bernsteinsäureamid Hydrochlorid, unpolareres Diastereoisomer

Wie für Beispiel 341 beschrieben wurden 0,96 mg N-[2-(4-Chlorphenyl)ethyl]bernsteinsäure und 1,0 g 4-(3-Chlorbenzyl)-4-dimethylaminocyclohexanon in 5 ml DMF in Gegenwart von 0,59 ml N,N-Diisopropylcarbodiimid und 0,5 g 1-Hydroxybenzotriazol umgesetzt und das Rohprodukt (2,23 g gelber Feststoff) analog isoliert. Durch Chromatographie an Kieselgel (3,0 x 20 cm) mit 200 ml Diethylether gefolgt von 200 ml Diethylether/Methanol (V:V = 5:1) wurden 947 mg des unpolareren Diastereoisomers von N-[4-(3-Chlorbenzyl)-4-dimethylaminocyclohexyl]-N'-[2-(4-chlorphenyl)ethyl]bernsteinsäureamid erhalten. 300 mg hiervon wurden in 5 ml 2-Butanon, 10 ml Ethylacetat und 1 ml Methanol gelöst und 10,7 µl Wasser und 76 µl Chlortrimethylsilan zugegeben, der Ansatz zur Trockne eingeengt, der Rückstand mit 10 ml 2-Butanon und 10 ml Diisopropylether über drei Tage bei Raumtemperatur gerührt und das ausgefallene Hydrochlorid durch Filtration isoliert und getrocknet (200 mg weißer feststoff).

### Beispiel 346: N-[4-(3-Chlorbenzyl)-4-dimethylaminocyclohexyl]-N'-[2-(4-chlor-phenyl)ethyl]bernsteinsäureamid Hydrochlorid, polareres Diastereoisomer

Wie für Beispiel 345 beschrieben wurden auch 360 mg des polareren Diastereoisomers von N-[4-(3-Chlorbenzyl)-4-dimethylaminocyclohexyl]-N'-[2-(4-chlorphenyl)ethyl]bernsteinsäureamid erhalten, die in 25 ml 2-Butanon und 10 ml Ethylacetat gelöst durch Zugabe von 12,5 µl Wasser, 88 µl Chlortrimethylsilan und 10 ml Diisopropylether in das korrespondierende Hydrochlorid überführt wurden (236 mg weißer Feststoff, Smp. 186 - 187,5 °C).

### Beispiel 347: Glutarsäure (4-benzyl-4-dimethylaminocyclohexyl)amid [2-(4-chlorphenyl)ethyl]amid Hydrochlorid, unpolarores Diastereoisomer

Wie für Beispiel 341 beschrieben wurden 0,87 mg 4-[2-(4-Chlorphenyl)ethyl-carbamoyl]buttersäure und 1,0 g 4-Benzyl-4-dimethylaminocyclohexanon in 5 ml DMF in Gegenwart von 0,59 ml N,N-Diisopropylcarbodiimid und 0,5 g 1-Hydroxybenzotriazol umgesetzt und das Rohprodukt (1,80 g) analog isoliert. Durch Chromatographie an Kieselgel (3,0 x 20 cm) mit 200 ml Diethylether gefolgt von 200 ml Diethylether/Methanol (V:V = 5:1) wurden 900 mg des unpolareren Diastereoisomers von Glutarsäure (4-benzyl-4-dimethylaminocyclohexyl)amid [2-(4-chlorphenyl)ethyl]amid erhalten, die in 20 ml 2-Butanon und 20 ml Ethylacetat gelöst durch Zugabe von 33,5 µl Wasser und 236 µl Chlortrimethylsilan und anschließendes Einengen zur Trockne in das korrespondierende Hydrochlorid überführt wurden (980 mg glasartiger Feststoff).

### Beispiel 348: Glutarsäure (4-benzyl-4-dimethylaminocyclohexyl)amid [2-(4-chlorphenyl)ethyl]amid Hydrochlorid, polareres Diastereoisomer

Wie für Beispiel 347 beschrieben wurden auch 335 mg des polareren Diastereoisomers von Glutarsäure (4-benzyl-4-dimethylaminocyclohexyl)amid [2-(4-chlorphenyl)ethyl]amid erhalten, die in 30 ml 2-Butanon, 30 ml Ethylacetat und 2 ml Methanol gelöst durch Zugabe von 12,5 µl Wasser, 88 µl Chlortrimethylsilan und 10 ml Diisopropylether in das korrespondierende Hydrochlorid überführt wurden (255 mg weißer Feststoff).

### Untersuchungen zur Wirksamkeit der erfindungsgemäßen Verbindungen:

### Messung der ORL1-Bindung

Die Cyclohexan-Derivate der allgemeinen Formel I wurde in einem Rezeptorbindungsassay mit ³H-Nociceptin/Orphanin FQ mit Membranen von rekombinanten CHO-ORL1 Zellen untersucht. Dieses Testsystem wurde gemäß der von Ardati et al. (Mol. Pharmacol., 51, 1997, S. 816-824) vorgestellten Methode durchgeführt. Die Konzentration von ³H-Nociceptin/Orphanin FQ betrug bei diesen Versuchen 0.5 nM. Die Bindungsassays wurden mit je 20 µg Membranprotein je 200 µl Ansatz in 50 mM Hepes, pH 7,4, 10 mM MgCl₂ und 1 mM EDTA durchgeführt. Die Bindung an den ORL1-Rezeptor wurde unter Verwendung von je 1 mg WGA-SPA Beads (Amersham-Pharmacia, Freiburg), durch einstündige Inkubation des Ansatzes bei RT und anschliessende Messung im Szintillationscounter Trilux (Wallac, Finnland), bestimmt. Die Affinität wird in Tabelle 1 als nanomolarer Kᵢ-Wert in oder % Inhibition bei c=1 µM angegeben.

### Messung der µ-Bindung

Die Rezeptoraffinität zum humanen µ-Opiatrezeptor wurde in einem homogenen Ansatz in Mikrotiterplatten bestimmt. Hierzu wurden Verdünnungsreihen des jeweils zu prüfenden substituierten substituierten Cyclohexyl-1,4-diamin -Derivates mit einer Rezeptormembranpräparation (15-40 µg Protein pro 250 µl Inkubationsansatz) von CHO-K1-Zellen, welche den humanen µ-Opiatrezeptor exprimieren (RB-HOM-Rezeptormembran-Präparation der Firma NEN, Zaventem, Belgien) in Gegenwart von 1 nmol/l des radioaktiven Liganden [³H]-Naloxon (NET719, Firma NEN, Zaventem, Belgien) sowie von 1 mg WGA-SPA-Beads (Wheat germ agglutinin SPA Beads der Firma Amersham/Pharmacia, Freiburg, Deutschland) in einem Gesamtvolumen von 250 µl für 90 Minuten bei Raumtemperatur inkubiert. Als Inkubationspuffer wurde 50 mmol/l Tris-HCl supplementiert mit 0,05 Gew.-% Natriumazid und mit 0,06 Gew.-% bovinem Serumalbumin verwendet. Zur Bestimmung der unspezifischen Bindung wurde zusätzlich 25 µmol/l Naloxon zugegeben. Nach Beendigung der neunzigminütigen Inkubationszeit wurden die Mikrotiterplatten für 20 Minuten bei 1000 g abzentrifugiert und die Radioaktivität in einem β-Counter (Microbeta-Trilux, Firma PerkinElmer Wallac, Freiburg, Deutschland) vermessen. Es wurde die prozentuale Verdrängung des radioaktiven Liganden aus seiner Bindung zum humanen µ-Opiatrezeptor bei einer Konzentration der Prüfsubstanzen von 1 µmol/l bestimmt und als prozentuale Hemmung (%Hemmung) der spezifischen Bindung angegeben. Teilweise wurden ausgehend von der prozentualen Verdrängung durch unterschiedliche Konzentrationen der zu prüfenden Verbindungen der allgemeinen Formel I IC₅₀ Hemmkonzentrationen berechnet, die eine 50-prozentige Verdrängung des radioaktiven Liganden bewirken. Durch Umrechnung mittels der Cheng-Prusoff-Beziehung wurden Ki-Werte für die Prüfsubstanzen erhalten.

### Messung der Serotonin-Wiederaufnahme

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den 5HT-Uptake werden diese vesikulären Partikel aus der Medulla + Pons-Region von männlichen Rattengehirnen isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

### Messung der Noradrenalin-Wiederaufnahme

Um diese in vitro Studien durchführen zu können, werden Synaptosomen aus Rattenhirnarealen frisch isoliert. Es findet jeweils eine sogenannte "P₂"-Fraktion Verwendung, die nach der Vorschrift von Gray und Whittaker (E.G. Gray und V.P. Whittaker (1962) J. Anat. 76, 79-88) präpariert wird. Für den NA-Uptake werden diese vesikulären Partikel aus dem Hypothalamus männlicher Rattengehirne isoliert.

Eine detaillierte Methodenbeschreibung kann der Literatur entnommen werden (M.Ch. Frink, H.-H. Hennies, W. Englberger, M. Haurand und B. Wilffert (1996) Arzneim.-Forsch./Drug Res. 46 (III), 11, 1029-1036).

Beispielhaft wurden die folgenden Bindungsdaten bestimmt:

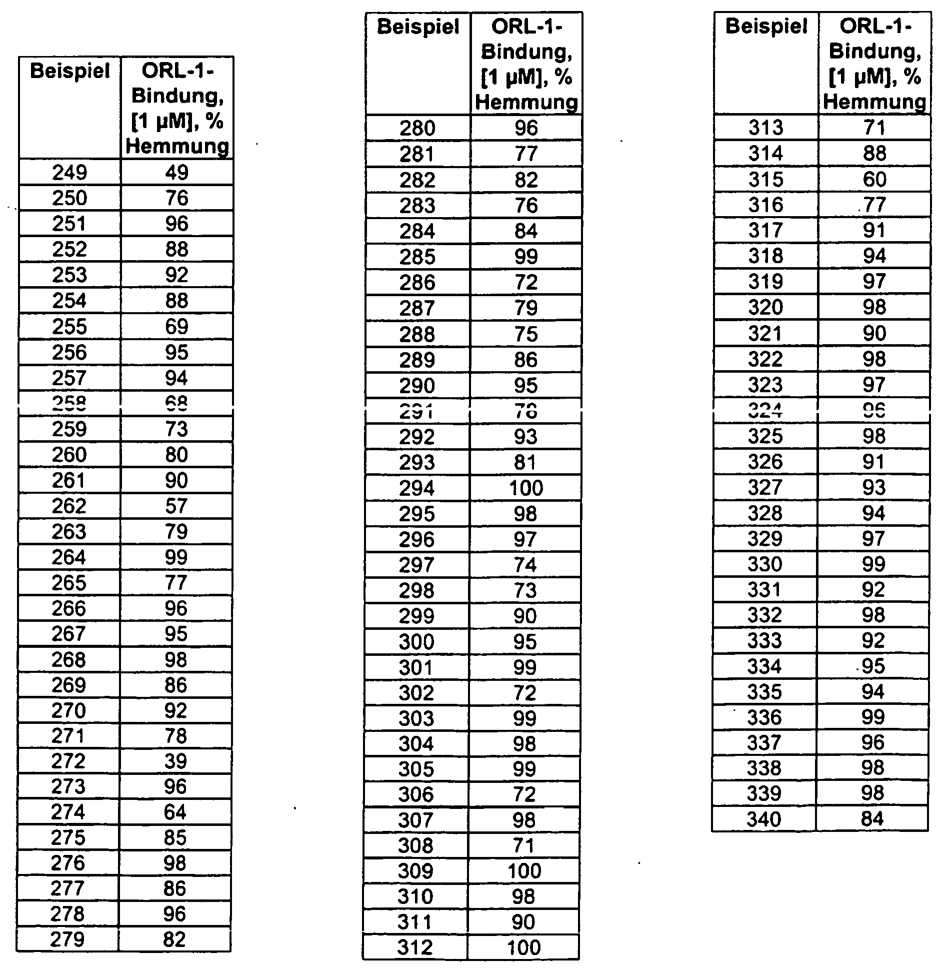

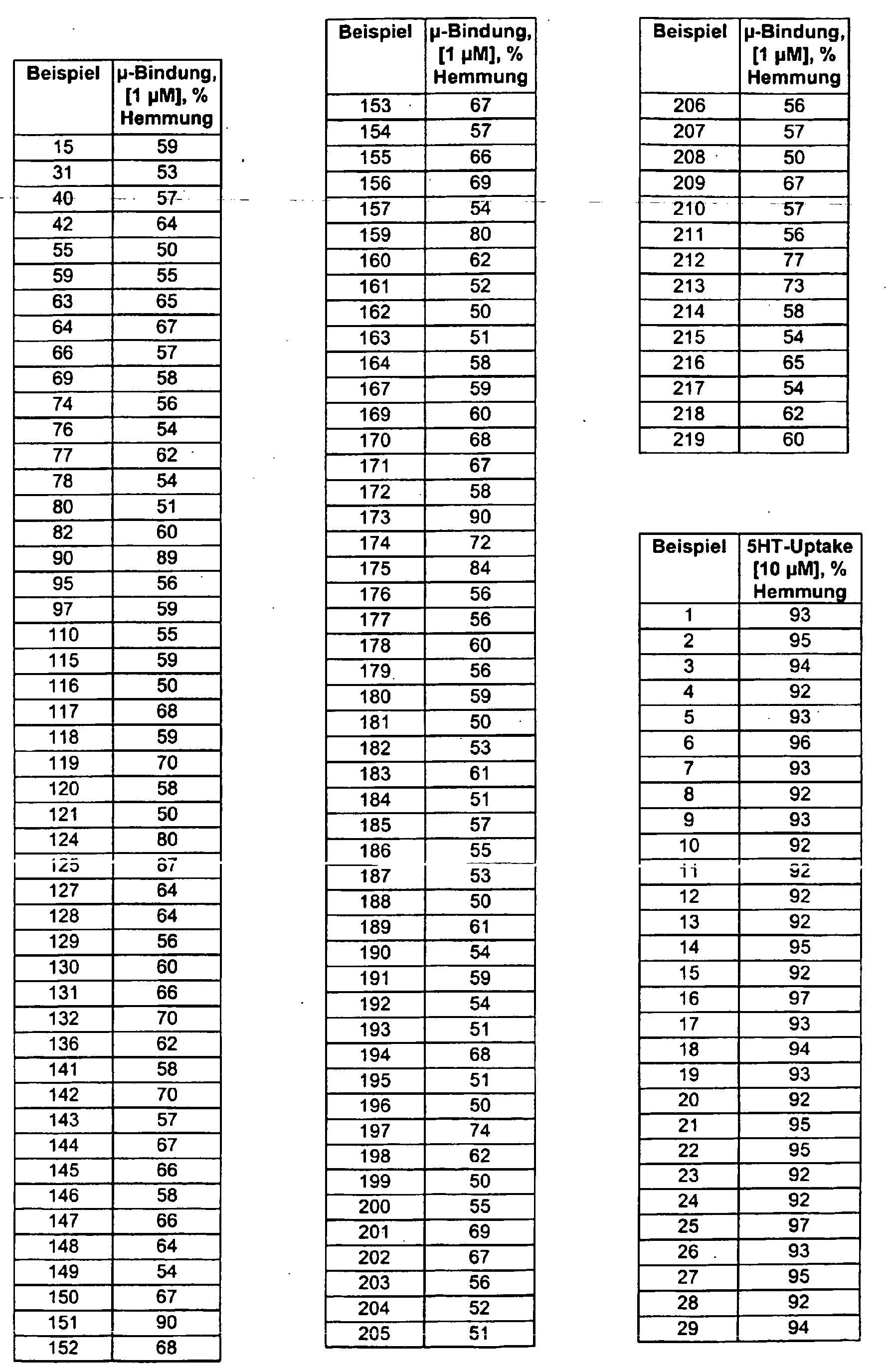

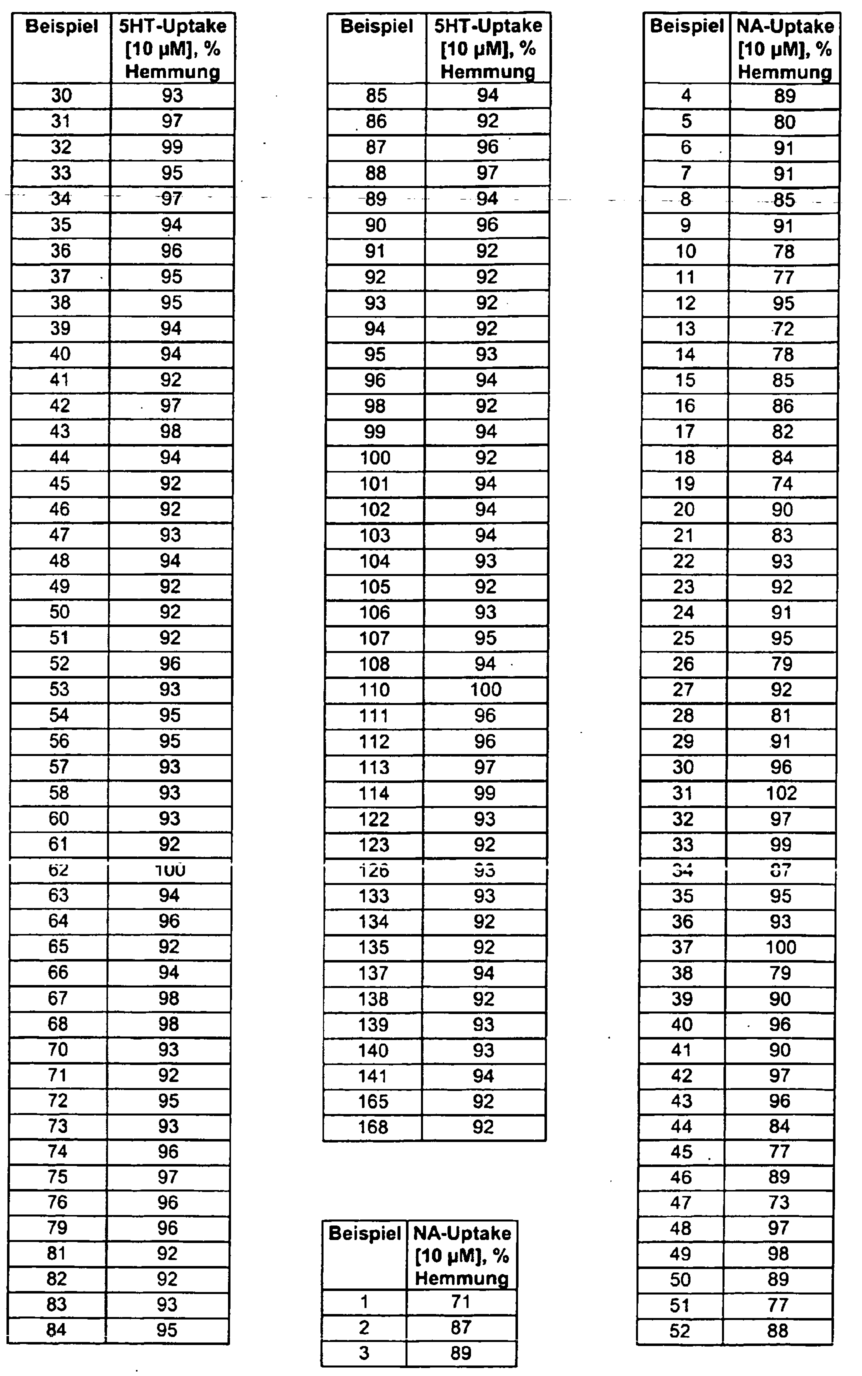

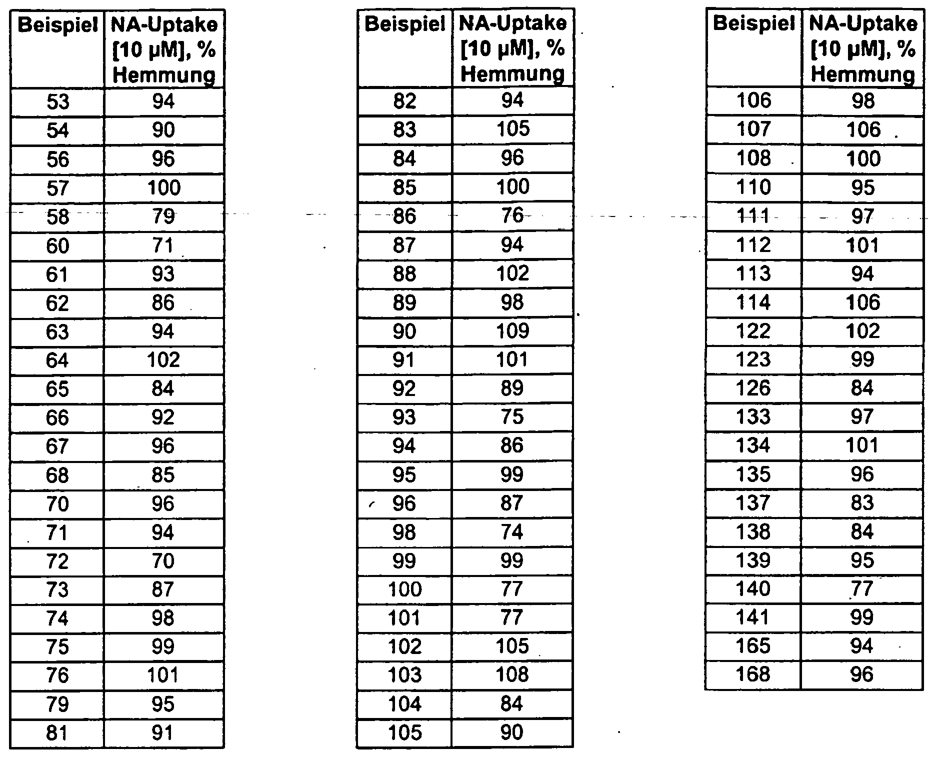

### Parenterale Lösung eines erfindungsgemäßen substituierten Cyclohexyl-1,4-diamin-Derivats

38 g eines der erfindungsgemäßen substituierten Cyclohexyl-1,4-diamin-Derivate, hier Beispiel 1, wird in 1 I Wasser für Injektionszwecke bei Raumtemperatur gelöst und anschließend durch Zugabe von wasserfreier Glukose für Injektionszwecke auf isotone Bedingungen eingestellt.

## Patentansprüche

1. Substituierte Cyclohexyl-1,4-diamin-Derivate der allgemeinen Formel I, 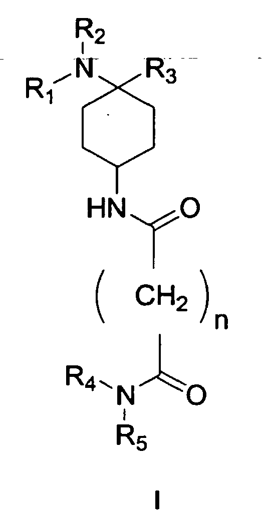 worin
n für 1, 2, 3, 4 oder 5 steht
R¹ und R², unabhängig voneinander für H; C₁₋₅-Alkyl jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, stehen;
oder die Reste R¹ und R² zusammen für CH₂CH₂OCH₂CH₂, CH₂CH₂NR¹⁰CH₂CH₂ oder (CH₂)₃₋₆ stehen,
wobei R¹⁰ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert; C(O)Phenyl, C(O)Heteroaryl, C(O)C₁₋₅-Alkyl, jeweils substituiert oder unsubstituiert, bedeutet;
R³ für C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils einfach oder mehrfach substituiert oder unsubstituiert; Aryl oder Heteroaryl, jeweils unsubstituiert oder einfach oder mehrfach substituiert; über C₁₋₃-Alkyl-Gruppe gebundenes Aryl, Heteroaryl oder C₃₋₈-Cycloalkyl, jeweils unsubstituiert oder einfach oder mehrfach substituiert, steht;
R⁴ für-(CR⁶R⁷)ₚR⁸ steht,
wobei p 0, 1, 2, 3 oder 4 bedeutet
R⁶ H oder C₁₋₅-Alkyl, jeweils gesättigt oder ungesättigt, verzweigt oder unverzweigt, unsubstituiert oder einfach oder mehrfach substituiert, bedeutet
R⁷ H, C₁₋₅-Alkyl, jeweils gesättigt oder ungesättigt, verzweigt oder unverzweigt, unsubstituiert oder einfach oder mehrfach substituiert, oder COOR⁹ bedeutet,
oder R⁶ und R⁷ einen Ring (CH₂)ₖCHR⁸(CH₂)ₘ bilden, mit k = 1, 2, 3, und
m=1,2
R⁸ C₁₋₆-Alkyl, jeweils gesättigt oder ungesättigt, verzweigt oder unverzweigt, unsubstituiert oder einfach oder mehrfach substituiert; C₃₋₈-Cycloalkyl, Aryl oder Heteroaryl, jeweils unsubstituiert oder einfach oder mehfach substituiert, bedeutet,
R⁹ H oder C₁₋₅-Alkyl bedeutet
R⁵ für H oder für -(CH₂)ₗR⁸ steht,
wobei I für 1, 2 oder 3 steht,
oder zusammen mit R⁴ für CH₂CHR¹⁴OCHR¹⁴CH₂, CH₂CH₂SCH₂CH₂, CH₂CH₂NR¹¹CH₂CH₂, (CR¹²R¹³)₃₋₆ oder 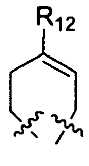 stehen,
wobei R¹¹ H; C₁₋₅-Alkyl, jeweils gesättigt oder ungesättigt, verzweigt oder unverzweigt, einfach oder mehrfach substituiert oder unsubstituiert; C₃₋₈-Cycloalkyl, jeweils gesättigt oder ungesättigt, einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
R¹² H; C₁₋₅-Alkyl, jeweils gesättigt oder ungesättigt, verzweigt oder unverzweigt, einfach oder mehrfach substituiert oder unsubstituiert; OH; C(O)Phenyl, unsubstituiert oder einfach oder mehrfach substituiert; C₃₋₈-Cycloalkyl, jeweils einfach oder mehrfach substituiert oder unsubstituiert; Aryl-, oder Heteroaryl, jeweils einfach oder mehrfach substituiert oder unsubstituiert; oder über C₁₋₃-Alkyl gebundenes Aryl, C₃₋₈-Cycloalkyl oder Heteroaryl, jeweils einfach oder mehrfach substituiert oder unsubstituiert, bedeutet;
und R¹³ H oder OH bedeutet oder mit R¹² zusammen über dasselbe C-Atom (Spiro-Verbindung) oder ein benachbartes C-Atom einen fünfgliedrigen oder sechsgliedrigen Ring bildet, der Heteroatome enthalten, gesättigt oder ungesättigt, substituiert oder unsubstituiert und/oder Teil eines polycyclischen Systems sein kann;
R¹⁴ H oder C₁₋₃-Alkyl, jeweils gesättigt oder ungesättigt, verzweigt oder unverzweigt, einfach oder mehrfach substituiert oder unsubstituiert, bedeutet;
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

2. Substituierte Cyclohexyl-1,4-diamin-Derivate gemäß Anspruch 1, **dadurch gekennzeichnet, dass**
R¹ und R² unabhängig voneinander für H; C₁₋₅-Alkyl, gesättigt oder ungesättigt, verzweigt oder unverzweigt, einfach oder mehrfach substituiert oder unsubstituiert, stehen;
oder die Reste R¹ und R² zusammen einen Ring bilden und CH₂CH₂OCH₂CH₂, CH₂CH₂NR¹⁰CH₂CH₂ oder (CH₂)₃₋₆ bedeuten,
wobei R¹⁰ H; C₁₋₅-Alkyl, gesättigt oder ungesättigt, verzweigt oder unverzweigt, einfach oder mehrfach substituiert oder unsubstituiert, bedeutet.

3. Substituierte Cyclohexyl-1,4-diamin -Derivate gemäß einem der Ansprüche 1 oder 2, **dadurch gekennzeichnet, dass** R¹ und R² unabhängig voneinander für CH₃ oder H stehen, wobei R¹ und R² nicht gleichzeitig H bedeuten, oder R¹ und R² für CH₂CH₂OCH₂CH₂ oder (CH₂)₆ stehen.

4. Substituierte Cyclohexyl-1,4-diamin -Derivate gemäß einem der Ansprüche 1-3 , **dadurch gekennzeichnet, dass**
R³ für Cyclopentyl, Cyclohexyl, Phenyl, Benzyl, Naphthyl, Anthracenyl, Thiophenyl, Benzothiophenyl, Furyl, Benzofuranyl, Benzodioxolanyl, Indolyl, Indanyl, Benzodioxanyl, Pyrrolyl, Pyridyl, Pyrimidyl oder Pyrazinyl, jeweils unsubstituiert oder einfach oder mehrfach substituiert; über eine gesättigte, unverzweigte C₁₋₂-Alkyl-Gruppe gebundenen C₅₋₆-Cycloalkyl, Phenyl, Naphthyl, Anthracenyl, Thiophenyl, Benzothiophenyl, Pyridyl, Furyl, Benzofuranyl, Benzodioxolanyl, Indolyl, Indanyl, Benzodioxanyl, Pyrrolyl, Pyrimidyl oder Pyrazinyl, jeweils unsubstituiert oder einfach oder mehrfach substituiert, steht;
insbesondere
R³ Phenyl, Furyl, Thiophenyl, Naphthyl, Benzyl, Benzofuranyl, Indolyl, Indanyl, Benzodioxanyl, Benzodioxolanyl, Pyridyl, Pyrimidyl, Pyrazinyl oder Benzothiophenyl, jeweils unsubstituiert oder einfach oder mehrfach substituiert; über eine gesättigte, unverzweigte C₁₋₂-Alkyl-Gruppe gebundenen Phenyl, Furyl oder Thiophenyl, jeweils unsubstituiert oder einfach oder mehrfach substituiert, bedeutet.

5. Substituierte Cyclohexyl-1,4-diamin -Derivate gemäß einem der Ansprüche 1-4 , **dadurch gekennzeichnet, dass** R³ für Phenyl, Thiophenyl, Pyridyl oder Benzyl, jeweils substituiert oder unsubstituiert, steht, besonders bevorzugt für 4-Methylbenzyl, 3-Chlorbenzyl, 4-Chlorbenzyl, 3-Methylbenzyl, Benzyl, Phenyl, Thiophenyl und 3-Fluorphenyl.

6. Substituierte Cyclohexyl-1,4-diamin-Derivate gemäß einem der Ansprüche 1-5 , **dadurch gekennzeichnet, dass** n für 2 oder 3 steht.

7. Substituierte Cyclohexyl-1,4-diamin-Derivate gemäß einem der Anspruch 1-6, **dadurch gekennzeichnet, dass** R⁴ für -(CR⁶R⁷)ₚR⁸ und R⁵ für H steht.

8. Substituierte Cyclohexyl-1,4-diamin-Derivate gemäß einem der Ansprüche 1-6, **dadurch gekennzeichnet, dass** R⁴ für (CH₂)ₚR⁸ und R⁵ für-(CH₂)ₗR⁸ steht.

9. Substituierte Cyclohexyl-1,4-diamin-Derivate gemäß einem der Ansprüche 1-6, **dadurch gekennzeichnet, dass** R⁴ und R⁵ zusammen für CH₂CHR¹⁴OCHR¹⁴CH₂, CH₂CH₂SCH₂CH₂, CH₂CH₂NR¹¹CH₂CH₂, (CR¹²R¹³)₃₋₆ oder 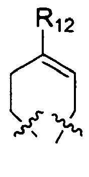 stehen.

10. Substituierte Cyclohexyl-1,4-diamin-Derivate gemäß einem der Ansprüche 1-7, **dadurch gekennzeichnet, dass** R⁶ H und R⁷ H, CH₃, Benzyl, unsubstituiert oder einfach oder mehrfach substituiert, oder COOR⁹ bedeutet oder R⁶ und R⁷ einen Ring (CH₂)ₖCHR⁸(CH₂)ₘ bilden.

11. Substituierte Cyclohexyl-1,4-diamin-Derivate gemäß einem der Ansprüche 1-8 oder 10 **dadurch gekennzeichnet, dass** R⁸ C₁₋₆-Alkyl, jeweils gesättigt oder ungesättigt, verzweigt oder unverzweigt, unsubstituiert oder einfach oder mehrfach substituiert; Cyclobutyl, Cyclopropyl, Cyclopentyl, Cyclohexyl, Cycloheptyl, Cyclooctyl, Anthracenyl, Indolyl, Naphthyl, Benzofuranyl, Benzothiophenyl, Indanyl, Benzodioxanyl, Benzodioxolanyl, Acenaphthyl, Carbazolyl, Phenyl, Thiophenyl, Furyl, Pyrrolidinyl, Tetrahydrofuryl, Piperazinyl, Pyridyl, Pyrrolyl, Pyrazinyl oder Pyrimidyl, Fluorenyl, Fluoranthenyl, Benzothiazolyl, Benzotriazolyl oder Benzo[1,2,5]thiazolyl oder 1,2-Dihydroacenaphtenyl, Pyridinyl, Furanyl, Benzofuranyl, Pyrazolinonyl, Oxopyrazolinonyl, Dioxolanyl, Adamantyl, Pyrimidinyl, Chinolinyl, Isochinolinyl, Phthalazinyl oder Chinazolinyl, jeweils unsubstituiert oder einfach oder mehrfach substituiert, bedeutet.

12. Substituierte Cyclohexyl-1,4-diamin-Derivate gemäß einem der Ansprüche 1-8 oder 10 **dadurch gekennzeichnet, dass** R⁸ C₁₋₆-Alkyl, jeweils gesättigt oder ungesättigt, verzweigt oder unverzweigt, unsubstituiert oder einfach oder mehrfach substituiert; Pyrrolidinyl, Morpholinyl, Tetrahydrofuryl, Tetrahydronaphthyl, Dihydroindolyl, Pyridyl, Thienyl, Piperazinyl, Naphthyl, Indanyl, Chinolinyl, Cyclopropyl, Cyclopentyl, Cyclohexyl, Cycloheptyl, Cyclooctyl, Adamantyl, Furyl, Benzofuryl, Phenyl oder Indolyl, jeweils unsubstituiert oder einfach oder mehrfach substituiert, bedeutet.

13. Substituierte Cyclohexyl-1,4-diamin-Derivate gemäß einem der Ansprüche 1-12 aus der Gruppe
N-[1-(2,6-Dichlor-benzyl)-pyrrolidin-3-yl]-N'-[4-dimethylamino-4-(4-methyl-benzyl)-cyclohexyl]-succinamid
N-[4-(3-Chlor-benzyl)-4-dimethylamino-cyclohexyl]-N'-(2-oxo-tetrahydro-furan-3-yl)-succinamid
N-[4-(4-Chlor-benzyl)-4-dimethylamino-cyclohexyl]-N'-(3,3-diphenyl-propyl)-succinamid
N-[4-Dimethylamino-4-(4-methyl-benzyl)-cyclohexyl]-4-[4-(7-methoxy-benzo[1,3]dioxol-5-ylmethyl)-piperazin-1-yl]-4-oxo-butyramid
N-[4-(4-Chlor-benzyl)-4-dimethylamino-cyclohexyl]-4-(3,6-dihydro-2H-pyridin-1-yl)-4-oxo-butyramid
N-[4-Dimethylamino-4-(4-methyl-benzyl)-cyclohexyl]-4-oxo-4-(4-m-tolyl-piperazin-1-yl)-butyramid
2-{3-[4-(4-Chlor-benzyl)-4-dimethylamino-cyclohexylcarbamoyl]-propionylamino}-propionsäureethylester
N-[4-(3-Chlor-benzyl)-4-dimethylamino-cyclohexyl]-4-(8-fluor-1,3,4,5-tetrahydro-pyrido[4,3-b]indol-2-yl)-4-oxo-butyramid
3-{3-[4-Dimethylamino-4-(4-methyl-benzyl)-cyclohexylcarbamoyl]-propionylamino}-propionsäureethylester
N-[4-Dimethylamino-4-(4-methyl-benzyl)-cyclohexyl]-4-[4-(5-methyl-pyrazin-2-carbonyl)-piperazin-1-yl]-4-oxo-butyramid
N-[4-Dimethylamino-4-(4-methyl-benzyl)-cyclohexyl]-N'-methyl-N'-[2-(2,2,2-trifluoracetylamino)-ethyl]-succinamid
N-[4-(3-Chlor-benzyl)-4-dimethylamino-cyclohexyl]-N'-(2-pyridin-2-yl-ethyl)-succinamid
N-[4-(4-Chlor-benzyl)-4-dimethylamino-cyclohexyl]-4-oxo-4-thiomorpholin-4-yl-butyramid
N-[4-(4-Chlor-benzyl)-4-dimethylamino-cyclohexyl]-4-[4-(5-methyl-pyrazin-2-carbonyl)-piperazin-1-yl]-4-oxo-butyramid
N-[4-Dimethylamino-4-(3-methyl-benzyl)-cyclohexyl]-N'-(2-thiophen-2-yl-ethyl)-succinamid
N-[4-(4-Chlor-benzyl)-4-dimethylamino-cyclohexyl]-4-[4-(4-ethoxy-phenyl)-piperazin-1-yl]-4-oxo-butyramid
N-Allyl-N'-[4-(4-chlor-benzyl)-4-dimethylamino-cyclohexyl]-N-methyl-succinamid
5-[4-(2,5-Dimethoxy-benzyl)-piperazin-1-yl]-5-oxo-valeriansäure (4-benzyl-4-dimethylamino-cyclohexyl)-amid
4-(3,6-Dihydro-2H-pyridin-1-yl)-N-[4-dimethylamino-4-(4-methyl-benzyl)-cyclohexyl]-4-oxo-butyramid
N-[4-Dimethylamino-4-(3-methyl-benzyl)-cyclohexyl]-4-(4-methyl-piperidin-1-yl)-4-oxo-butyramid
N-[4-Dimethylamino-4-(4-methyl-benzyl)-cyclohexyl]-4-oxo-4-(4-pyridin-2-yl-piperazin-1-yl)-butyramid
({3-[4-Dimethylamino-4-(3-methyl-benzyl)-cyclohexylcarbamoyl]-propionyl}-methyl-amino)-essigsäure benzyl ester
4-[4-(2,6-Dichlor-benzyl)-piperazin-1-yl]-N-[4-dimethylamino-4-(3-methyl-benzyl)-cyclohexyl]-4-oxo-butyramid
{[4-(4-Benzyl-4-dimethylamino-cyclohexylcarbamoyl)-butyryl]-methyl-amino}-essigsäure tert-butyl ester
5-[4-(2-Fluor-5-methoxy-benzyl)-piperazin-1-yl]-5-oxo-valeriansäure (4-benzyl-4-dimethylamino-cyclohexyl)-amid
N-[4-(4-Chlor-benzyl)-4-dimethylamino-cyclohexyl]-N'-(3-methoxy-propyl)-succinamid
4-[4-(4-Acetyl-phenyl)-piperazin-1-yl]-N-[4-dimethylamino-4-(4-methyl-benzyl)-cyclohexy-I]=4-oxo-butyramid
4-{3-[4-Dimethylamino-4-(4-methyl-benzyl)-cyclohexylcarbamoyl]-propionyl}-piperazin-1-carbonsäure-*tert*-butylester
N-[4-(3-Chlor-benzyl)-4-dimethylamino-cyclohexyl]-N'-(tetrahydro-furan-2-ylmethyl)-succinamid
2-{3-[4-(3-Chlor-benzyl)-4-dimethylamino-cyclohexylcarbamoyl]-propionylamino}-propionsäureethylester
N-(3-Brom-benzyl)-N'-[4-dimethylamino-4-(3-methyl-benzyl)-cyclohexyl]-succinamid
3-(4-Chlor-phenyl)-2-{3-[4-dimethylamino-4-(4-methyl-benzyl)-cyclohexylcarbamoyl]-propionylamino}-propionsäuremethylester
N-[4-(3-Chlor-benzyl)-4-dimethylamino-cyclohexyl]-4-[4-(5-methyl-pyrazin-2-carbonyl)-piperazin-1-yl]-4-oxo-butyramid
N-[4-(4-Chlor-benzyl)-4-dimethylamino-cyclohexyl]-N'-naphthalen-1-ylmethyl-succinamid
N-[4-Dimethylamino-4-(3-methyl-benzyl)-cyclohexyl]-4-[4-(3-fluor-4-methoxy-benzyl)-piperazin-1-yl]-4-oxo-butyramid
N-[4-Dimethylamino-4-(4-methyl-benzyl)-cyclohexyl]-N'-[2-(1H-indol-3-yl)-ethyl]-N'-methyl-succinamid
(4-{3-[4-(3-Chlor-benzyl)-4-dimethylamino-cyclohexylcarbamoyl]-propionylamino}-benzyl)-phosphonsäurediethylester
N-[4-Dimethylamino-4-(4-methyl-benzyl)-cyclohexyl]-N'-methyl-N'-(2-pyridin-2-yl-ethyl)-succinamid
N-[4-(3-Chlor-benzyl)-4-dimethylamino-cyclohexyl]-4-(3,6-dihydro-2H-pyridin-1-yl)-4-oxo-butyramid
N-[4-Dimethylamino-4-(3-methyl-benzyl)-cyclohexyl]-N'-(1-methyl-3-phenyl-propyl)-succinamid
5-[4-(5-Methyl-pyrazin-2-carbonyl)-piperazin-1-yl]-5-oxo-valeriansäure (4-benzyl-4-dimethylamino-cyclohexyl)-amid
N-[4-(3-Chlor-benzyl)-4-dimethylamino-cyclohexyl]-N'-(2-chlor-6-methyl-benzyl)-succinamid
N-Allyl-N'-[4-(3-chlor-benzyl)-4-dimethylamino-cyclohexyl]-N-methyl-succinamid
N-[4-(4-Chlor-benzyl)-4-dimethylamino-cyclohexyl]-N'-cyclopentyl-succinamid
N-[4-Dimethylamino-4-(4-methyl-benzyl)-cyclohexyl]-N'-(tetrahydro-furan-2-ylmethyl)-succinamid
(4-{3-[4-Dimethylamino-4-(3-methyl-benzyl)-cyclohexylcarbamoyl]-propionylamino}-benzyl)-phosphonsäurediethylester
N-[4-(4-Chlor-benzyl)-4-dimethylamino-cyclohexyl]-4-[4-(2-methoxy-phenyl)-piperazin-1-yl]-4-oxo-butyramid
N-[4-Dimethylamino-4-(3-methyl-benzyl)-cyclohexyl]-4-[4-(3-methoxy-phenyl)-piperazin-1-yl]-4-oxo-butyramid
N-[4-(4-Chlor-benzyl)-4-dimethylamino-cyclohexyl]-N'-(3,5-difluor-benzyl)-succinamid
N-[4-(4-Chlor-benzyl)-4-dimethylamino-cyclohexyl]-N'-(2-methyl-benzyl)-succinamid
N-[4-Dimethylamino-4-(4-methyl-benzyl)-cyclohexyl]-4-oxo-4-{4-[(2,2,2-trifluor-acetylamino)-methyl]-piperidin-1-yl}-butyramid
N-[4-(4-Chlor-benzyl)-4-dimethylamino-cyclohexyl]-N'-[2-(1H-indol-3-yl)-ethyl]-succinamid
N-[4-(3-Chlor-benzyl)-4-dimethylamino-cyclohexyl]-N'-[2-(1H-indol-3-yl)-ethyl]-N'-methyl-succinamid
5-[4-(7-Methoxy-benzo[1,3]dioxol-5-ylmethyl)-piperazin-1-yl]-5-oxo-valeriansäure (4-benzyl-4-dimethylamino-cyclohexyl)-amid
N-Benzyl-N'-[4-dimethylamino-4-(3-methyl-benzyl)-cyclohexyl]-N-(2-hydroxy-ethyl)-succinamid
5-[4-(2,3-Dimethyl-phenyl)-piperazin-1-yl]-5-oxo-valeriansäure (4-benzyl-4-dimethylamino-cyclohexyl)-amid
N-[4-(4-Chlor-benzyl)-4-dimethylamino-cyclohexyl]-N'-[2-(1H-indol-3-yl)-ethyl]-N'-methyl-succinamid
N-[4-Dimethylamino-4-(4-mehtyl-benzyl) cyclohexyl] 1-[1-(2,3-dimethyl-phenyl)-piperazin-1-yl]-4-oxo-butyramid
Glutarsäure-(4-benzyl-4-dimethylamino-cyclohexyl)-amid (1,2,3,4-tetrahydro-naphthalen-1-yl)-amid
N-[4-(4-Chlor-benzyl)-4-dimethylamino-cyclohexyl]-4-(4-methyl-piperidin-1-yl)-4-oxo-butyramid
2-{3-[4-Dimethylamino-4-(4-methyl-benzyl)-cyclohexylcarbamoyl]-propionylamino}-propionsäure-tert-butylester
5-(4-Cycloheptyl-piperazin-1-yl)-5-oxo-valeriansäure (4-benzyl-4-dimethylamino-cyclohexyl)-amid
Glutarsäure-(4-benzyl-4-dimethylamino-cyclohexyl)-amid [2-(4-fluor-phenyl)-ethyl]-amid
Glutarsäure-(4-benzyl-4-dimethylamino-cyclohexyl)-amid [1-(2,6-dichlor-benzyl)-pyrrolidin-3-yl]-amid
N-[4-(4-Chlor-benzyl)-4-dimethylamino-cyclohexyl]-4-(1,4-dioxa-8-aza-spiro[4.5]dec-8-yl)-4-oxo-butyramid
N-[4-(3-Chlor-benzyl)-4-dimethylamino-cyclohexyl]-4-oxo-4-thiomorpholin-4-yl-butyramid
4-(4-Benzoyl-piperidin-1-yl)-N-[4-dimethylamino-4-(3-methyl-benzyl)-cyclohexyl]-4-oxo-butyramid
N-[4-(4-Chlor-benzyl)-4-dimethylamino-cyclohexyl]-N'-(2,4-dimethoxy-benzyl)-succinamid
N-[4-Dimethylamino-4-(3-methyl-benzyl)-cyclohexyl]-N'-(1,2,3,4-tetrahydro-naphthalen-1-yl)-succinamid
N-[4-(4-Chlor-benzyl)-4-dimethylamino-cyclohexyl]-N'-ethyl-N'-pyridin-4-ylmethyl-succinamid
N-[2-(3,4-Dimethoxy-phenyl)-ethyl]-N'-[4-dimethylamino-4-(3-methyl-benzyl)-cyclohexyl]-N-methyl-succinamid
N-[4-(4-Chlor-benzyl)-4-dimethylamino-cyclohexyl]-N'-methyl-N'-pyridin-3-ylmethyl-succinamid
N-[4-(3-Chlor-benzyl)-4-dimethylamino-cyclohexyl]-N'-pyridin-2-ylmethyl-succinamid
N-[4-(3-Chlor-benzyl)-4-dimethylamino-cyclohexyl]-N'-(2-methyl-benzyl)-succinamid
N-[4-Dimethylamino-4-(3-methyl-benzyl)-cyclohexyl]-N'-(4-methoxy-phenyl)-succinamid
N-[4-Dimethylamino-4-(3-methyl-benzyl)-cyclohexyl]-N'-indan-2-yl-succinamid
N-[4-(3-Chlor-benzyl)-4-dimethylamino-cyclohexyl]-N'-(1-methyl-3-phenyl-propyl)-succinamid
N-(2-Chlor-6-methyl-benzyl)-N'-[4-dimethylamino-4-(3-methyl-benzyl)-cyclohexyl]-succinamid
N-[4-Dimethylamino-4-(4-methyl-benzyl)-cyclohexyl]-4-[4-(4-methoxy-phenyl)-piperazin-1-yl]-4-oxo-butyramid
N-[4-Dimethylamino-4-(4-methyl-benzyl)-cydohexyl]-N'-[2-(4-fluor-phenyl)-ethyl]-succinamid
Glutarsäure-(4-benzyl-4-dimethylamino-cyclohexyl)-amid 4-dimethylamino-benzylamid
N-[4-(3-Chlor-benzyl)-4-dimethylamino-cyclohexyl]-N'-(3,5-dichlor-benzyl)-succinamid
N-[4-(4-Chlor-benzyl)-4-dimethylamino-cyclohexyl]-N'-(1-methyl-3-phenyl-propyl)-succinamid
4-{3-[4-(3-Chlor-benzyl)-4-dimethylamino-cyclohexylcarbamoyl]-propionyl}-piperazin-1-carbonsäureethylester
N-[4-Dimethylamino-4-(3-methyl-benzyl)-cyclohexyl]-N'-(4-methoxy-benzyl)-succinamid
N-[4-(4-Chlor-benzyl)-4-dimethylamino-cyclohexyl]-4-(2,6-dimethyl-morpholin-4-yl)-4-oxo-butyramid
N-[4-Dimethylamino-4-(4-methyl-benzyl)-cyclohexyl]-4-oxo-4-(4-phenyl-3,6-dihydro-2H-pyridin-1-yl)-butyramid
N-[4-(4-Chlor-benzyl)-4-dimethylamino-cyclohexyl]-N'-[2-(3-fluor-phenyl)-ethyl]-succinamid
4-[4-(4-Allyloxy-benzyl)-piperazin-1-yl]-N-[4-dimethylamino-4-(3-methyl-benzyl)-cyclohexyl]-4-oxo-butyramid
Glutarsäure-(4-benzyl-4-dimethylamino-cyclohexyl)-amid [2-(5-brom-2-methoxy-phenyl)-ethyl]-amid
5-[4-(3-Methoxy-phenyl)-piperazin-1-yl]-5-oxo-valeriansäure (4-benzyl-4-dimethylamino-cyclohexyl)-amid
N-[4-(3-Chlor-benzyl)-4-dimethylamino-cyclohexyl]-4-(4-methyl-piperidin-1-yl)-4-oxo-butyramid
(4-{3-[4-(4-Chlor-benzyl)-4-dimethylamino-cyclohexylcarbamoyl]-propionylamino}-benzyl)-phosphonsäurediethylester
N-[4-Dimethylamino-4-(4-methyl-benzyl)-cyclohexyl]-4-oxo-4-(4-o-tolyl-piperazin-1-yl)-butyramid
N-[4-Dimethylamino-4-(3-methyl-benzyl)-cyclohexyl]-N'-[2-(3-fluor-phenyl)-ethyl]-succinamid
4-[4-(4-Chlor-phenyl)-piperazin-1-yl]-N-[4-dimethylamino-4-(4-methyl-benzyl)-cyclohexyl]-4-oxo-butyramid
N-[4-(3-Chlor-benzyl)-4-dimethylamino-cyclohexyl]-N'-[1-(4-fluor-phenyl)-ethyl]-succinamid
N-[4-(4-Chlor-benzyl)-4-dimethylamino-cyclohexyl]-N'-[1-(4-trifluormethyl-benzyl)-pyrrolidin-3-yl]-succinamid
N-[4-(3-Chlor-benzyl)-4-dimethylamino-cyclohexyl]-N'-[2-(1H-indol-3-yl)-ethyl]-succinamid
N-[4-(4-Chlor-benzyl)-4-dimethylamino-cyclohexyl]-N'-indan-5-yl-succinamid
N-(4-Benzyloxy-phenyl)-N'-[4-dimethylamino-4-(4-methyl-benzyl)-cyclohexyl]-succinamid
2-{3-[4-Dimethylamino-4-(3-methyl-benzyl)-cyclohexylcarbamoyl]-propionylamino}-3-methyl-valeriansäure tert-butyl ester
4-(4-Benzyl-piperidin-1-yl)-N-[4-dimethylamino-4-(3-methyl-benzyl)-cyclohexyl]-4-oxo-butyramid
N-[4-(4-Chlor-benzyl)-4-dimethylamino-cyclohexyl]-4-[4-(4-fluor-phenyl)-3,6-dihydro-2H-pyridin-1-yl]-4-oxo-butyramid
N-[4-Dimethylamino-4-(3-methyl-benzyl)-cyclohexyl]-N'-(1,2-dimethyl-propyl)succinamid
N-[4-Dimethylamino-4-(3-methyl-benzyl)-cyclohexyl]-4-(8-fluor-1,3,4,5-tetrahydro-pyrido[4,3-b]indol-2-yl)-4-oxo-butyramid
4-[4-(3-Chlor-benzoyl)-piperazin-1-yl]-N-[4-dimethylamino-4-(3-methyl-benzyl)-cyclohexyl]-4-oxo-butyramid
N-[4-(4-Chlor-benzyl)-4-dimethylamino-cyclohexyl]-N'-(4-methoxy-phenyl)-succinamid
5-[4-(2,6-Dichlor-benzyl)-piperazin-1-yl]-5-oxo-valeriansäure (4-benzyl-4-dimethylamino-cyclohexyl)-amid
N-[4-Dimethylamino-4-(3-methyl-benzyl)-cyclohexyl]-N'-[2-(1H-indol-3-yl)-ethyl]-N'-methyl-succinamid
N-[4-Dimethylamino-4-(3-methyl-benzyl)-cyclohexyl]-N'-[1-(4-fluor-phenyl)-ethyl]-succinamid
4-[4-(4-Chlor-phenyl)-piperazin-1-yl]-N-[4-dimethylamino-4-(3-methyl-benzyl)-cyclohexyl]-4-oxo-butyramid
N-[4-(3-Chlor-benzyl)-4-dimethylamino-cyclohexyl]-4-oxo-4-(4-oxo-1-phenyl-1,3,8-triaza-spiro[4.5]dec-8-yl)-butyramid
N-Benzyl-N'-[4-dimethylamino-4-(3-methyl-benzyl)-cyclohexyl]-N-methyl-succinamid
N-[4-(3-Chlor-benzyl)-4-dimethylamino-cyclohexyl]-N'-(3,5-difluor-benzyl)-succinamid
N-[4-Dimethylamino-4-(3-methyl-benzyl)-cyclohexyl]-N'-[2-(4-fluor-phenyl)-ethyl]-succinamid
N-[4-Dimethylamino-4-(3-methyl-benzyl)-cyclohexyl]-N'-[2-(1H-indol-3-yl)-ethyl]-succinamid
N-(2-Benzylsulfinyl-ethyl)-N'-[4-dimethylamino-4-(3-methyl-benzyl)-cyclohexyl]-succinamid
N-[4-(3-Chlor-benzyl)-4-dimethylamino-cyclohexyl]-N'-(2,4-difluor-benzyl)-succinamid
N-[4-(3-Chlor-benzyl)-4-dimethylamino-cyclohexyl]-N'-phenethyl-succinamid
N-[4-Dimethylamino-4-(3-methyl-benzyl)-cyclohexyl]-N'-(1-naphthalen-2-yl-ethyl)-succinamid
N-[4-(3-Chlor-benzyl)-4-dimethylamino-cyclohexyl]-N'-(1-methoxymethyl-2-phenyl-ethyl)-succinamid
N-[4-(3-Chlor-benzyl)-4-dimethyfamino-cyclohexyl]-N'-(3,4-dichlor-benzyl)-succinamid
N-[4-(3-Chlor-benzyl)-4-dimethylamino-cyclohexyl]-N'-furan-2-ylmethyl-succinamid
N-[4-Dimethylamino-4-(4-methyl-benzyl)-cyclohexyl]-N'-(1,2,3,4-tetrahydro-naphthalen-1 -yl)-succinamid
N-[4-(3-Chlor-benzyl)-4-dimethylamino-cyclohexyl]-N'-(4-fluor-benzyl)-succinamid
N-[4-Dimethylamino-4-(3-methyl-benzyl)-cyclohexyl]-N'-indan-1-yl-succinamid
N-[4-(3-Chlor-benzyl)-4-dimethylamino-cyclohexyl]-4-(3,5-dimethyl-piperidin-1-yl)-4-oxo-butyramid
N-[4-Dimethylamino-4-(3-methyl-benzyl)-cyclohexyl]-N'-phenethyl-succinamid
N-[4-Dimethylamino-4-(4-methyl-benzyl)-cyclohexyl]-N'-phenethyl-succinamid
N-(2-Benzylsulfanyl-ethyl)-N'-[4-(3-chlor-benzyl)-4-dimethylamino-cyclohexyl]-succinamid
N-[4-(3-Chlor-benzyl)-4-dimethylamino-cyclohexyl]-N'-(2,5-dichlor-benzyl)-succinamid
4-[4-(3-Chlor-benzoyl)-piperazin-1-yl]-N-[4-(4-chlor-benzyl)-4-dimethylamino-cyclohexyl]-4-oxo-butyramid
N-[4-(4-Chlor-benzyl)-4-dimethylamino-cyclohexyl]-N'-(1-phenyl-propyl)-succinamid
N-(4-Brom-2-fluor-benzyl)-N'-[4-dimethylamino-4-(3-methyl-benzyl)-cyclohexyl]-succinamid
N-[4-(3-Chlor-benzyl)-4-dimethylamino-cyclohexyl]-N'-methyl-N'-pyridin-3-ylmethyl-succinamid
N-[4-Dimethylamino-4-(4-methyl-benzyl)-cyclohexyl]-N'-(2-ethoxy-benzyl)-succinamid
N-[4-Dimethylamino-4-(3-methyl-benzyl)-cyclohexyl]-N'-[2-(5-methoxy-1H-indol-3-yl)-ethyl]-succinamid
N-(4-sec-Butyl-phenyl)-N'-[4-(4-chlor-benzyl)-4-dimethylamino-cyclohexyl]-succinamid
5-Oxo-5-(4-phenyl-3,6-dihydro-2H-pyridin-1-yl)-valeriansäure (4-benzyl-4-dimethylamino-cyclohexyl)-amid
5-Oxo-5-(4-phenyl-3,6-dihydro-2H-pyridin-1-yl)-valeriansäure (4-benzyl-4-dimethylamino-cyclohexyl)-amid
N-[4-(3-Chlor-benzyl)-4-dimethylamino-cyclohexyl]-N'-[2-(3-fluor-phenyl)-ethyl]-succinamid
N-(3-Brom-benzyl)-N'-[4-(3-chlor-benzyl)-4-dimethylamino-cyclohexyl]-succinamid
N-(4-Brom-2-fluor-benzyl)-N'-[4-(3-chlor-benzyl)-4-dimethylamino-cyclohexyl]-succinamid
N-[4-(3-Chlor-benzyl)-4-dimethylamino-cyclohexyl]-N'-(1-phenyl-propyl)-succinamid
N-[4-Dimethylamino-4-(3-methyl-benzyl)-cyclohexyl]-N'-(4-fluor-2-trifluormethyl-benzyl)-succinamid
N-[4-(3-Chlor-benzyl)-4-dimethylamino-cyclohexyl]-N'-indan-2-yl-succinamid
N-[4-Dimethylamino-4-(3-methyl-benzyl)-cyclohexyl]-4-oxo-4-(4-phenyl-3,6-dihydro-2H-pyridin-1-yl)-butyramid
N-[4-(4-Chlor-benzyl)-4-dimethylamino-cyclohexyl]-N'-[2-(2-fluor-phenyl)-ethyl]-succinamid
N-[4-Dimethylamino-4-(4-methyl-benzyl)-cyclohexyl]-N'-[2-(2-fluor-phenyl)-ethyl]-succinamid
Glutarsäure-(4-benzyl-4-dimethylamino-cyclohexyl)-amid [2-(2-fluor-phenyl)-ethyl]-amid
N-[2-(2-Chlor-phenyl)-ethyl]-N'-[4-dimethylamino-4-(4-methyl-benzyl)-cyclohexyl]-succinamid
N-[4-(3-Chlor-benzyl)-4-dimethylamino-cyclohexyl]-N'-(3,4-difluor-benzyl)-succinamid
N-Benzyl-N-(2-cyano-ethyl)-N'-[4-dimethylamino-4-(4-methyl-benzyl)-cyclohexyl]-succinamid
N-[4-(3-Chlor-benzyl)-4-dimethylamino-cyclohexyl]-N'-(3-methyl-benzyl)-succinamid
N-[4-(3-Chlor-benzyl)-4-dimethylamino-cyclohexyl]-N'-(4-fluor-3-trifluormethyl-benzyl)-succinamid
N-[4-(3-Chlor-benzyl)-4-dimethylamino-cyclohexyl]-N'-[2-(2-chlor-phenyl)-ethyl]-succinamid
N-[4-(3-Chlor-benzyl)-4-dimethylamino-cyclohexyl]-N'-[2-(4-chlor-phenyl)-ethyl]-succinamid
N-[4-(3-Chlor-benzyl)-4-dimethylamino-cydohexyl]-N'-[2-(2,4-dichlor-phenyl)-ethyl]-succinamid
N-[4-(3-Chlor-benzyl)-4-dimethylamino-cyclohexyl]-N'-indan-1-yl-succinamid
N-[4-(4-Chlor-benzyl)-4-dimethylamino-cyclohexyl]-N'-indan-1-yl-succinamid
N-[2-(4-Chlor-phenyl)-propyl]-N'-[4-dimethylamino-4-(3-methyl-benzyl)-cyclohexyl]-succinamid
N-(3-Brom-benzyl)-N'-[4-(4-chlor-benzyl)-4-dimethylamino-cyclohexyl]-succinamid
N-[4-(3-Chlor-benzyl)-4-dimethylamino-cyclohexyl]-N'-(3-phenyl-propyl)-succinamid
N-[4-Dimethylamino-4-(4-methyl-benzyl)-cyclohexyl]-N'-(2-p-tolyl-ethyl)-succinamid
N-[4-(3-Chlor-benzyl)-4-dimethylamino-cyclohexyl]-N'-(2-thiophen-2-yl-ethyl)-succinamid
Glutarsäure-(4-benzyl-4-dimethylamino-cyclohexyl)-amid [2-(4-chlor-phenyl)-propyl]-amid
N-Benzyl-N'-[4-(3-chlor-benzyl)-4-dimethylamino-cyclohexyl]-N-methyl-succinamid
N-[4-(3-Chlor-benzyl)-4-dimethylamino-cyclohexyl]-N'-[2-(3-chlor-phenyl)-ethyl]-succinamid
Glutarsäure-(4-benzyl-4-dimethylamino-cyclohexyl)-amid [2-(2-ethoxy-phenyl)-ethyl]-amid
Glutarsäure-(4-benzyl-4-dimethylamino-cyclohexyl)-amid phenethyl-amid
Glutarsäure-(4-benzyl-4-dimethylamino-cyclohexyl)-amid [2-(3-chlor-phenyl)-ethyl]-amid
N-[4-(3-Chlor-benzyl)-4-dimethylamino-cyclohexyl]-N'-[1-(3-methoxy-phenyl)-ethyl]-succinamid
Glutarsäure-(4-benzyl-4-dimethylamino-cyclohexyl)-amid (1,3-dimethyl-butyl)-amid
N-Benzyl-N'-[4-dimethylämino-4-(3-methyl-benzyl)-cyclohexyl]-N-ethyl-succinamid
N-(4-Chlor-benzyl)-N'-[4-(3-chlor-benzyl)-4-dimethylamino-cyclohexyl]-succinamid
N-[4-(3-Chlor-benzyl)-4-dimethylamino-cyclohexyl]-N'-[2-(2-fluor-phenyl)-ethyl]-succinamid
N-[4-Dimethylamino-4-(3-methyl-benzyl)-cyclohexyl]-N'-(2-fluor-benzyl)-succinamid
N-[2-(4-Chlor-phenyl)-ethyl]-N'-[4-dimethylamino-4-(3-methyl-benzyl)-cyclohexyl]-succinamid
N-[2-(2-Chlor-phenyl)-ethyl]-N'-[4-dimethylamino-4-(3-methyl-benzyl)-cyclohexyl]-succinamid
N-[2-(3-Chlor-phenyl)-ethyl]-N'-[4-dimethylamino-4-(3-methyl-benzyl)-cyclohexyl]-succinamid
N-Benzyl-N'-[4-(3-chlor-benzyl)-4-dimethylamino-cyclohexyl]-N-(2-cyano-ethyl)-succinamid
N-[4-(3-Chlor-benzyl)-4-dimethylamino-cyclohexyl]-N'-(3-chlor-4-fluor-benzyl)-succinamid
N-[4-Dimethylamino-4-(3-methyl-benzyl)-cyclohexyl]-N'-(2-trifluormethyl-benzyl)-succinamid
N-[2-(2,4-Dichlor-phenyl)-ethyl]-N'-[4-dimethylamino-4-(3-methyl-benzyl)-cyclohexyl]-succinamid
N-Benzyl-N'-[4-(3-chlor-benzyl)-4-dimethylamino-cyclohexyl]-N-ethyl-succinamid
N-[2-(4-Chlor-phenyl)-ethyl]-N'-[4-dimethylamino-4-(4-methyl-benzyl)-cyclohexyl]-succinamid
5-Oxo-5-(4-o-tolyl-piperazin-1-yl)-valeriansäure (4-benzyl-4-dimethylamino-cyclohexyl)-amid
N-[4-(3-Chlor-benzyl)-4-dimethylamino-cyclohexyl]-N'-[3-(methyl-phenyl-amino)-propyl]-succinamid
N-[4-(3-Chlor-benzyl)-4-dimethylamino-cyclohexyl]-4-[4-(5-chlor-2-methyl-phenyl)-piperazin-1-yl]-4-oxo-butyramid
N-[4-Dimethylammo-4-(3-methyl-benzyl)-cyclohexyl]-N'-[2-(2-ethoxy-phenyl)-ethyl]-succinamid
4-[4-(4-Chlor-benzoyl)-piperidin-1-yl]-N-[4-(3-chlor-benzyl)-4-dimethylamino-cyclohexyl]-4-oxo-butyramid
N-[4-(3-Chlor-benzyl)-4-dimethylamino-cyclohexyl]-N'-[2-(2-ethoxy-phenyl)-ethyl]-succinamid
N-[2-(2,6-Dichlor-phenyl)-ethyl]-N'-[4-dimethylamino-4-(3-methyl-benzyl)-cyclohexyl]-succinamid
N-Benzhydryl-N'-[4-(3-chlor-benzyl)-4-dimethylamino-cyclohexyl]-succinamid
N-[4-(3-Chlor-benzyl)-4-dimethylamino-cyclohexyl]-N'-cyclooctyl-succinamid
N-[4-(3-Chlor-benzyl)-4-dimethylamino-cyclohexyl]-N'-[2-(4-phenoxy-phenyl)-ethyl]-succinamid
N-[4-Dimethylamino-4-(3-methyl-benzyl)-cyclohexyl]-N'-(2,3-dimethyl-benzyl)-succinamid
4-[4-(5-Chlor-2-methyl-phenyl)-piperazin-1-yl]-N-[4-dimethylamino-4-(3-methyl-benzyl)-cyclohexyl]-4-oxo-butyramid
4-[4-(4-Chlor-benzoyl)-piperidin-1-yl]-N-[4-dimethylamino-4-(3-methyl-benzyl)-cyclohexyl]-4-oxo-butyramid
N-[4-(3-Chlor-benzyl)-4-dimethylamino-cyclohexyl]-N'-(1,2,3,4-tetrahydro-naphthalen-1-yl)-succinamid
N-(2-Chlor-benzyl)-N'-[4-dimethylamino-4-(3-methyl-benzyl)-cyclohexyl]-succinamid
N-(2-Chlor-benzyl)-N'-[4-(3-chlor-benzyl)-4-dimethylamino-cyclohexyl]-succinamid
N-[4-(3-Chlor-benzyl)-4-dimethylamino-cyclohexyl]-N'-(3-fluor-5-trifluormethyl-benzyl)-succinamid
N-[4-(3-Chlor-benzyl)-4-dimethylamino-cyclohexyl]-N'-(1-p-tolyl-ethyl)-succinamid
N-[2-(2,6-Dichlor-benzylsulfanyl)-ethyl]-N'-[4-dimethylamino-4-(4-methyl-benzyl)-cyclohexyl]-succinamid
N-[4-(3-Chlor-benzyl)-4-dimethylamino-cyclohexyl]-N'-[2-(2,6-dichlor-benzylsulfanyl)-ethyl]-succinamid
N-[4-(3-Chlor-benzyl)-4-dimethylamino-cyclohexyl]-N'-[2-(3-trifluormethyl-phenyl)-ethyl]-succinamid
N-[2-(3,4-Dichlor-phenyl)-ethyl]-N'-[4-dimethylamino-4-(3-methyl-benzyl)-cyclohexyl]-succinamid
N-[4-(3-Chlor-benzyl)-4-dimethylamino-cyclohexyl]-4-[4-(2-fluor-phenyl)-piperazin-1-yl]-4-oxo-butyramid
5-[4-(2,4-Dimethoxy-phenyl)-piperazin-1-yl]-5-oxo-valeriansäure (4-benzyl-4-dimethylamino-cyclohexyl)-amid
N-[4-(3-Chlor-benzyl)-4-dimethylamino-cyclohexyl]-N'-(1-naphthalen-1-yl-ethyl)-succinamid
N-[4-Dimethylamino-4-(3-methyl-benzyl)-cyclohexyl]-N'-(1-naphthalen-1-yl-ethyl)-succinamid
N-[4-(3-Chlor-benzyl)-4-dimethylamino-cyclohexyl]-N'-(2-fluor-benzyl)-N'-furan 2-ylmethyl-succinamid
N-[4-Dimethylamino-4-(3-methyl-benzyl)-cyclohexyl]-N'-(2-fluor-benzyl)-N'-furan-2-ylmethyl-succinamid
N-[4-(3-Chlor-benzyl)-4-dimethylamino-cyclohexyl]-N'-(2,5-dimethoxy-benzyl)-N'-furan-2-ylmethyl-succinamid
N-(4-Azepan-1-yl-4-benzyl-cyclohexyl)-4-(2,3-dihydro-indol-1-yl)-4-oxo-butyramid
4-(2,3-Dihydro-indol-1-yl)-N-[4-dimethylamino-4-(3-fluor-phenyl)-cyclohexyl]-4-oxo-butyramid
N-(4-Benzyl-4-morpholin-4-yl-cyclohexyl)-N'-(2-chlor-6-methyl-benzyl)-succinamid
N-(4-Benzyl-4-morpholin-4-yl-cyclohexyl)-N'-[2-(3-methoxy-phenyl)-ethyl]-succinamid
N-(4-Benzyl-4-morpholin-4-yl-cyclohexyl)-N'-(3-methyl-benzyl)-succinamid
N-(4-Benzyl-4-morpholin-4-yl-cyclohexyl)-N'-(4-brom-2-fluor-benzyl)-succinamid
N-(4-Benzyl-4-morpholin-4-yl-cyclohexyl)-N'-pyridin-4-ylmethyl-succinamid
N-(4-Benzyl-4-morpholin-4-yl-cyclohexyl)-N'-indan-1-yl-succinamid
N-(4-Benzyl-4-morpholin-4-yl-cyclohexyl)-N'-(2,4-dimethoxy-benzyl)-succinamid
N-(4-Benzyl-4-morpholin-4-yl-cyclohexyl)-N'-thiophen-2-ylmethyl-succinamid
N-Adamantan-1-ylmethyl-N'-(4-benzyl-4-morpholin-4-yl-cyclohexyl)-succinamid
N-(4-Benzyl-4-morpholin-4-yl-cyclohexyl)-N'-(2,5-difluor-benzyl)-succinamid
N-(4-Benzyl-4-morpholin-4-yl-cyclohexyl)-N'-(4-tert-butyl-phenyl)-succinamid
N-(4-Benzyl-4-morpholin-4-yl-cyclohexyl)-N'-(3,5-bis-trifluormethyl-benzyl)-succinamid
N-(4-Benzyl-4-morpholin-4-yl-cyclohexyl)-N'-[2-(2-ethoxy-phenyl)-ethyl]-succinamid
N-(4-Benzyl-4-morpholin-4-yl-cyclohexyl)-N'-(3-methoxy-propyl)-succinamid
N-(4-Benzyl-4-morpholin-4-yl-cyclohexyl)-N'-(2-morpholin-4-yl-ethyl)-succinamid
N-(4-Benzyl-4-morpholin-4-yl-cyclohexyl)-N'-(2,5-dichlor-benzyl)-succinamid
N-(4-Benzyl-4-morpholin-4-yl-cyclohexyl)-N'-[1-(2-benzyloxy-benzyl)-pyrrolidin-3-yl]-N'-methyl-succinamid
N-(4-Benzyl-4-morpholin-4-yl-cyclohexyl)-4-oxo-4-(2-pyrrolidin-1-ylmethyl-pyrrolidin-1-yl)-butyramid
N-(4-Benzyl-4-morpholin-4-yl-cyclohexyl)-4-morpholin-4-yl-4-oxo-butyramid
N-(4-Benzyl-4-morpholin-4-yl-cyclohexyl)-N'-(3-dimethylamino-propyl)-N'-methyl-succinamid
N-(4-Benzyl-4-morpholin-4-yl-cyclohexyl)-N'-(2-phenyl-cyclopropyl)-succinamid
N-(4-Benzyl-4-morpholin-4-yl-cyclohexyl)-N'-naphthalen-1-ylmethyl-succinamid
N-(4-Benzyl-4-morpholin-4-yl-cyclohexyl)-4-(4-methyl-[1,4]diazepan-1-yl)-4-oxo-butyramid
N-(4-Benzyl-4-morpholin-4-yl-cyclohexyl)-4-(4-hydroxy-piperidin-1-yl)-4-oxo-butyramid
N-(4-Benzyl-4-morpholin-4-yl-cyclohexyl)-N'-[2-(7-methyl-1H-indol-3-yl)-ethyl]-succinamid
N-(4-Benzyl-4-morpholin-4-yl-cyclohexyl)-N'-[2-(5-methoxy-1H-indol-3-yl)-ethyl]-succinamid
N-(4-Benzyl-4-morpholin-4-yl-cyclohexyl)-4-oxo-4-(4-chinolin-2-ylmethyl-piperazin-1-yl)-butyramid
N-(4-Dimethylamino-4-thiophen-2-yl-cyclohexyl)-4-oxo-4-[4-(5-trifluormethyl-pyridin-2-yl)-piperazin-1-yl]-butyra mid
Glutarsäure-(1-adamantan-1-yl-ethyl)-amid (4-dimethylamino-4-thiophen-2-yl-cyclohexyl)-amid
N-[2-(2,6-Dichlor-phenyl)-ethyl]-N'-(4-dimethylamino-4-thiophen-2-yl-cyclohexyl)-succinamid
N-(4-Dimethylamino-4-thiophen-2-yl-cyclohexyl)-N'-(1-methyl-3-phenyl-propyl)-succinamid
N-Benzyl-N'-(4-dimethylamino-4-thiophen-2-yl-cyclohexyl)-N-ethyl-succinamid
N-[2-(4-Brom-phenyl)-ethyl]-N'-(4-dimethylamino-4-thiophen-2-yl-cyclohexyl)-succinamid
4-[4-(4-Chlor-benzoyl)-piperidin-1-yl]-N-(4-dimethylamino-4-thiophen-2-yl-cyclohexyl)-4-oxo-butyramid
Glutarsäure-[1-(2,6-dichlor-benzyl)-pyrrolidin-3-yl]-amid (4-dimethylamino-4-thiophen-2-yl-cyclohexyl)-amid
Glutarsäure-(4-dimethylamino-4-thiophen-2-yl-cyclohexyl)-amid (3-phenyl-propyl)-amid
N-(4-Dimethylamino-4-phenyl-cyclohexyl)-N'-pentyl-succinamid
3-(4-Chlor-phenyl)-2-[4-(4-dimethylamino-4-thiophen-2-yl-cyclohexylcarbamoyl)-butyrylamino]-propionsäureethylester
Glutarsäure-(4-dimethylamino-4-thiophen-2-yl-cyclohexyl)-amid indan-1-ylamid
N-Adamantan-1-ylmethyl-N'-(4-dimethylamino-4-thiophen-2-yl-cyclohexyl)-succinamid
5-(4-Benzofuran-2-ylmethyl-piperazin-1-yl)-5-oxo-valeriansäure (4-dimethylamino-4-thiophen-2-yl-cyclohexyl)-amid
4-[4-(4-tert-Butyl-benzyl)-piperazin-1-yl]-N-(4-dimethylamino-4-phenyl-cyclohexyl)-4-oxo-butyramid
Glutarsäure-(4-dimethylamino-4-thiophen-2-yl-cyclohexyl)-amid [2-(1H-indol-3-yl)-ethyl]-amid
N-(4-Dimethylamino-4-phenyl-cyclohexyl)-4-[4-hydroxy-4-(3-trifluormethyl-phenyl)-piperidin-1-yl]-4-oxo-butyramid
5-(3,4-Dihydro-1 H-isochinolin-2-yl)-5-oxo-valeriansäure (4-dimethylamino-4-thiophen-2-yl-cyclohexyl)-amid
4-(4-Benzyl-piperidin-1-yl)-N-(4-dimethylamino-4-phenyl-cyclohexyl)-4-oxo-butyramid
{[4-(4-Dimethylamino-4-thiophen-2-yl-cyclohexylcarbamoyl)-butyryl]-methyl-amino}-essigsäurebenzylester
N-Benzyl-N'-(4-dimethylamino-4-phenyl-cyclohexyl)-N-phenethyl-succinamid
Glutarsäure-(4-dimethylamino-4-thiophen-2-yl-cyclohexyl)-amid 3-trifluormethoxy-benzylamid
Glutarsäure-(4-dimethylamino-4-thiophen-2-yl-cyclohexyl)-amid ethyl-(2-methyl-allyl)-amid
N,N-Bis-(2-cyano-ethyl)-N'-(4-dimethylamino-4-thiophen-2-yl-cyclohexyl)-succinamid
Glutarsäure-benzyl-methyl-amid (4-dimethylamino-4-thiophen-2-yl-cyclohexyl)-amid
Glutarsäure-(2-benzyloxy-cyclopentyl)-amid (4-dimethylamino-4-thiophen-2-yl-cyclohexyl)-amid
Glutarsäure-(4-dimethylamino-4-thiophen-2-yl-cyclohexyl)-amid 4-trifluormethyl-benzylamid
N-(4-Dimethylamino-4-thiophen-2-yl-cyclohexyl)-N'-[2-(4-phenoxy-phenyl)-ethyl]-succinamid
Glutarsäure-(4-dimethylamino-4-thiophen-2-yl-cyclohexyl)-amid (3,3-diphenyl-propyl)-amid
4-(4-Benzyl-piperidin-1-yl)-N-(4-dimethylamino-4-thiophen-2-yl-cyclohexyl)-4-oxo-butyramid
N-(4-Dimethylamino-4-thiophen-2-yl-cyclohexyl)-4-[4-hydroxy-4-(3-trifluormethyl-phenyl)-piperidin-1-yl]-4-oxo-butyramid
Glutarsäure-(4-dimethylamino-4-thiophen-2-yl-cyclohexyl)-amid 4-fluor-2-trifluormethyl-benzylamid
N-(4-Dimethylamino-4-thiophen-2-yl-cyclohexyl)-N'-pentyl-succinamid
5-[4-(2-Methoxy-naphthalen-1-ylmethyl)-piperazin-1-yl]-5-oxo-valeriansäure (4-dimethylamino-4-thiophen-2-yl-cyclohexyl)-amid
N-(4-Dimethylamino-4-thiophen-2-yl-cyclohexyl)-N'-(2-ethoxy-benzyl)-N'-furan-2-ylmethyl-succinamid
Glutarsäure-(4-dimethylamino-4-thiophen-2-yl-cyclohexyl)-amid (2-phenyl-cyclopropyl)-amid
N-[2-(3,4-Dichlor-phenyl)-ethyl]-N'-(4-dimethylamino-4-phenyl-cyclohexyl)-succinamid
N-Adamantan-1-ylmethyl-N'-(4-dimethylamino-4-phenyl-cyclohexyl)-succinamid
5-[4-(4-Fluor-phenyl)-3,6-dihydro-2H-pyridin-1-yl]-5-oxo-valeriansäure (4-dimethylamino-4-thiophen-2-yl-cyclohexyl)-amid
2-[4-(4-Dimethylamino-4-thiophen-2-yl-cyclohexylcarbamoyl)-butyrylamino]-4-methyl-valeriansäure benzyl ester
{4-[4-(4-Dimethylamino-4-thiophen-2-yl-cyclohexylcarbamoyl)-butyrylamino]-phenyl}-carbaminsäure-tert-butylester
Glutarsäure-(4-dimethylamino-4-thiophen-2-yl-cyclohexyl)-amid [2-(3-methoxy-phenyl)-ethyl]-amid
Glutarsäure-benzyl-ethyl-amid (4-dimethylamino-4-thiophen-2-yl-cyclohexyl)-amid
Glutarsäure-3-chlor-benzylamid (4-dimethylamino-4-thiophen-2-yl-cyclohexyl)-amid
Glutarsäure-(4-dimethylamino-4-thiophen-2-yl-cyclohexyl)-amid (4-methyl-cyclohexyl)-amid
N-(4-Dimethylamino-4-phenyl-cyclohexyl)-N'-[2-(1H-indol-3-yl)-ethyl]-N'-methyl-succinamid
Glutarsäure-2,5-dichlor-benzylamid (4-dimethylamino-4-thiophen-2-yl-cyclohexyl)-amid
Glutarsäure-(4-dimethylamino-4-thiophen-2-yl-cyclohexyl)-amid [2-(3-fluor-phenyl)-ethyl]-amid
N-(4-Dimethylamino-4-phenyl-cyclohexyl)-N'-[2-(4-methoxy-phenoxy)-ethyl]-succinamid
N-(4-Dimethylamino-4-thiophen-2-yl-cyclohexyl)-N'-[2-(ethyl-m-tolyl-amino)-ethyl]-succinamid
Glutarsäure-(4-benzyloxy-phenyl)-amid (4-dimethylamino-4-thiophen-2-yl-cyclohexyl)-amid
Glutarsäure-(4-dimethylamino-4-thiophen-2-yl-cyclohexyl)-amid [2-(4-fluor-phenyl)-ethyl]-amid
Glutarsaure-[2-(4-brom-phenyl)-ethyl]-amid (4-dimethylamino-4-thiophen-2-yl-cyclohexyl) amid
N-(4-Dimethylamino-4-thiophen-2-yl-cyclohexyl)-N'-(4-phenoxy-phenyl)-succinamid
N-(4-Dimethylamino-4-phenyl-cyclohexyl)-N'-[2-(3-trifluormethyl-phenyl)-ethyl]-succinamid
Glutarsäure-[2-(4-chlor-phenyl)-propyl]-amid (4-dimethylamino-4-thiophen-2-yl-cyclohexyl)-amid
N-(4-Dimethylamino-4-thiophen-2-yl-cyclohexyl)-N'-[2-(1H-indol-3-yl)-ethyl]-N'-methyl-succinamid
N-(4-Dimethylamino-4-thiophen-2-yl-cyclohexyl)-N'-[2-(4-methoxy-phenoxy)-ethyl]-succinamid
Glutarsäure-(4-dimethylamino-4-thiophen-2-yl-cyclohexyl)-amid (3-phenoxy-phenyl)-amid
N-(4-Dimethylamino-4-phenyl-cyclohexyl)-N'-[2-(ethyl-m-tolyl-amino)-ethyl]-succinamid
Glutarsäure-[2-(5-brom-2-methoxy-phenyl)-ethyl]-amid (4-dimethylamino-4-thiophen-2-yl-cyciohexyl)-amid
N-(4-Dimethylamino-4-thiophen-2-yl-cyclohexyl)-N'-[2-(3-trifluormethyl-phenyl)-ethyl]-succinamid
Glutarsäure-(4-dimethylamino-4-thiophen-2-yl-cyclohexyl)-amid (1-methyl-3-phenyl-propyl)-amid
N-[2-(2,6-Dichlor-benzylsulfanyl)-ethyl]-N'-(4-dimethylamino-4-thiophen-2-yl-cyclohexyl)-succinamid
Glutarsäure-(4-dimethylamino-4-thiophen-2-yl-cyclohexyl)-amid (2,2-diphenyl-propyl)-amid
N-(4-Dimethylamino-4-thiophen-2-yl-cyclohexyl)-N'-[3-(methyl-phenyl-amino)-propyl]-succinamid
N-(1-Biphenyl-4-ylmethyl-pyrrolidin-3-yl)-N'-(4-dimethylamino-4-thiophen-2-yl-cyclohexyl)-N-methyl-succinamid
Glutarsäure-[4-(cyano-phenyl-methyl)-phenyl]-amid (4-dimethylamino-4-thiophen-2-yl-cyclohexyl)-amid
Glutarsäure-(4-tert-butyl-cyclohexyl)-amid (4-dimethylamino-4-thiophen-2-yl-cyclohexyl)-amid
Glutarsäure-(4-sec-butyl-phenyl)-amid (4-dimethylamino-4-thiophen-2-yl-cyclohexyl)-amid
Glutarsäure-(4-butyl-phenyl)-amid (4-dimethylamino-4-thiophen-2-yl-cyclohexyl)-amid
N-(2-Benzylsulfanyl-ethyl)-N'-(4-dimethylamino-4-thiophen-2-yl-cyclohexyl)-succinamid
N-(4-Dimethylamino-4-thiophen-2-yl-cyclohexyl)-N'-(2-phenoxy-ethyl)-succinamid
Glutarsäure-(4-dimethylamino-4-thiophen-2-yl-cyclohexyl)-amid (naphthalen-1-ylmethyl)-amid
Glutarsäure-3-brom-benzylamid (4-dimethylamino-4-thiophen-2-yl-cyclohexyl)-amid
N-(4-Dimethylamino-4-thiophen-2-yl-cyclohexyl)-N'-(3-phenyl-propyl)-succinamid
N-[2-(3-Chlor-phenyl)-ethyl]-N'-(4-dimethylamino-4-phenyl-cyclohexyl)-succinamid
2-[3-(4-Dimethylamino-4-thiophen-2-yl-cyclohexylcarbamoyl)-propionylamino]-propionsäurebenzylester
N-[2-(2-Chlor-phenyl)-ethyl]-N'-(4-dimethylamino-4-phenyl-cyclohexyl)-succinamid
N-(4-Dimethylamino-4-phenyl-cyclohexyl)-N'-[2-(3-fluor-phenyl)-ethyl]-succinamid
Glutarsäure-(4-dimethylamino-4-thiophen-2-yl-cyclohexyl)-amid (2-thiophen-2-yl-ethyl)-amid
Glutarsäure-(4-dimethylamino-4-thiophen-2-yl-cyclohexyl)-amid [2-(4-phenoxy-phenyl)-ethyl]-amid
N-[2-(4-Chlor-phenyl)-ethyl]-N'-(4-dimethylamino-4-phenyl-cyclohexyl)-succinamid
N-[2-(3-Chlor-phenyl)-ethyl]-N'-(4-dimethylamino-4-thiophen-2-yl-cyclohexyl)-succinamid
N-Benzyl-N'-(4-dimethylamino-4-thiophen-2-yl-cyclohexyl)-N-ethyl-succinamid
Glutarsäure-[1-(2,6-dichlor-benzyl)-pyrrolidin-3-yl]-amid (4-dimethylamino-4-thiophen-2-yl-cyclohexyl)-amid
Glutarsäure-(4-dimethylamino-4-thiophen-2-yl-cyclohexyl)-amid (3-phenyl-propyl)-amid
Glutarsäure-(4-dimethylamino-4-thiophen-2-yl-cyclohexyl)-amid [2-(1H-indol-3-yl)-ethyl]-amid
5-(3,4-Dihydro-1 H-isochinolin-2-yl)-5-oxo-valeriansäure (4-dimethylamino-4-thlophen-2-yl-cyclohexyl)-amid
{[4-(4-Dimethylamino-4-thiophen-2-yl-cyclohexylcarbamoyl)-butyryl]-methyl-amino}-essigsäurebenzylester
N-(4-Dimethylamino-4-thiophen-2-yl-cyclohexyl)-N'-[2-(4-phenoxy-phenyl)-ethyl]-succinamid
Glutarsäure-(4-dimethylamino-4-thiophen-2-yl-cyclohexyl)-amid (2-phenyl-cyclopropyl)-amid
N-[4-(3-Chlorbenzyl)-4-dimethylaminocyclohexyl]-N'-furan-2-ylmethylbemsteinsäureamid Hydrochlorid, unpolareres Diastereoisomer
N-[4-(3-Chlorbenzyl)-4-dimethylaminocyclohexyl]-N'-furan-2-ylmethylbernsteinsäureamid Hydrochlorid, polareres Diastereoisomer
Glutarsäure (4-benzyl-4-dimethylaminocyclohexyl)amid [2-(2-fluorphenyl)ethyl]amid Hydrochlorid, unpolareres Diastereoisomer
Glutarsäure (4-benzyl-4-dimethylaminocyclohexyl)amid [2-(2-fluorphenyl)ethyl]amid Hydrochlorid, polareres Diastereoisomer
N-[4-(3-Chlorbenzyl)-4-dimethylaminocyclohexyl]-N'-[2-(4-chlorphenyl)ethyl]-bernsteinsäureamid Hydrochlorid, unpolareres Diastereoisomer
N-[4-(3-Chlorbenzyl)-4-dimethylaminocyclohexyl]-N'-[2-(4-chlorphenyl)ethyl]-bernsteinsäureamid Hydrochlorid, polareres Diastereoisomer
Glutarsäure (4-benzyl-4-dimethylaminocyclohexyl)amid [2-(4-chlorphenyl)ethyl]amid Hydrochlorid, unpolareres Diastereoisomer
in Form des Razemats; der Enantiomere, Diastereomere, Mischungen der Enantiomere oder Diastereomere oder eines einzelnen Enantiomers oder Diastereomers; der Basen und/oder Salze physiologisch verträglicher Säuren oder Kationen.

14. Verfahren zur Herstellung von substituierten Cyclohexyl-1,4-diamin-Derivaten gemäß einem der Ansprüche 1-13, **dadurch gekennzeichnet, dass** substituierte Cyclohexan-1,4-diamine über Anhydride, offenkettige Dicarbonsäuren oder deren aktivierte Analoga, insbesondere deren Säurehalogenide, mit weiteren primären oder sekundären Aminen unter Zugabe von Kupplungsreagenzien verknüpft werden.

15. Verfahren zur Herstellung von substituierten Cyclohexyl-1,4-diamin-Derivaten gemäß einem der Ansprüche 1-13, **dadurch gekennzeichnet, dass** Anhydride, offenkettige Dicarbonsäuren oder deren aktivierte Analoga, insbesondere deren Säurehalogenide, mit primären oder sekundären Aminen und anschließend mit substituierten Cyclohexan-1,4-diaminen unter Zugabe von Kupplungsreagenzien verknüpft werden.

16. Arzneimittel enthaltend wenigstens ein substituiertes Cyclohexyl-1,4-diamin-Derivats gemäß einem der Ansprüche 1 bis 13, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate, sowie gegebenenfalls enthaltend geeignete Zusatz- und/oder Hilfsstoffe und/oder gegebenenfalls weiterer Wirkstoffe.

17. Verwendung eines substituierten Cyclohexyl-1,4-diamin-Derivats gemäß einem der Ansprüche 1 bis 13, gegebenenfalls in Form seines Razemats, der reinen Stereoisomeren, insbesondere Enantiomeren und Diastereomeren, in einem beliebigen Mischungsverhältnis; in Form seiner Säuren oder seiner Basen oder in Form seiner Salze, insbesondere der physiologisch verträglichen Salze oder Salze physiologisch verträglicher Säuren oder Kationen; oder in Form seiner Solvate, insbesondere der Hydrate; zur Herstellung eines Arzneimittels zur Behandlung von Schmerz, insbesondere von akutem, neuropathischem oder chronischem Schmerz.

18. Verwendung eines substituierten Cyclohexyl-1,4-diamin-Derivats gemäß einem der Ansprüche 1 bis 13 zur Herstellung eines Arzneimittels zur Behandlung von Angstzuständen, von Stress und mit Stress verbundenen Syndromen, Depressionen, Epilepsie, Alzheimer Erkrankung, seniler Demenz, Katalepsie, allgemeinen kognitiven Dysfunktionen, Lern- und Gedächtnis-Störungen (als Nootropikum), Entzugserscheinungen, Alkohol- und/oder Drogen- und/oder Medikamentenmißbrauch und/oder -abhängigkeit, sexuellen Dysfunktionen, cardiovaskulären Erkrankungen, Hypotension, Hypertension, Tinitus, Pruritus, Migräne, Schwerhörigkeit, mangelnder Darmmotilität, gestörter Nahrungsaufnahme, Anorexie, Fettsucht, Iokomotorischen Störungen, Diarrhoe, Kachexie, Harninkontinenz bzw. als Muskelrelaxanz, Antikonvulsivum oder Anesthetikum bzw. zur Coadministration bei Behandlung mit einem opioiden Analgetikum oder mit einem Anesthetikum, zur Diurese oder Antinatriurese, Anxiolyse, zur Modulation der Bewegungsaktivität, zur Modulation der Neurotransmitter-Ausschüttung und Behandlung damit verbundener neurodegenerativer Erkrankungen, zur Behandlung von Entzugserscheinungen und/oder zur Reduzierung des Suchtpotentials von Opioiden.

## Claims

1. Substituted cyclohexyl-1,4-diamine derivatives of the general formula I, 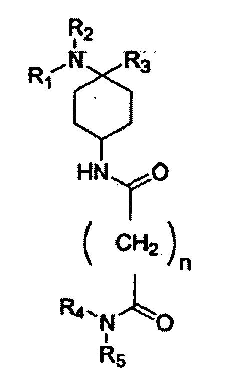 wherein
n stands for 1, 2, 3, 4 or 5
R¹ and R², independently of one another, stand for H; C₁₋₅ alkyl, respectively saturated or unsaturated, branched or unbranched, mono- or polysubstituted or unsubstituted; C₃₋₈-cycloalkyl, respectively mono- or polysubstituted or unsubstituted; or aryl, C₃₋₈-cycloalkyl or heteroaryl, respectively mono- or polysubstituted or unsubstituted, bonded via C₁₋₃-alkyl;
or the residues R¹ and R² together stand for CH₂CH₂OCH₂CH₂, CH₂CH₂NR¹⁰CH₂CH₂ or (CH₂)₃₋₆;
wherein R¹⁰ represents H; C₁₋₅ alkyl, respectively saturated or unsaturated, branched or unbranched, mono- or polysubstituted or unsubstituted; C₃₋₈-cycloalkyl, respectively mono- or polysubstituted or unsubstituted; aryl or heteroaryl, respectively mono- or polysubstituted or unsubstituted; or aryl, C₃₋₈-cycloalkyl or heteroaryl, respectively mono- or polysubstituted or unsubstituted, bonded via C₁₋₃-alkyl; C(O)phenyl, C(O)heteroaryl, C(O)C₁₋₅-alkyl, respectively substituted or unsubstituted;
R³ stands for C₁₋₅ alkyl, respectively saturated or unsaturated, branched or unbranched, mono- or polysubstituted or unsubstituted; C₃₋₈-cycloalkyl, respectively mono- or polysubstituted or unsubstituted; aryl or heteroaryl, respectively unsubstituted or mono- or polysubstituted; aryl, heteroaryl or C₃₋₈-cycloalkyl, respectively unsubstituted or mono- or polysubstituted, bonded via C₁₋₃-alkyl group;
R⁴ stands for -(CR⁶R⁷)ₚR⁸;
wherein p represents 0, 1, 2, 3 or 4
R⁶ represents H or C₁₋₅ alkyl, respectively saturated or unsaturated, branched or unbranched, unsubstituted or mono- or polysubstituted
R⁷ represents H or C₁₋₅ alkyl, respectively saturated or unsaturated, branched or unbranched, unsubstituted or mono- or polysubstituted, or COOR⁹,
or R⁶ and R⁷ form a (CH₂)ₖCHR⁸(CH₂)ₘ ring, where k = 1, 2, 3 and m = 1, 2
R⁸ represents C₁₋₆ alkyl, respectively saturated or unsaturated, branched or unbranched, unsubstituted or mono- or polysubstituted; C₃₋₈-cycloalkyl, aryl or heteroaryl, respectively unsubstituted or mono- or polysubstituted,
R⁹ represents H or C₁₋₅ alkyl
R⁵ stands for H or for -(CH₂)ₗR⁸,
wherein l stands for 1, 2 or 3,
or together with R⁴ stands for CH₂CHR¹⁴OCHR¹⁴CH₂, CH₂CH₂SCH₂CH₂, CH₂CH₂NR¹¹CH₂CH₂, (CR¹²R¹³)₃₋₆ or 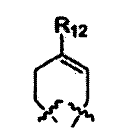 wherein R¹¹ represents H; C₁₋₅ alkyl, respectively saturated or unsaturated, branched or unbranched, mono- or polysubstituted or unsubstituted; C₃₋₈-cycloalkyl, respectively saturated or unsaturated, mono- or polysubstituted or unsubstituted; aryl or heteroaryl, respectively mono- or polysubstituted or unsubstituted; or aryl, C₃₋₈-cycloalkyl or heteroaryl, respectively mono- or polysubstituted or unsubstituted, bonded via C₁₋₃-alkyl;
R¹² represents H; C₁₋₅ alkyl, respectively saturated or unsaturated, branched or unbranched, mono- or polysubstituted or unsubstituted; OH; C(O)phenyl, unsubstituted or mono- or polysubstituted; C₃₋₈-cycloalkyl, respectively mono- or polysubstituted or unsubstituted; aryl or heteroaryl, respectively mono- or polysubstituted or unsubstituted; or aryl, C₃₋₈-cycloalkyl or heteroaryl, respectively mono- or polysubstituted or unsubstituted, bonded via C₁₋₃-alkyl;
and R¹³ represents H or OH, or together with R¹², via the same C atom (spiro compound) or an adjacent C atom, forms a five-membered or six-membered ring, which can contain heteroatoms, saturated or unsaturated, substituted or unsubstituted and/or can be a part of a polycyclic system;
R¹⁴ represents H or C₁₋₃-alkyl, respectively saturated or unsaturated, branched
or unbranched, mono- or polysubstituted or unsubstituted;
in the form of the racemate; the enantiomers, diastereomers, mixtures of the enantiomers or diastereomers or a single enantiomer or diastereomer; of the bases and/or salts of physiologically compatible acids or cations.

2. Substituted cyclohexyl-1,4-diamine derivatives according to claim 1, **characterised in that**
R¹ and R², independently of one another, stand for H; C₁₋₅alkyl, saturated or unsaturated, branched or unbranched, mono- or polysubstituted or unsubstituted;
or the residues R¹ and R² together form a ring and represent CH₂CH₂OCH₂CH₂ CH₂CH₂NR¹⁰CH₂CH₂ or (CH₂)₃₋₆,
wherein R¹⁰ represents H; C₁₋₅-alkyl, saturated or unsaturated, branched or unbranched, mono- or polysubstituted or unsubstituted.

3. Substituted cyclohexyl-1,4-diamine derivatives according to one of claims 1 or 2, **characterised in that** R¹ and R², independently of one another, stand for CH₃ or H,
wherein R¹ and R² do not represent H simultaneously, or R¹ and R² stand for CH₂CH₂OCH₂CH₂ or (-CH₂)₆.

4. Substituted cyclohexyl-1,4-diamine derivatives according to one of claims 1-3,
**characterised in that**
R³ stands for cyclopentyl, cyclohexyl, phenyl, benzyl, naphthyl, anthracenyl, thiophenyl, benzothiophenyl, furyl, benzofuranyl, benzodioxolanyl, indolyl, indanyl, benzodioxanyl, pyrrolyl, pyridyl, pyrimidyl or pyrazinyl, respectively unsubstituted or mono- or polysubstituted; C₅₋₆-cycloalkyl, phenyl, naphthyl, anthracenyl, thiophenyl, benzothiophenyl, pyridyl, furyl, benzofuranyl, benzodioxolanyl, indolyl, indanyl, benzodioxanyl, pyrrolyl, pyrimidyl or pyrazinyl, respectively substituted or mono- or polysubstituted, bonded via a saturated, unbranched C₁₋₂-alkyl group;
in particular
R³ stands for phenyl, furyl, thiophenyl, naphthyl, benzyl, benzofuranyl, indolyl, indanyl, benzodioxanyl, benzodioxolanyl, pyridyl, pyrimidyl, pyrazinyl or benzothiophenyl, respectively unsubstituted or mono- or polysubstituted; phenyl, furyl or thiophenyl, respectively unsubstituted or mono- or polysubstituted, bonded via a saturated, unbranched C₁₋₂-alkyl group.

5. Substituted cyclohexyl-1,4-diamine derivatives according to one of claims 1-4, **characterised in that** R³ stands for phenyl, thiophenyl, pyridyl or benzyl, respectively substituted or unsubstituted, particularly preferred for 4-methylbenzyl, 3-chlorobenzyl, 4-chlorobenzyl, 3-methylbenzyl, benzyl, phenyl, thiophenyl and 3-fluorophenyl.

6. Substituted cyclohexyl-1,4-diamine derivatives according to one of claims 1-5, **characterised in that** n stands for 2 or 3.

7. Substituted cyclohexyl-1,4-diamine derivatives according to one of claims 1-6, **characterised in that** R⁴ stands for -(CR⁶R⁷)ₚR⁸ and R⁵ stands for H.

8. Substituted cyclohexyl-1,4-diamine derivatives according to one of claims 1-6, **characterised in that** R⁴ stands for (CH₂)ₚR⁸ and R⁵ stands for (CH₂)ₗR⁸.

9. Substituted cyclohexyl-1,4-diamine derivatives according to one of claims 1-6, **characterised in that** R⁴ and R⁵ together stand for CH₂CHR¹⁴OCHR¹⁴CH₂, CH₂CH₂SCH₂CH₂, CH₂CH₂NR¹¹CH₂CH₂, (CR¹²R¹³)₃₋₆ or

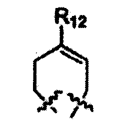

10. Substituted cyclohexyl-1,4-diamine derivatives according to one of claims 1-7, **characterised in that** R⁶ represents H, CH₃, benzyl, unsubstituted or mono- or polysubstituted, or COOR⁹, or R⁶ and R⁷ form a (CH₂)ₖCHR⁸(CH₂)ₘ ring.

11. Substituted cyclohexyl-1,4-diamine derivatives according to one of claims 1-8 or 10, **characterised in that** R⁸ represents C₁₋₆-alkyl, respectively saturated or unsaturated, branched or unbranched, unsubstituted or mono- or polysubstituted; cyclobutyl, cyclopropyl, cyclopentyl, cyclohexyl, cycloheptyl, cyclooctyl, anthracenyl, indolyl, naphthyl, benzofuranyl, benzothiophenyl, indanyl, benzodioxanyl, benzodioxolanyl, acenaphthyl, carbazolyl, phenyl, thiophenyl, furyl, pyrrolidinyl, tetrahydrofuryl, piperazinyl, pyridyl, pyrrolyl, pyrazinyl or pyrimidyl, fluoroenyl, fluoroanthenyl, benzothiazolyl, benzotriazolyl orbenzo[1,2,5]thiazolyl or 1,2-dihydroacenaphthenyl, pyridinyl, furanyl, benzofuranyl, pyrazolinonyl, oxopyrazolinonyl, dioxolanyl, adamantyl, pyrimidinyl, quinolinyl, isoquinolinyl, phthalazinyl or quinazolinyl, respectively unsubstituted or mono- or polysubstituted.

12. Substituted cyclohexyl-1,4-diamine derivatives according to one of claims 1-8 or 10, **characterised in that** R⁸ represents C₁₋₆-alkyl, respectively saturated or unsaturated, branched or unbranched, unsubstituted or mono- or polysubstituted; pyrrolidinyl, morpholinyl, tetrahydrofuryl, tetrahydronaphthyl, dihydroindolyl, pyridyl, thienyl, piperazinyl, naphthyl, indanyl, quinolinyl, cyclopropyl, cyclopentyl, cyclohexyl, cycloheptyl, cyclooctyl, adamantyl, furyl, benzofuryl, phenyl or indolyl, respectively unsubstituted or mono- or polysubstituted.

13. Substituted cyclohexyl-1,4-diamine derivatives according to one of claims 1-12 from the group
N-[1-(2,6-dichlorobenzyl)-pyrrolidin-3-yl]-N'-[4-dimethylamino-4-(4-methyl-benzyl)-cyclohexyl] succinamide
N-[4-(3-chlorobenzyl)-4-dimethylamino-cyclohexyl)-N'-(2-oxo-tetrahydrofuran-3-yl) succinamide
N-[4-(4-chlorobenzyl)-4-dimethylamino-cyclohexyl]-N'-(3,3-diphenyl-propyl) succinamide
N-[4-dimethylamino-4-(4-methyl-benzyl)-cyclohexyl]-4-[4-(7-methoxybenzo-[1,3]dioxol-5-ylmethyl)-piperazin-1-yl]-4-oxo-butyramide
N-[4-(4-chlorobenzyl)-4-dimethylamino-cyclohexyl]-4-(3,6-dihydro-2H-pyridin-1-yl)-4-oxo-butyramide
N-[4-dimethylamino-4-(4-methyl-benzyl)-cyclohexyl]-4-oxo-4-(4-m-tolyl-piperazin-1-yl) butyramide
2-{3-[4-(4-chlorobenzyl)-4-dimethylamino-cyclohexylcarbamoyl]-propionylamino}-ethyl propionate
N-[4-(3-chlorobenzyl)-4-dimethylamino-cyclohexyl]-4-(8-fluoro-1,3,4,5-tetrahydro-pyrido[4,3-b]indol-2-yl)-4-oxo-butyramide
3- {3-[4-dimethylamino-4-(4-methyl-benzyl)-cyclohexylcarbamoyl]-propionylamino}-ethyl propionate
N-[4-dimethylamino-4-(4-methyl-benzyl)-cyclohexyl]-4-[4-(5-methyl-pyrazine-2-carbonyl)-piperazin-1-yl]-4-oxo-butyramide
N-[4-dimethylamino-4-(4-methyl-benzyl)-cyclohexyl]-N'-[2-(2,2,2-trifluoro-acetylamino)-ethyl] succinamide
N-[4-(3-chlorobenzyl)-4-dimethylamino-cyclohexyl]-N'-(2-pyridin-2-yl-ethyl) succinamide
N-[4-(4-chlorobenzyl)-4-dimehtylamino-cyclohexyl]-4-oxo-4-thiomorpholin-4-yl butyramide
N-[4-(4-chlorobenzyl)-4-dimethylamino-cyclohexyl]-4-[4-(5-methyl-pyrazine-2-carbonyl)-piperazin-1-yl]-4-oxo-butyramide
N-[4-dimethylamino-4-(3-methyl-benzyl)-cyclohexyl]-N'-(2-thiophen-2-yl-ethyl) succinamide
N-[4-(4-chlorobenzyl)-4-dimethylamino-cyclohexyl]-4-[4-(4-ethoxyphenyl)-piperazin-1-yl]-4-oxo-butyramide
N-allyl-N'-[4-(4-chlorobenzyl)-4-dimethylamino-cyclohexyl]-N-methyl succinamide
5-[4-(2,5-dimethyoxy-benzyl)-piperazin-1-yl]-5-oxo-valeric acid (4-benzyl-4-dimethylamino-cyclohexyl) amide
4-(3,6-dihydro-2H-pyridin-1-yl)-N-[4-dimethylamino-4-(4-methyl-benzyl)-cyclohexyl]-4-oxo-butyramide
N-[4-dimethylamino-4-(3-methyl-benzyl)-cyclohexyl]-4-(4-methyl-piperidin-1-yl)-4-oxo-butyramide
N-[4-dimethylamino-4-(4-methyl-benzyl)-cyclohexyl]-4-oxo-4-(4-pyridin-2-yl-piperazin-1-yl) butyramide
({3-[4-dimethylamino-4-(3-methyl-benzyl)-cyclohexylcarbamoyl]-propionyl}-methylamino) benzyl acetate
4-[4-(2,6-dichlorobenzyl)-piperazin-1-yl]-N-(4-dimethylamino-4-(3-methyl-benzyl)-cyclohexyl]-4-oxo-butyramide
{[4-(4-benzyl-4-dimethylamino-cyclohexylcarbamoyl)-butyryl]-methylamino}-tert-butyl acetate
5-[4-(2-fluoro-5-methoxybenzyl)-piperazin-1-yl]-5-oxo-valeric acid (4-benzyl-4-dimethylamino-cyclohexyl) amide
N-[4-(4-chlorobenzyl)-4-dimethylamino-cyclohexyl]-N'-(3-methoxypropyl) succinamide
4-[4-(4-acetyl-phenyl)-piperazin-1-yl]-N-[4-dimethylamino-4-(4-methyl-benzyl)-cyclohexyl]-4-oxo-butyramide
4-{3[4-dimethylamino-4-(4-methyl-benzyl)-cyclohexylcarbamoyl]-propionyl}-piperazine-1-tert-butyl carboxylate
N-[4-(3-chlorobenzyl)-4-dimethylamino-cyclohexyl]-N'-(tetrahydrofuran-2-ylmethyl) succinamide
2-{3-[4-(3-chlorobenzyl)-4-dimethylamino-cyclohexylcarbamoyl]-propionylamino} ethyl propionate
N-(3-bromobenzyl)-N'-[4-dimethylamino-4-(3-methyl-benzyl)-cyclohexyl] succinamide
3-(4-chlorophenyl)-2- {3-[4-dimethylamino-4-(4-methyl-benzyl)-cyclohexylcarbamoyl]-propionylamino} methyl propionate
N-[4-(3-chlorobenzyl)-4-dimethylamino-cyclohexyl]-4-[4-(5-methyl-pyrazine-2-carbonyl)-piperazin-1-yl]-4-oxo-butyramide
N-[4-(4-chlorobenzyl)-4-dimethylamino-cyclohexyl]-N'-naphthalen-1-ylmethyl succinamide
N-[4-dimethylamino-4-(3-methyl-benzyl)-cyclohexyl]-4-[4-(3-fluoro-4-methoxybenzyl)-piperazin-1-yl]-4-oxo-butyramide
N-[4-dimethylamino-4-(4-methyl-benzyl)-cyclohexyl]-N'-[2-(1H-indol-3-yl)-ethyl]-N'-methyl succinamide
(4-{3-[4-(3-chlorobenzyl)-4-dimethylamino-cyclohexylcarbamoyl]-propionylamino}-benzyl diethyl phosphonate
N-[4-dimethylamino-4-(4-methyl-benzyl)-cyclohexyl)-N'-methyl-N'-(2-pyridin-2-yl-ethyl) succinamide
N-[4-(3-chlorobenzyl)-4-dimethylamino-cyclohexyl]-4-(3,6-dihydro-2H-pyridin-1-yl)-4-oxo-butyramide
N-[4-dimethylamino-4-(3-methyl-benzyl)-cyclohexyl]-N'-(1-methyl-3-phenyl-propyl) succinamide
5-[4-(5-methyl-pyrazine-2-carbonyl)-piperazin-1-yl]-5-oxo-valeric acid (4-benzyl-4-dimethylamino-cyclohexyl) amide
N-[4-(3-chlorobenzyl)-4-dimethylamino-cyclohexyl]-N'-(2-chloro-6-methyl-benzyl) succinamide
N-allyl-N'-[4-(3-chlorobenzyl)-4-dimethylamino-cyclohexyl]-N-methyl succinamide
N-[4-(4-chlorobenzyl)-4-dimethylamino-cyclohexyl]-N'-cyclopentyl succinamide
N-[4-dimethylamino-4-(4-methyl-benzyl)-cyclohexyl]-N'-(tetrahydrofuran-2-ylmethyl) succinamide
(4-{3-[4-dimethylamino-4-(3-methyl-benzyl)-cyclohexylcarbamoyl]-propionylamino}-benzyl) diethyl phosphonate
N-[4-(4-chlorobenzyl)-4-dimethylamino-cyclohexyl]-4-[4-(2-methoxyphenyl)-piperazin-1-yl)-4-oxo-butyramide
N-[4-dimethylamino-4-(3-methyl-benzyl)-cyclohexyl]-4-[4-(3-methoxyphenyl)-piperazin-1-yl]-4-oxo-butyramide
N-[4-(4-chlorobenzyl)-4-dimethylamino-cyclohexyl]-N'-(3,5-difluorobenzyl) succinamide
N-[4-(4-chlorobenzyl)-4-dimethylamino-cyclohexyl]-N'-(2-methyl-benzyl) succinamide
N-[4-dimethylamino-4-(4-methyl-benzyl)-cyclohexyl]-4-oxo-4-{4-[(2,2,2-trifluoro-acetylamino)-methyl]-piperidin-1-yl}butyramide
N-[4-(4-chlorobenzyl)-4-dimethylamino-cyclohexyl]-N'-[2-(1H-indol-3-yl)-ethyl] succinamide
N-[4-(3-chlorobenzyl)-4-dimethylamino-cyclohexyl]-N'-[2(1H-indol-3-yl)-ethyl]-N'-methyl succinamide
5-[4-(7-methoxybenzyl[1,3]dioxol-5-ylmethyl)-piperazin-1-yl]-5-oxo-valeric acid (4-benzyl-4-dimethylamino-cyclohexyl) amide
N-benzyl-N'-[4-dimethylamino-4-(3-methyl-benzyl)-cyclohexyl]-N-(2-hydroxyethyl) succinamide
5-[4-(2,3-dimethyl-phenyl)-piperazin-1-yl]-5-oxo-valeric acid (4-benzyl-4-dimethylamino-cyclohexyl) amide
N-[4-(4-chlorobenzyl)-4-dimethylamino-cyclohexyl]-N'-[2-(1H-indol-3-yl)-ethyl]-N'-methyl succinamide
N-[4-dimethylamino-4-(4-methyl-benzyl)-cyclohexyl]-4-[4-(2, 3-dimethyl-phenyl)-piperazin-1-yl]-4-oxo-butyramide
glutaric acid-(4-benzyl-4-dimethylamino-cyclohexyl) amide (1,2,3,4-tetrahydro-naphthalen-1-yl) amide
N-[4-(4-chlorobenzyl)-4-dimethylamino-cyclohexyl]-4-(4-methyl-piperidin-1-yl)-4-oxo-butyramide
2-{3-[4-dimethylamino-4-(4-methyl-benzyl)-cyclohexylcarbamoyl]-propionylamino}-tert-butyl propionate
5-(4-cycloheptyl-piperazin-1-yl)-5-oxo-valeric acid (4-benzyl-4-dimethylamino-cyclohexyl) amide
glutaric acid-(4-benzyl-4-dimethylamino-cyclohexyl) amide [2-(4-fluorophenyl)-ethyl] amide
glutaric acid-(4-benzyl-4-dimethylamino-cyclohexyl) amide [1-(2,6-dichlorobenzyl)-pyrrolidin-3-yl] amide
N-[4-(4-chlorobenzyl)-4-dimethylamino-cyclohexyl]-4-(1,4-dioxa-8-aza-spiro[4.5]dec-8-yl)-4-oxo-butyramide
N-[4-(3-chlorobenzyl)-4-dimethylamino-cyclohexyl]-4-oxo-4-thiomorpholin-4-yl butyramide
4-(4-benzoyl-piperidin-1-yl)-N-[4-dimethylamino-4-(3-methyl-benzyl)-cyclohexyl]-4-oxo-butyramide
N-[4-(4-chlorobenzyl)-4-dimethylamino-cyclohexyl]-N'-(2,4-dimethoxy-benzyl) succinamide
N-[4-dimethylamino-4-(3-methyl-benzyl)-cyclohexyl]-N'-(1, 2, 3,4-tetrahydro-naphthalen-1-yl) succinamide
N-[4-(4-chlorobenzyl)-4-dimethylamino-cyclohexyl]-N'-ethyl-N'-pyridin-4-ylmethyl succinamide
N-[2-(3,4-dimethoxy-phenyl)-ethyl]-N'-[4-dimethylamino-4-(3-methyl-benzyl)-cyclohexyl]-N-methyl succinamide
N-[4-(4-chlorobenzyl)-4-dimethylamino-cyclohexyl]-N'-methyl-N'-pyridin-3-ylmethyl succinamide
N-[4-(3-chlorobenzyl)-4-dimethylamino-cyclohexyl]-N'-pyridin-2-ylmethyl succinamide
N-[4-(3-chlorobenzyl)-4-dimethylamino-cyclohexyl]-N'-(2-methyl-benzyl) succinamide
N-[4-dimethylamino-4-(3-methyl-benzyl)-cyclohexyl]-N'-(4-methoxyphenyl) succinamide
N-[4-dimethylamino-4-(3-methyl-benzyl)-cyclohexyl]-N'-indan-2-yl succinamide
N-[4-(3-chlorobenzyl)-4-dimethylamino-cyclohexyl]-N'-(1-methyl-3-phenyl-propyl) succinamide
N-(2-chloro-6-methyl-benzyl)-N'-[4-dimethylamino-4-(3-methyl-benzyl)-cyclohexyl] succinamide
N-[4-dimethylamino-4-(4-methyl-benzyl)-cyclohexyl]-4-[4-(4-methoxyphenyl)-piperazin-1-yl]-4-oxo-butyramide
N-[4-dimethylamino-4-(4-methyl-benzyl)-cyclohexyl]-N'-[2-(4-fluorophenyl)-ethyl] succinamide
glutaric acid-(4-benzyl-4-dimethylamino-cyclohexyl) amide 4-dimethylamino-benzylamide
N-[4-(3-chlorobenzyl)-4-dimethylamino-cyclohexyl]-N'-(3,5-dichlorobenzyl) succinamide
N-[4-(4-chlorobenzyl)-4-dimethylamino-cyclohexyl]-N'-(1-methyl-3-phenyl-propyl) succinamide
4- {3-[4-(3-chlorobenzyl)-4-dimethylamino-cyclohexylcarbamoyl]-propionyl}-piperazin-1-ethyl carboxylate
N-[4-dimethylamino-4-(3-methyl-benzyl)-cyclohexyl]-N'-(4-methoxybenzyl) succinamide
N-[4-(4-chlorobenzyl)-4-dimethylamino-cyclohexyl]-4-(2,6-dimethyl-morpholin-4-yl)-4-oxo-butyramide
N-[4-dimethylamino-4-(4-methyl-benzyl)-cyclohexyl]-4-oxo-4-(4-phenyl-3,6-dihydro-2H-pyridin-1-yl) butyramide
N-[4-(4-chlorobenzyl)-4-dimethylamino-cyclohexyl]-N'-[2-(3-fluorophenyl)-ethyl] succinamide
4-[4-(4-allyloxy-benzyl)-piperazin-1-yl]-N-[4-dimethylamino-4-(3-methyl-benzyl)-cyclohexyl]-4-oxo-butyramide
glutaric acid-(4-benzyl-4-dimethylamino-cyclohexyl) amide [2-(5-bromo-2-methoxyphenyl)-ethyl] amide
5-[4-(3-methoxyphenyl)-piperazin-1-yl]-5-oxo-valeric acid (4-benzyl-4-dimethylamino-cyclohexyl) amide
N-[4-(3-chlorobenzyl)-4-dimethylamino-cyclohexyl]-4-(4-methyl-piperidin-1-yl)-4-oxo-butyramide
(4-{3-[4-(4-chlorobenzyl)-4-dimethylamino-cyclohexylcarbamoyl]-propionylamino}-benzyl) diethyl phosphonate
N-[4-dimethylamino-4-(4-methyl-benzyl)-cyclohexyl]-4-oxo-4-(4-o-tolyl-piperazin-1-yl) butyramide
N-[4-dimethylamino-4-(3-methyl-benzyl)-cyclohexyl]-N'-[2-(3-fluorophenyl)-ethyl] succinamide
4-[4-(4-chlorophenyl)-piperazin-1-yl]-N-[4-dimethylamino-4-(4-methyl-benzyl)-cyclohexyl]-4-oxo-butyramide
N-[4-(3-chlorobenzyl)-4-dimethylamino-cyclohexyl]-N'-[1-(4-fluorophenyl)-ethyl] succinamide
N-[4-(4-chlorobenzyl)-4-dimethylamino-cyclohexyl]-N'-[1-(4-trifluoromethylbenzyl)-pyrrolidin-3-yl] succinamide
N-[4-(3-chlorobenzyl)-4-dimethylamino-cyclohexyl]-N'-[2-(1H-indol-3-yl)-ethyl] succinamide
N-[4-(4-chlorobenzyl)-4-dimethylamino-cyclohexyl]-N'-indan-5-yl succinamide
N-(4-benzyloxy-phenyl)-N'-[4-dimethylamino-4-(4-methyl-benzyl)-cyclohexyl ]succinamide
2- {3-[4-dimethylamino-4-(3-methyl-benzyl)-cyclohexylcarbamoyl]-propionylamino}-3-methyl tert-butyl valerate
4-(4-benzyl-piperidin-1-yl)-N-[4-dimethylamino-4-(3-methyl-benzyl)-cyclohexyl]-4-oxo-butyramide
N-[4-(4-chlorobenzyl)-4-dimethylamino-cyclohexyl]-4-[4-(4-fluorophenyl)-3,6-dihydro-2H-pyridin-1-yl]-4-oxo-butyramide
N-[4-dimethylamino-4-(4-methyl-benzyl)-cyclohexyl]-N'-(1,2-dimethyl-propyl) succinamide
N-[4-dimethylamino-4-(3-methyl-benzyl)-cyclohexyl]-4-(8-fluoro-1,2,3,4-tetrahydro-pyrido[4,3-b]indol-2-yl)-4-oxo-butyramide
4-[4-(3-chlorobenzoyl)-piperazin-1-yl]-N-[4-dimethylamino-4-(3-methyl-benzyl)-cyclohexyl]-4-oxo-butyramide
N-[4-(4-chlorobenzyl)-4-dimethylamino-cyclohexyl]-N'-(4-methoxyphenyl) succinamide
5-[4-(2,6-dichlorobenzyl)-piperazin-1-yl]-5-oxo valeric acid (4-benzyl-4-dimethylamino-cyclohexyl) amide
N-[4-dimethylamino-4-(3-methyl-benzyl)-cyclohexyl]-N'-[2-(1H-indol-3-yl)-ethyl]-N'-methyl succinamide
N-[4-dimethylamino-4-(3-methyl-benzyl)-cyclohexyl]-N'-[1-(4-fluorophenyl)-ethyl] succinamide
4-[4-(4-chlorophenyl)-piperazin-1-yl]-N-[4-dimethylamino-4-(3-methyl-benzyl)-cyclohexyl]-4-oxo-butyramide
N-[4-(3-chlorobenzyl)-4-dimethylamino-cyclohexyl]-4-oxo-4-(4-oxo-1-phenyl-1,3,8-triaza-spiro[4.5]dec-8-yl) butyramide
N-benzyl-N'-[4-dimethylamino-4-(3-methyl-benzyl)-cyclohexyl]-N-methyl succinamide
N-[4-(3-chlorobenzyl)-4-dimethylamino-cyclohexyl]-N'-(3,5-difluorobenzyl) succinamide
N-[4-dimethylamino-4-(3-methyl-benzyl)-cyclohexyl]-N'-[2-(4-fluorophenyl)-ethyl] succinamide
N-[4-dimethylamino-4-(3-methyl-benzyl)-cyclohexyl]-N'-[2-(1H-indol-3-yl)-ethyl] succinamide
N-(2-benzylsulphanyl-ethyl)-N'-[4-dimethylamino-4-(3-methyl-benzyl)-cyclohexyl] succinamide
N-[4-(3-chlorobenzyl)-4-dimethylamino-cyclohexyl]-N'-(2,4-difluorobenzyl) succinamide
N-[4-(3-chlorobenzyl)-4-dimethylamino-cyclohexyl]-N'-phenethyl succinamide
N-[4-dimethylamino-4-(3-methyl-benzyl)-cyclohexyl]-N'-(1-naphthalen-2-yl-ethyl) succinamide
N-[4-(3-chlorobenzyl)-4-dimethylamino-cyclohexyl]-N'-(1-methoxymethyl-2-phenylethyl) succinamide
N-[4-(3-chlorobenzyl)-4-dimethylamino-cyclohexyl]-N'-(3,4-dichlorobenzyl) succinamide
N-[4-(3-chlorobenzyl)-4-dimethylamino-cyclohexyl]-N'-furan-2-ylmethyl succinamide
N-[4-dimethylamino-4-(4-methyl-benzyl)-cyclohexyl]-N'-(1,2,3,4-tetrahydro-naphthalen-1-yl) succinamide
N-[4-(3-chlorobenzyl)-4-dimethylamino-cyclohexyl]-N'-(4-fluorobenzyl) succinamide
N-[4-dimethylamino-4-(3-methyl-benzyl)-cyclohexyl]-N'-indan-1-yl succinamide
N-[4-(3-chlorobenzyl)-4-dimethylamino-cyclohexyl]-4-(3,5-dimethyl-piperidin-1-yl)-4-oxo-butyramide
N-[4-dimethylamino-4-(3-methyl-benzyl)-cyclohexyl]-N'-phenethyl succinamide N-[4-dimethylamino-4-(4-methyl-benzyl)-cyclohexyl]-N'-phenethyl succinamide
N-(2-benzylsulphanyl-ethyl)-N'-[4-(3-chlorobenzyl)-4-dimethylamino-cyclohexyl] succinamide
N-[4-(3-chlorobenzyl)-4-dimethylamino-cyclohexyl-N'-(2,5-dichlorobenzyl) succinamide
4-[4-(3-chlorobenzyl)-piperazin-1-yl]-N-[4-(4-chlorobenzyl)-4-dimethylamino-cyclohexyl]-4-oxo-butyramide
N-[4-(4-chlorobenzyl)-4-dimethylamino-cyclohexyl]-N'-(1-phenyl-propyl) succinamide
N-(4-bromo-2-fluorobenzyl)-N'-[4-dimethylamino-4-(3-methyl-benzyl)-cyclohexyl] succinamide
N-[4-(3-chlorobenzyl)-4-dimethylamino-cyclohexyl]-N'-methyl-N'-pyridin-3-ylmethyl succinamide
N-[4-dimethylamino-4-(4-methyl-benzyl)-cyclohexyl]-N'-(2-ethoxy-benzyl) succinamide
N-[4-dimethylamino-4-(3-methyl-benzyl)-cyclohexyl]-N'-[2-(5-methoxy-1H-indol-3-yl)-ethyl] succinamide
N-(4-sec-butyl-phenyl)-N'-[4-(4-chlorobenzyl)-4-dimethylamino-cyclohexyl] succinamide
5-oxo-5-(4-phenyl-3,6-dihydro-2H-pyridin-1-yl) valeric acid (4-benzyl-4-dimethylamino-cyclohexyl) amide
N-[4-(3-chlorobenzyl)-4-dimethylamino-cyclohexyl]-N'-[2-(3-fluorophenyl)-ethyl] succinamide
N-(3-bromo-benzyl)-N'-[4-(3-chlorobenzyl)-4-dimethylamino-cyclohexyl] succinamide
N-(4-bromo-2-fluorobenzyl)-N'-[4-(3-chlorobenzyl)-4-dimethylamino-cyclohexyl] succinamide
N-[4-(3-chlorobenzyl)-4-dimethylamino-cyclohexyl]-N'-(1-phenyl-propyl) succinamide
N-[4-dimethylamino-4-(3-methyl-benzyl)-cyclohexyl]-N'-(4-fluoro-2-trifluoromethylbenzyl) succinamide
N-[4-(3-chlorobenzyl)-4-dimethylamino-cyclohexyl]-N'-indan-2-yl succinamide
N-[4-dimethylamino-4-(3-methyl-benzyl)-cyclohexyl]-4-oxo-4-(4-phenyl-3,6-dihydro-2H-pyridin-1-yl) butyramide
N-[4-(4-chlorobenzyl)-4-dimethylamino-cyclohexyl]-N'-[2-(2-fluorophenyl)-ethyl] succinamide
N-[4-dimethylamino-4-(4-methyl-benzyl)-cyclohexyl]-N'-[2-(2-fluorophenyl)-ethyl] succinamide
glutaric acid-(4-benzyl-4-dimethylamino-cyclohexyl) amide [2-(2-fluorophenyl)-ethyl] succinamide
N-[2-(2-chlorophenyl)-ethyl]-N'-[4-dimethylamino-4-(4-methyl-benzyl)-cyclohexyl] succinamide
N-[4-(3-chlorobenzyl)-4-dimethylamino-cyclohexyl]-N'-(3,4-difluorobenzyl) succinamide
N-benzyl-N-(2-cyano-ethyl)-N'-[4-dimethylamino-4-(4-methyl-benzyl)-cyclohexyl] succinamide
N-[4-(3-chlorobenzyl)-4-dimethylamino-cyclohexyl]-N'-(3-methyl-benzyl) succinamide
N-[4-(3-chlorobenzyl)-4-dimethylamino-cyclohexyl]-N'-(4-fluoro-3-trifluoromethylbenzyl) succinamide
N-[4-(3-chlorobenzyl)-4-dimethylamino-cyclohexyl]-N'-[2-(2-chlorophenyl)-ethyl] succinamide
N-[4-(3-chlorobenzyl)-4-dimethylamino-cyclohexyl]-N'-[2-(4-chlorophenyl)-ethyl] succinamide
N-[4-(3-chlorobenzyl)-4-dimethylamino-cyclohexyl]-N'-[2-(2,4-dichlorophenyl)-ethyl] succinamide
N-[4-(3-chlorobenzyl)-4-dimethylamino-cyclohexyl]-N'-indan-1-yl succinamide N-[4-(4-chlorobenzyl)-4-dimethylamino-cyclohexyl]-N'-indan-1-yl succinamide
N-[2-(4-chlorophenyl)-propyl]-N'-[4-dimethylamino-4-(3-methyl-benzyl)-cyclohexyl] succinamide
N-(3-bromobenzyl)-N'-[4-(4-chlorobenzyl)-4-dimethylamino-cyclohexyl] succinamide
N-[4-(3-chlorobenzyl)-4-dimethylamino-cyclohexyl]-N'-(3-phenyl-propyl) succinamide
N-[4-dimethylamino-4-(4-methyl-benzyl)-cyclohexyl]-N'-(2-p-tolyl-ethyl succinamide
N-[4-(3-chlorobenzyl)-4-dimethylamino-cyclohexyl]-N'-(2-thiophen-2-yl-ethyl) succinamide
glutaric acid-(4-benzyl-4-dimethylamino-cyclohexyl) amide [2-(4-chlorophenyl)-propyl] amide
N-benzyl-N'-[4-(3-chlorobenzyl)-4-dimethylamino-cyclohexyl]-N-methyl succinamide
N-[4-(3-chlorobenzyl)-4-dimethylamino-cyclohexyl]-N'-[2-(3-chlorophenyl)-ethyl] succinamide
glutaric acid-(4-benzyl-4-dimethylamino-cyclohexyl) amide [2-(2-ethoxy-phenyl)-ethyl] amide
glutaric acid-(4-benzyl-4-dimethylamino-cyclohexyl) amide phenethylamide
glutaric acid-(4-benzyl-4-dimethylamino-cyclohexyl) amide [2-(3-chlorophenyl)-ethyl] amide
N-[4-(3-chlorobenzyl)-4-dimethylamino-cyclohexyl]-N'-[1-(3-methoxyphenyl)-ethyl] succinamide
glutaric acid-(4-benzyl-4-dimethylamino-cyclohexyl) amide (1,3-dimethyl-butyl) amide
N-benzyl-N'-[4-dimethylamino-4-(3-methyl-benzyl)-cyclohexyl]-N-ethyl succinamide
N-(4-chlorobenzyl)-N'-[4-(3-chlorobenzyl)-4-dimethylamino-cyclohexyl] succinamide
N-[4-(3-chlorobenzyl)-4-dimethylamino-cyclohexyl]-N'-[2-(2-fluorophenyl)-ethyl] succinamide
N-[4-dimethylamino-4-(3-methyl-benzyl)-cyclohexyl]-N'-(2-fluorobenzyl) succinamide
N-[2-(4-chlorophenyl)-ethyl]-N'-[4-dimethylamino-4-(3-methyl-benzyl)-cyclohexyl] succinamide
N-[2-(2-chlorophenyl)-ethyl]-N'-[4-dimethylamino-4-(3-methyl-benzyl)-cyclohexyl] succinamide
N-[2-(3-chlorophenyl)-ethyl]-N'-[4-dimethylamino-4-(3-methyl-benzyl)-cyclohexyl] succinamide
N-benzyl-N'-[4-(3-chlorobenzyl)-4-dimethylamino-cyclohexyl]-N-(2-cyano-ethyl) succinamide
N-[4-(3-chlorobenzyl)-4-dimethylamino-cyclohexyl]-N'-(3-chloro-4-fluorobenzyl) succinamide
N-[4-dimethylamino-4-(3-methyl-benzyl)-cyclohexyl]-N'-(2-trifluoromethyl-benzyl) succinamide
N-[2-(2,4-dichlorophenyl)-ethyl[-N'-[4-dimethylamino-4-(3-methyl-benzyl)-cyclohexyl] succinamide
N-benzyl-N'-[4-(3-chlorobenzyl)-4-dimethylamino-cyclohexyl]-N-ethyl succinamide
N-[2-(4-chlorophenyl)-ethyl]-N'-[4-dimethylamino-4-(4-methyl-benzyl)-cyclohexyl] succinamide
5-oxo-5-(4-o-tolyl-piperazin-1-yl)-valeric acid (4-benzyl-4-dimethylamino-cyclohexyl) amide
N-[4-(3-chlorobenzyl)-4-dimethylamino-cyclohexyl]-N'-[3-(methyl-phenyl-amino)-propyl] succinamide
N-[4-(3-chlorobenzyl)-4-dimethylamino-cyclohexyl]-4-[4-(5-chloro-2-methylphenyl)-piperazin-1-yl]-4-oxo-butyramide
N-[4-dimethylamino-4-(3-methyl-benzyl)-cyclohexyl-N'-[2-(2-ethoxyphenyl)-ethyl] succinamide
4-[4-(4-chlorobenzoyl)-piperidin-1-yl]-N-[4-(3-chlorobenzyl)-4-dimethylamino-cyclohexyl]-4-oxo-butyramide
N-[4-(3-chlorobenzyl)-4-dimethylamino-cyclohexyl]-N'-[2-(2-ethoxyphenyl)-ethyl] succinamide
N-[2-(2,6-dichlorophenyl)-ethyl]-N'-[4-dimethylamino-4-(3-methyl-benzyl)-cyclohexyl] succinamide
N-benzohydryl-N'-[4-(3-chlorobenzyl)-4-dimethylamino-cyclohexyl] succinamide N-[4-(3-chlorobenzyl)-4-dimethylamino-cyclohexyl]-N'-cyclooctyl succinamide
N-[4-(3-chlorobenzyl)-4-dimethylamino-cyclohexyl]-N'-[2-(4-phenoxy-phenyl)-ethyl] succinamide
N-[4-dimethylamino-4-(3-methyl-benzyl)-cyclohexyl]-N'-(2,3-dimethyl-benzyl) succinamide
4-[4-(5-chloro-2-methyl-phenyl)-piperazin-1-yl]-N-[4-dimethylamino-4-(3-methyl-benzyl)-cyclohexyl]-4-oxo-butyramide
4-[4-(4-chlorobenzoyl)-piperidin-1-yl]-N-[4-dimethylamino-4-(3-methyl-benzyl)-cyclohexyl]-4-oxo-butyramide
N-[4-(3-chlorobenzyl)-4-dimethylamino-cyclohexyl]-N'-(1,2, 3,4-tetrahydro-naphthalen-1-yl) succinamide
N-(2-chlorobenzyl)-N'-[4-dimethylamino-4-(3-methyl-benzyl)-cyclohexyl] succinamide
N-(2-chlorobenzyl)-N'-[4-(3-chlorobenzyl)-4-dimethylamino-cyclohexyl] succinamide
N-[4-(3-chlorobenzyl)-4-dimethylamino-cyclohexyl]-N'-(3-fluoro-5-trifluoromethylbenzyl) succinamide
N-[4-(3-chlorobenzyl)-4-dimethylamino-cyclohexyl]-N'-(1-p-tolyl-ethyl) succinamide
N-[2-(2,6-dichloro-benzylsulphanyl)-ethyl]-N'-[4-dimethylamino-4-(4-methyl-benzyl)-cyclohexyl] succinamide
N-[4-(3-chlorobenzyl)-4-dimethylamino-cyclohexyl-N'-[2-(2,6-dichloro-benzylsulphanyl)-ethyl] succinamide
N-[4-(3-chlorobenzyl)-4-dimethylamino-cyclohexyl]-N'-[2-(3-trifluoromethylphenyl)-ethyl] succinamide
N-[2-(3,4-dichlorophenyl)-ethyl]-N'-[4-dimethylamino-4-(3-methyl-benzyl)-cyclohexyl] succinamide
N-[4-(3-chlorobenzyl)-4-dimethylamino-cyclohexyl]-4-[4-(2-fluorophenyl)-piperazin-1-yl]-4-oxo-butyramide
5-[4-(2,4-dimethoxy-phenyl)-piperazin-1-yl]-5-oxo-valeric acid (4-benzyl-4-dimethylamino-cyclohexyl) amide
N-[4-(3-chlorobenzyl)-4-dimethylamino-cyclohexyl]-N'-(1-naphthalen-1-yl-ethyl) succinamide
N-[4-dimethylamino-4-(3-methyl-benzyl)-cyclohexyl]-N'-(1-naphthalen-1-yl-ethyl) succinamide
N-[4-(3-chlorobenzyl)-4-dimethylamino-cyclohexyl]-N'-(2-fluorobenzyl)-N'-furan-2-ylmethyl succinamide
N-[4-dimethylamino-4-(3-methyl-benzyl)-cyclohexyl]-N'-(2-fluorobenzyl)-N'-furan-2-ylmethyl succinamide
N-[4-(3-chlorobenzyl)-4-dimethylamino-cyclohexyl]-N'-(2,5-dimethoxy-benzyl)-N'-furan-2-ylmethyl succinamide
N-(4-azepan-1-yl-4-benzyl-cyclohexyl)-4-(2,3-dihydro-indol-1-yl)-4-oxo-butyramide
4-(2,3-dihydro-indol-1-yl)-N-[4-dimethylamino-4-(3-fluorophenyl)-cyclohexyl]-4-oxo-butyramide
N-(4-benzyl-4-morpholin-4-yl-cyclohexyl)-N'-(2-chloro-6-methyl-benzyl) succinamide
N-(4-benzyl-4-morpholin-4-yl-cyclohexyl)-N'-[2-(3-methoxyphenyl)-ethyl] succinamide
N-(4-benzyl-4-morpholin-4-yl-cyclohexyl)-N'-(3-methyl-benzyl) succinamide
N-(4-benzyl-4-morpholin-4-yl-cyclohexyl)-N'-(4-bromo-2-fluorobenzyl) succinamide
N-(4-benzyl-4-morpholin-4-yl-cyclohexyl)-N'-pyridin-4-ylmethyl succinamide
N-(4-benzyl-4-morpholin-4-yl-cyclohexyl)-N'-indan-1-yl succinamide
N-(4-benzyl-4-morpholin-4-yl-cyclohexyl)-N'-(2,4-dimethoxy-benzyl) succinamide
N-(4-benzyl-4-morpholin-4-yl-cyclohexyl)-N'-thiophen-2-ylmethyl succinamide
N-adamantan-1-ylmethyl-N'-(4-benzyl-4-morpholin-4-yl-cyclohexyl) succinamide
N-(4-benzyl-4-morpholin-4-yl-cyclohexyl)-N'-(2,5-difluorobenzyl) succinamide
N-(4-benzyl-4-morpholin-4-yl-cyclohexyl)-N'-(4-tert-butyl-phenyl) succinamide
N-(4-benzyl-4-morpholin-4-yl-cyclohexyl)-N'-(3,5-bis-trifluoromethyl-benzyl) succinamide
N-(4-benzyl-4-morpholin-4-yl-cyclohexyl)-N'-[2-(2-ethoxyphenyl)-ethyl] succinamide
N-(4-benzyl-4-morpholin-4-yl-cyclohexyl)-N'-(3-methoxypropyl) succinamide
N-(4-benzyl-4-morpholin-4-yl-cyclohexyl)-N'-(2-morpholin-4-yl-ethyl) succinamide
N-(4-benzyl-4-morpholin-4-yl-cyclohexyl)-N'-(2,5-dichlorobenzyl) succinamide
N-(4-benzyl-4-morpholin-4-yl-cyclohexyl)-N'-[1-(2-benzyloxy-benzyl)-pyrrolidin-3-yl]-N'-methyl succinamide
N-(4-benzyl-4-morpholin-4-yl-cyclohexyl)-4-oxo-4-(2-pyrrolidin-1-ylmethyl-pyrrolidin-1-yl) butyramide
N-(4-benzyl-4-morpholin-4-yl-cyclohexyl)-4-morpholin-4-yl-4-oxo-butyramide
N-(4-benzyl-4-morpholin-4-yl-cyclohexyl)-N'-(3-dimethylamino-propyl)-N'-methyl succinamide
N-(4-benzyl-4-morpholin-4-yl-cyclohexyl)-N'-(2-phenyl-cyclopropyl) succinamide
N-(4-benzyl-4-morpholin-4-yl-cyclohexyl)-N'-naphthalen-1-ylmethyl succinamide
N-(4-benzyl-4-morpholin-4-yl-cyclohexyl)-4-(4-methyl-[1,4]diazepan-1-yl)-4-oxo butyramide
N-(4-benzyl-4-morpholin-4-yl-cyclohexyl)-4-(4-hydroxy-piperidin-1-yl)-4-oxo-butyramide
N-(4-benzyl-4-morpholin-4-yl-cyclohexyl)-N'-[2-(7-methyl-1H-indol-3-yl)-ethyl] succinamide
N-(4-benzyl-4-morpholin-4-yl-cyclohexyl)-N'-[2-(5-methoxy-1H-indol-3-yl)-ethyl] succinamide
N-(4-benzyl-4-morpholin-4-yl-cyclohexyl)-4-oxo-4-(4-quinolin-2-ylmethyl-piperazin-1-yl) butyramide
N-(4-dimethylamino-4-thiophen-2-yl-cyclohexyl)-4-oxo-4-[4-(5-trifluoromethyl-pyridin-2-yl)-piperazin-1-yl] butyramide
glutaric acid-(1-adamantan-1-yl-ethyl) amide (4-dimethylamino-4-thiophen-2-yl-cyclohexyl) amide
N-[2-(2,6-dichlorophenyl)-ethyl]-N'-(4-dimethylamino-4-thiophen-2-yl-cyclohexyl) succinamide
N-(4-dimethylamino-4-thiophen-2-yl-cyclohexyl)-N'-(1-methyl-3-phenyl-propyl) succinamide
N-benzyl-N'-(4-dimethylamino-4-thiophen-2-yl-cyclohexyl)-N-ethyl succinamide
N-[2-(4-bromophenyl)-ethyl]-N'-(4-dimethylamino-4-thiophen-2-yl-cyclohexyl) succinamide
4-[4-(4-chlorobenzoyl)-piperidin-1-yl]-N-(4-dimethylamino-4-thiophen-2-yl-cyclohexyl)-4-oxo-butyramide
glutaric acid-[1-(2,6-dichlorobenzyl)-pyrrolidin-3-yl] amide (4-dimethylamino-4-thiophen-2-yl-cyclohexyl) amide
glutaric acid-(4-dimethylamino-4-thiophen-2-yl-cyclohexyl) amide (3-phenyl-propyl) amide
N-(4-dimethylamino-4-phenyl-cyclohexyl)-N'-pentyl succinamide
3-(4-chlorophenyl)-2-[4-(4-dimethylamino-4-thiophen-2-yl-cyclohexylcarbamoyl)-butyrylamino] ethyl propionate
glutaric acid-(4-dimethylamino-4-thiophen-2-yl-cyclohexyl) amide indan-1-ylamide
N-adamantan-1-ylmethyl-N'-(4-dimethylamino-4-thiophen-2-yl-cyclohexyl) succinamide
5-(4-benzofuran-3-ylmethyl-piperazin-1-yl)-5-oxo-valeric acid (4-dimethylamino-4-thiophen-2-yl-cyclohexyl) amide
4-[4-(4-tert-butyl-benzyl)-piperazin-1-yl]-N-(4-dimethylamino-4-phenyl-cyclohexyl)-4-oxo-butyramide
glutaric acid-(4-dimethylamino-4-thiophen-2-yl-cyclohexyl) amide [2-(1H-indol-3-yl)-ethyl] amide
N-(4-dimethylamino-4-phenyl-cyclohexyl)-4-[4-hydroxy-4-(3-trifluoromethylphenyl)-piperidin-1-yl]-4-oxo-butyramide
5-(3,4-dihydro-1H-isoquinolin-2-yl)-5-oxo-valeric acid (4-dimethylamino-4-thiophen-2-yl-cyclohexyl) amide
4-(4-benzyl-piperidin-1-yl)-N-(4-dimethylamino-4-phenyl-cyclohexyl)-4-oxo-butyramide
{[4-(4-dimethylamino-4-thiophen-2-yl-cyclohexylcarbamoyl)-butyryl]-methylamino} benzyl acetate
N-benzyl-N'-(4-dimethylamino-4-phenyl-cyclohexyl)-N-phenethyl succinamide glutaric acid-(4-dimethylamino-4-thiophen-2-yl-cyclohexyl) amide 3-trifluoromethoxy-benzylamide
glutaric acid-(4-dimethylamino-4-thiophen-2-yl-cyclohexyl) amide ethyl-(2-methylallyl) amide
N,N-bis-(2-cyano-ethyl)-N'-(4-dimethylamino-4-thiophen-2-yl-cyclohexyl) succinamide
glutaric acid-benzyl-methyl amide (4-dimethylamino-4-thiophen-2-yl-cyclohexyl) amide
glutaric acid-(2-benzyloxy-cyclopentyl) amide (4-dimethylamino-4-thiophen-2-yl-cyclohexyl) amide
glutaric acid-(4-dimethylamino-4-thiophen-2-yl-cyclohexyl) amide 4-trifluoromethyl-benzylamide
N-(4-dimethylamino-4-thiophen-2-yl-cyclohexyl)-N'-[2-(4-phenoxy-phenyl)-ethyl] succinamide
glutaric acid-(4-dimethylamino-4-thiophen-2-yl-cyclohexyl) amide (3,3-diphenyl-propyl) amide
4-(4-benzyl-piperidin-1-yl)-N-(4-dimethylamino-4-thiophen-2-yl-cyclohexyl)-4-oxo-butyramide
N-(4-dimethylamino-4-thiophen-2-yl-cyclohexyl)-4-[4-hydroxy-4-(4-trifluoromethylphenyl)-piperidin-1-yl]-4-oxo-butyramide
glutaric acid-(4-dimethylamino-4-thiophen-2-yl-cyclohexyl) amide 4-trifluoro-2-trifluoromethyl-benzylamide
N-(4-dimethylamino-4-thiophen-2-yl-cyclohexyl)-N'-pentyl succinamide
5-[4-(2-methoxy-naphthalen-1-ylmethyl)-piperazin-1-yl]-5-oxo-valeric acid (4-dimethylamino-4-thiophen-2-yl-cyclohexyl) amide
N-(4-dimethylamino-4-thiophen-2-yl-cyclohexyl)-N'-(2-ethoxybenzyl)-N'-furan-2-ylmethyl succinamide
glutaric acid-(4-dimethylamino-4-thiophen-2-yl-cyclohexyl) amide (2-phenylcyclopropyl) amide
N-[2-(3,4-dichlorophenyl)-ethyl]-N'-(4-dimethylamino-4-phenyl-cyclohexyl) succinamide
N-adamantan-1-ylmethyl-N'-(4-dimethylamino-4-phenyl-cyclohexyl) succinamide
5-[4-(4-fluorophenyl)-3,6-dihydro-2H-pyridin-1-yl]-5-oxo-valeric acid (4-dimethylamino-4-thiophen-2-yl-cyclohexyl) amide
2-[4-(4-dimethylamino-4-thiophen-2-yl-cyclohexylcarbamoyl)-butyrylamino]-4-methyl benzyl valerate
{4-[4-(4-dimethylamino-4-thiophen-2-yl-cyclohexylcarbamoyl)-butyrylamino]-phenyl}tert-butyl carbamate
glutaric acid-(4-dimethylamino-4-thiophen-2-yl-cyclohexyl) amide [2-(3-methoxyphenyl)-ethyl] amide
glutaric acid benzyl-ethylamide (4-dimethylamino-4-thiophen-2-yl-cyclohexyl) amide
glutaric acid 3-chloro-benzylamide (4-dimethylamino-4-thiophen-2-yl-cyclohexyl) amide
glutaric acid-(4-dimethylamino-4-thiophen-2-yl-cyclohexyl) amide (4-methylcyclohexyl) amide
N-(4-dimethylamino-4-phenyl-cyclohexyl)-N'-[2-(1H-indol-3-yl)-ethyl]-N'-methyl succinamide
glutaric acid 2,5-dichloro-benzylamide (4-dimethylamino-4-thiophen-2-yl-cyclohexyl) amide
glutaric acid-(4-dimethylamino-4-thiophen-2-yl-cyclohexyl) amide [2-(3-fluorophenyl)-ethyl] amide
N-(4-dimethylamino-4-phenyl-cyclohexyl)-N'-[2-(4-methoxy-phenoxy)-ethyl] succinamide
N-(4-dimethylamino-4-thiophen-2-yl-cyclohexyl)-N'-[2-(ethyl-m-tolyl-amino)-ethyl] succinamide
glutaric acid-(4-benzyloxy-phenyl) amide (4-dimethylamino-4-thiophen-2-yl-cyclohexyl) amide
glutaric acid-(4-dimethylamino-4-thiophen-2-yl-cyclohexyl) amide [2-(4-fluorophenyl)-ethyl] amide
glutaric acid-[2-(4-bromophenyl)-ethyl] amide (4-dimethylamino-4-thiophen-2-yl-cyclohexyl) amide
N-(4-dimethylamino-4-thiophen-2-yl-cyclohexyl)-N'-(4-phenoxy-phenyl) succinamide
N-(4-dimethylamino-4-phenyl-cyclohexyl)-N'-[2-(3-trifluoromethyl-phenyl)-ethyl] succinamide
glutaric acid-[2-(4-chlorophenyl)-propyl] amide (4-dimethylamino-4-thiophen-2-yl-cyclohexyl) amide
N-(4-dimethylamino-4-thiophen-2-yl-cyclohexyl)-N'-[2-(1H-indol-3-yl)-ethyl]-N'-methyl succinamide
N-(4-dimethylamino-4-thiophen-2-yl-cyclohexyl)-N'-[2-(4-methoxy-phenoxy)-ethyl] succinamide
glutaric acid-(4-dimethylamino-4-thiophen-2-yl-cyclohexyl) amide (3-phenoxyphenyl) amide
N-(4-dimethylamino-4-phenyl-cyclohexyl)-N'-[2-(ethyl-m-tolyl-amino)-ethyl] succinamide
glutaric acid-[2-(5-bromo-2-methoxyphenyl)-ethyl] amide (4-dimethylamino-4-thiophen-2-yl-cyclohexyl) amide
N-(4-dimethylamino-4-thiophen-2-yl-cyclohexyl)-N'-[2-(3-trifluoromethyl-phenyl)-ethyl] succinamide
glutaric acid-(4-dimethylamino-4-thiophen-2-yl-cyclohexyl) amide (1-methyl-3-phenyl-propyl) amide
N-[2-(2,6-dichloro-benzylsulphanyl)-ethyl]-N'-(4-dimethylamino-4-thiophen-2-yl-cyclohexyl) succinamide
glutaric acid-(4-dimethylamino-4-thiophen-2-yl-cyclohexyl) amide (2,2-diphenyl-propyl) amide
N-(4-dimethylamino-4-thiophen-2-yl-cyclohexyl)-N'-[3-(methyl-phenyl-amino)-propyl] succinamide
N-(1-biphenyl-4-ylmethyl-pyrrolidin-3-yl)-N'-(4-dimethylamino-4-thiophen-2-yl-cyclohexyl)-N-methyl succinamide
glutaric acid-[4-(cyano-phenyl-methyl)-phenyl] amide (4-dimethylamino-4-thiophen-2-yl-cyclohexyl) amide
glutaric acid- (4-tert-butyl-cyclohexyl) amide (4-dimethylamino-4-thiophen-2-yl-cyclohexyl) amide
glutaric acid-(4-sec-butyl-phenyl) amide (4-dimethylamino-4-thiophen-2-yl-cyclohexyl) amide
glutaric acid-(4-butyl-phenyl)-amide (4-dimethylamino-4-thiophen-2-yl-cyclohexyl) amide
N-(2-benzylsulphanyl-ethyl)-N'-(4-dimethylamino-4-thiophen-2-yl-cyclohexyl) succinamide
N-(4-dimethylamino-4-thiophen-2-yl-cyclohexyl)-N'-(2-phenoxy-ethyl) succinamide
glutaric acid-(4-dimethylamino-4-thiophen-2-yl-cyclohexyl) amide (naphthalen-1-ylmethyl) amide
glutaric acid 3-bromo-benzylamide (4-dimethylamino-4-thiophen-2-yl-cyclohexyl) amide
N-(4-dimethylamino-4-thiophen-2-yl-cyclohexyl)-N'-(3-phenyl-propyl) succinamide
N-[2-(3-chlorophenyl)-ethyl]-N'-(4-dimethylamino-4-phenyl-cyclohexyl) succinamide
2-[3-(4-dimethylamino-4-thiophen-2-yl-cyclohexylcarbamoyl)-propionylamino] benzyl propionate
N-[2-(2-chlorophenyl)-ethyl]-N'-(4-dimethylamino-4-phenyl-cyclohexyl) succinamide
N-(4-dimethylamino-4-phenyl-cyclohexyl)-N'-[2-(3-fluorophenyl)-ethyl] succinamide
glutaric acid-(4-dimethylamino-4-thiophen-2-yl-cyclohexyl) amide (2-thiophen-2-yl-ethyl) amide
glutaric acid-(4-dimethylamino-4-thiophen-2-yl-cyclohexyl) amide [2-(4-phenoxy-phenyl)-ethyl] amide
N-[2-(4-chlorophenyl)-ethyl]-N'-(4-dimethylamino-4-phenyl-cyclohexyl) succinamide
N-[2-(3-chlorophenyl)-ethyl]-N'-(4-dimethylamino-4-thiophen-2-yl-cyclohexyl) succinamide
N-benzyl-N'-(4-dimethylamino-4-thiophen-2-yl-cyclohexyl)-N-ethyl succinamide glutaric acid-[1-(2,6-dichloro-benzyl)-pyrrolidin-3-yl] amide (4-dimethylamino-4-thiophen-2-yl-cyclohexyl) amide
glutaric acid-(4-dimethylamino-4-thiophen-2-yl-cyclohexyl) amide (3-phenyl-propyl) amide
glutaric acid-(4-dimethylamino-4-thiophen-2-yl-cyclohexyl) amide [2-(1H-indol-3-yl)-ethyl] amide
5-(3,4-dihydro-1H-isoquinolin-2-yl)-5-oxo-valeric acid (4-dimethylamino-4-thiophen-2-yl-cyclohexyl) amide
{[4-(4-dimethylamino-4-thiophen-2-yl-cyclohexylcarbamoyl)-butyryl]-methylamino} benzyl acetate
N-(4-dimethylamino-4-thiophen-2-yl-cyclohexyl)-N'-[2-(4-phenoxy-phenyl)-ethyl] succinamide
glutaric acid-(4-dimethylamino-4-thiophen-2-yl-cyclohexyl) amide (2-phenyl-cyclopropyl) amide
N-[4-(3-chlorobenzyl)-4-dimethylaminocyclohexyl]-N'-furan-2-ylmethyl succinamide hydrochloride, non-polar diastereoisomer
N-[4-(3-chlorobenzyl)-4-dimethylaminocyclohexyl]-N'-furan-2-ylmethyl succinamide hydrochloride, polar diastereoisomer
glutaric acid (4-benzyl-4-dimethylaminocyclohexyl) amide [2-(2-fluorophenyl)ethyl] amide hydrochloride, non-polar diastereoisomer
glutaric acid (4-benzyl-4-dimethylaminocyclohexyl) amide [2-(2-fluorophenyl)ethyl] amide hydrochloride, polar diastereoisomer
N-[4-(3-chlorobenzyl)-4-dimethylaminocyclohexyl]-N'-[2-(4-chlorophenyl)ethyl] succinamide hydrochloride, non-polar diastereoisomer
N-[4-(3-chlorobenzyl)-4-dimethylaminocyclohexyl]-N'-[2-(4-chlorophenyl)ethyl] succinamide hydrochloride, polar diastereoisomer
glutaric acid (4-benzyl-4-dimethylaminocyclohexyl)amide [2-(4-chlorophenyl)ethyl] amide hydrochloride, non-diastereoisomer
in the form of the racemate; the enantiomers, diastereomers, mixtures of the enantiomers or diastereomers or a single enantiomer or diastereomer; of the bases and/or salts of physiologically compatible acids or cations.

14. Method for producing substituted cyclohexyl-1,4-diamine derivatives according to one of claims 1 to 13, **characterised in that** substituted cyclohexyl-1,4-diamines are bonded via anhydrides, open-chain dicarboxylic acids or their activated analogues, in particular their acid halides, with further primary or secondary amines by adding coupling reagents.

15. Method for producing substituted cyclohexyl-1,4-diamine derivatives according to one of claims 1 to 13, **characterised in that** anhydrides, open-chain dicarboxylic acids or their activated analogues, in particular their acid halides, are bonded with primary or secondary amines and then with substituted cyclohexyl-1,4-diamines by adding coupling reagents.

16. Medication containing at least one substituted cyclohexyl-1,4-diamine derivative according to one of claims 1 to 13, possibly in the form of its racemate, the pure stereoisomers, in particular enantiomers and diastereomers, in any desired mixture ratio; in the form of its acids or its bases or in the form of its salts, in particular the physiologically compatible salts or salts of physiologically compatible acids or cations; or in the form of its solvates, in particular hydrates, and also possibly containing suitable additives and/or adjuvants and/or possibly further active substances.

17. Use of a substituted cyclohexyl-1,4-diamine derivative according to one of claims 1 to 13, possibly in the form of its racemate, the pure stereoisomers, in particular enantiomers and diastereomers, in any desired mixture ratio; in the form of its acids or its bases or in the form of its salts, in particular the physiologically compatible salts or salts of physiologically compatible acids or cations; or in the form of its solvates, in particular hydrates, for the production of a medication for the treatment of pain, in particular acute, neuropathic or chronic pain.

18. Use of a substituted cyclohexyl-1,4-diamine derivative according to one of claims 1 to 13, for the production of a medication for the treatment of anxiety conditions, stress and stress-related syndromes, depressive illnesses, epilepsy, Alzheimer's disease, senile dementia, catalepsy, general cognitive dysfunctions, learning and memory disabilities (as nootropic), withdrawal symptoms, alcohol and/or drug and/or medication misuse and/or dependence, sexual dysfunctions, cardiovascular diseases, hypotension, hypertension, tinitus, pruritus, migraine, hearing impairment, deficient intestinal motility, eating disorders, anorexia, bulimia, mobility disorders, diarrhoea, cachexia, urinary incontinence, or as muscle relaxant, anticonvulsive or anaesthetic, or for coadministration in the treatment with an opioid analgesic or with an anaesthetic, for diuresis or anti-natriuresis, anxiolysis, for modulating movement activity, for modulating neurotransmitter release and for treating neuro-degenerative diseases associated therewith, for treating withdrawal symptoms and/or for reducing the addiction potential of opioids.

## Revendications

1. Dérivés de cyclohexyl-1,4-diamine substitués de formule générale I, 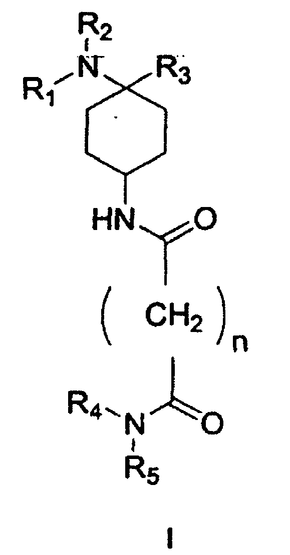 dans laquelle
n représente 1, 2, 3, 4 ou 5
R¹ et R² représentent, indépendamment l'un de l'autre, H ; un groupe alkyle en C₁-C₅ dans chaque cas saturé ou insaturé, ramifié ou non ramifié, non substitué ou une ou plusieurs fois substitué ; un groupe cycloalkyle en C₃-C₈ chaque fois non substitué ou une ou plusieurs fois substitué ; ou un groupe aryle, cycloalkyle en C₃-C₈ ou hétéroaryle, chaque fois non substitué ou une ou plusieurs fois substitué, lié par un groupe alkyle en C₁-C₃ ;
ou les radicaux R¹ et R² représentent ensemble un groupe CH₂CH₂OCH₂CH₂, CH₂CH₂NR¹⁰CH₂CH₂ ou (CH₂)₃₋₆ ;
R¹⁰ représentant H ; un groupe alkyle en C₁-C₅ respectivement saturé ou insaturé, ramifié ou non ramifié, non substitué ou une ou plusieurs fois substitué ; un groupe cycloalkyle en C₃-C₈ respectivement non substitué ou une ou plusieurs fois substitué ; un groupe aryle ou hétéroaryle, chacun non substitué ou une ou plusieurs fois substitué ; ou un groupe aryle, cycloalkyle en C₃-C₈ ou hétéroaryle, chacun non substitué ou une
ou plusieurs fois substitué, lié par un groupe alkyle en C₁-C₃ ; un groupe C(O)phényle, C(O)hétéroaryle, C(O)-alkyle(C₁-C₅), chacun substitué ou non substitué ;
R³ représente un groupe alkyle en C₁-C₅ respectivement saturé ou insaturé, ramifié ou non ramifié, non substitué ou une ou plusieurs fois substitué ; un groupe cycloalkyle en C₃-C₈ respectivement non substitué ou une ou plusieurs fois substitué ; un groupe aryle ou hétéroaryle, chacun non substitué ou une ou plusieurs fois substitué ; un groupe aryle, hétéroaryle ou cycloalkyle en C₃-C₈, chacun non substitué ou une ou plusieurs fois substitué, lié par un groupe alkyle en C₁-C₃ ;
R⁴ représente un groupe -(CR⁶R⁷)ₚR⁸,
dans lequel p représente 0, 1, 2, 3 ou 4 R⁶ représente H ou un groupe alkyle en C₁-C₅, dans chaque cas saturé ou insaturé, ramifié ou non ramifié, non substitué ou une ou plusieurs fois substitué,
R⁷ représente H, un groupe alkyle en C₁-C₅, dans chaque cas saturé ou insaturé, ramifié ou non ramifié, non substitué ou une ou plusieurs fois substitué, ou COOR⁹,
ou R⁶ et R⁷ forment un cycle (CH₂)ₖCHR⁸(CH₂)ₘ, où k = 1, 2, 3 et m = 1, 2,
R⁸ représente un groupe alkyle en C₁-C₆, dans chaque cas saturé ou insaturé, ramifié ou non ramifié, non substitué ou une ou plusieurs fois substitué ; un groupe cycloalkyle en C₃-C₈, aryle
ou hétéroaryle, chaque fois non substitué ou une
ou plusieurs fois substitué,
R⁹ représente H ou un groupe alkyle en C₁-C₅,
R⁵ représente H ou un groupe -(CH₂)₁R⁸,
l représentant 1, 2 ou 3,
ou représente conjointement avec R⁴ un groupe CH₂CHR¹⁴OCHR¹⁴CH₂, CH₂CH₂SCH₂CH₂, CH₂CH₂NR¹¹CH₂CH₂, (CR¹²R¹³)₃₋₆ ou 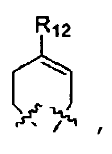 R¹¹ représentant H ; un groupe alkyle en C₁-C₅ dans chaque cas saturé ou insaturé, ramifié ou non ramifié, non substitué ou une ou plusieurs fois substitué ; un groupe cycloalkyle en C₃-C₈ chaque fois saturé ou non saturé, non substitué ou une ou plusieurs fois substitué ; un groupe aryle ou hétéroaryle, chaque fois non substitué ou une ou plusieurs fois substitué ; ou un groupe aryle, cycloalkyle en C₃-C₈ ou hétéroaryle, chaque fois non substitué ou une ou plusieurs fois substitué, lié par un groupe alkyle en C₁-C₃ ;
R¹² représentant H ; un groupe alkyle en C₁-C₅ dans chaque cas saturé ou insaturé, ramifié
ou non ramifié, non substitué ou une ou plusieurs fois substitué ; OH, C(O)phényle, non substitué ou une ou plusieurs fois substitué ; un groupe cycloalkyle en C₃-C₈ chaque fois non substitué ou une ou plusieurs fois substitué ; un groupe aryle ou hétéroaryle, chaque fois non substitué ou une ou plusieurs fois substitué ; ou un groupe aryle, cycloalkyle en C₃-C₈ ou hétéroaryle, chaque fois non substitué ou une ou plusieurs fois substitué, lié par un groupe alkyle en C₁-C₃ ;
et R¹³ représentant H ou OH ou bien forme conjointement avec R¹², par l'intermédiaire du même atome de carbone (liaison spiro) ou d'un atome de carbone voisin, un cycle à cinq chaînons ou à six chaînons, contenant des hétéroatomes, saturé ou insaturé, substitué
ou non substitué et/ou pouvant faire partie d'un système polycyclique ;
R¹⁴ représentant H ; un groupe alkyle en C₁-C₃ dans chaque cas saturé ou insaturé, ramifié
ou non ramifié, non substitué ou une ou plusieurs fois substitué ;
sous forme du racémate ; des énantiomères, des diastéréoisomères, de mélanges des énantiomères ou diastéréoisomères ou d'un énantiomère individuel ou d'un diastéréoisomère individuel ; des bases et/ou des sels avec des acides ou cations physiologiquement acceptables.

2. Dérivés de cyclohexyl-1,4-diamine substitués selon la revendication 1, **caractérisés en ce que**
R¹ et R² représentent, indépendamment l'un de l'autre, H ; un groupe alkyle en C₁-C₅ saturé ou insaturé, ramifié ou non ramifié, non substitué
ou une ou plusieurs fois substitué ;
ou les radicaux R¹ et R² forment ensemble un cycle et représentent CH₂CH₂OCH₂CH₂, CH₂CH₂NR¹⁰CH₂CH₂ ou (CH₂)₃₋₆,
R¹⁰ représentant H ; un groupe alkyle en C₁-C₅ saturé ou insaturé, ramifié ou non ramifié, non substitué ou une
ou plusieurs fois substitué.

3. Dérivés de cyclohexyl-1,4-diamine substitués selon la revendication 1 ou 2, **caractérisés en ce que** R¹ et R² représentent, indépendamment l'un de l'autre, CH₃
ou H, R¹ et R² ne représentant pas simultanément H, ou R¹ et R² représentent CH₂CH₂OCH₂CH₂ ou (CH₂)₆.

4. Dérivés de cyclohexyl-1,4-diamine substitués selon l'une quelconque des revendications 1 à 3, **caractérisés en ce que**
R³ représente un groupe cyclopentyle, cyclohexyle, phényle, benzyle, naphtyle, anthracényle, thiophényle, benzothiophényle, furyle, benzofurannyle, benzodioxolanyle, indolyle, indanyle, benzodioxannyle, pyrrolyle, pyridyle, pyrimidyle ou pyrazinyle, chacun non substitué ou une ou plusieurs fois substitué ; un groupe cycloalkyle en C₅-C₆, phényle, naphtyle, anthracényle, thiophényle, benzothiophényle, pyridyle, furyle, benzofurannyle, benzodioxolanyle, indolyle, indanyle, benzodioxannyle, pyrrolyle, pyridyle, pyrimidyle ou pyrazinyle, chacun non substitué ou une ou plusieurs fois substitué, lié par un groupe alkyle en C₁-C₂ saturé, non ramifié ;
en particulier
R³ représente un groupe phényle, furyle, thiophényle, naphtyle, benzyle, benzofurannyle, indolyle, indanyle, benzodioxanyle, benzodioxolanyle, pyridyle, pyrimidyle, pyrazinyle ou benzothiophényle, chacun non substitué ou une ou plusieurs fois substitué ; un groupe phényle, furyle ou thiophényle, chacun non substitué ou une ou plusieurs fois substitué, lié par un groupe alkyle en C₁-C₂ saturé, non ramifié.

5. Dérivés de cyclohexyl-1,4-diamine substitués selon l'une quelconque des revendications 1 à 4, **caractérisés en ce que** R³ représente un groupe phényle, thiophényle, pyridyle ou benzyle, chacun substitué ou non substitué, de façon particulièrement préférée le groupe 4-méthylbenzyle, 3-chlorobenzyle, 4-chlorobenzyle, 3-méthylbenzyle, benzyle, phényle, thiophényle ou 3-fluorophényle.

6. Dérivés de cyclohexyl-1,4-diamine substitués selon l'une quelconque des revendications 1 à 5, **caractérisés en ce que** n représente 2 ou 3.

7. Dérivés de cyclohexyl-1,4-diamine substitués selon l'une quelconque des revendications 1 à 6, **caractérisés en ce que** R⁴ représente -(CR⁶R⁷)ₚR⁸ et R⁵ représente H.

8. Dérivés de cyclohexyl-1,4-diamine substitués selon l'une quelconque des revendications 1 à 6, **caractérisés en ce que** R⁴ représente (CH₂)ₚR⁸ et R⁵ représente -(CH₂)₁R⁸.

9. Dérivés de cyclohexyl-1,4-diamine substitués selon l'une quelconque des revendications 1 à 6, **caractérisés en ce que** R⁴ et R⁵ représentent ensemble un groupe CH₂CHR¹⁴OCHR¹⁴CH₂, CH₂CH₂SCH₂CH₂, CH₂CH₂NR¹¹CH₂CH₂, (CR¹²R¹³)₃₋₆ ou

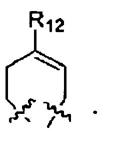

10. Dérivés de cyclohexyl-1,4-diamine substitués selon l'une quelconque des revendications 1 à 7, **caractérisés en ce que** R⁶ représente H et R⁷ représente H, CH₃, un groupe benzyle non substitué ou une ou plusieurs fois substitué, ou COOR⁹, ou R⁶ et R⁷ forment un cycle (CH₂)ₖCHR⁸(CH₂)ₘ.

11. Dérivés de cyclohexyl-1,4-diamine substitués selon l'une quelconque des revendications 1 à 8 ou 10, **caractérisés en ce que** R⁸ représente un groupe alkyle en C₁-C₆, chaque fois saturé ou insaturé, ramifié ou non ramifié, non substitué ou une ou plusieurs fois substitué ; cyclobutyle, cyclopropyle, cyclopentyle, cyclohexyle, cycloheptyle, cyclo-octyle, anthracényle, indolyle, naphtyle, benzofurannyle, benzothiophényle, indanyle, benzodioxannyle, benzodioxolanyle, acénaphtyle, carbazolyle, phényle, thiophényle, furyle, pyrrolidinyle, tétrahydrofuryle, pipérazinyle, pyridyle, pyrrolyle, pyrazinyle ou pyrimidyle, fluorényle, fluoranthényle, benzothiazolyle, benzotriazolyle ou benzo[1,2,5]thiazolyle ou 1,2-dihydroacénaphtényle, pyridinyle, furannyle, benzofurannyle, pyrazolinonyle, oxopyrazolinonyle, dioxolannyle, adamantyle, pyrimidinyle, quinolinyle, isoquinolinyle, phtalazinyle ou quinazolinyle, chacun non substitué ou une ou plusieurs fois substitué.

12. Dérivés de cyclohexyl-1,4-diamine substitués selon l'une quelconque des revendications 1 à 8 ou 10, **caractérisés en ce que** R⁸ représente un groupe alkyle en C₁-C₆, chaque fois saturé ou insaturé, ramifié ou non ramifié, non substitué ou une ou plusieurs fois substitué ; un groupe pyrrolidinyle, morpholinyle, tétrahydrofuryle, tétrahydronaphtyle, dihydro-indolyle, pyridyle, thiényle, pipérazinyle, naphtyle, indanyle, quinolinyle, cyclopropyle, cyclopentyle, cyclohexyle, cycloheptyle, cyclo-octyle, adamantyle, furyle, benzofuryle, phényle ou indolyle, chacun non substitué
ou une ou plusieurs fois substitué.

13. Dérivés de cyclohexyl-1,4-diamine substitués selon l'une quelconque des revendications 1 à 12, choisis dans le groupe
N-[1-(2,6-dichloro-benzyl)-pyrrolidin-3-yl]-N'-[4-diméthylamino-4-(4-méthyl-benzyl)-cyclohexyl]-succinamide
N-[4-(3-chloro-benzyl)-4-diméthylamino-cyclohexyl]-N'-(2-oxo-tétrahydro-furann-3-yl)-succinamide
N-[4-(4-chloro-benzyl)-4-diméthylamino-cyclohexyl]-N'-(3,3-diphényl-propyl)-succinamide
N-[4-diméthylamino-4-(4-méthyl-benzyl)-cyclohexyl]-4-[4-(7-méthoxy-benzo[1,3]dioxol-5-ylméthyl)-pipérazin-1-yl]-4-oxo-butyramide
N-[4-(4-chloro-benzyl)-4-diméthylamino-cyclohexyl]-4-(3,6-dihydro-2H-pyridin-1-yl)-4-oxo-butyramide
N-[4-diméthylamino-4-(4-méthyl-benzyl-cyclohexyl)-4-oxo-4-(4-m-tolyl-pipérazin-1-yl)-butyramide
2-{3-[4-(4-chloro-benzyl)-4-diméthylamino-cyclohexylcarbamoyl]-propionylamino}-propionate d'éthyle
N-[4-(3-chloro-benzyl)-4-diméthylamino-cyclohexyl]-4-(8-fluoro-1,3,4,5-tétrahydro-pyrido[4,3-b]indol-2-yl)-4-oxo-butyramide
3-{3-[4-diméthylamino-4-(4-méthyl-benzyl)-cyclohexyl-carbamoyl]-propionylamino}-propionate d'éthyle
N-[4-diméthylamino-4-(4-méthyl-benzyl)-cyclohexyl]-4-[4-(5-méthyl-pyrazin-2-carbonyl)-pipérazin-1-yl]-4-oxo-butyramide
N-[4-diméthylamino-4-(4-méthyl-benzyl)-cyclohexyl]-N'-méthyl-N'-[2-(2,2,2-trifluoro-acétylamino)-éthyl]-succinamide
N-[4-(3-chloro-benzyl)-4-diméthylamino-cyclohexyl]-N'-(2-pyridin-2-yl-éthyl)-succinamide
N-[4-(4-chloro-benzyl)-4-diméthylamino-cyclohexyl]-4-oxo-4-thiomorpholin-4-yl-butyramide
N-[4-(4-chloro-benzyl)-4-diméthylamino-cyclohexyl]-4-[4-(5-méthyl-pyrazin-2-carbonyl)-pipérazin-1-yl]-4-oxo-butyramide
N-[4-diméthylamino-4-(3-méthyl-benzyl)-cyclohexyl]-N'-(2-thiophén-2-yl-éthyl)-succinamide
N-[4-(4-chloro-benzyl)-4-diméthylamino-cyclohexyl]-4-[4-(4-éthoxy-phényl)-pipérazin-1-yl]-4-oxo-butyramide
N-allyl-N'-[4-(4-chloro-benzyl)-4-diméthylamino-cyclohexyl]-N-méthyl-succinamide
(4-benzyl-4-diméthylamino-cyclohexyl)-amide d'acide 5-[4-(2,5-diméthoxy-benzyl)-pipérazin-1-yl]-5-oxo-valérique
4-(3,6-dihydro-2H-pyridin-1-yl)-N-[4-diméthylamino-4-(4-méthyl-benzyl)-cyclohexyl]-4-oxo-butyramide
N-[4-diméthylamino-4-(3-méthyl-benzyl)-cyclohexyl]-4-(4-méthyl-pipéridin-1-yl)-4-oxo-butyramide
N-[4-diméthylamino-4-(4-méthyl-benzyl)-cyclohexyl]-4-oxo-4-(4-pyridin-2-yl-pipérazin-1-yl)-butyramide
({3-[4-diméthylamino-4-(3-méthyl-benzyl)-cyclohexyl-carbamoyl]-propionyl}-méthyl-amino)-acétate de benzyle
4-[4-(2,6-dichloro-benzyl)-pipérazin-1-yl]-N-[4-diméthylamino-4-(3-méthyl-benzyl)-cyclohexyl]-4-oxo-butyramide
{[4-(4-benzyl-4-diméthylamino-cyclohexylcarbamoyl)-butyryl]-méthyl-amino}-acétate de tert-butyle (4-benzyl-4-diméthylamino-cyclohexyl)-amide d'acide 5-[4-(2-fluoro-5-méthoxy-benzyl)-pipérazin-1-yl]-5-oxo-valérique
N-[4-(4-chloro-benzyl)-4-diméthylamino-cyclohexyl]-N'-(3-méthoxy-propyl)-succinamide
4-[4-(4-acétyl-phényl)-pipérazin-1-yl]-N-[4-diméthylamino-4-(4-méthyl-benzyl)-cyclohexyl]-4-oxo-butyramide
4-{3-[4-diméthylamino-4-(4-méthyl-benzyl)-cyclohexyl-carbamoyl]-propionyl}-pipérazin-1-carboxylate de tert-butyle
N-[4-(3-chloro-benzyl)-4-diméthylamino-cyclohexyl]-N'-(tétrahydro-furann-2-ylméthyl)-succinamide
2-{3-[4-(3-chloro-benzyl)-4-diméthylamino-cyclohexyl-carbamoyl]-propionylamino}-propionate d'éthyle
N-(3-bromo-benzyl)-N'-[4-diméthylamino-4-(3-méthyl-benzyl)-cyclohexyl]-succinamide
3-(4-chloro-phényl)-2-{3-[4-diméthylamino-4-(4-méthyl-benzyl)-cyclohexylcarbamoyl]-propionylamino}-propionate de méthyle
N-[4-(3-chloro-benzyl)-4-diméthylamino-cyclohexyl]-4-[4-(5-méthyl-pyrazin-2-carbonyl)-pipérazin-1-yl]-4-oxo-butyramide
N-[4-(4-chloro-benzyl)-4-diméthylamino-cyclohexyl]-N'-naphtalén-1-ylméthyl-succinamide
N-[4-diméthylamino-4-(3-méthyl-benzyl)-cyclohexyl]-4-[4-(3-fluoro-4-méthoxy-benzyl)-pipérazin-1-yl]-4-oxo-butyramide
N-[4-diméthylamino-4-(4-méthyl-benzyl)-cyclohexyl]-N'-[2-(1H-indol-3-yl)-éthyl]-N'-méthyl-succinamide
(4-{3-[4-(3-chloro-benzyl)-4-diméthylamino-cyclohexyl-carbamoyl]-propionylamino}-benzyl)-phosphonate de diéthyle
N-[4-diméthylamino-4-(4-méthyl-benzyl)-cyclohexyl]-N'-méthyl-N'-(2-pyridin-2-yl-éthyl)-succinamide
N-[4-(3-chloro-benzyl)-4-diméthylamino-cyclohexyl]-4-(3,6-dihydro-2H-pyridin-1-yl)-4-oxo-butyramide
N-[4-diméthylamino-4-(3-méthyl-benzyl)-cyclohexyl]-N'-(1-méthyl-3-phényl-propyl)-succinamide
(4-benzyl-4-diméthylamino-cyclohexyl)-amide d'acide 5-[4-(5-méthyl-pyrazin-2-carbonyl)-pipérazin-1-yl]-5-oxo-valérique
N-[4-(3-chloro-benzyl)-4-diméthylamino-cyclohexyl]-N'-(2-chloro-6-méthyl-benzyl)-succinamide
N-allyl-N'-[4-(3-chloro-benzyl)-4-diméthylamino-cyclohexyl]-N-méthyl-succinamide
N-[4-(4-chloro-benzyl)-4-diméthylamino-cyclohexyl]-N'-cyclopentyl-succinamide
N-[4-diméthylamino-4-(4-méthyl-benzyl)-cyclohexyl]-N'-(tétrahydro-furann-2-ylméthyl)-succinamide
(4-{3-[4-diméthylamino-4-(3-méthyl-benzyl)-cyclohexyl-carbamoyl]-propionylamino}-benzyl)-phosphonate de diéthyle
N-[4-(4-chloro-benzyl)-4-diméthylamino-cyclohexyl]-4-[4-(2-méthoxy-phényl)-pipérazin-1-yl]-4-oxo-butyramide
N-[4-diméthylamino-4-(3-méthyl-benzyl)-cyclohexyl]-4-[4-(3-méthoxy-phényl)-pipérazin-1-yl]-4-oxo-butyramide
N-[4-(4-chloro-benzyl)-4-diméthylamino-cyclohexyl]-N'-(3,5-difluoro-benzyl)-succinamide
N-[4-(4-chloro-benzyl)-4-diméthylamino-cyclohexyl]-N'-(2-méthyl-benzyl)-succinamide
N-[4-diméthylamino-4-(4-méthyl-benzyl)-cyclohexyl]-4-oxo-4-{4-[(2,2,2-trifluoro-acétylamino)-méthyl]-pipéridin-1-yl}-butyramide
N-[4-(4-chloro-benzyl)-4-diméthylamino-cyclohexyl]-N'-[2-(1H-indol-3-yl)-éthyl]-succinamide
N-[4-(3-chloro-benzyl)-4-diméthylamino-cyclohexyl]-N'-[2-(1H-indol-3-yl)-éthyl]-N'-méthyl-succinamide
(4-benzyl-4-diméthylamino-cyclohexyl)-amide d'acide 5-[4-(7-méthoxy-benzo[1,3]dioxol-5-ylméthyl)-pipérazin-1-yl]-5-oxo-valérique
N-benzyl-N'-[4-diméthylamino-4-(3-méthyl-benzyl)-cyclohexyl]-N-(2-hydroxy-éthyl)-succinamide
(4-benzyl-4-diméthylamino-cyclohexyl)-amide d'acide 5-[4-(2,3-diméthyl-phényl)-pipérazin-1-yl]-5-oxo-valérique
N-[4-(4-chloro-benzyl)-4-diméthylamino-cyclohexyl]-N'-[2-(1H-indol-3-yl)-éthyl]-N'-méthyl-succinamide
N-[4-diméthylamino-4-(4-méthyl-benzyl)-cyclohexyl]-1-[1-(2,3-diméthyl-phényl)-pipérazin-1-yl]-4-oxo-butyramide
(4-benzyl-4-diméthylamino-cyclohexyl)-amide (1,2,3,4-tétrahydro-naphtalén-1-yl)-amide d'acide glutarique
N-[4-(4-chloro-benzyl)-4-diméthylamino-cyclohexyl]-4-(4-méthyl-pipéridin-1-yl)-4-oxo-butyramide
2-{3-[4-diméthylamino-4-(4-méthyl-benzyl)-cyclohexyl-carbamoyl]-propionylamino)-propionate de tert-butyle
(4-benzyl-4-diméthylamino-cyclohexyl)-amide d'acide 5-(4-cycloheptyl-pipérazin-1-yl)-5-oxo-valérique
(4-benzyl-4-diméthylamino-cyclohexyl)-amide [2-(4-fluoro-phényl)-éthyl]-amide d'acide glutarique
(4-benzyl-4-diméthylamino-cyclohexyl)-amide [1-(2,6-dichloro-benzyl)-pyrrolidin-3-yl]-amide d'acide glutarique
N-[4-(4-chloro-benzyl)-4-diméthylamino-cyclohexyl]-4-(1,4-dioxa-8-aza-spiro[4.5]déc-8-yl)-4-oxo-butyramide
N-[4-(3-chloro-benzyl)-4-diméthylamino-cyclohexyl]-4-oxo-4-thiomorpholin-4-yl-butyramide
4-(4-benzoyl-pipéridin-1-yl)-N-[4-diméthylamino-4-(3-méthyl-benzyl)-cyclohexyl]-4-oxo-butyramide
N-[4-(4-chloro-benzyl)-4-diméthylamino-cyclohexyl]-N'-(2,4-diméthoxy-benzyl)-succinamide
N-[4-diméthylamino-4-(3-méthyl-benzyl)-cyclohexyl]-N'-(1,2,3,4-tétrahydro-naphtalén-1-yl)-succinamide
N-[4-(4-chloro-benzyl)-4-diméthylamino-cyclohexyl]-N'-éthyl-N'-pyridin-4-ylméthyl-succinamide
N-[2-(3,4-diméthoxy-phényl)-éthyl]-N'-[4-diméthylamino-4-(3-méthyl-benzyl)-cyclohexyl]-N-méthyl-succinamide
N-[4-(4-chloro-benzyl)-4-diméthylamino-cyclohexyl]-N'-méthyl-N'-pyridin-3-yl-méthyl-succinamide
N-[4-(3-chloro-benzyl)-4-diméthylamino-cyclohexyl]-N'-pyridin-2-ylméthyl-succinamide
N-[4-(3-chloro-benzyl)-4-diméthylamino-cyclohexyl]-N'-(2-méthyl-benzyl)-succinamide
N-[4-diméthylamino-4-(3-méthyl-benzyl)-cyclohexyl]-N'-(4-méthoxy-phényl)-succinamide
N-[4-diméthylamino-4-(3-méthyl-benzyl)-cyclohexyl]-N'-indan-2-yl-succinamide
N-[4-(3-chloro-benzyl)-4-diméthylamino-cyclohexyl]-N'-(1-méthyl-3-phényl-propyl)-succinamide
N-(2-chloro-6-méthyl-benzyl)-N'-[4-diméthylamino-4-(3-méthyl-benzyl)-cyclohexyl]-succinamide
N-[4-diméthylamino-4-(4-méthyl-benzyl)-cyclohexyl]-4-[4-(4-méthoxy-phényl)-pipérazin-1-yl]-4-oxo-butyramide
N-[4-diméthylamino-4-(4-méthyl-benzyl)-cyclohexyl]-N'-[2-(4-fluoro-phényl)-éthyl]-succinamide
(4-benzyl-4-diméthylamino-cyclohexyl)-amide 4-diméthylamino-benzylamide d'acide glutarique
N-[4-(3-chloro-benzyl)-4-diméthylamino-cyclohexyl]-N'-(3,5-dichloro-benzyl)-succinamide
N-[4-(4-chloro-benzyl)-4-diméthylamino-cyclohexyl]-N'-(1-méthyl-3-phényl-propyl)-succinamide
4-{3-[4-(3-chloro-benzyl)-4-diméthylamino-cyclohexyl-carbamoyl]-propionyl}-pipérazin-1-carboxylate d'éthyle
N-[4-diméthylamino-4-(3-méthyl-benzyl)-cyclohexyl]-N'-(4-méthoxy-benzyl)-succinamide
N-[4-(4-chloro-benzyl)-4-diméthylamino-cyclohexyl]-4-(2,6-diméthyl-morpholin-4-yl)-4-oxo-butyramide
N-[4-diméthylamino-4-(4-méthyl-benzyl)-cyclohexyl)-4-oxo-4-(4-phényl-3,6-dihydro-2H-pyridin-1-yl)-butyramide
N-[4-(4-chloro-benzyl)-4-diméthylamino-cyclohexyl]-N'-[2-(3-fluoro-phényl)-éthyl]-succinamide
4-[4-(4-allyloxy-benzyl)-pipérazin-1-yl]-N-[4-diméthylamino-4-(3-méthyl-benzyl)-cyclohexyl]-4-oxo-butyramide
(4-benzyl-4-diméthylamino-cyclohexyl)-amide [2-(5-bromo-2-méthoxy-phényl)-éthyl]-amide d'acide glutarique
(4-benzyl-4-diméthylamino-cyclohexyl}amide d'acide 5-[4-(3-méthoxy-phényl)-pipérazin-1-yl]-5-oxo-valérique
N-[4-(3-chloro-benzyl)-4-diméthylamino-cyclohexyl]-4-(4-méthyl-pipéridin-1-yl)-4-oxo-butyramide
(4-{3-[4-(4-chloro-benzyl)-4-diméthylamino-cyclohexyl-carbamoyl]-propionylamino}-benzyl)-phosphonate de diéthyle
N-[4-diméthylamino-4-(4-méthyl-benzyl)-cyclohexyl]-4-oxo-4-(4-o-tolyl-pipérazin-1-yl)-butyramide
N-[4-diméthylamino-4-(3-méthyl-benzyl)-cyclohexyl]-N'-[2-(3-fluoro-phényl)-éthyl]-succinamide
4-[4-(4-chloro-phényl)-pipérazin-1-yl]-N-[4-diméthylamino-4-(4-méthyl-benzyl)-cyclohexyl]-4-oxo-butyramide
N-[4-(3-chloro-benzyl)-4-diméthylamino-cyclohexyl]-N'-[1-(4-fluoro-phényl)-éthyl]-succinamide
N-[4-(4-chloro-benzyl)-4-diméthylamino-cyclohexyl]-N'-[1-(4-trifluorométhyl-benzyl)-pyrrolidin-3-yl]-succinamide
N-[4-(3-chloro-benzyl)-4-diméthylamino-cyclohexyl]-N'-[2-(1H-indol-3-yl)-éthyl]-succinamide
N-[4-(4-chloro-benzyl)-4-diméthylamino-cyclohexyl]-N'-indan-5-yl-succinamide
N-(4-benzyloxy-phényl)-N'-[4-diméthylamino-4-(4-méthyl-benzyl)-cyclohexyl]-succinamide
2-{3-[4-diméthylamino-4-(3-méthyl-benzyl)-cyclohexyl-carbamoyl]-propionylamino}-3-méthyl-valérate de tert-butyle
4-(4-benzyl-pipéridin-1-yl)-N-[4-diméthylamino-4-(3-méthyl-benzyl)-cyclohexyl]-4-oxo-butyramide
N-[4-(4-chloro-benzyl)-4-diméthylamino-cyclohexyl]-4-[4-(4-fluoro-phényl)-3,6-dihydro-2H-pyridin-1-yl]-4-oxo-butyramide
N-[4-diméthylamino-4-(4-méthyl-benzyl)-cyclohexyl]-N'-(1,2-diméthyl-propyl)-succinamide
N-[4-diméthylamino-4-(3-méthyl-benzyl)-cyclohexyl]-4-(8-fluoro-1,3,4,5-tétrahydro-pyrido[4,3-b]indol-2-yl)-4-oxo-butyramide
4-[4-(3-chloro-benzoyl)-pipérazin-1-yl]-N-[4-diméthylamino-4-(3-méthyl-benzyl)-cyclohexyl]-4-oxo-butyramide
N-[4-(4-chloro-benzyl)-4-diméthylamino-cyclohexyl]-N'-(4-méthoxy-phényl)-succinamide
(4-benzyl-4-diméthylamino-cyclohexyl)-amide d'acide 5-[4-(2,6-dichloro-benzyl)-pipérazin-1-yl]-5-oxo-valérique
N-[4-diméthylamino-4-(3-méthyl-benzyl)-cyclohexyl]-N'-[2-(1H-indol-3-yl)-éthyl]-N'-méthyl-succinamide
N-[4-diméthylamino-4-(3-méthyl-benzyl)-cyclohexyl]-N'-[1-(4-fluoro-phényl)-éthyl]-succinamide
4-[4-(4-chloro-phényl)-pipérazin-1-yl]-N-[4-diméthylamino-4-(3-méthyl-benzyl)-cyclohexyl]-4-oxo-butyramide
N-[4-(3-chloro-benzyl)-4-diméthylamino-cyclohexyl]-4-oxo-4-(4-oxo-1-phényl-1,3,8-triazaspiro[4.5]déc-8-yl)-butyramide
N-benzyl-N'-[4-diméthylamino-4-(3-méthyl-benzyl)-cyclohexyl]-N-méthyl-succinamide
N-[4-(3-chloro-benzyl)-4-diméthylamino-cyclohexyl]-N'-(3,5-difluoro-benzyl)-succinamide
N-[4-diméthylamino-4-(3-méthyl-benzyl)-cyclohexyl]-N'-[2-(4-fluoro-phényl)-éthyl]-succinamide
N-[4-diméthylamino-4-(3-méthyl-benzyl)-cyclohexyl]-N'-[2-(1H-indol-3-yl)-éthyl]-succinamide
N-(2-benzylsulfanyl-éthyl)-N'-[4-diméthylamino-4-(3-méthyl-benzyl)-cyclohexyl]-succinamide
N-[4-(3-chloro-benzyl)-4-diméthylamino-cyclohexyl]-N'-(2,4-difluoro-benzyl)-succinamide
N-[4-(3-chloro-benzyl)-4-diméthylamino-cyclohexyl]-N'-phénéthyl-succinamide
N-[4-diméthylamino-4-(3-méthyl-benzyl)-cyclohexyl]-N'-(1-naphtalén-2-yl-éthyl)-succinamide
N-[4-(3-chloro-benzyl)-4-diméthylamino-cyclohexyl]-N'-(1-méthoxyméthyl-2-phényl-éthyl)-succinamide
N-[4-(3-chloro-benzyl)-4-diméthylamino-cyclohexyl]-N'-(3,4-dichloro-benzyl)-succinamide
N-[4-(3-chloro-benzyl)-4-diméthylamino-cyclohexyl]-N'-furann-2-ylméthyl-succinamide
N-[4-diméthylamino-4-(4-méthyl-benzyl)-cyclohexyl]-N'-(1,2,3,4-tétrahydro-naphtalén-1-yl)-succinamide
N-[4-(3-chloro-benzyl)-4-diméthylamino-cyclohexyl]-N'-(4-fluoro-benzyl)-succinamide
N-[4-diméthylamino-4-(3-méthyl-benzyl)-cyclohexyl]-N'-indan-1-yl-succinamide
N-[4-(3-chloro-benzyl)-4-diméthylamino-cyclohexyl]-4-(3,5-diméthyl-pipéridin-1-yl)-4-oxo-butyramide
N-[4-diméthylamino-4-(3-méthyl-benzyl)-cyclohexyl]-N'-phénéthyl-succinamide
N-[4-diméthylamino-4-(4-méthyl-benzyl)-cyclohexyl]-N'-phénéthyl-succinamide
N-(2-benzylsulfanyl-éthyl)-N'-[4-(3-chloro-benzyl)-4-diméthylamino-cyclohexyl]-succinamide
N-[4-(3-chloro-benzyl)-4-diméthylamino-cyclohexyl]-N'-(2,5-dichloro-benzyl)-succinamide
4-[4-(3-chloro-benzoyl)-pipérazin-1-yl]-N-[4-(4-chlorobenzyl)-4-diméthylamino-cyclohexyl]-4-oxo-butyramide
N-[4-(4-chloro-benzyl)-4-diméthylamino-cyclohexyl]-N'-(1-phényl-propyl)-succinamide
N-(4-bromo-2-fluoro-benzyl)-N'-[4-diméthylamino-4-(3-méthyl-benzyl)-cyclohexyl]-succinamide
N-[4-(3-chloro-benzyl)-4-diméthylamino-cyclohexyl]-N'-méthyl-N'-pyridin-3-ylméthyl-succinamide
N-[4-diméthylamino-4-(4-méthyl-benzyl)-cyclohexyl]-N'-(2-éthoxy-benzyl)-succinamide
N-[4-diméthylamino-4-(3-méthyl-benzyl)-cyclohexyl]-N'-[2-(5-méthoxy-1H-indol-3-yl)-éthyl]-succinamide
N-(4-sec-butyl-phényl)-N'-[4-(4-chloro-benzyl)-4-diméthylamino-cyclohexyl]-succinamide
(4-benzyl-4-diméthylamino-cyclohexyl)-amide d'acide 5-oxo-5-(4-phényl-3,6-dihydro-2H-pyridin-1-yl)-valérique
(4-benzyl-4-diméthylaminocyclohexyl)-amide d'acide 5-oxo-5-(4-phényl-3,6-dihydro-2H-pyridin-1-yl)-valérique
N-[4-(3-chloro-benzyl)-4-diméthylamino-cyclohexyl]-N'-[2-(3-fluoro-phényl)-éthyl]-succinamide
N-(3-bromo-benzyl)-N'-[4-(3-chloro-benzyl)-4-diméthylamino-cyclohexyl]-succinamide
N-(4-bromo-2-fluoro-benzyl)-N'-[4-(3-chloro-benzyl)-4-diméthylamino-cyclohexyl]-succinamide
N-[4-(3-chloro-benzyl)-4-diméthylamino-cyclohexyl]-N'-(1-phényl-propyl)-succinamide
N-[4-diméthylamino-4-(3-méthyl-benzyl)-cyclohexyl]-N'-(4-fluoro-2-trifluorométhyl-benzyl)-succinamide
N-[4-(3-chloro-benzyl)-4-diméthylamino-cyclohexyl]-N'-indan-2-yl-succinamide
N-[4-diméthylamino-4-(3-méthyl-benzyl)-cyclohexyl]-4-oxo-4-(4-phényl-3,6-dihydro-2H-pyridin-1-yl)-butyramide
N-[4-(4-chloro-benzyl)-4-diméthylamino-cyclohexyl]-N'-[2-(2-fluoro-phényl)-éthyl]-succinamide
N-[4-diméthylamino-4-(4-méthyl-benzyl)-cyclohexyl]-N'-[2-(2-fluoro-phényl)-éthyl]-succinamide
(4-benzyl-4-diméthylamino-cyclohexyl)-amide [2-(2-fluoro-phényl)-éthyl]-amide d'acide glutarique
N-[2-(2-chloro-phényl)-éthyl)-N'-[4-diméthylamino-4-(4-méthyl-benzyl)-cyclohexyl]-succinamide
N-[4-(3-chloro-benzyl)-4-diméthylamino-cyclohexyl]-N'-(3,4-difluoro-benzyl)-succinamide
N-benzyl-N-(2-cyano-éthyl)-N'-[4-diméthylamino-4-(4-méthyl-benzyl)-cyclohexyl]-succinamide
N-[4-(3-chloro-benzyl)-4-diméthylamino-cyclohexyl]-N'-(3-méthyl-benzyl)-succinamide
N-[4-(3-chloro-benzyl)-4-diméthylamino-cyclohexyl]-N'-(4-fluoro-3-trifluorométhyl-benzyl)-succinamide
N-[4-(3-chloro-benzyl)-4-diméthylamino-cyclohexyl]-N'-[2-(2-chloro-phényl)-éthyl]-succinamide
N-[4-(3-chloro-benzyl)-4-diméthylamino-cyclohexyl]-N'-[2-(4-chloro-phényl)-éthyl]-succinamide
N-[4-(3-chloro-benzyl)-4-diméthylamino-cyclohexyl]-N'-[2-(2,4-dichloro-phényl)-éthyl]-succinamide
N-[4-(3-chloro-benzyl)-4-diméthylamino-cyclohexyl]-N'-indan-1-yl-succinamide
N-[4-(4-chloro-benzyl)-4-diméthylamino-cyclohexyl]-N'-indan-1-yl-succinamide
N-[2-(4-chloro-phényl)-propyl]-N'-[4-diméthylamino-4-(3-méthyl-benzyl)-cyclohexyl]-succinamide
N-(3-bromo-benzyl)-N'-[4-(4-chloro-benzyl)-4-diméthylamino-cyclohexyl]-succinamide
N-[4-(3-chloro-benzyl)-4-diméthylamino-cyclohexyl]-N'-(3-phényl-propyl)-succinamide
N-[4-diméthylamino-4-(4-méthyl-benzyl)-cyclohexyl]-N'-(2-p-tolyl-éthyl)-succinamide
N-[4-(3-chloro-benzyl)-4-diméthylamino-cyclohexyl]-N'-(2-thiophén-2-yl-éthyl)-succinamide
(4-benzyl-4-diméthylamino-cyclohexyl)-amide [2-(4-chloro-phényl)-propyl]-amide d'acide glutarique
N-benzyl-N'-[4-(3-chloro-benzyl)-4-diméthylamino-cyclohexyl]-N-méthyl-succinamide
N-[4-(3-chloro-benzyl)-4-diméthylamino-cyclohexyl]-N'-[2-(3-chloro-phényl)-éthyl]-succinamide
(4-benzyl-4-diméthylamino-cyclohexyl)-amide [2-(2-éthoxy-phényl)-éthyl]-amide d'acide glutarique (4-benzyl-4-diméthylamino-cyclohexyl)-amide phénéthyl-amide d'acide glutarique
(4-benzyl-4-diméthylamino-cyclohexyl)-amide [2-(3-chloro-phényl)-éthyl]-amide d'acide glutarique
N-[4-(3-chloro-benzyl)-4-diméthylamino-cyclohexyl]-N'-[1-(3-méthoxy-phényl)-éthyl]-succinamide
(4-benzyl-4-diméthylamino-cyclohexyl)-amide (1,3-diméthyl-butyl)-amide d'acide glutarique
N-benzyl-N'-[4-diméthylamino-4-(3-méthyl-benzyl)-cyclohexyl]-N-éthyl-succinamide
N-(4-chloro-benzyl)-N'-[4-(3-chloro-benzyl)-4-diméthylamino-cyclohexyl]-succinamide
N-[4-(3-chloro-benzyl)-4-diméthylamino-cyclohexyl]-N'-[2-(2-fluoro-phényl)-éthyl]-succinamide
N-[4-diméthylamino-4-(3-méthyl-benzyl)-cyclohexyl]-N'-(2-fluoro-benzyl)-succinamide
N-[2-(4-chloro-phényl)-éthyl]-N'-[4-diméthylamino-4-(3-méthyl-benzyl)-cyclohexyl]-succinamide
N-[2-(2-chloro-phényl)-éthyl]-N'-[4-diméthylamino-4-(3-méthyl-benzyl)-cyclohexyl]-succinamide
N-[2-(3-chloro-phényl)-éthyl]-N'-[4-diméthylamino-4-(3-méthyl-benzyl)-cyclohexyl]-succinamide
N-benzyl-N'-[4-(3-chloro-benzyl)-4-diméthylamino-cyclohexyl]-N-(2-cyano-éthyl)-succinamide
N-[4-(3-chloro-benzyl)-4-diméthylamino-cyclohexyl]-N'-(3-chloro-4-fluoro-benzyl)-succinamide
N-[4-diméthylamino-4-(3-méthyl-benzyl)-cyclohexyl]-N'-(2-trifluorméthyl-benzyl)-succinamide
N-[2-(2,4-dichloro-phényl)-éthyl]-N'-[4-diméthylamino-4-(3-méthyl-benzyl)-cyclohexyl]-succinamide
N-benzyl-N'-[4-(3-chloro-benzyl)-4-diméthylamino-cyclohexyl]-N-éthyl-succinamide
N-[2-(4-chloro-phényl)-éthyl]-N'-[4-diméthylamino-4-(4-méthyl-benzyl)-cyclohexyl]-succinamide
(4-benzyl-4-diméthylamino-cyclohexyl)-amide d'acide 5-oxo-5-(4-o-tolyl-pipérazin-1-yl)-valérique
N-[4-(3-chloro-benzyl)-4-diméthylamino-cyclohexyl]-N'-[3-(méthyl-phényl-amino)-propyl]-succinamide
N-[4-(3-chloro-benzyl)-4-diméthylamino-cyclohexyl]-4-[4-(5-chloro-2-méthyl-phényl)-pipérazin-1-yl]-4-oxo-butyramide
N-[4-diméthylamino-4-(3-méthyl-benzyl)-cyclonexyl]-N'-[2-(2-éthoxy-phényl)-éthyl]-succinamide
4-[4-(4-chloro-benzoyl)-pipéridin-1-yl]-N-[4-(3-chlorobenzyl)-4-diméthylamino-cyclohexyl]-4-oxo-butyramide
N-(4-(3-chloro-benzyl)-4-diméthylamino-cyclohexyl)-N'-[2-(2-éthoxy-phényl)-éthyl]-succinamide
N-[2-(2,6-dichloro-phényl)-éthyl]-N'-[4-diméthylamino-4-(3-méthyl-benzyl)-cyclohexyl]-succinamide
N-benzhydryl-N'-(4-(3-chloro-benzyl)-4-diméthylamino-cyclohexyl]-succinamide
N-[4-(3-chloro-benzyl)-4-diméthylamino-cyclohexyl]-N'-cyclo-octyl-succinamide
N-[4-(3-chloro-benzyl)-4-diméthylamino-cyclohexyl]-N'-[2-(4-phénoxy-phényl)-éthyl]-succinamide
N-[4-diméthylamino-4-(3-méthyl-benzyl)-cyclohexyl]-N'-(2,3-diméthyl-benzyl)-succinamide
4-[4-(5-chloro-2-méthyl-phényl)-pipérazin-1-yl]-N-[4-diméthylamino-4-(3-méthyl-benzyl)-cyclohexyl]-4-oxo-butyramide
4-[4-(4-chloro-benzoyl)-pipéridin-1-yl]-N-[4-diméthylamino-4-(3-méthyl-benzyl)-cyclohexyl]-4-oxo-butyramide
N-[4-(3-chloro-benzyl)-4-diméthylamino-cyclohexyl]-N'-(1,2,3,4-tétrahydro-naphtalén-1-yl)-succinamide
N-(2-chloro-benzyl)-N'-[4-diméthylamino-4-(3-méthyl-benzyl)-cyclohexyl]-succinamide
N-(2-chloro-benzyl)-N'-[4-(3-chloro-benzyl)-4-diméthylamino-cyclohexyl]-succinamide
N-[4-(3-chloro-benzyl)-4-diméthylamino-cyclohexyl]-N'-(3-fluoro-5-trifluorméthyl-benzyl)-succinamide
N-[4-(3-chloro-benzyl)-4-diméthylamino-cyclohexyl]-N'-(1-p-tolyl-éthyl)-succinamide
N-[2-(2,6-dichloro-benzylsulfanyl)-éthyl]-N'-[4-diméthylamino-4-(4-méthyl-benzyl)-cyclohexyl]-succinamide
N-[4-(3-chloro-benzyl)-4-diméthylamino-cyclohexyl]-N'-[2-(2,6-dichloro-benzylsulfanyl)-éthyl]-succinamide
N-[4-(3-chloro-benzyl)-4-diméthylamino-cyclohexyl]-N'-[2-(3-trifluorométhyl-phényl)-éthyl]-succinamide
N-[2-(3,4-dichloro-phényl)-éthyl]-N'-[4-diméthylamino-4-(3-méthyl-benzyl)-cyclohexyl]-succinamide
N-[4-(3-chloro-benzyl)-4-diméthylamino-cyclohexyl]-4-[4-(2-fluoro-phényl)-pipérazin-1-yl]-4-oxo-butyramide
(4-benzyl-4-diméthylamino-cyclohexyl)-amide d'acide 5-[4-(2,4-diméthoxy-phényl)-pipérazin-1-yl]-5-oxo-valérique
N-[4-(3-chloro-benzyl)-4-diméthylamino-cyclohexyl]-N'-(1-naphtalén-1-yl-éthyl)-succinamide
N-[4-diméthylamino-4-(3-méthyl-benzyl)-cyclohexyl]-N'-(1-naphtalén-1-yl-éthyl)-succinamide
N-[4-(3-chloro-benzyl)-4-diméthylamino-cyclohexyl]-N'-(2-fluoro-benzyl)-N'-furann-2-ylméthyl-succinamide
N-[4-diméthylamino-4-(3-méthyl-benzyl)-cyclohexyl]-N'-(2-fluoro-benzyl)-N'-furann-2-ylméthyl-succinamide
N-[4-(3-chloro-benzyl)-4-diméthylamino-cyclohexyl]-N'-(2,5-diméthoxy-benzyl)-N'-furann-2-ylméthyl-succinamide
N-(4-azépan-1-yl-4-benzyl-cyclohexyl)-4-(2,3-dihydro-indol-1-yl)-4-oxo-butyramide
4-(2,3-dihydro-indol-1-yl)-N-[4-diméthylamino-4-(3-fluoro-phényl)-cyclohexyl]-4-oxo-butyramide
N-(4-benzyl-4-morpholin-4-yl-cyclohexyl)-N'-(2-chloro-6-méthyl-benzyl)-succinamide
N-(4-benzyl-4-morpholin-4-yl-cyclohexyl)-N'-[2-(3-méthoxy-phényl)-éthyl]-succinamide
N-(4-benzyl-4-morpholin-4-yl-cyclohexyl)-N'-(3-méthyl-benzyl)-succinamide
N-(4-benzyl-4-morpholin-4-yl-cyclohexyl)-N'-(4-bromo-2-fluoro-benzyl)-succinamide
N-(4-benzyl-4-morpholin-4-yl-cyclohexyl)-N'-pyridin-4-ylméthyl-succinamide
N-(4-benzyl-4-morpholin-4-yl-cyclohexyl)-N'-indan-1-yl-succinamide
N-(4-benzyl-4-morpholin-4-yl-cyclohexyl)-N'-(2,4-diméthoxy-benzyl)-succinamide
N-(4-benzyl-4-morpholin-4-yl-cyclohexyl)-N'-thiophén-2-ylméthyl-succinamide
N-adamantan-1-ylméthyl-N'-(4-benzyl-4-morpholin-4-yl-cyclohexyl)-succinamide
N-(4-benzyl-4-morpholin-4-yl-cyclohexyl)-N'-(2,5-difluoro-benzyl)-succinamide
N-(4-benzyl-4-morpholin-4-yl-cyclohexyl)-N'-(4-tert-butyl-phényl)-succinamide
N-(4-benzyl-4-morpholin-4-yl-cyclohexyl)-N'-(3,5-bis-trifluorméthyl-benzyl)-succinamide
N-(4-benzyl-4-morpholin-4-yl-cyclohexyl)-N'-[2-(2-éthoxy-phényl)-éthyl]-succinamide
N-(4-benzyl-4-morpholin-4-yl-cyclohexyl)-N'-(3-méthoxy-propyl)-succinamide
N-(4-benzyl-4-morpholin-4-yl-cyclohexyl)-N'-(2-morpholin-4-yl-éthyl)-succinamide
N-(4-benzyl-4-morpholin-4-yl-cyclohexyl)-N'-(2,5-dichloro-benzyl)-succinamide
N-(4-benzyl-4-morpholin-4-yl-cyclohexyl)-N'-[1-(2-benzyloxy-benzyl)-pyrrolidin-3-yl]-N'-méthyl-succinamide
N-(4-benzyl-4-morpholin-4-yl-cyclohexyl)-4-oxo-4-(2-pyrrolidin-1-ylméthyl-pyrrolidin-1-yl)-butyramide
N-(4-benzyl-4-morpholin-4-yl-cyclohexyl)-4-morpholin-4-yl-4-oxo-butyramide
N-(4-benzyl-4-morpholin-4-yl-cyclohexyl)-N'-(3-diméthylamino-propyl)-N'-méthyl-succinamide
N-(4-benzyl-4-morpholin-4-yl-cyclohexyl)-N'-(2-phényl-cyclopropyl)-succinamide
N-(4-benzyl-4-morpholin-4-yl-cyclohexyl)-N'-naphtalén-1-ylméthyl-succinamide
N-(4-benzyl-4-morpholin-4-yl-cyclohexyl)-4-(4-méthyl-[1,4]diazépan-1-yl)-4-oxo-butyramide
N-(4-benzyl-4-morpholin-4-yl-cyclohexyl)-4-(4-hydroxy-pipéridin-1-yl)-4-oxo-butyramide
N-(4-benzyl-4-morpholin-4-yl-cyclohexyl)-N'-[2-(7-méthyl-1H-indol-3-yl)-éthyl]-succinamide
N-(4-benzyl-4-morpholin-4-yl-cyclohexyl)-N'-[2-(5-méthoxy-1H-indol-3-yl)-éthyl]-succinamide
N-(4-benzyl-4-morpholin-4-yl-cyclohexyl)-4-oxo-4-(4-quinolin-2-ylméthyl-pipérazin-1-yl)-butyramide
N-(4-diméthylamino-4-thiophén-2-yl-cyclohexyl)-4-oxo-4-[4-(5-trifluorméthyl-pyridin-2-yl)-pipérazin-1-yl]-butyramide
(1-adamantan-1-yl-éthyl)-amide (4-diméthylamino-4-thiophén-2-yl-cyclohexyl)-amide d'acide glutarique
N-[2-(2,6-dichloro-phényl)-éthyl]-N'-(4-diméthylamino-4-thiophén-2-yl-cyclohexyl)-succinamide
N-(4-diméthylamino-4-thiophén-2-yl-cyclohexyl)-N'-(1-méthyl-3-phényl-propyl)-succinamide
N-benzyl-N'-(4-diméthylamino-4-thiophén-2-yl-cyclohexyl)-N-éthyl-succinamide
N-[2-(4-bromo-phényl)-éthyl]-N'-(4-diméthylamino-4-thiophén-2-yl-cyclohexyl)-succinamide
4-[4-(4-chloro-benzoyl)-pipéridin-1-yl]-N-(4-diméthylamino-4-thiophén-2-yl-cyclohexyl)-4-oxo-butyramide
[1-(2,6-dichloro-benzyl)-pyrrolidin-3-yl]-amide (4-diméthylamino-4-thiophén-2-yl-cyclohexyl)-amide d'acide glutarique
(4-diméthylamino-4-thiophén-2-yl-cyclohexyl)-amide (3-phényl-propyl)-amide d'acide glutarique
N-(4-diméthylamino-4-phényl-cyclohexyl)-N'-pentyl-succinamide
3-(4-chloro-phényl)-2-[4-(4-diméthylamino-4-thiophén-2-yl-cyclohexylcarbamoyl)-butyrylamino]-propionate d'éthyle
(4-diméthylamino-4-thiophén-2-yl-cyclohexyl)-amide indan-1-ylamide d'acide glutarique
N-adamantan-1-ylméthyl-N'-(4-diméthylamino-4-thiophén-2-yl-cyclohexyl)-succinamide
(4-diméthylamino-4-thiophén-2-yl-cyclohexyl)-amide d'acide 5-(4-benzofurann-2-ylméthyl-pipérazin-1-yl)-5-oxo-valérique
4-[4-(4-tert-butyl-benzyl)-pipérazin-1-yl]-N-(4-diméthylamino-4-phényl-cyclohexyl)-4-oxo-butyramide
(4-diméthylamino-4-thiophén-2-yl-cyclohexyl)-amide [2-(1H-indol-3-yl)-éthyl]-amide d'acide glutarique
N-(4-diméthylamino-4-phényl-cyclohexyl)-4-[4-hydroxy-4-(3-trifluorométhyl-phényl)-pipéridin-1-yl)-4-oxo-butyramide
(4-diméthylamino-4-thiophén-2-yl-cyclohexyl)-amide d'acide 5-(3,4-dihydro-1H-isoquinolin-2-yl)-5-oxo-valérique
4-(4-benzyl-pipéridin-1-yl)-N-(4-diméthylamino-4-phényl-cyclohexyl)-4-oxo-butyramide
{[4-(4-diméthylamino-4-thiophén-2-yl-cyclohexyl-carbamoyl)-butyryl]-méthyl-amino}-acétate de benzyle
N-benzyl-N'-(4-diméthylamino-4-phényl-cyclohexyl)-N-phénéthyl-succinamide
(4-diméthylamino-4-thiophén-2-yl-cyclohexyl)-amide 3-trifluorméthoxy-benzylamide d'acide glutarique
(4-diméthylamino-4-thiophén-2-yl-cyclohexyl)-amide éthyl-(2-méthyl-allyl)-amide d'acide glutarique
N,N-bis-(2-cyano-éthyl)-N'-(4-diméthylamino-4-thiophén-2-yl-cyclohexyl)-succinamide
benzyl-méthyl-amide (4-diméthylamino-4-thiophén-2-yl-cyclohexyl)-amide d'acide glutarique
(2-benzyloxy-cyclopentyl)-amide (4-diméthylamino-4-thiophén-2-yl-cyclohexyl)-amide d'acide glutarique
(4-diméthylamino-4-thiophén-2-yl-cyclohexyl)-amide 4-trifluorméthyl-benzylamide d'acide glutarique
N-(4-diméthylamino-4-thiophén-2-yl-cyclohexyl)-N'-[2-(4-phénoxy-phényl)-éthyl]-succinamide
(4-diméthylamino-4-thiophén-2-yl-cyclohexyl)-amide (3,3-diphényl-propyl)-amide d'acide glutarique
4-(4-benzyl-pipéridin-1-yl)-N-(4-diméthylamino-4-thiophén-2-yl-cyclohexyl)-4-oxo-butyramide
N-(4-diméthylamino-4-thiophén-2-yl-cyclohexyl)-4-[4-hydroxy-4-(3-trifluorméthyl-phényl)-pipéridin-1-yl]-4-oxo-butyramide
(4-diméthylamino-4-thiophén-2-yl-cyclohexyl)-amide 4-fluoro-2-trifluorméthyl-benzylamide d'acide glutarique
N-(4-diméthylamino-4-thiophén-2-yl-cyclohexyl)-N'-pentyl-succinamide
(4-diméthylamino-4-thiophén-2-yl-cyclohexyl)-amide d'acide 5-[4-(2-méthoxy-naphtalén-1-ylméthyl)-pipérazin-1-yl]-5-oxo-valérique
N-(4-diméthylamino-4-thiophén-2-yl-cyclohexyl)-N'-(2-éthoxy-benzyl)-N'-furann-2-ylméthyl-succinamide
(4-diméthylamino-4-thiophén-2-yl-cyclohexyl)-amide (2-phényl-cyclopropyl)-amide d'acide glutarique
N-[2-(3,4-dichloro-phényl)-éthyl]-N'-(4-diméthylamino-4-phényl-cyclohexyl)-succinamide
N-adamantan-1-ylméthyl-N'-(4-diméthylamino-4-phényl-cyclohexyl)-succinamide
(4-diméthylamino-4-thiophén-2-yl-cyclohexyl)-amide d'acide 5-[4-(4-fluoro-phényl)-3,6-dihydro-2H-pyridin-1-yl]-5-oxo-valérique
2-[4-(4-diméthylamino-4-thiophén-2-yl-cyclohexyl-carbamoyl)-butyrylamino]-4-méthyl-valérate de benzyle
{4-[4-(4-diméthylamino-4-thiophén-2-yl-cyclohexyl-carbamoyl)-butyrylamino]-phényl}-carbamate de tert-butyle
(4-diméthylamino-4-thiophén-2-yl-cyclohexyl)-amide [2-(3-méthoxy-phényl)-éthyl]-amide d'acide glutarique
benzyl-éthyl-amide (4-diméthylamino-4-thiophén-2-yl-cyclohexyl)-amide d'acide glutarique
3-chloro-benzylamide (4-diméthylamino-4-thiophén-2-yl-cyclohexyl)-amide d'acide glutarique
(4-diméthylamino-4-thiophén-2-yl-cyclohexyl)-amide (4-méthyl-cyclohexyl)-amide d'acide glutarique
N-(4-diméthylamino-4-phényl-cyclohexyl)-N'-[2-(1H-indol-3-yl)-éthyl]-N'-méthyl-succinamide
2,5-dichloro-benzylamide (4-diméthylamino-4-thiophén-2-yl-cyclohexyl)-amide d'acide glutarique
(4-diméthylamino-4-thiophén-2-yl-cyclohexyl)-amide [2-(3-fluoro-phényl)-éthyl]-amide d'acide glutarique
N-(4-diméthylamino-4-phényl-cyclohexyl)-N'-[2-(4-méthoxy-phénoxy)-éthyl]-succinamide
N-(4-diméthylamino-4-thiophén-2-yl-cyclohexyl)-N'-[2-(éthyl-m-tolyl-amino)-éthyl]-succinamide
(4-benzyloxy-phényl)-amide (4-diméthylamino-4-thiophén-2-yl-cyclohexyl)-amide d'acide glutarique
(4-diméthylamino-4-thiophén-2-yl-cyclohexyl)-amide [2-(4-fluoro-phényl)-éthyl]-amide d'acide glutarique
[2-(4-bromo-phényl)-éthyl]-amide (4-diméthylamino-4-thiophén-2-yl-cyclohexyl)amide d'acide glutarique
N-(4-diméthylamino-4-thiophén-2-yl-cyclohexyl)-N'-(4-phénoxy-phényl)-succinamide
N-(4-diméthylamino-4-phényl-cyclohexyl)-N'-[2-(3-trifluorméthyl-phényl)-éthyl]-succinamide
[2-(4-chloro-phényl)-propyl]-amide (4-diméthylamino-4-thiophén-2-yl-cyclohexyl)-amide d'acide glutarique
N-(4-diméthylamino-4-thiophén-2-yl-cyclohexyl)-N'-[2-(1H-indol-3-yl)-éthyl]-N'-méthyl-succinamide
N-(4-diméthylamino-4-thiophén-2-yl-cyclohexyl)-N'-[2-(4-méthoxy-phénoxy)-éthyl]-succinamide
(4-diméthylamino-4-thiophén-2-yl-cyclohexyl)-amide (3-phénoxy-phényl)-amide d'acide glutarique
N-(4-diméthylamino-4-phényl-cyclohexyl)-N'-[2-(éthyl-m-tolyl-amino)-éthyl]-succinamide
[2-(5-bromo-2-méthoxy-phényl)-éthyl]-amide (4-diméthylamino-4-thiophén-2-yl-cyclohexyl)-amide d'acide glutarique
N-(4-diméthylamino-4-thiophén-2-yl-cyclohexyl)-N'-[2-(3-trifluorométhyl-phényl)-éthyl]-succinamide
(4-diméthylamino-4-thiophén-2-yl-cyclohexyl)-amide (1-méthyl-3-phényl-propyl)-amide d'acide glutarique
N-[2-(2,6-dichloro-benzylsulfanyl)-éthyl]-N'-(4-diméthylamino-4-thiophén-2-yl-cyclohexyl)-succinamide
(4-diméthylamino-4-thiophén-2-yl-cyclohexyl)-amide (2,2-diphényl-propyl)-amide d'acide glutarique
N-(4-diméthylamino-4-thiophén-2-yl-cyclohexyl)-N'-[3-(méthyl-phényl-amino)-propyl]-succinamide
N-(1-biphényl-4-ylméthyl-pyrrolidin-3-yl)-N'-(4-diméthylamino-4-thiophén-2-yl-cyclohexyl)-N-méthyl-succinamide
[4-(cyano-phényl-méthyl)-phényl]-amide (4-diméthylamino-4-thiophén-2-yl-cyclohexyl)-amide d'acide glutarique
(4-tert-butyl-cyclohexyl)-amide (4-diméthylamino-4-thiophén-2-yl-cyclohexyl)-amide d'acide glutarique
(4-sec-butyl-phényl)-amide (4-diméthylamino-4-thiophén-2-yl-cyclohexyl)-amide d'acide glutarique
(4-butyl-phényl)-amide (4-diméthylamino-4-thiophén-2-yl-cyclohexyl)-amide d'acide glutarique
N-(2-benzylsulfanyl-éthyl)-N'-(4-diméthylamino-4-thiophén-2-yl-cyclohexyl)-succinamide
N-(4-diméthylamino-4-thiophén-2-yl-cyclohexyl)-N'-(2-phénoxy-éthyl)-succinamide
(4-diméthylamino-4-thiophén-2-yl-cyclohexyl)-amide (naphtalén-1-ylméthyl)-amide d'acide glutarique
3-bromo-benzylamide (4-diméthylamino-4-thiophén-2-yl-cyclohexyl)-amide d'acide glutarique
N-(4-diméthylamino-4-thiophén-2-yl-cyclohexyl)-N'-(3-phényl-propyl)-succinamide
N-[2-(3-chloro-phényl)-éthyl]-N'-(4-diméthylamino-4-phényl-cyclohexyl)-succinamide
2-[3-(4-diméthylamino-4-thiophén-2-yl-cyclohexylcarbamoyl)-propionylamino]-propionate de benzyle
N-[2-(2-chloro-phényl)-éthyl]-N'-(4-diméthylamino-4-phényl-cyclohexyl)-succinamide
N-(4-diméthylamino-4-phényl-cyclohexyl)-N'-[2-(3-fluoro-phényl)-éthyl]-succinamide
(4-diméthylamino-4-thiophén-2-yl-cyclohexyl)-amide (2-thiophén-2-yl-éthyl)-amide d'acide glutarique
(4-diméthylamino-4-thiophén-2-yl-cyclohexyl)-amide [2-(4-phénoxy-phényl)-éthyl]-amide d'acide glutarique
N-[2-(4-chloro-phényl)-éthyl]-N'-(4-diméthylamino-4-phényl-cyclohexyl)-succinamide
N-[2-(3-chloro-phényl)-éthyl]-N'-(4-diméthylamino-4-thiophén-2-yl-cyclohexyl)-succinamide
N-benzyl-N'-(4-diméthylamino-4-thiophén-2-yl-cyclohexyl)-N-éthyl-succinamide
[1-(2,6-dichloro-benzyl)-pyrrolidin-3-yl]-amide (4-diméthylamino-4-thiophén-2-yl-cyclohexyl)-amide d'acide glutarique
(4-diméthylamino-4-thiophén-2-yl-cyclohexyl)-amide (3-phényl-propyl)-amide d'acide glutarique
(4-diméthylamino-4-thiophén-2-yl-cyclohexyl)-amide [2-(1H-indol-3-yl)-éthyl]-amide d'acide glutarique
(4-diméthylamino-4-thiophén-2-yl-cyclohexyl)-amide d'acide 5-(3,4-dihydro-1H-isoquinolin-2-yl)-5-oxo-valérique
{[4-(4-diméthylamino-4-thiophén-2-yl-cyclohexyl-carbamoyl)-butyryl]-méthyl-amino}-acétate de benzyle
N-(4-diméthylamino-4-thiophén-2-yl-cyclohexyl)-N'-[2-(4-phénoxy-phényl)-éthyl]-succinamide
(4-diméthylamino-4-thiophén-2-yl-cyclohexyl)-amide (2-phényl-cyclopropyl)-amide d'acide glutarique chlorhydrate de N-[4-(3-chlorobenzyl)-4-diméthylamino-cyclohexyl]-N'-furann-2-ylméthylsuccinamide, diastéréoisomère non polaire
chlorhydrate de N-[4-(3-chlorobenzyl)-4-diméthylamino-cyclohexyl]-N'-furann-2-ylméthylsuccinamide, diastéréoisomère polaire
chlorhydrate de (4-benzyl-4-diméthylaminocyclohexyl)-amide [2-(2-fluorophényl)éthyl]amide d'acide glutarique, diastéréoisomère non polaire,
chlorhydrate de (4-benzyl-4-diméthylaminocyclohexyl)-amide [2-(2-fluorophényl)éthyl]amide d'acide glutarique, diastéréoisomère polaire,
chlorhydrate de N-[4-(3-chlorobenzyl)-4-diméthylaminocyclohexyl]-N'-[2-(4-chlorophényl)éthyl]-succinamide, diastéréoisomère non polaire,
chlorhydrate de N-[4-(3-chlorobenzyl)-4-diméthylaminocyclohexyl]-N'-[2-(4-chlorophényl)éthyl]-succinamide, diastéréoisomère polaire,
chlorhydrate de (4-benzyl-4-diméthylaminocyclohexyl)-amide [2-(4-chlorophényl)éthyl]amide d'acide glutarique, diastéréoisomère non polaire,
sous forme du racémate ; des énantiomères, des diastéréoisomères, de mélanges des énantiomères ou diastéréoisomères ou d'un énantiomère individuel ou d'un diastéréoisomère individuel ; des bases et/ou des sels avec des acides ou cations physiologiquement acceptables.

14. Procédé pour la préparation de dérivés de cyclohexyl-1,4-diamine substitués selon l'une quelconque des revendications 1 à 13, **caractérisé en ce qu'**on fait fusionner des cyclohexane-1,4-diamines substituées, au moyen d'anhydrides, d'acides dicarboxyliques à chaîne ouverte ou de leurs analogues activés, en particulier de leurs halogénures d'acides, avec d'autres amines primaires ou secondaires, avec addition de réactifs de couplage.

15. Procédé pour la préparation de dérivés de cyclohexyl-1,4-diamine substitués selon l'une quelconque des revendications 1 à 13, **caractérisé en ce qu'**on fait fusionner des anhydrides, des acides dicarboxyliques à chaîne ouverte ou leurs analogues activés, en particulier leurs halogénures d'acides, avec des amines primaires ou secondaires et ensuite avec des cyclohexane-1,4-diamines substituées, avec addition de réactifs de couplage.

16. Médicament contenant au moins un dérivé de cyclohexyl-1,4-diamine substitué selon l'une quelconque des revendications 1 à 13, éventuellement sous forme de son racémate, des stéréoisomères purs, en particulier énantiomères et diastéréoisomères, en un rapport de mélange quelconque ; sous forme de ses acides ou de ses bases ou sous forme de ses sels, en particulier des sels physiologiquement acceptables ou des sels avec des cations ou acides physiologiquement acceptables ; ou sous forme de ses produits de solvatation, en particulier des hydrates, ainsi qu'éventuellement contenant des additifs et/ou adjuvants et/ou éventuellement d'autres substances actives.

17. Utilisation d'un dérivé de cyclohexyl-1,4-diamine substitué selon l'une quelconque des revendications 1 à 13, éventuellement sous forme de son racémate, des stéréoisomères purs, en particulier énantiomères et diastéréoisomères, en un rapport de mélange quelconque ; sous forme des ses acides ou de ses bases ou sous forme de ses sels, en particulier des sels physiologiquement acceptables ou des sels avec des cations ou acides physiologiquement acceptables ; ou sous forme de ses produits de solvatation, en particulier des hydrates ; pour la fabrication d'un médicament destiné au traitement de la douleur, en particulier de la douleur aiguë, névropathique ou chronique.

18. Utilisation d'un dérivé de cyclohexyl-1,4-diamine substitué selon l'une quelconque des revendications 1 à 13, pour la fabrication d'un médicament destiné au traitement d'états anxieux, du stress et de syndromes liés au stress, de dépressions, de l'épilepsie, de la maladie d'Alzheimer, de la démence sénile, de la catalepsie, de dysfonctionnements cognitifs généraux, de troubles de la mémoire et de l'apprentissage (en tant qu'agent nootrope), de manifestations inflammatoires, d'abus d'alcool et/ou de drogues et/ou de médicaments et/ou de leur dépendance, de dysfonctionnements sexuels, de maladies cardiovasculaires, de l'hypotension, de l'hypertension, de l'acouphène, du prurit, de la migraine, de troubles de l'audition, de la motilité intestinale insuffisante, de l'alimentation perturbée, de l'anorexie, de l'adiposité, de troubles locomoteurs, de la diarrhée, de la cachexie, de l'incontinence urinaire ou en tant que myorelaxant, anticonvulsivant ou anesthésique, ou à l'administration simultanée dans le traitement par un analgésique opiacé ou par un anesthésique, à la diurèse ou à l'antinatriurèse, à l'anxiolyse, à la modulation de l'activité motrice, à la modulation de la sécrétion de neurotransmetteurs et au traitement de maladies neurodégénératives qui y sont liées, au traitement de manifestations inflammatoires et/ou à la réduction du potentiel de dépendance des opiacés.
